(19) Europäisches Patentamt
European Patent Office
Office européen des brevets

(11) Publication number: **0 318 860 B1**

(12) **EUROPEAN PATENT SPECIFICATION**

(45) Date of publication of patent specification: **16.06.93**

(51) Int. Cl.[5]: **C07C 217/54**, C07C 233/64, C07C 235/42, C07D 307/12, C07C 255/36, C07D 307/42, C07D 263/32, C07D 277/24, C07D 213/30, C07D 295/08, C07D 261/08

(21) Application number: **88119667.9**

(22) Date of filing: **25.11.88**

# (54) Substituted alkylamine derivatives.

(30) Priority: **27.11.87 JP 299584/87**
**19.04.88 JP 96286/88**
**10.05.88 JP 113310/88**

(43) Date of publication of application:
**07.06.89 Bulletin 89/23**

(45) Publication of the grant of the patent:
**16.06.93 Bulletin 93/24**

(84) Designated Contracting States:
**AT BE CH DE ES FR GB GR IT LI LU NL SE**

(56) References cited:
**EP-A- 0 051 196**
**EP-A- 0 066 628**
**GB-A- 2 185 980**
**US-A- 4 609 732**

(73) Proprietor: **BANYU PHARMACEUTICAL CO., LTD.**
**2-3, Nihonbashi Honcho 2-chome**
**Chuo-ku Tokyo 103(JP)**

(72) Inventor: **Takezawa, Hiroshi**
**879-91, Uchikoshi-machi**
**Hachioji-shi Tokyo(JP)**
Inventor: **Hayashi, Masahiro**
**311, 19-15, Fukuei 3-chome**
**Ichikawa-shi Chiba-ken(JP)**
Inventor: **Iwasawa, Yoshikazu**
**235, Nishi Koiso Oiso-machi**
**Naka-gun Kanagawa-ken(JP)**
Inventor: **Hosoi, Masaaki**
**1-51, Kajigaya 5-chome Takatsu-ku**
**Kawasaki-shi Kanagawa-ken(JP)**
Inventor: **Iida, Yoshiaki**
**922, Imai-cho Hodogaya-ku**
**Yokohama-shi Kanagawa-ken(JP)**
Inventor: **Tsuchiya, Yoshimi**
**402, 45-7, Higashifunabashi 3-chome**
**Funabashi-shi Chiba-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

Inventor: **Horie, Masahiro**
**5-17, Tokiwadai 2-chome**
**Itabashi-ku Tokyo(JP)**
Inventor: **Kamei, Toshio**
**11-8, Hagoromo-cho 2-chome**
**Tachikawa-shi Tokyo(JP)**

(74) Representative: **Kraus, Walter, Dr. et al**
**Patentanwälte Kraus, Weisert & Partner**
**Thomas-Wimmer-Ring 15**
**W-8000 München 22 (DE)**

## Description

This invention relates to novel substituted alkylamine derivatives. More specifically, it relates to substituted alkylamine derivatives and their salts which are useful as pharmaceuticals, particularly for the treatment and prevention of hypercholesterolemia, hyperlipemia and arteriosclerosis, processes for production thereof, and their use.

Arteriosclerosis is a degenerative arterial disease which has closely to do with aging and diet, and is regarded as the cause of coronary and cerebral arterial diseases, the principal cause of death in the present day. Arteriosclerosis begins in early ages as deposition of lipid on the endothelia of large vessels, and with age, its degree increases. It will finally show clinical symptoms as ischemic heart diseases such as myocardial infarction and angina pectoris, cerebral arteriosclerosis such as cerebral infarction, and aneurism. It is known that the increase of various blood lipids is involved in this lipid deposition. In particular, the increase of blood cholesterol is the most prominent risk factor, and decreasing the blood cholesterol level to a normal value is the most effective therapeutic and prophylactic means against arteriosclerosis. It is said that in humans, more than 50 % of cholesterol is derived from de novo biosynthesis. Nowadays, lovastatin and pravastatin which are inhibitors of enzymes in the process of de novo biosynthesis are clinically used as hypocholesterolemic agents (see, for example, A. W. Alberts et al., Proc. Natl. Acad. Sci., vol. 77, page 3957, 1980; and Tsujita et al., Biochim. Biophs. Acta, vol. 877, page 50, 1986). However, since 3-hydroxymethyl glutaryl-coenzyme A reductase, a target enzyme of these inhibitors, is positioned in the early stage of the cholesterol biosynthesis pathway, the administration of these drugs will also inhibit formation of dolichol and ubiquinone which are other biologically important metabolites. Furthermore, it was reported that triparanol, an inhibitor of the later stage of the cholesterol biosynthesis pathway, becomes the cause of cataract due to the accumulation of desmosterol. Since squalene epoxidase is positioned in the middle stage of the cholesterol biosynthesis pathway, an inhibitor of this enzyme is expected to solve these problems and serve as a hypocholesterolemic agent with high safety.

Some compounds have already been known as inhibitors of squalene epoxidase [see G. Petranyi et al., Science, vol. 224, page 1239 (1984)]. All of these, however, were developed as antimycotic agents which inhibit fungal squalene epoxidase selectively. No inhibitor has been known which inhibits mammalian enzyme and has utility as an hypocholesterolemic agent.

It is a primary object of this invention to provide an hypolipemic agent, and a therapeutic and prophylactic agent for arteriosclerosis which is safer and better than conventional hypolipemic agents.

The present inventors investigated squalene epoxidase inhibitors having hypocholesterolemic activity in order to develop a novel antiarteriosclerotic agent, and have found that substituted alkylamine derivatives of general formula [I] given below selectively inhibit squalene epoxidase of mammals, and have strong hypocholesterolemic activity.

Thus, the present invention provides substituted alkylamine derivatives represented by the general formula

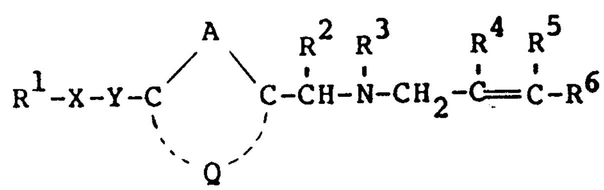

[I]

wherein A represents a methine group, a nitrogen atom, an oxygen atom or a sulfur atom; Q represents a group which may contain one or two hetero atoms selected from the group consisting of nitrogen, oxygen and sulfur atoms and forms a 5- or 6-membered aromatic ring together with the adjacent carbon atoms and A (the aromatic ring may contain one substituent, or two identical or different substituents, selected from the class consisting of halogen atoms, hydroxyl groups, lower alkyl groups and lower alkoxy groups); X and Y may be identical or different and each represents an oxygen atom, a sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group, or when taken together, X and Y represent a vinylene or ethynylene group; $R^1$ represents a lower alkenyl group which may be substituted, a cycloalkenyl group which may be substituted, a lower alkynyl group which may be substituted, an aryl group which may be substituted, or a heterocyclic group which may be substituted; $R^2$ represents a hydrogen atom or a lower alkyl group; $R^3$ represents a hydrogen atom, a lower alkyl group, a

lower alkenyl group, a lower alkynyl group or a cycloalkyl group; $R^4$ and $R^5$ may be identical or different and each represents a hydrogen atom or a halogen atom; and $R^6$ represents an acyclic hydrocarbon group which may be substituted (which may contain 1 or 2 unsaturated bonds selected from double and triple bonds), a cycloalkyl group which may be substituted, or a phenyl group which may be substituted; provided that when either one of X and Y represents an oxygen or sulfur atom, or the group $-NR^b-$ in which $R^b$ is as defined above, the other represents a carbonyl group or the group $-CHR^a-$ in which $R^a$ is as defined above; nontoxic salts of these, processes for production thereof, and the use thereof in the treatment of hypercholesterolemia, hyperlipemia and arteriosclerosis.

The invention will be described below in more detail.

It has previously been known that allylamine derivatives typified by naftifine and terbinafine represented by the following structural formulae

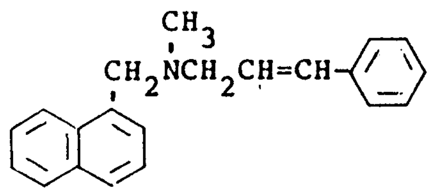

naftifine

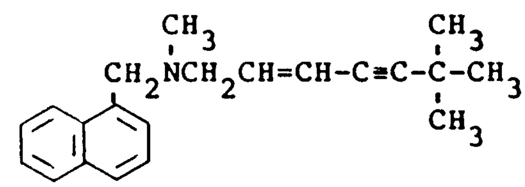

terbinafine

show strong inhibiting activity on the fungal squalene epoxidase, and therefore are useful as an antifungal agent (see G. Petranyi et al., Science, vol. 224, page 1239, 1984). However, these compounds hardly show an inhibitory action on the mammalian squalene epoxidase including human squalene epoxidase, and cannot be inhibitors of cholesterol biosynthesis (see N. S. Ryder et al., Biochem. J., vol. 230, page 765, 1985).

The present inventors extensively made investigations in order to develop a drug which selectively acts on the mammalian squalene epoxidase and shows anticholesterol activity, and have found that if a 1,3-substituted 5- or 6-membered aromatic ring of the formula

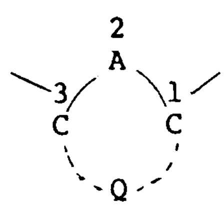

wherein A and Q are as defined above,

in which its 3-position is substituted by a group of the formula $R^1$-X-Y- ($R^1$, X and Y are as defined above) is substituted for the naphthalene ring moiety of the naftifine and terbinafine, a compound showing strong inhibitory activity on the mammalian squalene epoxidase can be obtained.

The inventors have also found that the squalene epoxidase inhibitory activity of the compounds of general formula [I] is very selective, and these compounds show little activity on the enzymes of fungi and are very valuable as drugs for treatment or prevention of hypercholesterolemia, hyperlipemia and arteriosclerosis.

The term "lower", as used in the present specification and the appended claims to qualify a group or a compound, means that the group or compound so qualified has not more than 6, preferably not more than 4, carbon atoms.

The lower alkyl group may be, for example, a linear or branched alkyl group having 1 to 6 carbon atoms, such as methyl, ethyl, propyl, isopropyl, butyl, isobutyl, sec-butyl, tert-butyl, pentyl, isopentyl, neopentyl and hexyl groups. The lower alkenyl group may be, for example, a linear or branched alkenyl group of 2 to 6 carbons containing 1 or 2 double bonds in the carbon chain, such as vinyl, 1-propenyl, isopropenyl, allyl, 1-methyl-1-propenyl, 2-methyl-1-propenyl, 1-methyl-2-propenyl, 2-methyl-2-propenyl, 1-butenyl, 2-butenyl, 3-butenyl, 1,3-butadienyl, 2-methyl-1-butenyl, 3-methyl-1,3-butadienyl, 2-ethyl-1-butenyl, 3-methyl-2-butenyl, 1-pentenyl, 2-pentenyl, 1,3-pentadienyl, 2,4-pentadienyl, 3-methyl-2-pentenyl, 1-hexenyl and 2-hexenyl groups. The lower alkynyl group may be, for example, a linear or branched alkynyl group of 2 to 6 carbon atoms containing 1 or 2 triple bonds in the carbon chain, such as ethynyl, 1-propynyl, propargyl, 1-butynyl, 2-butynyl, 3-butynyl, 3-methyl-1-butynyl, 3,3-dimethyl-1-butynyl, 1-pentynyl, 2-pentynyl, 3-pentynyl, 1,3-pentanediynyl, 1-ethynyl-2-propynyl, 4-methyl-2-pentynyl and 2-hexynyl groups.

The lower alkoxy group may be, for example, a linear or branched alkoxy group having 1 to 4 carbon atoms such as methoxy, ethoxy, propoxy, isopropoxy, butoxy, isobutoxy, sec-butoxy and tert-butoxy groups. The lower alkenyloxy group includes linear or branched alkenyloxy groups having 3 to 6 carbon atoms such as 2-propenyloxy, 2-methyl-2-propenyloxy, 2-methyl-2-butenyloxy and 3-methyl-2-butenyloxy groups. Examples of the cycloalkyl groups are cycloalkyl groups having 3 to 7 carbon atoms such as cyclopropyl, cyclobutyl, cyclopentyl, cyclohexyl and cycloheptyl groups. Examples of the cycloalkenyl groups are cycloalkenyl groups of 5 to 7 carbon atoms containing 1 or 2 double bonds in the ring, such as 1-cyclopentenyl, 2-cyclopentenyl, 3-cyclopentenyl, 2,4-cyclopentadienyl, 1-cyclohexenyl, 1,3-cyclohexadienyl, 1,4-cyclohexadienyl, and 1-cycloheptenyl groups.

Examples of the aryl group include monocyclic or polycyclic aromatic groups such as phenyl, naphthyl and tetrahydronaphthyl groups. The heterocyclic group may be, for example, 5- to 12-membered, preferably 5- to 6-membered, heterocyclic groups having 1 to 3 hetero atoms selected from the group consisting of nitrogen, oxygen and sulfur atoms on the ring, such as furyl, tetrahydrofuryl, pyrrolyl, pyrrolidinyl, imidazolyl, pyrazolyl, oxazolyl, isoxazolyl, furazanyl, thiazolyl, isothiazolyl, thiadiazolyl, thienyl, pyridyl, piperidinyl, pyrazinyl, pyrimidinyl, pyridazinyl, piperazinyl, morpholinyl, thiomorpholinyl, triazinyl, quinolyl, isoquinolyl, phthalazinyl, naphthyridinyl, quinoxalinyl, quinazolinyl, benzofuranyl, benzothienyl, benzoisoxazolyl, benzothiazolyl and benzofurazanyl groups.

The halogen atoms may be, for example, fluorine, chlorine, bromine or iodine.

The acyclic hydrocarbon group which may contain 1 or 2 unsaturated bond selected from double and triple bonds may be, for example, a $C_1$-$C_{17}$, preferably $C_5$-$C_{10}$, linear or branched saturated hydrocarbon group, or a $C_1$-$C_{17}$, preferably $C_5$-$C_{10}$, linear or branched unsaturated hydrocarbon group having 1 or 2 double bonds and/or triple bonds. Specific examples include methyl, ethyl, propyl, isopropyl, butyl, isobutyl, sec-butyl, tert-butyl, pentyl, isopentyl, tert-pentyl, neopentyl, hexyl, 1,2,2-trimethylpropyl, 1,1-dimethylbutyl, 2,2-dimethylbutyl, 3,3-dimethylbutyl, 1,1-dimethylpentyl, 3,3-dimethylpentyl, 4,4-dimethylpentyl, 2,4,4-trimethylpentyl, heptyl, octyl, nonyl, decanyl, undecyl, dodecyl, tridecyl, tetradecyl, pentadecyl, hexadecyl, heptadecyl, vinyl, ethynyl, allyl, isopentenyl, 1-pentenyl, propargyl, 1-propynyl, 1-butenyl, 2-butenyl, 3-butenyl, 1-butynyl, 2-butynyl, 3-butynyl, 1-methyl-1-propenyl, 2-methyl-1-propenyl, 1-methyl-2-propenyl, 2-methyl-2-propenyl, 1,2-dimethyl-1-propenyl, 1,2-dimethyl-2-propenyl, 2-methyl-1-butenyl, 2-methyl-2-butenyl, 3-methyl-1-butenyl, 3-methyl-1-butynyl, 3,3-dimethyl-1-butenyl, 3,3-dimethyl-1-butynyl, 4,4-dimethyl-1-pentenyl, 4,4-dimethyl-1-pentynyl, 3-ethyl-1-pentenyl, 3-ethyl-1-pentenyl, 4-ethyl-1-hexenyl, 4-ethyl-1-hexynyl, 1,1-dimethyl-2-hexenyl, 1,1,4,4-tetramethyl-2-pentenyl, 1,1,4,4-tetramethyl-2-pentynyl, 1,3-butadienyl, 2-methyl-1,3-butadienyl, 1,3-pentadienyl, 4-penten-2-ynyl, 3-penten-2-ynyl, 3-methyl-3-buten-1-ynyl, 5,5-dimethyl-1,3-hexadienyl and 5,5-dimethyl-3-hexen-1-ynyl groups.

Examples of the substituents on the lower alkenyl, lower alkynyl and cycloalkenyl groups which may be substituted include a hydroxyl group, halogen atoms, a cyano group, lower alkoxy groups, aryl groups and heterocyclic groups. The above alkenyl, alkynyl and cycloalkenyl groups may be substituted by 1 or 2 of these substituents.

Examples of the substituents in the aryl groups (or phenyl group) and heterocyclic groups which may be substituted include a hydroxyl group, halogen atoms, a cyano group, a formyl group, lower alkyl groups, lower haloalkyl groups, lower hydroxyalkyl groups, lower alkenyl groups, lower alkenyloxy groups, aryl groups and heterocyclic groups. The above aryl groups and heterocyclic groups may be substituted by 1 to 3 such substituents, preferably 1 or 2 such substituents.

In order to disclose the compounds of the invention represented by general formula [I] more specifically, the various symbols used in formula [I] will be explained in detail by citing preferred examples.

The lower alkenyl group $R^1$ which may be substituted is a linear or branched lower alkenyl group which may be substituted, for example, by a hydroxyl group, a halogen atom, a cyano group, a lower alkoxy group, an aryl group or a heterocyclic group. Preferably, it may be an unsubstituted lower alkenyl group

such as vinyl, allyl, 1-propenyl, isopropenyl, 1-methyl-1-propenyl, 2-methyl-1-propenyl, 1-methyl-2-propenyl, 2-methyl-2-propenyl, 1-butenyl, 2-butenyl, 3-butenyl, 2-methyl-1-butenyl, 2-ethyl-1-butenyl, 3-methyl-2-butenyl, 1-pentenyl, 2-pentenyl, 3-methyl-2-pentenyl, 1-hexenyl and 2-hexenyl groups; or a substituted lower alkenyl group such as 2-fluoro-2-propenyl, 2-chloro-2-propenyl, 2-bromo-2-propenyl, 2,3-dichloro-2-propenyl, 1,1-difluoro-2-propenyl, 3-fluoro-2-butenyl, 3-chloro-2-butenyl, 3-hydroxy-1-propenyl, 2-cyanoethenyl, 3-cyano-2-propenyl, 3-methoxy-1-propenyl, 3-methoxy-1-butenyl, 4-methoxy-2-butenyl, 4-methoxy-3-methyl-2-butenyl, 3-methoxy-1-methylene-1-butenyl, 3-methoxy-2-methyl-1-methylene-1-butenyl, styryl, cinnamyl, 2-(2-furyl)ethenyl, 2-(3-furyl)ethenyl, 3-(2-furyl)-2-propenyl, 4-phenyl-1,3-butadienyl, alpha-methylenecinnamyl, alpha-ethylidenecinnamyl, 1,1-dimethyl-3-phenyl-2-propenyl, beta-methyl-alpha-methylenecinnamyl, 4-(2-furyl)-1,2-butadienyl, 4-(3-furyl)-1,3-butadienyl, 3-(2-furyl)-1-methylene-2-propenyl, 3-(3-furyl)-1-methylene-2-propenyl, (2-oxazolyl)ethenyl, 2-(5-oxazolyl)ethenyl, 2-(2-thiazolyl)ethenyl, 2-(4-thiazolyl)ethenyl and 2-(5-thiazolyl)ethenyl groups.

Examples of especially preferred lower substituted alkenyl groups $R^1$ include unsubstituted alkenyl groups of 3 to 5 carbon atoms, such as 1-propenyl, isopropenyl, 1-methyl-1-propenyl, 2-methylpropenyl, 1-methyl-2-propenyl, 2-methyl-2-propenyl, 1-butenyl and 2-methyl-1-butenyl groups.

The cycloalkenyl group $R^1$ which may be substituted is a cycloalkenyl group which may be substituted by, for example, a hydroxyl group, a halogen atom, a cyano group, a lower alkoxy group, an aryl group or a heterocyclic group. It may be a substituted or unsubstituted cycloalkenyl group having 5 to 6 carbon atoms. Specific examples include 1-cyclopentenyl, 2-cyclohexenyl, 1,4-cyclohexadienyl, 2-methyl-1-cyclopentenyl, 2-methyl-1-cyclohexenyl, 3-hydroxy-1-cyclohexenyl, 3-methoxy-1-cyclohexenyl, 2-fluoro-1-cyclopentenyl, 2-chloro-1-cyclopentenyl, 2-fluoro-1-cyclohexenyl, 2-chloro-1-cyclohexenyl, 2-cyano-1-cyclohexenyl, 4-methoxy-1,3-cyclohexadienyl, 2-(2-furyl)-1-cyclohexenyl, 2-phenyl-1-cyclohexenyl, 3-(2-furyl)-2-cyclohexenyl, 3-phenyl-1-cyclohexenyl, 2-(5-oxazolyl)-1-cyclohexenyl, 2-(2-thiazolyl)-1-cyclohexenyl, and 2-(5-thiazolyl)-1-cyclohexenyl groups.

The lower alkynyl group $R^1$ which may be substituted may be a lower alkynyl group which may be substituted, for example, by a hydroxyl group, a halogen atom, a cyano group, a lower alkyl group, a lower alkoxy group, an aryl group, or a heterocyclic group. Specific preferred examples include substituted or unsubstituted lower alkynyl groups such as ethynyl, 1-propynyl, propargyl, 1-butynyl, 2-butynyl, 3-methyl-1-butynyl, 3,3-dimethyl-1-butynyl, 1-pentynyl, 2-pentynyl, 2-phenylethynyl, 2-(2-furyl)ethynyl, 2-(5-oxazolyl)-ethynyl, 2-(5-thiazolyl)ethynyl and 3-methoxy-3-methyl-1-butynyl.

The aryl group $R^1$ which may be substituted may be an aryl group which may be substituted, for example, by a hydroxyl group, a halogen atom, a cyano group, a formyl group, a lower alkyl group, a halogenoalkyl group having 1 or 2 carbon atoms such as a trifluoromethyl or 2,2,2-trifluoroethyl group, a hydroxyalkyl group having 1 or 2 carbon atoms such as a hydroxymethyl or 1-hydroxyethyl group, a lower alkenyl group, a lower alkoxy group, a linear or branched lower alkenyloxy group having 3 to 5 carbon atoms such as a 2-propenyloxy, 2-methyl-2-propenyloxy or 3-methyl-2-butenyloxy group, an aryl group, or a heterocyclic group. Examples of preferred aryl groups $R^1$ include unsubstituted aryl groups such as phenyl, 1-naphthyl and 2-naphthyl groups; and substituted phenyl groups such as 2-hydroxyphenyl, 3-hydroxyphenyl, 2-fluorophenyl, 3-fluorophenyl, 4-fluorophenyl, 2-chlorophenyl, 3-chlorophenyl, 2,6-difluorophenyl, 2-cyanophenyl, 3-cyanophenyl, 2-formylphenyl, 3-formylphenyl, 2-methylphenyl, 3-methylphenyl, 4-methylphenyl, 2-ethylphenyl, 2-propylphenyl, 2,3-dimethylphenyl, 2,6-dimethylphenyl, 2-methoxyphenyl, 3-methoxyphenyl, 2-ethoxyphenyl, 2-propoxyphenyl, 3-propoxyphenyl, 3-isopropoxyphenyl, 3-butoxyphenyl, 3-isobutoxyphenyl, 2-hydroxymethylphenyl, 3-hydroxymethylphenyl, 2-trifluoromethylphenyl, 3-trifluoromethylphenyl, 2-vinylphenyl, 3-vinylphenyl, 3-allylphenyl, 3-isopropenylphenyl, 3-(1-propenyl)-phenyl, 3-(2-methyl-1-propenyl)phenyl, 3-(2-methyl-2-propenyl)phenyl, 3-(1-butenyl)phenyl, 3-(2-butenyl)-phenyl, 3-(2-methyl-1-butenyl)phenyl, 3-(3-methyl-1-butenyl)phenyl, 3-(1-pentenyl)phenyl, 3-(2-pentenyl)-phenyl, 3-(1,3-butadienyl)phenyl, 3-(1-vinyl-1-propenyl)phenyl, 2-allyloxyphenyl, 3-allyloxyphenyl, 3-(2-methyl-2-propenyloxy)phenyl, 3-(3-methyl-2-butenyloxy)phenyl, 2-biphenylyl, 3-biphenylyl, 2-(2-furyl)phenyl, 3-(2-furyl)phenyl, 2-(3-furyl)phenyl, 3-(3-furyl)phenyl, 4-(2-furyl)phenyl, 4-(3-furyl)phenyl, 3-(2-furyl)-2-methylphenyl, 3-(3-furyl)-2-methylphenyl, 3-(2-furyl)-6-methylphenyl, 3-(3-furyl)-6-methylphenyl, 3-(2-thienyl)phenyl, 3-(3-thienyl)phenyl, 2-methyl-3-(2-thienyl)phenyl, 6-methyl-2-(2-thienyl)phenyl, 3-(2-oxazolyl)-phenyl, 3-(4-oxazolyl)phenyl, 3-(5-oxazolyl)phenyl, 3-(2-thiazolyl)phenyl, 3-(4-thiazolyl)phenyl, 3-(5-thiazolyl)-phenyl, 3-(3-isoxazolyl)phenyl, 3-(4-isoxazolyl)phenyl, 3-(5-isoxazolyl)phenyl, 3-(3-isothiazolyl)phenyl, 3-(4-isothiazolyl)phenyl, 3-(5-isothiazolyl)phenyl, 3-(1-pyrrolyl)phenyl, 3-(2-pyrrolyl)phenyl, 3-(3-pyrrolyl)phenyl, 3-(1-imidazolyl)phenyl, 3-(2-imidazolyl)phenyl, 3-(4-imidazolyl)phenyl, 3-(5-imidazolyl)phenyl, 3-(3-furazanyl)phenyl, 3-(2-pyridyl)phenyl, 3-(3-pyridyl)phenyl, 3-(4-pyridyl)phenyl, 3-(2-pyrimidinyl)phenyl, 3-(4-pyrimidinyl)phenyl, 3-(2-pyrazinyl)phenyl, 3-(3-pyridazinyl)phenyl, 3-(4-pyridazinyl)phenyl, 3-(1-pyrazolyl)-phenyl, 3-[2-(1,3,5-triazinyl)]phenyl, 3-[2-(1,3,4-oxadiazolyl)]phenyl, and 3-[2-(1,3,4-thiadiazolyl)]phenyl

groups. Preferred among them are a phenyl group and a naphthyl group; groups resulting from substitution of the o- or m-position of the phenyl group by a hydroxyl group, a halogen atom, a cyano group, a lower alkyl group having 1 to 3 carbon atoms, or a lower alkoxy group having 1 or 2 carbon atoms, such as 2-hydroxyphenyl, 3-hydroxyphenyl, 2-fluorophenyl, 2-chlorophenyl, 2-cyanophenyl, 3-cyanophenyl, 2-methylphenyl, 2-ethylphenyl, 2-methoxyphenyl and 2-ethoxyphenyl groups; and groups resulting from substitution of the m-position of the phenyl group by a 5-membered aromatic heterocyclic group, such as 3-(2-furyl)-phenyl, 3-(3-furyl)phenyl, 3-(2-thienyl)phenyl, 3-(3-thienyl)phenyl, 3-(1-pyrrolyl)phenyl, 3-(1-imidazolyl)-phenyl, 3-(2-oxazolyl)phenyl, 3-(4-oxazolyl)phenyl, 3-(5-oxazolyl)phenyl, 3-(2-thiazolyl)phenyl, 3-(4-thiazolyl)-phenyl, 3-(5-thiazolyl)phenyl, 3-(3-isoxazolyl)phenyl, 3-(4-isoxazolyl)phenyl, 3-(5-isoxazolyl)phenyl, and 3-(1-pyrazolyl)phenyl groups. The 2-methylphenyl, 3-cyanophenyl, 2-(2-furyl)phenyl, 3-(3-furyl)phenyl, 3-(1-pyrrolyl)phenyl, 3-(4-oxazolyl)phenyl, 3-(5-oxazolyl)phenyl, 3-(1-imidazolyl)phenyl, 3-(4-thiazolyl)phenyl and 3-(5-thiazolyl)phenyl groups are especially preferred.

The heterocyclic group $R^1$ which may be substituted may be, for example, a heterocyclic group which may be substituted by, for example, a hydroxyl group, a halogen atom, a cyano group, a formyl group, a lower alkyl group, a halogenoalkyl group having 1 or 2 carbon atoms (e.g., a trifluoromethyl or 2,2,2-trifluoroethyl group), a hydroxyalkyl group having 1 or 2 carbon atoms (e.g, a hydroxymethyl or 1-hydroxyethyl group), a lower alkenyl group, a lower alkoxy group, a lower alkenyloxy group, an aryl group or a heterocyclic group. Examples of preferred heterocyclic groups $R^1$ include 5- to 12-membered, preferably 5- to 6-membered, unsubstituted aromatic heterocyclic groups containing 1 to 3 hetero atoms selected from nitrogen, oxygen and sulfur atoms, such as 2-furyl, 3-furyl, 2-thienyl, 3-thienyl, 2-benzo[b]-furanyl, 3-benzo[b]furanyl, 4-benzo[b]furanyl, 2-benzo[b]thienyl, 4-benzo[b]thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-benzoxazolyl, 4-benzoxazolyl, 7-benzoxazolyl, 2-benzothiazolyl, 4-benzothiazolyl, 7-benzothiazolyl, 2-pyridyl, 3-pyridyl, 4-pyridyl, 2-pyrimidyl, 4-pyrimidyl, 2-quinolyl, 8-quinolyl and 3-isoquinolyl groups; and 5- to 6-membered substituted aromatic heterocyclic groups containing 1 or 2 hetero atoms selected from the group consisting of nitrogen, oxygen and sulfur atoms, such as 3-methyl-2-furyl, 2-methyl-3-furyl, 4-methyl-3-furyl, 5-methyl-2-furyl, 3-chloro-2-furyl, 2-chloro-3-furyl, 3-fluoro-2-furyl, 5-chloro-2-furyl, 4-cyano-2-furyl, 5-cyano-2-furyl, 3-methyl-2-thienyl, 2-methyl-3-thienyl, 5-methyl-2-thienyl, 5-chloro-2-thienyl, 4-cyano-2-thienyl, 5-cyano-2-thienyl, 4-methyl-2-oxazolyl, 5-methyl-2-oxazolyl, 5-methyl-4-oxazolyl, 4-methyl-5-oxazolyl, 5-cyano-2-oxazolyl, 2-cyano-5-oxazolyl, 4-methyl-2-thiazolyl, 5-methyl-2-thiazolyl, 5-cyano-2-thiazolyl, 2-cyano-5-thiazolyl, 5-methyl-3-isoxazolyl, 3-methyl-5-isoxazolyl, 5-cyano-3-isoxazolyl, 3-cyano-5-isoxazolyl, 3-methyl-2-pyridyl, 4-methyl-2-pyridyl, 2-methyl-3-pyridyl, 4-methyl-3-pyridyl, 2-chloro-3-pyridyl, 4-chloro-3-pyridyl, 2-methyl-4-pyridyl, 3-methyl-4-pyridyl, 2-chloro-4-pyridyl, 6-methoxy-2-pyridyl, 2-methoxy-3-pyridyl, 4-methoxy-3-pyridyl, 2-methoxy-4-pyridyl, 4-cyano-2-pyridyl, 6-cyano-2-pyridyl, 2-cyano-4-pyridyl, 4-(2-furyl)-2-furyl, 5-(5-furyl)-2-furyl, 5-(3-furyl)-2-furyl, 5-(2-oxazolyl)-2-furyl, 5-(2-thiazolyl)-2-furyl, 5-phenyl-2-furyl, 2-(2-furyl)-4-oxazolyl, 2-(2-furyl)-5-oxazolyl, 4-(2-furyl)2-oxazolyl, 5-(2-furyl)-2-oxazolyl, 4-(2-oxazolyl)-2-oxazolyl, 5-(2-oxazolyl)-2-oxazolyl, 2-(2-oxazolyl)-4-oxazolyl, 2-(5-oxazolyl)-4-oxazolyl, 2-(5-oxazolyl)-5-oxazolyl, 2-(2-oxazolyl)-5-oxazolyl, 4-phenyl-2-oxazolyl, 5-phenyl-2-oxazolyl, 2-phenyl-4-oxazolyl, 2-phenyl-5-oxazolyl, 5-phenyl-3-isoxazolyl, 3-phenyl-5-isoxazolyl, 2-(2-furyl)-4-thiazolyl, 2-(2-furyl)-5-thiazolyl, 4-(2-furyl)-2-thiazolyl, 5-(2-furyl)-2-thiazolyl, 5-phenyl-2-thiazolyl, 2-phenyl-4-thiazolyl, 2-phenyl-5-thiazolyl, 2-(2-thienyl)-4-thiazolyl, 2-(5-oxazolyl)-4-thiazolyl, 2-(2-thienyl)-5-thiazolyl, 2-(5-oxazolyl)-5-thiazolyl, 2-(4-thiazolyl)-4-thiazolyl, 2-(4-thiazolyl)-5-thiazolyl, 2-(5-thiazolyl)-4-thiazolyl, 2-(5-thiazolyl)-5-thiazolyl, 4-(5-thiazolyl)-2-thiazolyl, 5-(5-thiazolyl)-2-thiazolyl, 4-(2-thiazolyl)-2-thiazolyl, 2-(2-thiazolyl)-5-thiazolyl, 5-phenyl-2-thienyl, 4-phenyl-2-pyridyl, 2-phenyl-4-pyridyl, 4-phenyl-2-pyrimidyl and 2-phenyl-4-pyrimidyl groups. Preferred among them are 5- or 6-membered unsubstituted aromatic heterocyclic groups such as 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-pyridyl, 3-pyridyl, 4-pyridyl, 2-pyrimidyl and 4-pyrimidyl groups; and 5-membered substituted aromatic heterocyclic groups such as 5-chloro-2-furyl, 2-chloro-3-furyl, 3-methyl-2-furyl, 2-methyl-3-furyl, 4-methyl-3-furyl, 5-methyl-2-furyl, 5-cyano-2-furyl, 4-cyano-2-furyl, 3-methyl-2-thienyl, 2-methyl-3-thienyl, 5-methyl-2-thienyl, 4-cyano-2-thienyl, 5-cyano-2-thienyl, 2-chloro-4-oxazolyl, 2-chloro-5-oxazolyl, 4-methyl-2-oxazolyl, 5-methyl-2-oxazolyl, 2-methyl-4-oxazolyl, 5-methyl-4-oxazolyl, 2-methyl-5-oxazolyl, 4-methyl-5-oxazolyl, 5-cyano-2-oxazolyl, 2-cyano-5-oxazolyl, 2-chloro-4-thiazolyl, 2-chloro-5-thiazolyl, 4-methyl-2-thiazolyl, 5-methyl-2-thiazolyl, 2-methyl-4-thiazolyl, 2-methyl-5-thiazolyl, 5-cyano-2-thiazolyl, 2-cyano-5-thiazolyl, 5-methyl-3-isoxazolyl, 3-methyl-5-isoxazolyl, 5-cyano-3-isoxazolyl, 3-cyano-5-isoxazolyl, 5-(2-furyl)-2-furyl, 4-phenyl-2-furyl, 5-phenyl-2-furyl, 4-phenyl-2-thienyl, 5-phenyl-2-thienyl, 2-phenyl-5-oxazolyl, 4-phenyl-2-oxazolyl, 5-phenyl-2-oxazolyl, 2-(2-furyl)-4-oxazolyl, 2-(2-furyl)-5-oxazolyl, 2-(2-thienyl)-4-oxazolyl, 2-(2-thienyl)-5-oxazolyl, 2-(5-oxazolyl)-4-oxazolyl, 2-(5-oxazolyl)-5-oxazolyl, 2-phenyl-5-thiazolyl, 4-phenyl-2-thiazolyl, 5-phenyl-2-thiazolyl, 2-(2-furyl)-4-thiazolyl, 2-(2-furyl)-5-thiazolyl, 2-(4-thiazolyl)-4-thiazolyl, 2-(5-thiazolyl)-4-thiazolyl, 2-(4-thiazolyl)-5-thiazolyl and 2-(5-thiazolyl)-5-thiazolyl groups.

X and Y are identical or different and each represents an oxygen atom, a sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group; or taken together, X and Y represent a vinylene or ethynylene group. If either one of X and Y represents an oxygen atom, a sulfur atom or the group $-NR^b-$, the other represents a carbonyl group or the group $-CHR^a-$. Examples of groups represented by the formula -X-Y- include $-(CHR^a)_2-$, $-CHR^aO-$, $-OCHR^a-$, $-CHR^aS$, $-SCHR^a-$, $-CHR^aNR^b-$, $-NR^bCHR^a-$, $-CHR^aCO-$, $-COCHR^a-$, -COO-, -OCO-, -COS-, -SCO-, $-CONR^b$, $-NR^bCO-$,-CH=CH-, and -C≡C- (in these formulae, $R^a$ and $R^b$ are as defined hereinabove). Of these, the ethylene group, (E)-vinylene group, the group $-CH_2O-$, and the group $-CH_2NH-$ are preferred.

$R^2$ is preferably a hydrogen atom or a linear or branched lower alkyl group having 1 to 4 carbon atoms such as a methyl, ethyl, propyl or isopropyl group. The hydrogen atom is preferred.

$R^3$ represents a hydrogen atom, a lower alkyl group, a lower alkenyl group, a lower alkynyl group or a cycloalkyl group, for example linear or branched lower alkyl groups having 1 to 5 carbon atoms such as methyl, ethyl, propyl, isopropyl, butyl, isobutyl and pentyl groups, linear or branched lower alkenyl groups having 3 to 5 carbon atoms such as allyl, 2-butenyl, 2-pentenyl, 2-methyl-2-propenyl and 3-methyl-2-butenyl groups, linear or branched lower alkynyl groups having 3 to 5 carbon atoms such as propargyl, 2-butynyl and 2-pentynyl groups, and cycloalkyl groups having 3 to 5 carbon atoms such as cyclopropyl, cyclobutyl and cyclopentyl groups. Methyl, ethyl, propyl, allyl and propargyl groups are preferred, and the methyl, ethyl and propyl groups are most preferred.

$R^4$ and $R^5$ represent a hydrogen atom or a halogen atom, preferably a hydrogen atom, a fluorine atom or a chlorine atom. The hydrogen atom is especially preferred.

A trans-form (E-form) geometric isomer and a cis-form (Z-form) geometric isomer exist at the double bond formed by the two carbon atoms to which $R^4$ and $R^5$ are bonded respectively. The trans-form geometric isomer is preferred.

The acyclic hydrocarbon group $R^6$ which may be substituted (the acyclic hydrocarbon groups may contain 1 or 2 unsaturated bonds selected from the group consisting of double and triple bonds) means a linear or branched acyclic hydrocarbon group having 1 to 17, preferably 3 to 12, carbon atoms which may be substituted, for example, by a hydroxyl group, a halogen atom, a cycloalkyl group, a lower alkoxy group, or a phenyl group which may be substituted by a halogen atom, a lower alkyl group or a lower alkoxy group and in which the carbon chain may contain 1 or 2 unsaturated bonds selected from the group consisting of double and triple bonds. Examples of preferred acyclic hydrocarbon groups include substituted or unsubstituted saturated hydrocarbons such as isopropyl, tert-butyl, isopentyl, tert-pentyl, neopentyl, isopropoxymethyl, tert-butoxymethyl, 2-(isopropoxy)ethyl, 2-(tert-butoxy)ethyl, 2-methoxy-2-methylpropyl, p-(tert-butyl)benzyl, phenethyl, alpha-methylbenzyl, alpha,alpha-dimethylbenzyl, 3-phenylpropyl, 2-(p-fluorophenyl)ethyl, 2-[p-(tert-butyl)phenyl]ethyl, alpha,alpha-dimethyl-p-fluorobenzyl and alpha,alpha-dimethyl-p-(tert-butyl)benzyl group; groups represented by the formula $-CH=CH-R^g$ (in which $R^g$ represents a lower alkyl group which may be substituted, a lower alkenyl group which may be substituted, a cycloalkyl group which may be substituted, or a phenyl group which may be substituted), such as 2-cyclopropylvinyl, 2-(1-methylcyclopropyl)vinyl, 1-propenyl, 1-butenyl, 3-methyl-1-butenyl, 3,3-dimethyl-1-butenyl, 3-methoxy-3-methyl-1-butenyl, 1-pentenyl, 3-methyl-1-pentenyl, 3,3-dimethyl-1-pentenyl, 3-ethyl-1-pentenyl, 1-hexenyl, 1-heptenyl, 1-octenyl, styryl, 2-(p-fluorophenyl)vinyl, 2-[p-(tert-butyl)phenyl]vinyl, 3-methyl-3-phenyl-1-butenyl, 3-methyl-3-(p-fluorophenyl)-1-butenyl, 3-methyl-3-[p-(tert-butyl)phenyl]-1-butenyl, 1,3-butadienyl, 3-methyl-1,3-butadienyl, 1,3-pentadienyl, 3-methyl-1,3-pentadienyl, 4-methyl-1,3-pentadienyl, 3,4-dimethyl-1,3-pentadienyl, 1,3-hexadienyl and 5,5-dimethyl-1,3-hexadienyl groups; and groups represented by the formula $-C{\equiv}C-R^g$ in which $R^g$ is as defined hereinabove, such as 2-cyclopropylethynyl, 2-(1-methylcyclopropyl)ethynyl, 1-propynyl, 1-butynyl, 3-methyl-1-butynyl, 3,3-dimethyl-1-butynyl, 3-methoxy-3-methyl-1-butynyl, 1-pentynyl, 3-methyl-1-pentynyl, 3,3-dimethyl-1-pentynyl, 3-ethyl-1-pentynyl, 1-hexynyl, 1-heptynyl, 1-octynyl, 2-phenylethynyl, 2-(p-fluorophenyl)ethynyl, 2-[p-(tert-butyl)phenyl]ethynyl, 3-methyl-3-phenyl-1-butynyl, 3-methyl-3-(p-fluorophenyl)-1-butynyl, 3-methyl-3-[p-(tert-butyl)phenyl]-1-butynyl, 3-buten-1-ynyl, 3-methyl-3-buten-1-ynyl, 3-penten-1-ynyl, 3-methyl-3-penten-1-ynyl, 4-methyl-3-penten-1-ynyl, 3,4-dimethyl-3-penten-1-ynyl, 3-hexen-1-ynyl and 5,5-dimethyl-3-hexen-1-ynyl groups. Preferred among them are groups of the formula $-CH=CH-R^c$ (in which $R^c$ represents an alkyl or alkenyl group having 3 to 6 carbon atoms which may be substituted by one lower alkoxy group having 1 to 4 carbon atoms, or a cycloalkyl group having 3 to 6 carbon atoms which may be substituted by an alkyl group having 1 to 4 carbon atoms, such as 3,3-dimethyl-1-butenyl, 3-methoxy-3-methyl-1-butenyl, 1-pentenyl, 3,3-dimethyl-1-pentenyl, 3-ethyl-1- pentenyl, 1-hexenyl, 3,3-dimethyl-1-hexenyl, 1-heptenyl, 1-octenyl, 3-methyl-1,3-butadienyl, 1,3-pentadienyl, 4-methyl-1,3-pentadienyl, 1,3-hexadienyl, 5,5-dimethyl-1,3-hexadienyl, 2-cyclopropylvinyl, 2-(1-methylcyclopropyl)vinyl, 2-cyclopentylvinyl and 2-cyclohexylvinyl groups; and groups of the formula -C≡C-$R^c$ in which $R^c$ is as defined above, such as 3,3-dimethyl-1-butynyl, 3-methoxy-3-methyl-1-butynyl, 1-pentynyl, 3,3-dimethyl-1-pentynyl, 3-ethyl-1-pentynyl, 1-hexynyl, 3,3-dimethyl-1-hexynyl, 1-heptynyl, 1-octynyl, 3-methyl-3-butyn-1-ynyl, 3-penten-1-ynyl, 4-methyl-3-penten-1-ynyl, 3-hexen-1-ynyl, 5,5-dimethyl-3-hexen-1-ynyl, 2-cyclopropylethynyl, 2-(1-methylcyclopropyl)ethynyl, 2-cyclopentylethynyl and 2-cyclohexylethynyl groups. More preferred are groups of the following formula

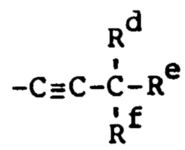

in which $R^d$ represents a hydrogen atom, an alkyl group having 1 to 4 carbon atoms, or an alkoxy group having 1 to 4 carbon atoms, and $R^e$ and $R^f$ are identical or different and each represents a methyl group or an ethyl group or taken together represents a cyclopropyl group,
such as 3-methyl-1-butynyl, 3,3-dimethyl-1-butynyl, 3-methoxy-3-methyl-1-butynyl, 3-methyl-1-pentynyl, 3-ethyl-1-pentynyl, 3-methyl-1-hexynyl, 2-cyclopropylethynyl and 2-(1-methylcyclopropyl)ethynyl groups. Of these, the 3,3-dimethyl-1-butynyl and 3-methoxy-3-methyl-1-butynyl groups are most preferred.

The cycloalkenyl group $R^6$ which may be substituted may be, for example, a cycloalkenyl group which may be substituted by, for example, a hydroxyl group, a halogen atom, a lower alkoxy group or a phenyl group which may be substituted by a halogen atom, a lower alkyl group, or a lower alkoxy group. Examples of preferred cycloalkenyl groups include 1-cyclopentenyl, 3-methyl-1-cyclopentenyl, 3-ethyl-1-cyclopentenyl, 3,3-dimethyl-1-cyclopentenyl, 3-methoxy-1-cyclopentenyl, 3-methoxy-3-methyl-1-cyclopentenyl, 3-cyclopropyl-1-cyclopentenyl, 1-cyclohexenyl, 2-methyl-1-cyclohexenyl, 3-methyl-1-cyclohexenyl, 3,3-dimethyl-1-cyclohexenyl and 3-cyclopropyl-1-cyclohexenyl groups. The phenyl group $R^6$ which may be substituted may be, for example, a phenyl group which may be substituted by a hydroxyl group, a halogen atom, a lower alkyl group or a lower alkoxy group. Examples of preferred phenyl groups include phenyl, 4-hydroxyphenyl, 4-fluorophenyl, 4-chlorophenyl, 4-methylphenyl, 4-ethylphenyl, 4-propylphenyl, 4-isopropylphenyl, 4-butylphenyl, 4-isobutylphenyl, 4-(tert-butyl)phenyl, 2-methoxyphenyl, 3-methoxyphenyl, 4-methoxyphenyl, 3,4-dimethoxyphenyl and 2,6-dichlorophenyl.

Examples of preferred 5- or 6-membered aromatic rings represented by the formula

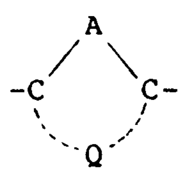

in which A and Q are as defined,
include benzene, pyrrole, furan, thiophene, oxazole, isoxazole, thiazole, imidazole, pyridine, pyrimidine, pyrazine, pyridazine and triazine rings. The benzene, furan, thiophene, oxazole, isoxazole, thiazole, pyridine and pyrimidine rings are preferred, and the benzene ring is most preferred.

Most preferably, the aromatic ring is unsubstituted. As required, it may have one substituent, or two identical or different substituents, selected from the group consisting of halogen atoms, a hydroxyl group, lower alkyl groups and lower alkoxy groups. Of these, the hydroxyl group, fluorine and chlorine atoms, the methyl group and the ethyl group are preferred.

A preferred group of the compounds provided by this invention are substituted alkylamines of general formula [I] in which $R^1$ represents an aryl or heterocyclic group which may be substituted; X represents a methylene group and Y represents an oxygen atomm or an imino group, or X and Y, taken together, represent an ethylene group or an (E)-vinylene group; the aromatic ring of the formula

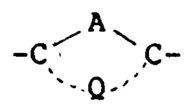

is a benzene, furan, thiophene, oxazole, isoxazole, thiazole, pyridine or pyrimidine ring; $R^2$ represents a hydrogen atom; $R^3$ represents an alkyl group having 1 to 5 carbon atoms, an alkenyl group having 3 to 5 carbon atoms, an alkynyl group having 3 to 5 carbon atoms, or a cycloalkyl group having 3 to 5 carbon atoms; $R^4$ and $R^5$ each represent a hydrogen atom, and the double bond formed by the two carbon atoms to which they are bonded is of a trans-form (E-form); and $R^6$ is a group of the formula $-CH=CH-R^c$ in which $R^c$ represents an alkyl or alkenyl group having 3 to 6 carbon atoms which may be substituted by one alkoxy group having 1 to 4 carbon atoms, or a cycloalkyl group having 3 to 6 carbon atoms which may be substituted by 1 or 2 alkyl groups having 1 to 4 carbon atoms, or a group of the formula $-C{\equiv}C-R^c$ in which $R^c$ is as defined. In this group, $R^1$ is preferably an aryl group or an aromatic heterocyclic group, which may have one substituent or two identical or different substituents, selected from the group consisting of halogen atoms and cyano, hydroxyl, formyl, hydroxymethyl, lower alkyl, lower alkenyl, lower alkynyl, lower alkoxy, lower alkenyloxy, phenyl, furyl, thienyl, oxazolyl, thiazolyl, isoxazolyl, isothiazolyl, pyrrolyl group, imidazolyl, furazanyl, pyridyl, pyrazinyl, pyrimidinyl, pyridazinyl, 1,3,5-triazinyl, 1,3,4-oxadiazolyl and 1,3,4-thiadiazolyl groups. Desirably, the aromatic heterocyclic group is a 5- to 12-membered, preferably 5- to 6-membered, aromatic heterocyclic group containing 1 to 3 hetero atoms selected from the group consisting of nitrogen, oxygen and sulfur atoms. More preferably, $R^1$ is a phenyl, naphthyl, furyl, thienyl, pyridyl, oxazolyl, thiazolyl, isoxazolyl, isothiazolyl, pyrazinyl, pyrimidyl, pyridazinyl, 1,3,5-triazinyl, benzofuranyl, benzothienyl, benzoxazolyl, benzothiazolyl or benzofurazanyl group which may have one substituent, or two identical or different substituents, selected from the class consisting of halogen atoms and hydroxyl, cyano, formyl, hydroxymethyl, $C_1$-$C_3$ alkyl, $C_3$-$C_5$ alkenyl, $C_3$-$C_5$ alkynyl, $C_1$-$C_3$ alkoxy, $C_3$-$C_5$ alkenyloxy, phenyl, furyl, thienyl, oxazolyl, thiazolyl, isoxazolyl, pyrrolyl and imidazolyl groups.

Especially preferred substituted alkylamines of general formula [I] are those in which $R^1$ is a 2-methylphenyl, 2-fluorophenyl, 3-cyanophenyl, 3-(2-furyl)phenyl, 3-(2-thienyl)phenyl, 3-(5-oxazolyl)phenyl, 3-(1-pyrrolyl)phenyl, 3-(1-imidazolyl)phenyl or 3-(5-thiazolyl)phenyl group.

On the other hand, in the preferred group of compounds of general formula [I], $R^3$ desirably represents an alkyl group having 1 to 3 carbon atoms, an allyl group, a propargyl group or a cyclopropyl group; and $R^6$ represents a group of the formula

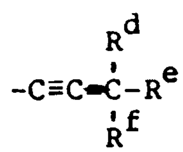

in which $R^d$ represents a hydrogen atom, an alkyl group having 1 to 4 carbon atoms or an alkoxy group having 1 to 4 carbon atoms; $R^e$ and $R^f$ may be identical or different and each represents a methyl or ethyl group, or taken together, may represent a cyclopropyl group.

Particularly, $R^3$ is preferably a methyl, ethyl or propyl group.

The above substituted alkylamine derivatives may exist in the form of an acid addition salt. Examples of the acid addition salts are inorganic acid salts such as hydrochlorides, hydrobromides, hydroiodides, sulfates, nitrates, perchlorates and phosphates; and organic acid salts such as p-toluenesulfonates, benzenesulfonates, methanesulfonates, oxalates, succinates, tartarates, citrates, fumarates and maleates. Preferably, they are nontoxic salts which are pharmaceutically acceptable. Furthermore, depending upon the embodiments of the substituents, the compounds of formula [I] provided by this invention may contain stereoisomers such as geometric isomers and optical isomers. The compounds [I] of this invention include all of these stereoisomers and their mixtures.

General processes for producing the compounds of this invention will now be described.

The compounds [I] of this invention may be produced by any one of the following processes A, B, C, D and E.

## Reaction Scheme 1

[Process A]

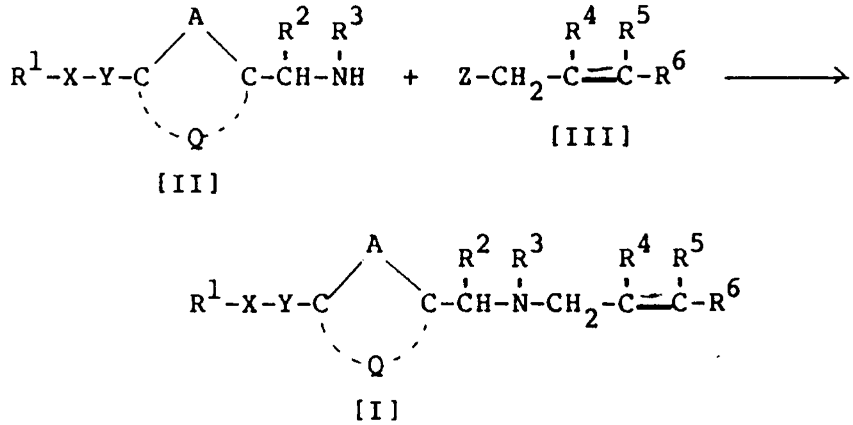

[II] [III] [I]

[Process B]

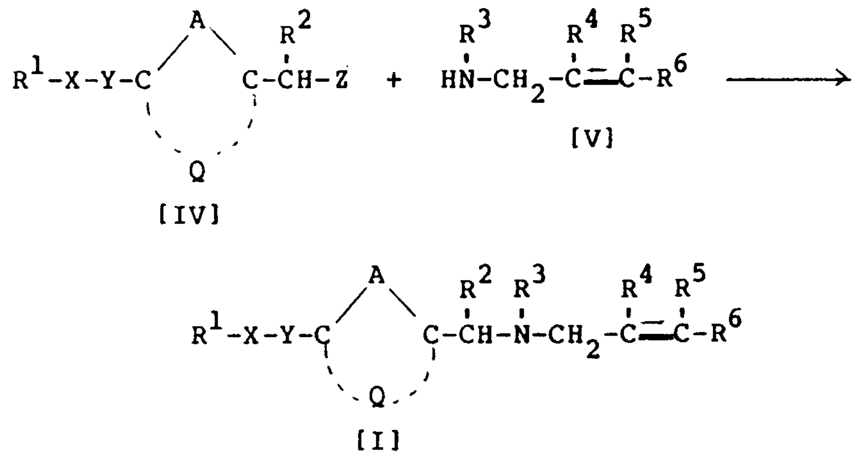

[IV] [V] [I]

[Process C]

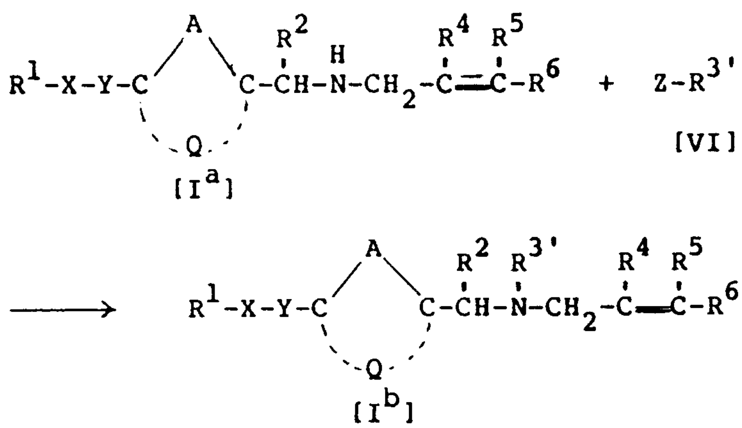

[I^a] [VI] [I^b]

[Process D]

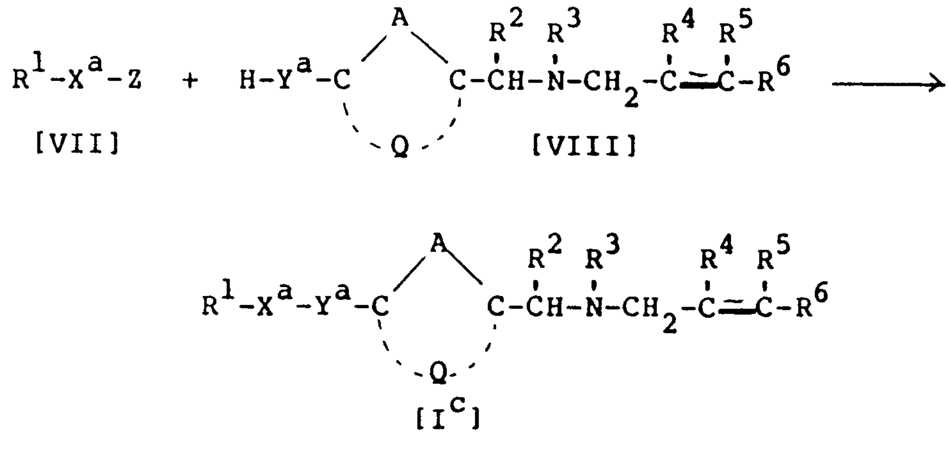

[VII] [VIII] [I^c]

[Process E]

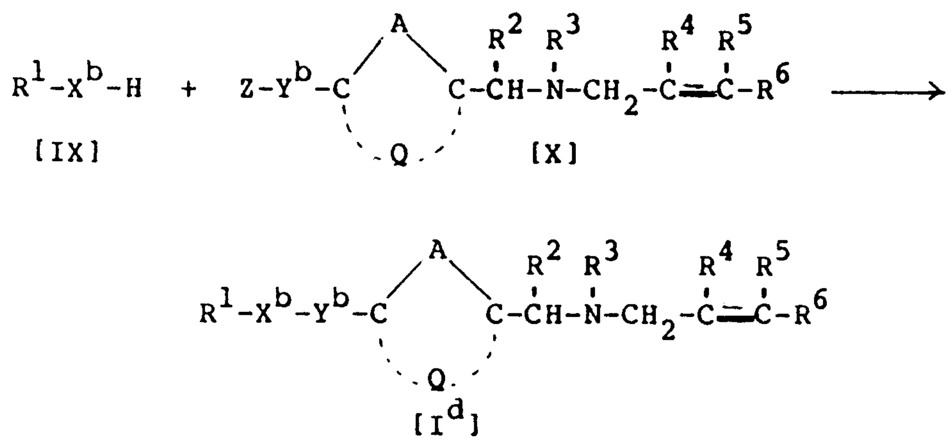

[IX] [X] [I^d]

In the above formulae, Z represents a leaving group; $R^{3'}$ represents a lower alkyl group, a lower alkenyl group, a lower alkynyl group or a cycloalkyl group, $x^a$ and $Y^b$ represent a carbonyl group or a group of the formula $-CHR^a-$ in which $R^a$ is as defined above; $X^b$ and $Y^a$ represent an oxygen atom, a sulfur atom or a group of the formula $-NR^b-$ in which $R^b$ is as defined above; and A, Q, $R^1$, $R^2$, $R^3$, $R^4$, $R^5$ and $R^6$ are as defined above.

The above processes A, B and C are alkylation of amines which are well known in the field of organic syntheses, and can therefore be carried out by using ordinary means known per se. These processes are carried out by using a solvent which does not adversely affect the reactions, and reacting compounds [II] and [III] in process A, compounds [IV] and [V] in process B and compounds [$I^a$] and [VI] in process C in nearly equimolar proportions or using one of them in a slightly excessive proportion. Examples of the solvent include aromatic hydrocarbons such as benzene, toluene and xylene; ethers such as ethyl ether, tetrahydrofuran and dioxane; halogenated hydrocarbons such as methylene chloride, chloroform and dichloroethane; alcohols such as ethanol and isopropanol; dimethylformamide, acetonitrile and dimethyl sulfoxides; and mixtures of these. The reaction temperature is generally -20 °C to 150 °C, preferably from room temperature to the boiling point of the solvent used. The reaction time may be usually 5 minutes to 10 days, preferably 1 to 24 hours. Advantageously, the reactions are carrried out in the presence of a base in order to carry them out smoothly. Examples of the base are alkali metal hydrides such as sodium hydride, lithium hydride and potassium hydride; alkali metal or alkaline earth metal hydroxides such as sodium hydroxide, potassium hydroxide and calcium hydroxide; alkali metal carbonates such as sodium carbonate, potassium carbonate and sodium hydrogen carbonate; and organic amines such as triethylamine and pyridine. The amount of the base used is not critical, and can be varied over a broad range. Generally, it is nearly 1 mole, or slightly more, preferably 1 to 2 moles, per mole of the starting materials.

Processes D and E are for the production of compounds [$I^c$] or [$I^d$] corresponding to the compounds of general formula [I] in which the group of the formula -X-Y- is -COO-, -OCO-, -CONR$^b$-, -NR$^b$CO-, CHR$^a$O-, -OCHR$^a$-, -CHR$^a$S-, or -SCHR$^a$- (in which R$^a$ and R$^b$ are as defined). Processes D and E are usually carried out in a solvent which does not adversely affect the reaction (such as tetrahydrofuran, dioxane, chloroform, benzene, acetone, dimethylformamide or dimethyl sulfoxide) by reacting compounds [VII] and [VIII] in process D and compounds [IX] and [X] in process E in nearly equimolar proportions or using one of them in a slightly excessive molar proportion. The reaction conditions used at this time vary depending upon the starting compounds used. Generally, the reaction temperature is in the range of -70 °C to 100 °C, preferably -20 °C to 50 °C, and the reaction time is 1 minute to 24 hours, preferably 30 minutes to 5 hours. Preferably, the reaction is carried out in the presence of a base so as to perform the action smoothly. Examples of the base used at this time are inorganic bases such as sodium hydride, lithium hydride, sodium hydroxide, potassium hydroxide, sodium carbonate, potassium carbonate, sodium hydrogen carbonate and potassium hydrogen carbonate and organic bases such as pyridine, triethylamine and dimethylaminopyridine. The amount of the base used is not critical, and can be varied over a broad range. Generally, it is nearly 1 mole, or slightly more, preferably 1 to 2 moles, per mole of the starting materials.

If the starting compounds [$I^a$], [II], [III], [IV], [V], [VII], [VIII], [IX] and [X] contain reactive functional groups such as a hydroxyl or amino group in addition to the amino groups which are involved in the reaction, these reactive functional groups may, as required, be protected prior to the reaction, and the protected groups may be removed after the reaction. Protective groups which can be easily eliminated by hydrolysis under acidic or alkaline conditions may be used for this purpose. Examples of the protective groups are methoxymethyl, tetrahydropyranyl, trityl, dimethyl(tert-butyl)silyl, formyl, acetyl, methoxycarbonyl, ethoxycarbonyl and tert-butoxycarbonyl groups.

The desired compound of formula [I] obtained by the above processes in accordance with this invention can be isolated and purified by, for example, column chromatography, solvent extraction, precipitation and recrystallization, either alone or in combination. As required, the compound [I] of the invention as a free base may be converted to its acid addition salt, or vice versa. The step of converting the free base of the compound [I] into its acid addition salt or the step of converting the acid addition salt to its free base can be carried out easily by ordinary methods using the corresponding acids or bases.

The leaving group represented by Z may be, for example, a halogen atom such as a chlorine, bromine or iodine atom, or an organic sulfonyloxy group such as a methanesulfonyloxy or p-toluenesulfonyloxy group.

The starting compounds [$I^a$] to [X] used in processes A to E can be purchased as commercial goods, or may be produced and obtained by the methods described in the literature (see, for example, J. Med. Chem., vol. 27, page 1539, 1984; J. Med. Chem., vol. 29, page 112, 1086; and Japanese Laid-Open Patent Publications Nos. 32440/1981, 123177/1982, 208252/1983, 45/1986, 201850/1987 and 5059/1988), the general processes shown below, or processes substantially in accordance with them.

For example, the starting compounds used in this invention may be produced by the following synthesizing methods.

## Reaction Scheme 2

### Production Route (a)

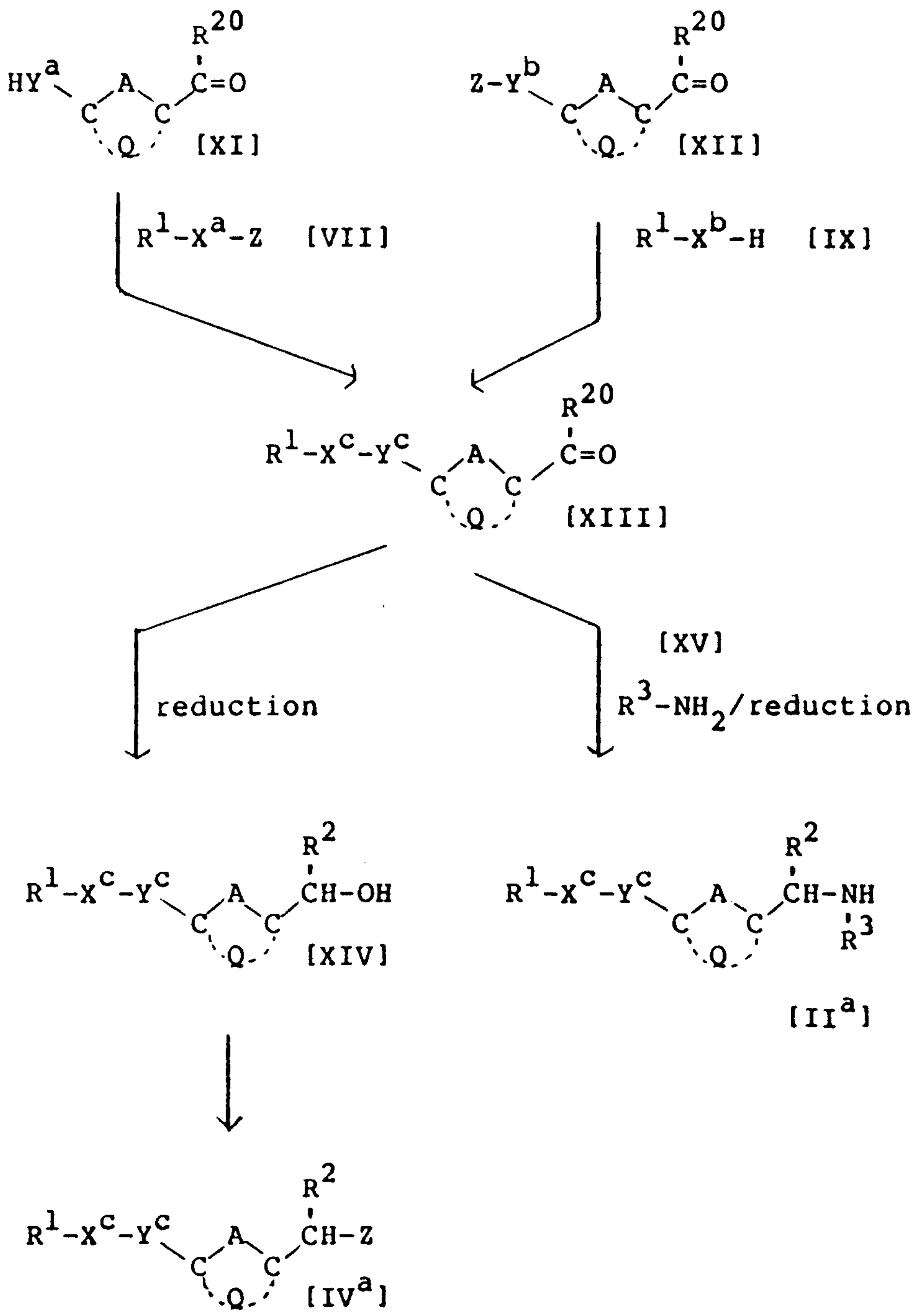

Production Route (b)

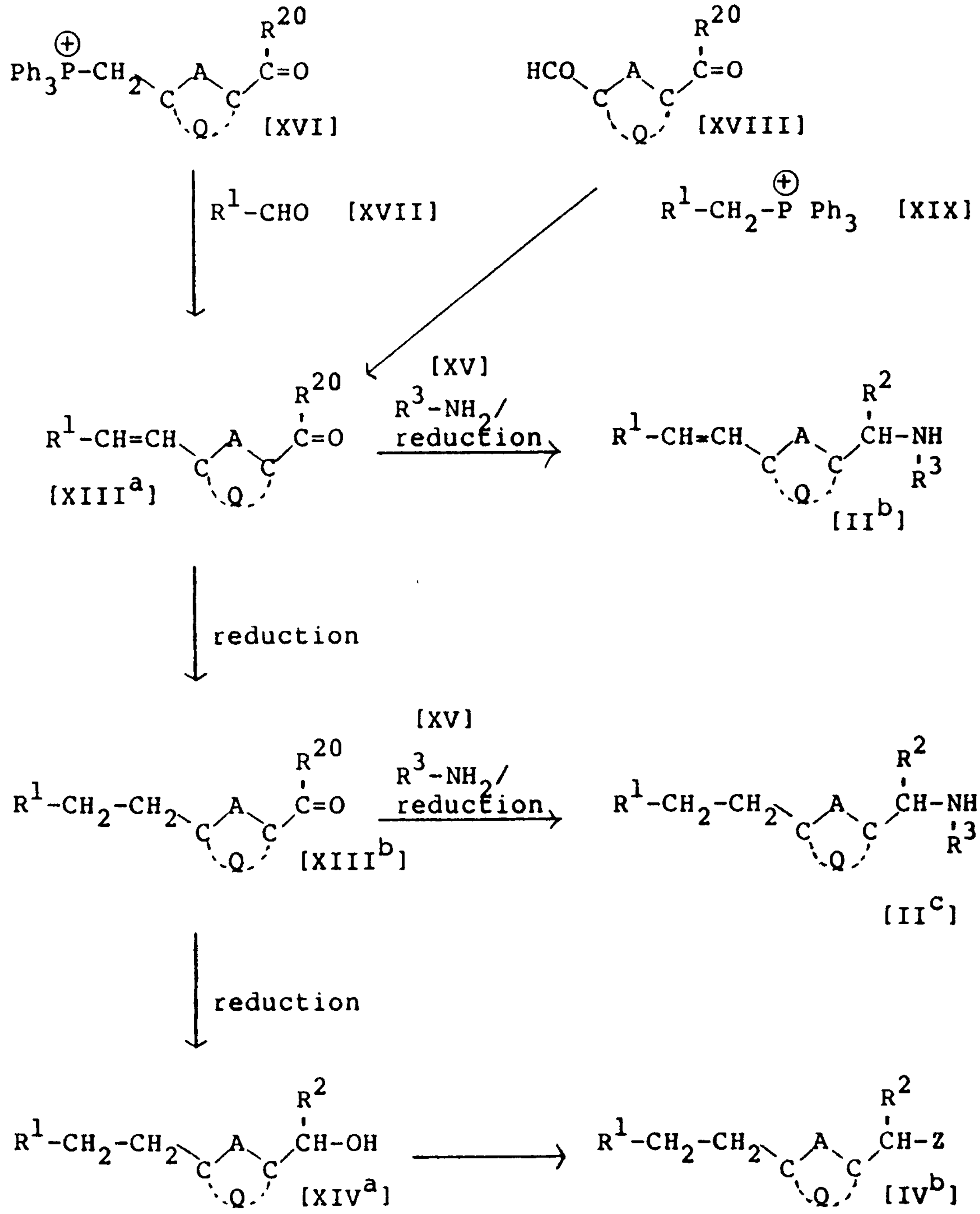

Production Route (c)

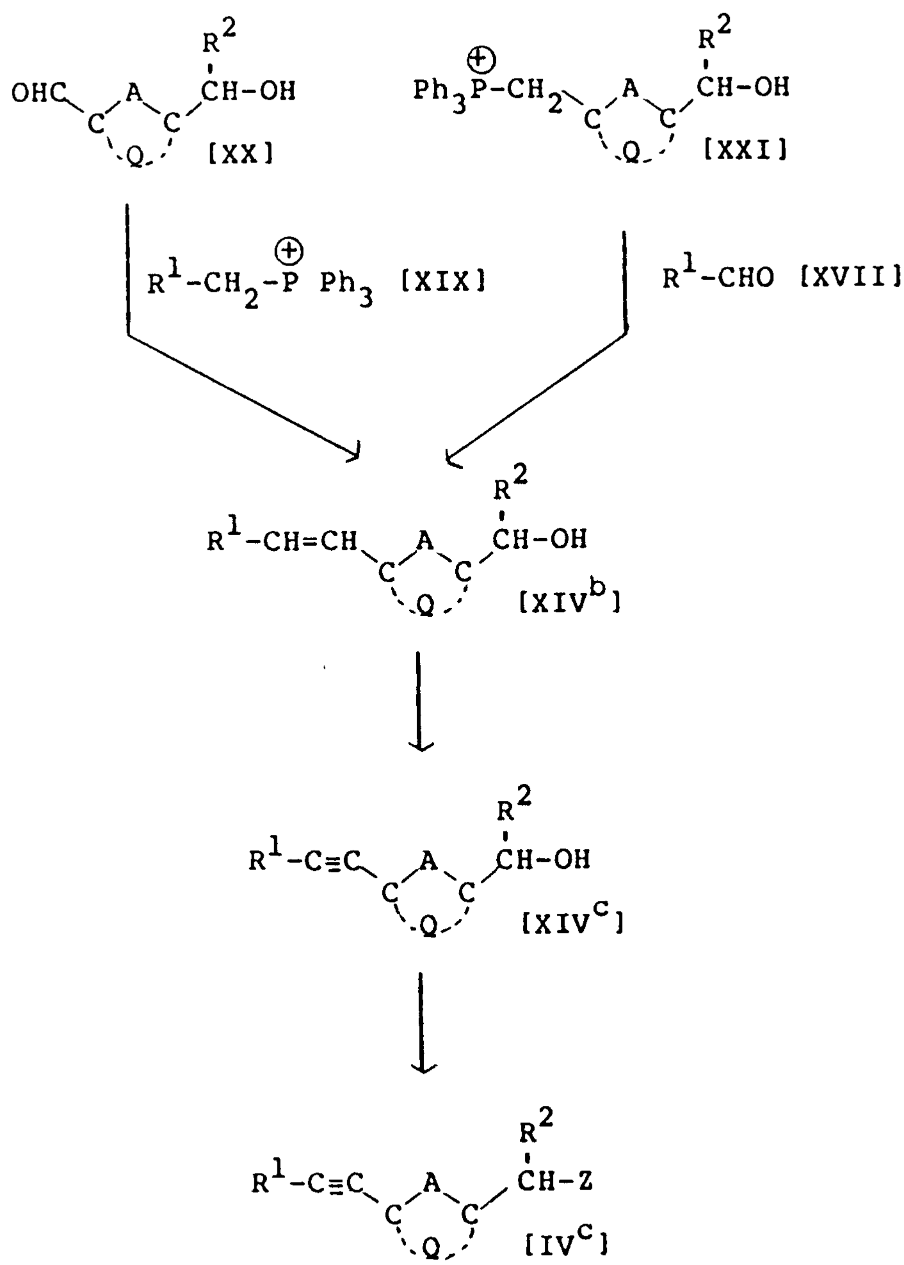

Production Route (d)

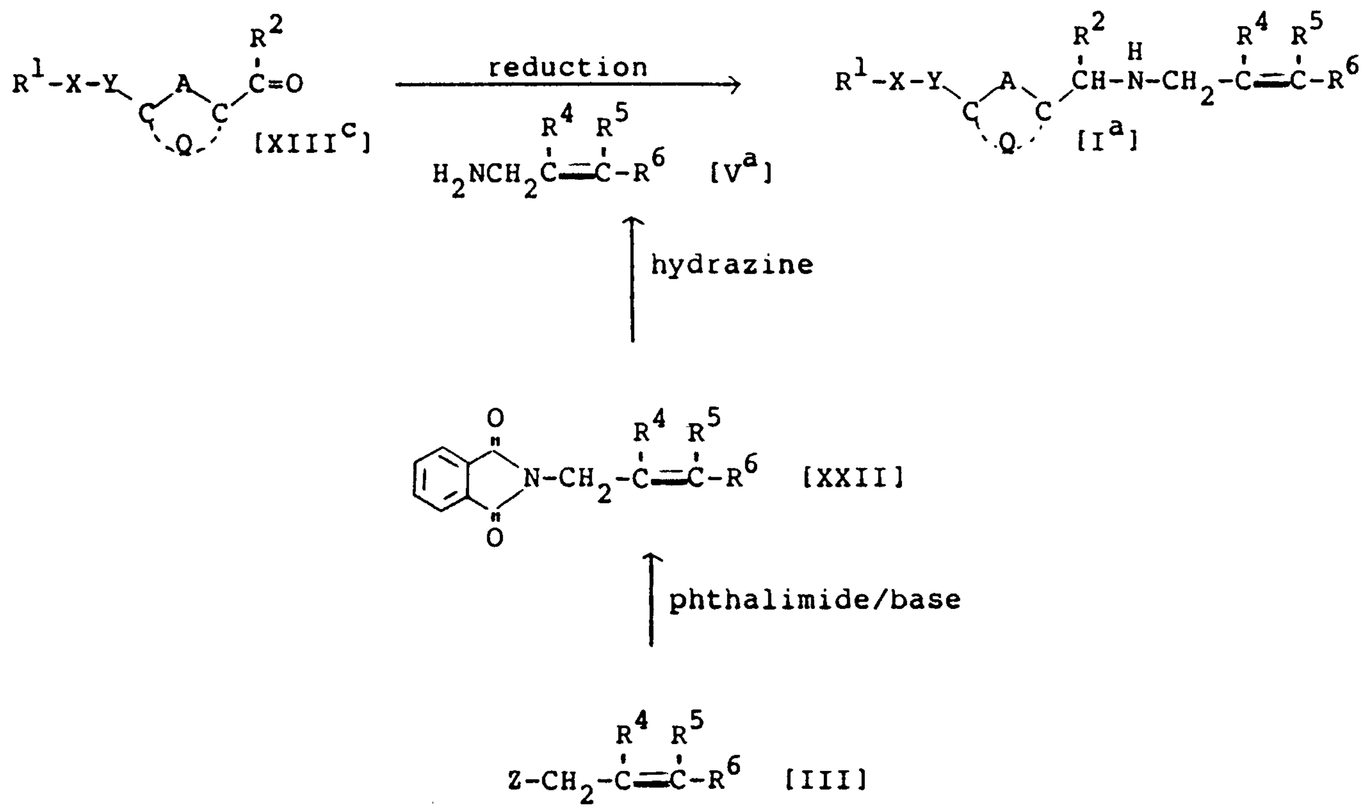


[XIII$^c$]

[V$^a$]

[I$^a$]

[XXII]

[III]

Production Route (e)

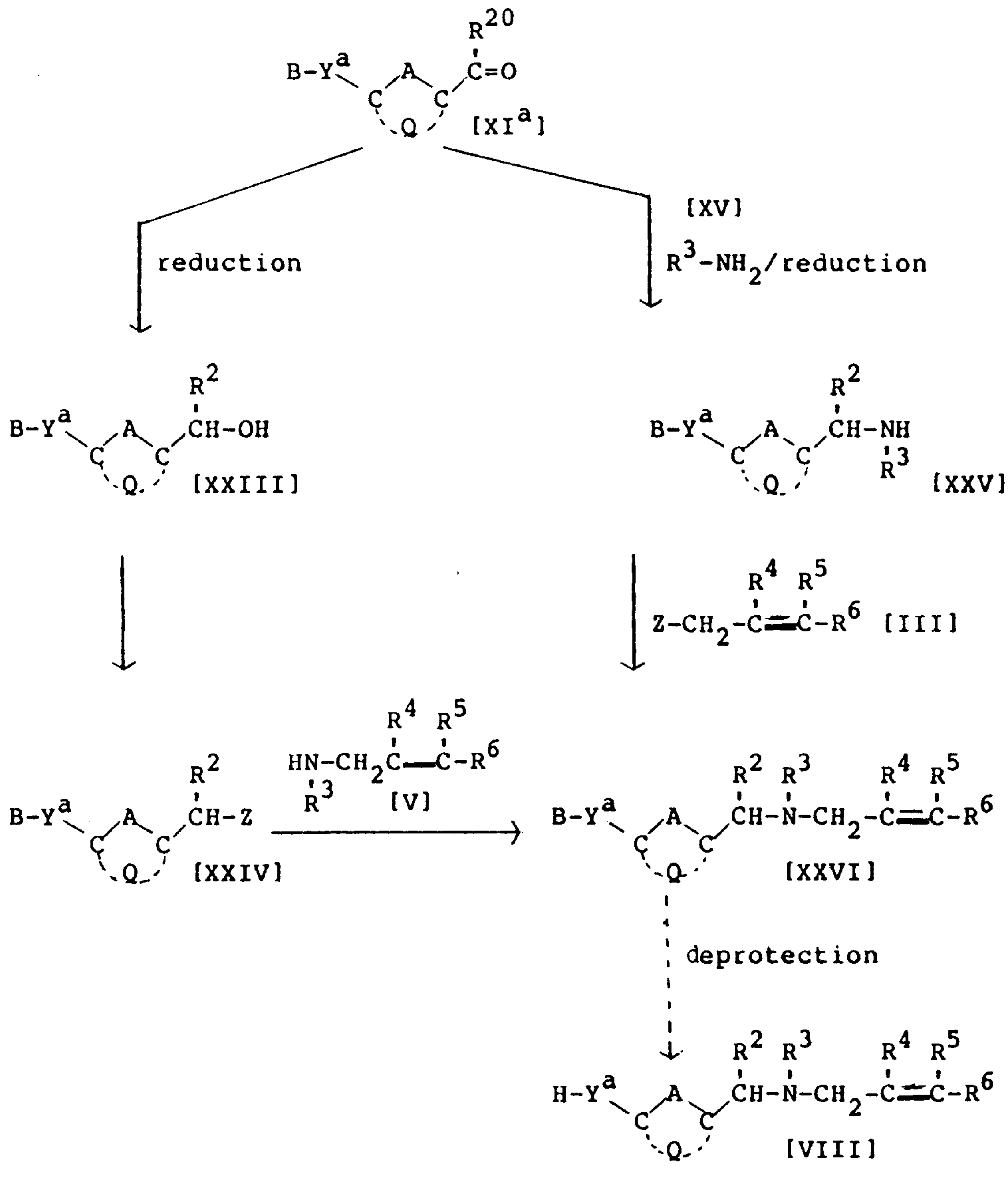

Production Route (f)

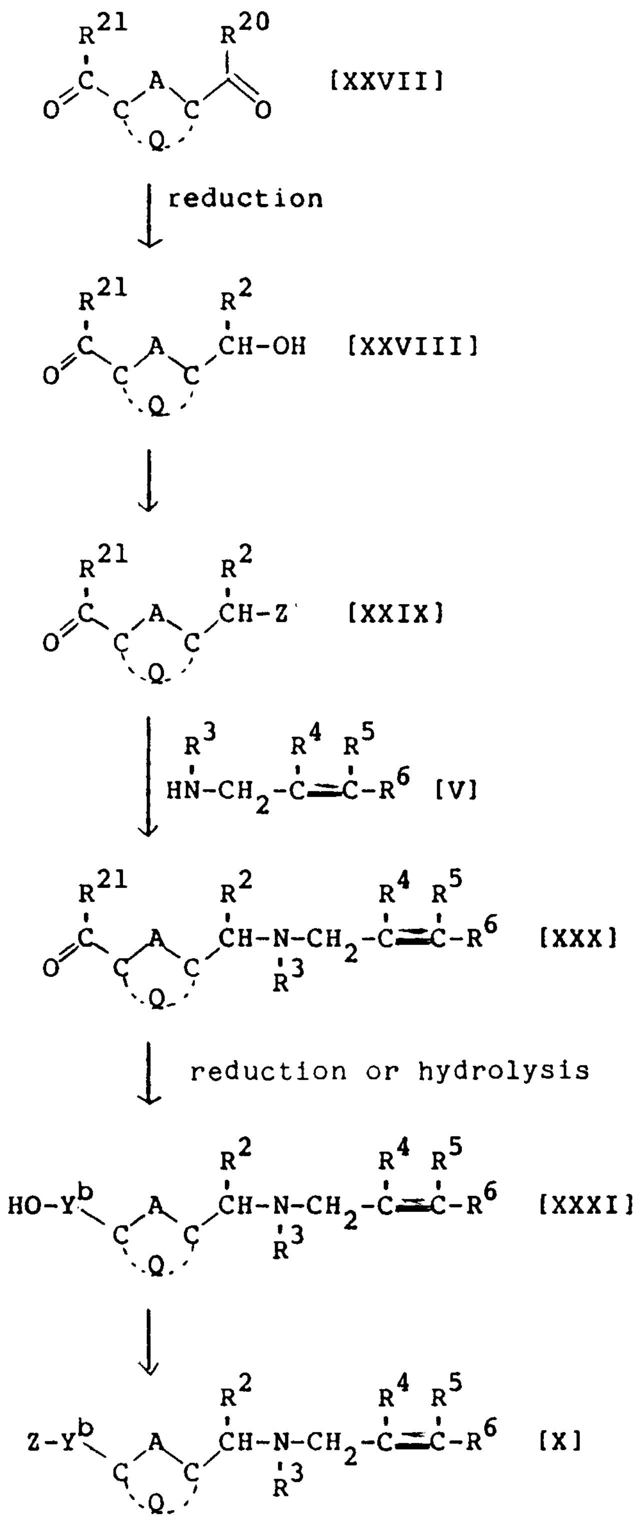


[XXVII] [XXVIII] [XXIX] [V] [XXX] [XXXI] [X]

In the above formulae, $X^c$ and $Y^c$ are identical or different, and each represents an oxygen atom, a sulfur atom, a carbonyl group, a group of the formula $-CHR^a-$ in which $R^a$ is as defined above or a group of the formula $-NR^b-$ in which $R^b$ is as defined above; each of $R^{20}$ and $R^{21}$ represents a hydrogen atom, a lower alkyl group or a lower alkoxy group; B represents a hydrogen atom or a protective group; and A, Q, X, Y, $X^a$, $X^b$, $Y^a$, $Y^b$, Z, $R^1$, $R^2$, $R^3$, $R^4$, $R^5$ and $R^6$ are as defined above. When either one of $X^c$ and $Y^c$

represents an oxygen atom, a sulfur atom or the group $-NR^b-$, the other represents a carbonyl group or the group $-CHR^a-$.

Production Route (a)

The step of reacting compound [XI] with compound [VII] to produce compound [XIII] is carried out in the presence of a base such as sodium hydride, sodium hydroxide or potassium carbonate in a solvent, for example tetrahydrofuran, ethanol or dimethylformamide, at a temperature of -20 °C to 100 °C for 2 to 3 hours using the compounds [XI] and [VII] in nearly equimolar proportions. The step of reacting compound [XII] with compound [IX] to produce compound [XIII] can be performed in the same way as the step of reacting compound [XI] with compound [VII].

The step of reacting compound [XIII] with compound [XV] to produce compound [$II^a$] can be performed by condensing compound [VIII] with compound [XV] in benzene or an alcohol to form an imine or amide and reducing it, or by reacting an excessive amount of compound [XV] with compound [XIII] and simultaneously performing reduction. Reagents used for reduction at this time are, for example, sodium borohydride, sodium cyanoborohydride and aluminum lithium hydride. The reaction may be carried out, for example, in methanol, etanol or tetrahydrofuran at 0 °C to room temperature for 1 to 6 hours.

The step of reducing compound [XIII] to produce compound [XIV] may be performed by, for example, treating the compound [XIII] with sodium borohydride, sodium cyanoborohydride or lithium aluminum hydride in a solvent such as methanol, ethanol or tetrahydrofuran at 0 °C to room temperature for 1 to 5 hours.

The step of converting compound [XIV] to compound [$IV^a$] may be performed by, for example, treating the compound [XIV] with halogenation reagents such as thionyl chloride or phosphorus tribromide or with sulfonation reagents such as methane sulfonyl chloride in the presence of triethylamine in a solvent such as chloroform or methylene chloride at -20 °C to room temperature for 1 to 5 hours.

Production Route (b)

The step of reacting compound [XVI] with compound [XVII] to produce compound [$XIII^a$] may be performed, for example, by treating the compounds with a base such as butyl lithium or sodium hydride in a solvent such as tetrahydrofuran at 0 °C to room temperature for 1 to 6 hours.

The step of reacting compound [XVIII] with compound [XIX] may be carried out in the same way as the step of reacting compound [XVI] with compound [XVII].

The step of producing compound [$XIII^b$] by reducing compound [$XIII^a$] may be carried out, for example, by catalytically reducing compound [$XIII^a$] in a solvent such as methanol or ethanol, at room temperature and atmospheric pressure for 1 to 10 hours in the presence of a catalyst such as palladium-carbon.

The step of producing compounds [$II^b$] or compound [$II^c$] by reacting compound [$XIII^a$] or compound [$XIII^b$] with compound [XV] may be carried out in the same way as in the above step of reacting compound [XIII] with compound [XV] to produce compound [$II^a$]. Furthermore, the step of reducing compound [$XIII^b$] to form compound [$XIV^a$] and then converting the compound [$XIV^a$] into compound [$IV^b$] may be carried out in the same way as a series of steps of producing compound [$IV^a$] from compound [XIII] via compound [XIV] described above.

Production Route (c)

The step of producing compound [$XIV^b$] by reacting compound [XX] with compound [XIX] or reacting compound [XXI] with compound [XVII] may be carried out in the same way as in the above-described step of producing compound [$XIII^a$] by reacting compound [XVI] with compound [XVII].

The step of converting compound [$XIV^b$] into compound [$XIV^c$] may be performed by reacting compound [$XIV^b$], either as such or after protection by a suitable protecting group, with bromine in an organic solvent such as methylene chloride, chloroform or ethyl ether at 0 to 60 °C for 0.5 to 3 hours, concentrating the reaction mixture to dryness, and then treating the residue in the presence of a base such as sodium hydroxide or potassium hydroxide in an alcohol solution such as methanol, ethanol or isopropanol at the boiling point of the solvent for 1 to 10 hours.

The step of converting compound [$XIV^c$] into compound [$IV^c$] may be carried out in the same way as in the step of converting compound [XIV] into compound [$IV^a$].

Production Route (d)

The step of producing compound [$I^a$] by reacting compound [$XIII^c$] with compound [$V^a$] may be performed in the same way as in the production of compound [$II^a$] by reacting compound [XIII] with compound [XV].

The compound [$V^a$] used at this time can be produced by the so-called Gabriel method which comprises reacting compound [III] with phthalimide at 10 to 100 °C in the presence of a base such as sodium hydroxide or potassium carbonate in a solvent such as tetrahydrofuran or dimethylformamide to produce compound [XXII] and then reacting this compound with hydrazine in ethanol or dimethylformamide to produce compound [$V^a$].

Production Route (e)

The step of producing compound [XXV] by reacting compound [$XI^a$] with compound [XV] may be performed in the same way as in the step of producing compound [$II^a$] by reacting compound [XIII] with compound [XV], the step of reducing compound [$XI^a$] to produce compound [XXIII] and then converting it into compound [XXIV] may be performed in the same way as the step of reducing compound [XIII] to produce compound [XIV] and converting it into compound [$IV^a$]. The step of reacting compound [XXIV] with compound [V] or reacting compound [XXV] with compound [III] to produce compound [XXVI], and then, as required, deprotecting this compound to obtain compound [VIII] may be carried out by the same method as in process A or B described above.

In the starting compound of formula [$XI^a$] in Production Route (e), the protecting group B may be any of various protective groups normally used in organic syntheses as protective groups for the hydroxyl group, mercapto group or amino group. Specific examples are methoxymethyl, tetrahydropyranyl, trityl, tert-butoxycarbonyl and dimethyl-tert-butylsilyl groups.

Production Route (f)

The step of producing compound [XXVIII] by reducing compound [XXVII] is the partial reduction utilizing the difference in reactivity in the reduction of the coexisting carbonyl groups, and can be carried out, for example, by reducing compound [XXVII] with 1 to 2 equivalents, based on compound [XXVII], of a reducing agent such as sodium borohydride at -20 °C to room temperature for 1 to 5 hours in a solvent such as ethanol or tetrahydrofuran, or catalytically reducing compound [XXVII] in the presence of a catalyst such as palladium-carbon for 1 to 5 hours. The step of producing compound [XXX] by converting compound [XXVIII] into compound [XXIX], and then reacting it with compound [V] may be carried out in the same way as in the step of producing compound [XXVI] by converting compound [XXIII] into compound [XXIV] and reacting the resulting compound with compound [V].

The step of producing compound [XXXI] by converting compound [XXX] may be carried out by reduction when the product is an alcohol product in which $Y^b$ is a group of the formula $-CHR^a-$ in which $R^a$ is as defined above, or by hydrolysis when the product is a carboxylic acid product in which $Y^b$ is a carbonyl group.

The step of producing the corresponding alcohol [XXXI] by reducing the compound [XXX] can be carried out in the same way as in the step of producing compound [XIV] by reducing the compound [XIII]. The step of hydrolyzing compound [XXX] to produce the corresponding carboxylic acid compound [XXXI] can be carried out, for example, by dissolving the compound [XXX] in a solvent such as hydrous ethanol or hydrous tetrahydrofuran in which an equimolar proportion or an excessive molar proportion of a base such as sodium hydroxide is present, and hydrolyzing it at room temperature to 100 °C for 1 to 10 hours.

The step of producing compound [X] by converting compound [XXXI] can be performed, for example, by treating it with a halogenation reagent such as thionyl chloride or phosphorus tribromide in the absence of a solvent or in a solvent such as chloroform or methylene chloride at -20 °C to room temperature for 1 to 5 hours.

The products obtained by the above steps may, as required, be purified or isolated by known purifying methods such as chromatography, recrystallization, solvent extraction, precipitation and distillation either singly or in combination.

Starting compounds for these starting intermediates can be purchased as commercial goods or can be easily obtained by the known methods of organic syntheses described in the literature (see, for example, J. Med. Chem., vol. 27, page 1539, 1984; J. Med. Chem., vol. 29, page 112, 1086; and Japanese Laid-Open Patent Publications Nos. 32440/1981, 123177/1982, 208252/1983, 45/1986, 201850/1987 and 5059/1988).

The compounds of this invention represented by general formula [I] inhibit the mammalian squalene epoxidase very selectively and strongly, and are expected to be useful as an hypolipemic agent or an antiarteriosclerotic agent.

The following Pharmacological Test Examples, Antimycotic Test Example, and Acute Toxicity Test Example given below demonstrate this fact.

PHARMACOLOGICAL TEST EXAMPLE 1

Squalene epoxidase inhibiting activity

(1) Preparation of squalene epoxidase

The rat squalene epoxidase was prepared by the method described in J. Biol. Chem., vol. 245, page 1670, 1970; ibid., vol. 250, page 1572, 1975).

SD-strain female rats were killed by exsanguination. Livers were extracted and homogenized with in the presence of 2 volumes of 0.1M Tris-HCl buffer (pH 7.5). The homogenate was centrifuged at 9750 Xg for 10 minutes. The supernatant fraction was further centrifuged at 105000 Xg for 1 hour. The sediment was washed with 0.1M Tris-HCl buffer (pH 7.5), and then centrifuged at 105000 Xg for 1 hour. The microsomes obtained were suspended in 0.1M Tris-HCl buffer (pH 7.5) so that the amount of proteins was 40 mg/ml, and under ice cooling, the suspension was stirred in the presence of 2 % Triton X-100 to solubilize the enzyme. After the solubilization, the solution was diluted to a Triton X-100 concentration of 0.5 % with 1 mM EDTA and 1 mM dithiothreitol, and centrifuged at 105000 Xg for 1 hour. The resulting supernatant fraction was used in the following test as a squalene epoxidase fraction.

(2) Method of assaying the squalene epoxidase activity

The squalene epoxidase activity was assayed in accordance with the method described in J. Biol. Chem., vol. 245, page 1670, 1970.

Three microliters of a dimethyl sulfoxide solution of a test drug was added to a solution composed of 0.2 ml of the squalene epoxidase fraction prepared in (1) [proteins 0.4 mg, 0.5 % Triton X-100, 20 $\mu$M Tris-HCl buffer (pH 7.5)], 100 $\mu$M FAD, 1 mM NADPH, 1 mM EDTA and 8 $\mu$M $^3$H-squalene-Tween 80 emulsion to adjust the total amount of the solution to 0.3 ml. The solution was incubated at 37 °C for 30 minutes with shaking. Then, 0.3 ml of a 10 % methanolic potassium hydroxide was added to stop the reaction, and the reaction mixture was left to stand at room temperature for 1 hour. The non-saponified material was extracted with petroleum ether, and the solvent was evaporated under a nitrogen stream. The resulting residue was dissolved in a small amount of ethyl ether and spotted on pre-coatd silica gel TLC plate, followed by developing with benzene/ethyl acetate (99.5:0.5). The position of the resulting $^3$H-squalene-2,3-epoxide on TLC was determined using ergosterol acetate as a marker, and the $^3$H-squalene-2,3-epoxide portion on the TLC was cut off. The TLC strip was immersed in a toluene-type scintillator, and measured by a liquid scintillation counter. As a result, the 50 % inhibitory concentrations ($IC_{50}$ values) of the compounds of this invention on squalene epoxidase were determined. The results are shown in Table 1.

The numbers designating the compounds correspond to the numbers given in Examples given hereinafter. The same applies to Tables 2 to 4.

Table 1

| Drug | 50 % inhibitory concentration ($IC_{50}$, $\mu M$) |
|---|---|
| Compound 1 | 6.80 |
| 2 | 1.40 |
| 6 | 0.56 |
| 8 | 2.90 |
| 10 | 4.70 |
| 12 | 6.00 |
| 16 | 1.40 |
| 18 | 0.25 |
| 19 | 3.10 |
| 28 | 5.60 |
| 34 | 1.70 |
| 36 | 0.30 |
| 37 | 0.27 |
| 41 | 2.10 |
| 42 | 9.60 |
| 43 | 3.90 |
| 44 | 0.16 |
| 45 | 0.12 |
| 46 | 0.15 |
| 47 | 0.15 |
| 48 | 5.10 |
| 49 | 4.90 |
| 50 | 2.10 |
| 51 | 1.10 |
| 52 | 1.40 |
| 53 | 0.66 |
| 54 | 1.80 |
| 55 | 0.60 |
| 56 | 2.10 |
| 57 | 3.10 |

- to be continued -

Table 1 (continued)

| Drug | 50 % inhibitory concentration ($IC_{50}$, $\mu M$) |
|---|---|
| Compound 58 | 7.90 |
| 61 | 6.10 |
| 62 | 0.36 |
| 63 | 5.40 |
| 64 | 0.51 |
| 65 | 4.60 |
| 66 | 1.20 |
| 67 | 2.40 |
| 68 | 1.20 |
| 69 | 0.51 |
| 70 | 3.10 |
| 71 | 0.54 |
| 72 | 9.70 |
| 73 | 2.30 |
| 74 | 0.57 |
| 75 | 0.14 |
| 76 | 1.20 |
| 77 | 1.20 |
| 78 | 0.063 |
| 79 | 0.089 |
| 81 | 0.014 |
| 82 | 0.87 |
| 83 | 1.90 |
| 86 | 3.30 |
| 87 | 3.50 |
| 90 | 5.50 |
| 92 | 3.30 |
| 95 | 6.80 |
| 96 | 0.84 |
| 97 | 0.32 |

- to be continued -

Table 1 (continued)

| Drug | 50 % inhibitory concentration ($IC_{50}$, μM) |
|---|---|
| Compound 99 | 2.00 |
| 100 | 0.12 |
| 101 | 2.10 |
| 102 | 2.10 |
| 103 | 0.36 |
| 104 | 7.70 |
| 107 | 3.60 |
| 108 | 0.45 |
| 110 | 1.50 |
| 112 | 0.69 |
| 115 | 0.27 |
| 117 | 0.52 |
| 118 | 0.29 |
| 119 | 0.0071 |
| 120 | 0.0027 |
| 121 | 0.026 |
| 122 | 0.013 |
| 123 | 0.034 |
| 126 | 2.20 |
| 131 | 1.00 |
| 132 | 5.40 |
| terbinafine | >100 |
| naftifine | >100 |

PHARMACOLOGICAL TEST EXAMPLE 2

Inhibitory activity on cholesterol biosynthesis in cultured cells:-

Human hepatoma (Hep-G2) cells were cultured in 10 $cm^2$ dishes until they formed a monolayer. One milliliter of the culture medium was replaced, and 1 μCi of [$^{14}C$] sodium acetate and 1 microliter of a dimethyl sulfoxide solution of a test drug were added, and the cells were cultured at 37 °C in air containing 5 % of carbon dioxide for 6 hours.

After the cultivation, the medium was aspirated, and the cells were cooled with ice, and washed with Dulbecco's phosphate buffered saline solution. The resulting cells were scraped by a rubber policeman, and collected by centrifugation. The cells collected were dissolved in 400 microliters of 0.3N sodium hydroxide. A 200 microliters aliquot of the solution was used for extraction, and the remainder, for protein determination.

To 200 microliters of the extracted cells, 15 % ethanolic potassium hydroxide was added, and saponification was carried out at 75 °C for 1 hour. Water (1 ml) was then added, and the mixture was extracted with 2 ml of petroleum ether twice to remove non-saponified materials. The petroleum ether extracts were washed with 1 ml of water, and evaporated under a nitrogen stream. The residue was spotted on a pre-coated silica gel TLC plate using a small amount of chloroform, and developed with hexane/ethyl ether/acetic acid (85:15:4). Cholesterol and squalene portions on the TLC were detected with iodine, and the corresponding TLC portions were cut out. The TLC strips were immersed in a toluene-type scintillator, and radioactivity was counted by a liquid scintillation counter. The results were corrected by the amount of proteins measured by the method described in J. Biol. Chem., vol. 193, page 265, 1951. The 50 % inhibitory concentration ($IC_{50}$ value) of the compound of the invention on cholesterol biosynthesis in the cultured Hep-G2 cells was calculated. The results are shown in Table 2.

Table 2

| Compound | 50% inhibitory concentration ($IC_{50}$, $\mu$M) |
|---|---|
| 2 | 1.50 |
| 6 | 0.34 |
| 36 | 0.064 |
| 37 | 0.085 |
| 44 | 0.050 |
| 45 | 0.083 |
| 46 | 0.11 |
| 47 | 0.090 |
| 64 | 0.12 |
| 78 | 0.020 |
| 79 | 0.023 |
| 103 | 0.45 |

PHARMACOLOGICAL TEST EXAMPLE 3

Test on inhibition of cholesterol biosynthesis in vivo:-

Male SD rats, 5 weeks of age, were used in the in vivo test. The rats were kept for 9 days in an environment of reversed light cycle (i.e., dark between 6:00 am and 6:00 pm). The rats were allowed to take a solid diet and water freely. The test drug was orally administered two hours before dark sixth hour when the cholesterol synthesis reached a maximum. Compounds 6 and 44 were administered as a suspension in a 0.5 % aqueous solution of methyl cellulose. Compound 45 was administered in a 0.5 % aqueous solution of methyl cellulose containing 5 % of dimethyl sulfoxide and 1 % Tween 80. The dose was 1 ml/100 g of body weight.

An equal volume of 0.5 % methyl cellulose was administered to a control group. One hour after administration of the test drug, [$^{14}C$] sodium acetate (56 mCi/mmole) was intraperitoneally administered to the rats in a dose of 20 $\mu$Ci/100 g of body weight. At dark sixth hour, a blood sample was obtained from the abdominal artery under ether anesthesia, and the plasma was separated by centrifugation.

Two milliliters of plasma was mixed with a 15 % methanolic potassium hydroxide and saponified by heating at 75 °C for 3 hours. The resulting sample was extracted with 2 ml of petroleum ether twice. The extracts were washed with 2 ml of distilled water, and finally evaporated under a nitrogen stream. The resulting residue was dissolved in a small amount of ethyl ether, and all the solution was spotted on a pre-coated silica gel TLC plate. The plate was developed with a solvent system composed of hexane/ethyl ether/acetic acid (85:15:4). Color formation was carried out by iodine, and the radioactivity of the cholesterol portion was measured by a liquid scintillation counter.

The results were expressed in dpm of the resulting $^{14}C$-cholesterol present in 1 ml of the plasma. The inhibition of cholesterol biosynthesis was calculated by comparing the amounts of $^{14}C$-cholesterol biosynthesized in the test group and that in the control group. The results are shown in Table 3.

Table 3

| | $^{14}$C-cholesterol | Inhibition of cholesterol biosynthesis |
|---|---|---|
| Control group<br>Compound 6 (100 mg/kg) | 1816±857<br>862±387 | -<br>53 % |
| Control group<br>Compound 44 (100 mg/kg) | 1913±668<br>568±313 | -<br>69 % |
| Control group<br>Compound 45 (100 mg/kg) | 1701±517<br>274±155 | -<br>85 % |

ANTIMYCOTIC ACTIVITY TEST EXAMPLE

Each of the test fungi was cultured (Candida albicans was cultured overnight in Sabouraud broth; Trichophyton was inoculated in Sabouraud's agar and cultured for 2 days, after which the spores were harvested). Thereafter, one platinum loopful of Candida albicans or Trichophyton ($10^6$ cells/ml for C. albicans; $10^6$ spores/ml for Trichophyton) was inoculated in Sabouraud's agar containing the test drug in various concentrations, and cultured (at 37 °C for 2 days for C. albicans; at 28 °C for 5 days for Trichophyton). Then, the minimum growth inhibition concentration (MIC) was measured. The results given in Table 4 show that the test drugs had very weak antimycotic activity and did not inhibit growth of the test fungi even in a concentration of as high as 100 micrograms/ml.

Table 4

| Test drug | MIC (µg/ml) | |
|---|---|---|
| | C. albicans | Trichophyton |
| Compound 2 | >100 | >100 |
| Compound 18 | >100 | >100 |
| Compound 45 | >100 | >100 |
| Compound 61 | >100 | >100 |
| Compound 64 | >100 | >100 |
| Compound 97 | >100 | >100 |
| Compound 107 | >100 | >100 |
| Compound 110 | >100 | >100 |
| terbinafine | 0.3 | <0.01 |

ACUTE TOXICITY TEST EXAMPLE

Each of test drugs (compounds 2, 6, 44, 45, 82, 97 and 103) was suspended or dissolved in 0.5 % methyl cellulose or olive oil, and orally administered to mice (ddy, male, body weight 28±2 g, five per group). The acute toxicity value ($LD_{50}$) was calculated from the mortality determined one hour after administration.

The test drug had very low toxicity, and even in a high dose of 1000 mg/kg, no case of death was occurred.

As can be seen from the results of the foregoing tests, the compounds of this invention strongly inhibit squalene epoxidase and thus the biosynthesis of cholesterol. Accordingly, they are effective for the treatment and prevention of various diseases induced by the increase of the biosynthesis of cholesterol, for example obesity, hyperlipemia and arteriosclerosis. Furthermore, the squalene epoxidase inhibiting activity of the compounds of this invention is not observed on fungi, and is specific for mammals. The compounds of this invention also have low toxicity. Accordingly, they are very useful as pharmaceuticals.

The compound of formula [I] provided by this invention is formulated into a form suitable for oral or parenteral administration, and can be used in the treatment and remedy of hypercholesterolemia, hyperlipemia and arteriosclerosis. In using the compounds of the invention clinically, they can be formulated

together with pharmaceutically acceptable adjuvants suitable for dosage forms, and then administered. Various adjuvants usually used in the pharmaceutical field can be used. Examples of the adjuvants include gelatin, lactose, sucrose, titanium dioxide, starch, crystalline cellulose, hydroxypropylmethyl cellulose, carboxymethyl cellulose, corn starch, microcrystalline wax, white Vaseline, magnesium metasilicate aluminate, anhydrous calcium phosphate, citric acid, trisodium citrate, hydroxypropyl cellulose, sorbitol, sorbitan fatty acid esters, polyvinylpyrrolidone, magnesium stearate, light anhydrous silicic acid, talc, vegetable oil, benzyl alcohol, gum arabic, propylene glycol or polyalkylene glycols.

Formulations prepared as mixtures with these adjuvants include, for example, solid preparations such as tablets, capsules, granules, powders and suppositories and liquid preparations such as syrups, elixirs and injectable preparations. These preparations can be formed by ordinary methods known in the field of pharmaceutical preparation. The liquid preparations may be in the form of a solution or suspension in water or another suitable medium. As required, the injectable preparations may be dissolved in physiological saline or glucose solution, or a buffer or a preservative may be added.

These formulations may contain 1.0 to 100 % by weight, preferably 1.0 to 60 % by weight, of the compound of this invention based on the total drugs. They may also contain other therapeutically effective compounds.

When the compound of this invention is used as an hypolipemic agent, an antiarteriosclerotic agent or an hypocholesterolemic agent, its dosage and the number of administration differ depending upon the sex, age, body weight and the severity of symptom of a patient and the type and range of the intended therapeutic effect. Generally, in oral administration, it is administered preferably in a dose of 0.01 to 20 mg/kg once or in several divided portions. In parenteral administration, it is preferably administered in a dose of 0.001 to 20 mg/kg once or in several divided portions.

The following Examples and Referential Examples illustrate the present invention more specifically. It should be understood that the present invention is not limited to these examples alone.

EXAMPLE 1

Production of (E)-N-(6,6-dimethyl-2-hepten-4-ynyl)-3-benzyloxybenzylamine (compound 1)

3-Benzyloxybenzylamine hydrochloride (110 mg) was dissolved in 3 ml of dimethylformamide, and 89 mg of 1-bromo-6,6-dimethyl-2-hepten-4-yne (a 3:1 E/Z form mixture; the same in the following examples) and 61 mg of potassium carbonate were added, and the mixture was stirred at room temperature for 2 hours. Water (15 ml) was added to the reaction mixture to dilute it, and the diluted reaction mixture was extracted with 10 ml of ethyl ether twice. The extracts were combined, washed with 10 ml of a saturated aqueous sodium chloride solution, and dried over anhydrous magnesium sulfate. The solvent was then evaporated. The residue was purified by preparative thin-layer chromatography [thin layer plate: Kieselgel 60$F_{254}$, ART. 5744 (E. Merck Co.); developing solvent: chloroform/methanol = 5/1] to give 16 mg (yield 11 %) of the captioned compound as a colorless oil.

$$IR\ \nu_{max}^{neat}\ cm^{-1}:$$

2968, 1599, 1491, 1458, 1263, 1155, 1029, 738, 696.

NMR($CDCl_3$)δ: 1.24(9H, s), 2.30(1H, br), 3.28(2H, dd, J = 6.4Hz, 1.5Hz), 3.77(2H, s), 5.06(2H, s), 5.64-(1H, dt, J = 15.9Hz, 1.5Hz), 6.09(1H, dt, J = 15.9Hz, 6.4Hz), 6.84-6.92(2H, m), 6.97-6.98(1H, m), 7.20-7.25(1H, m), 7.31-7.45(5H, m).

As an alternative method of synthesizing the above compound, phthalimide and 1-bromo-6,6-dimethyl-2-hept-4-yne (a mixture of E and Z forms in a ratio of about 3:1) are reacted in dimethylformamide. The resulting (E)-N-(6,6-dimethyl-2-hepten-4-ynyl)phthalimide by recrystallization from hexane, it is separated into an E form and a Z form, m.p. 106-108 °C) is reacted with hydrazine to produce (E)-6,6-dimethyl-2-hepten-4-ynyl-amine. This compound is reductively alkylated by using equimolar proportions of 3-benzyloxybenzaldehyde and sodium borohydride in methanol to give the captioned compound.

EXAMPLE 2

Production of (E)- and (Z)-N-(6,6-dimethyl-2-hepten-4-ynyl)-N-methyl-3-benzyloxybenzylamine hydrochloride

N-methyl-3-benzyloxybenzylamine hydrochloride (1.72 g) was dissolved in 20 ml of dimethylformamide, and 1.31 g of 1-bromo-6,6-dimethyl-2-hepten-4-yne and 0.76 g of sodium carbonate were added. The mixture was stirred overnight at room temperature. The reaction mixture was diluted with 80 ml of water, and extracted with 50 ml of ethyl ether twice. The extracts were combined, washed with 20 ml of a saturated aqueous sodium chloride solution, and then dried over anhydrous magnesium sulfate.

The desiccant was removed by filtration, and the solvent was evaporated. The residue was purified by medium-pressure liquid chromatography [column: Lobar column, size B, Lichroprep Si 60F (made by E. Merck Co.); eluting solvent: hexane/ethyl acetate = 6/1] to give a fraction containing the captioned (E)-form and a fraction containing the captioned (Z)-form. These fractions were separately evaporated under reduced pressure, dissolved in methanol containing hydrogen chloride, again concentrated under reduced pressure, and recrystallized from a mixture of tetrahydrofuran and ethyl ether. The amounts of the products obtained and their properties are shown below.

(E)-N-(6,6-dimethyl-2-hepten-4-ynyl)-N-methyl-3-benzyloxybenzylamine hydrochloride (compound 2)

Amount: 1.09 g (yield 44 %)
Melting point: 142-143 °C

$$IR\nu_{max}^{KBr}\ cm^{-1}:$$

2968, 2482, 1458, 1266, 696.

NMR($CDCl_3$)δ: 1.23(9H, s), 2.57(3H, s), 3.55(2H, d, J = 7.6Hz), 4.04(2H, s), 5.14(2H, s), 5.80(1H, d, J = 15.7Hz), 6.22(1H, dt, J = 15.7Hz, 7.6Hz), 7.00-7.04(1H, m), 7.05-7.08(1H, m), 7.28-7.39(5H, m), 7.43-7.46(2H, m).

(Z)-N-(6,6-dimethyl-2-hepten-4-ynyl)-N-methyl-3-benzyloxybenzylamine hydrochloride (compound 3)

Amount: 0.46 g (yield 19 %)
Melting point: 150-152 °C

$$IR\nu_{max}^{KBr}\ cm^{-1}:$$

2506, 1452, 1263, 1182, 1020, 747.

NMR($CD_3OD$)δ: 1.21(9H, s), 2.76(3H, s), 3.88-3.90 (2H, m), 4.30(2H, s), 5.14(2H, s), 6.00-6.04(2H, m), 7.08-7.15(2H, m), 7.17-7.18(1H, m), 7.30-7.46(6H, m).

Compounds of Examples 3 to 11 were prepared by repeating Example 2 except that instead of the starting N-methyl-3-benzyloxybenzylamine hydrochloride, the corresponding amine hydrochlorides were used (when the reaction product was a free base, the hydrochloride producing step in the after-treatment was not included).

EXAMPLE 3

(E)-N-(6,6-dimethyl-2-hepten-4-ynyl)-N-isopropyl-3-benzyloxybenzylamine hydrochloride (compound 4)

Melting point: 167-169 °C

$$IR\nu_{max}^{KBr}\ cm^{-1}:$$

2974, 1458, 1269, 696.

NMR($CDCl_3$)δ: 1.23(9H, s), 1.40(6H, br), 3.50 (3H, br), 4.00(2H, br), 5.17(2H, s), 5.74(1H, d, J=15.9Hz), 6.33-6.45 (1H, m), 6.98-7.02(1H, m), 7.11-7.14 (1H, m), 7.26-7.39(4H, m), 7.46-7.49 (2H, m), 7.50(1H, br).

(Z)-N-(6,6-dimethyl-2-hepten-4-ynyl)-N-isopropyl-3-benzyloxybenzylamine hydrochloride (compound 5)

$$IR\ \nu_{max}^{neat}\ cm^{-1}:$$

2968, 2932, 1602, 1491, 1458, 1266, 1167, 735.

NMR($CDCl_3$)δ: 1.04(6H, d, J=6.6Hz), 1.25(9H, s), 2.96(1H, sept, J=6.6Hz), 3.27(2H, dd, J=6.8Hz, 1.4Hz), 3.54(2H, s), 5.06 (2H, s), 5.49(1H, dt, J=10.7Hz, 1.4Hz), 5.82(1H, dt, J=10.7Hz, 6.8Hz), 6.81-6.84(1H, m), 6.92-6.96(1H, m), 7.04-7.05(1H, m), 7.16-7.22(1H, m), 7.30-7.45(5H, m).

EXAMPLE 4

(E)-N-(6,6-dimethyl-2-hepten-4-ynyl)-N-methyl-3-(2-methylbenzyloxy)benzylamine hydrochloride (compound 6)

Melting point: 150-152 °C

$$IR\ \nu_{max}^{KBr}\ cm^{-1}:$$

2974, 2680, 2626, 2506, 1602, 1497, 1458, 1263, 1164, 747.

NMR($CDCl_3$)δ: 1.25(9H, s), 2.40(3H, s), 2.62(3H, s), 3.45-3.58(1H, m), 3.65-3.75(1H, m), 3.95-4.07(1H, m), 4.12-4.25(1H, m), 5.14(2H, s), 5.82(1H, d, J=15.5Hz), 6.26(1H, dt, J=15.5Hz, 7.6Hz), 7.06 (1H, m), 7.11(1H, d, J=7.9Hz), 7.17-7.28(3H, m), 7.35(1H, t, J=7.9Hz), 7.40-7.45(2H, m).

(Z)-N-(6,6-dimethyl-2-hepten-4-ynyl)-N-methyl-3-(2-methylbenzyloxy)benzylamine hydrochloride (compound 7)

$$IR\ \nu_{max}^{neat}\ cm^{-1}:$$

2974, 1599, 1491, 1458, 1263, 1152, 1032, 747.

NMR($CDCl_3$)δ: 1.24(9H, s), 2.23(3H, s), 2.38(3H, s), 3.27(2H, dd, J=6.8Hz, 1.5Hz), 3.50 (2H, s), 5.03-(2H, s), 5.61(1H, dt, J=10.7Hz, 1.5Hz), 5.95(1H, dt, J=10.7Hz, 6.8Hz), 6.85-6.89(1H, m), 6.91-6.94 (1H, m), 6.99-7.00(1H, m), 7.19-7.26 (4H, m), 7.39-7.43(1H, m).

EXAMPLE 5

(E)-N-(6,6-dimethyl-2-hepten-4-ynyl)-N-methyl-3-(3-chlorobenzyloxy)benzylamine (compound 8)

$$IR\ \nu_{max}^{neat}\ cm^{-1}:$$

2974, 1602, 1458, 1266, 780.

NMR($CDCl_3$)δ: 1.24(9H, s), 2.18(3H, s), 3.03(2H, dd, J = 6.5Hz, 1.5Hz), 3.46(2H, s), 5.03 (2H, s), 5.64-(1H, dt, J = 15.9Hz, 1.5Hz), 6.07(1H, dt, J = 15.9Hz, 6.6Hz), 6.82-6.86(1H, m), 6.89-6.92(1H, m), 6.96-6.97(1H, m), 7.19-7.25(1H, m), 7.27-7.32(3H, m), 7.45(1H, br).

(Z)-N-(6,6-dimethyl-2-hepten-4-ynyl)-N-methyl-3-(3-chlorobenzyloxy)benzylamine (compound 9)

$$IR\ \nu_{max}^{neat}\ cm^{-1}:$$

2974, 1602, 1491, 1458, 1263, 1152, 1035, 780.

NMR($CDCl_3$)δ: 1.24(9H, s), 2.22(3H, s), 3.27(2H, dd, J = 6.8Hz, 1.5Hz), 3.50(2H, s), 5.04 (2H, s), 5.62-(1H, dt, J = 10.7Hz, 1.5Hz), 5.94(1H, dt, J = 10.7Hz, 6.8Hz), 6.82-6.86(1H, m), 6.91-6.94(1H, m), 6.97-6.98(1H, m), 7.20-7.26(1H, m), 7.27-7.32(3H, m), 7.43-7.44(1H, m).

EXAMPLE 6

(E)-N-(6,6-dimethyl-2-hepten-4-ynyl)-N-methyl-3-(4-fluorobenzyloxy)benzylamine (compound 10)

$$IR\ \nu_{max}^{neat}\ cm^{-1}:$$

2974, 1608, 1518, 1458, 1266, 1227, 1155.

NMR($CDCl_3$)δ: 1.20(9H, s), 2.18(3H, s), 3.03(2H, dd, J = 6.6Hz, 1.4Hz), 3.48(2H, s), 5.02 (2H, s), 5.64-(1H, dt, J = 15.6Hz, 1.4Hz), 6.08(1H, dt, J = 15.6Hz, 6.6Hz), 6.81-6.86(1H, m), 6.88-6.92(1H, m), 6.90-6.97(1H, m), 7.03-7.09(2H, m), 7.19-7.24(1H, m), 7.38-7.43(2H, m).

(Z)-N-(6,6-dimethyl-2-hepten-4-ynyl)-N-methyl-3-(4-fluorobenzyloxy)benzylamine (compound 11)

$$IR\ \nu_{max}^{neat}\ cm^{-1}:$$

2974, 1608, 1518, 1266, 1227, 1155.

NMR($CDCl_3$)δ: 1.24(9H, s), 2.21(3H, s), 3.26(2H, dd, J = 6.8Hz, 1.5Hz), 3.49(2H, s), 5.01 (2H, s), 5.61-(1H, dt, J = 10.7Hz, 1.5Hz), 5.93(1H, dt, J = 10.7Hz, 6.8Hz), 6.82-6.86(1H, m), 6.90-6.93(1H, m), 6.96-6.97(1H, m), 7.02-7.09(2H, m), 7.19-7.25(1H, m), 7.37-7.42(2H, m).

EXAMPLE 7

(E)-N-(6,6-dimethyl-2-hepten-4-ynyl)-N-methyl-3-(4-methoxybenzyloxy)benzylamine (compound 12)

Melting point: 35-36 °C

$$IR\ \nu_{max}^{KBr}\ cm^{-1}:$$

2968, 1611, 1587, 1518, 1458, 1251, 1173, 1032.

NMR($CDCl_3$)δ: 1.24(9H, s), 2.18(3H, s), 3.03(2H, dd, J = 6.3Hz, 1.4Hz), 3.46(2H, s), 3.81 (3H, s), 4.98-(2H, s), 5.64(1H, dt, J = 15.9Hz, 1.4Hz), 6.08(1H, dt, J = 15.9Hz, 6.3Hz), 6.83-6.94(4H, m), 6.95-6.96(1H, m), 7.18-7.23(1H, m), 7.34-7.38(2H, m).

(Z)-N-(6,6-dimethyl-2-hepten-4-ynyl)-N-methyl-3-(4-methoxybenzyloxy)benzylamine (compound 13)

$$IR\ \nu_{max}^{neat}\ cm^{-1}:$$

2968, 1614, 1518, 1458, 1251, 1173, 1032.

NMR($CDCl_3$)δ: 1.25(9H, s), 2.22(3H, s), 3.26(2H, dd, J = 6.8Hz, 1.5Hz), 3.49(2H, s), 3.81 (3H, s), 4.98-(2H, s), 5.61(1H, dt, J = 10.7Hz, 1.5Hz), 5.94(1H, dt, J = 10.7Hz, 6.8Hz), 6.83-6.87(1H, m), 6.89-6.94(3H, m), 6.97-6.98(1H, m), 7.19-7.24(1H, m), 7.31-7.37(2H, m).

EXAMPLE 8

(E)-N-(6,6-dimethyl-2-hepten-4-ynyl)-N-methyl-3-(beta-phenethyloxy)benzylamine (compound 14)

$$IR\ \nu_{max}^{neat}\ cm^{-1}:$$

2974, 1605, 1458, 1266, 1029, 696.

NMR($CDCl_3$)δ: 1.24(9H, s), 2.17(3H, s), 3.03(2H, dd, J = 6.6Hz, 1.4Hz), 3.10(2H, t, J = 7.2Hz), 3.44(2H, s), 4.17(2H, t, J = 7.4Hz), 5.64(1H, dt, J = 15.9Hz, 1.4Hz), 6.08 (1H, dt, J = 15.9Hz, 6.6Hz), 6.75-6.79 (1H, m), 6.85-6.89(2H, m), 7.16-7.35 (6H, m).

(Z)-N-(6,6-dimethyl-2-hepten-4-ynyl)-N-methyl-3-(beta-phenethyloxy)benzylamine (compound 15)

$$IR\ \nu_{max}^{neat}\ cm^{-1}:$$

2968, 1605, 1458, 1266, 1152, 1032, 696.

NMR($CDCl_3$)δ: 1.24(9H, s), 2.21(3H, s), 3.09(2H, t, J = 7.0Hz), 3.26(2H, dd, J = 6.8Hz, 1.5Hz), 3.47(2H, s), 4.17(2H, t, J = 7.0Hz), 5.60 (1H, dt, J = 10.7Hz, 1.5Hz), 5.94(1H, dt, J = 10.7Hz, 6.8Hz), 6.76-6.80(1H, m), 6.87-6.90(2H, m), 7.17-7.35(6H, m).

EXAMPLE 9

(E)-N-(6,6-dimethyl-2-hepten-4-ynyl)-N-methyl-3-(alpha-phenethyloxy)benzylamine (compound 16)

$$IR\ \nu_{max}^{neat}\ cm^{-1}:$$

2974, 1584, 1491, 1455, 1152, 1071, 699.

NMR($CDCl_3$)δ: 1.25(9H, s), 1.63(3H, d, J=6.5Hz), 2.11 (3H, s), 2.96(2H, dd, J=6.7Hz, 1.4Hz), 3.99-(2H, s), 5.33(1H, q, J=6.5Hz), 5.60 (1H, dt, J=15.9Hz, 1.4Hz), 6.03(1H, dt, J=15.9Hz, 6.7Hz), 6.70-7.40(1H, m), 6.78-6.81(1H, m), 6.84-6.86(1H, m), 7.08-7.13(1H, m), 7.21-7.59(5H, m).

(Z)-N-(6,6-dimethyl-2-hepten-4-ynyl)-N-methyl-3-(alpha-phenethyloxy)benzylamine (compound 17)

$$IR\ \nu_{max}^{neat}\ cm^{-1}:$$

2974, 1584, 1491, 1455, 1263, 1152, 1071, 699.

NMR($CDCl_3$)δ: 1.24(9H, s), 1.62(3H, d, J=6.3Hz), 2.15 (3H, s), 3.21(2H, dd, J=6.8Hz, 1.5Hz), 3.42-(2H, s), 5.32(1H, q, J=6.3Hz), 5.58 (1H, dt, J=10.7Hz, 1.5Hz), 5.89(1H, dt, J=10.7Hz, 6.8Hz), 6.70-6.74(1H, m), 6.81-6.84(1H, m), 6.86-6.87(1H, m), 7.09-7.14(1H, m), 7.19-7.39(5H, m).

EXAMPLE 10

(E)-N-(6,6-dimethyl-2-hepten-4-ynyl)-N-methyl-3-(1-naphthylmethyloxy)benzylamine (compound 18)

$$IR\ \nu_{max}^{neat}\ cm^{-1}:$$

2974, 1599, 1458, 1263, 1020, 792, 777.

NMR($CDCl_3$)δ: 1.25(9H, s), 2.21(3H, s), 3.06(2H, dd, J=6.6Hz, 1.4Hz), 3.49(2H, s), 5.50 (2H, s), 5.66-(1H, dt, J=15.9Hz, 1.4Hz), 6.10(1H, dt, J=15.9Hz, 6.6Hz), 6.92-6.96 (2H, m), 7.06-7.07(1H, m), 7.23-7.28 (1H, m), 7.44-7.58(3H, m), 7.60-7.63 (1H, m), 7.84-7.92(2H, m), 8.06-8.09 (1H, m).

(Z)-N-(6,6-dimethyl-2-hepten-4-ynyl)-N-methyl-3-(1-naphthylmethyloxy)benzylamine (compound 19)

$$IR\ \nu_{max}^{neat}\ cm^{-1}:$$

2968, 1602, 1458, 1263, 1026, 792, 777.

NMR($CDCl_3$)δ: 1.24(9H, s), 2.23(3H, s), 3.28(2H, dd, J=6.8Hz, 1.5Hz), 3.52(2H, s), 5.49 (2H, s), 5.62-(1H, dt, J=10.7Hz, 1.5Hz), 5.95(1H, dt, J=10.7Hz, 6.8Hz), 6.92-6.97(2H, m), 7.06-7.07(1H, m), 7.23-7.28(1H, m), 7.43-7.56(3H, m), 7.59-7.62(1H, m), 7.83-7.91(2H, m), 8.04-8.08(1H, m).

EXAMPLE 11

(E)-N-(6,6-dimethyl-2-hepten-4-ynyl)-N-methyl-3-benzyloxy-alpha-phenethylamine (compound 20)

$$IR\ \nu_{max}^{neat}\ cm^{-1}:$$

2974, 1602, 1584, 1458, 1284, 1266, 696.

NMR($CDCl_3$)δ: 1.24(9H, s), 1.32(3H, d, J=6.5Hz), 2.16 (3H, s), 2.88(1H, ddd, J=14.4Hz, 6.9Hz, 1.3Hz), 3.07(1H, ddd, J=14.4Hz, 6.4Hz, 1.6Hz), 3.52(1H, q, J=6.5Hz), 5.06 (2H, s), 5.58(1H, ddd, J=15.9Hz, 1.6Hz, 1.3Hz), 6.02(1H, ddd, J=15.9Hz, 6.9Hz, 6.2Hz), 6.83-6.87(1H, m), 6.88-6.91(1H, m), 6.96-6.98(1H, m), 7.18-7.24(1H, m), 7.29-7.45(5H, m).

(Z)-N-(6,6-dimethyl-2-hepten-4-ynyl)-N-methyl-3-benzyloxy-alpha-phenethylamine (compound 21)

$$IR\ \nu_{max}^{neat}\ cm^{-1}:$$

2974, 1602, 1584, 1491, 1458, 1266, 1026, 735, 696.

NMR($CDCl_3$)δ: 1.21(9H, s), 1.37(3H, d, J=6.8Hz), 2.19 (3H, s), 3.18(1H, ddd, J=14.4Hz, 7.1Hz, 1.5Hz), 3.33(1H, ddd, J=14.4Hz, 6.8Hz, 1.5Hz), 3.54(1H, q, J=6.8Hz), 5.06 (2H, s), 5.57(1H, dt, J=10.7Hz, 1.5Hz), 5.90(1H, ddd, J=10.7Hz, 7.1Hz, 6.8Hz), 6.83-6.87(1H, m), 6.91-6.94(1H, m), 6.97-6.99(1H, m), 7.19-7.25(1H, m), 7.28-7.45(5H, m).

EXAMPLE 12

Production of (E) and (Z)-N-(6,6-dimethyl-2-hepten-4-ynyl)-N-methyl-3-benzoylaminobenzylamine

N-methyl-3-benzoylaminobenzylamine hydrochloride (277 mg) was dissolved in 3 ml of dimethylformamide, and 221 mg of 1-bromo-6,6-dimethyl-2-hepten-4-yne and 552 mg of potassium carbonate were stirred overnight at room temperature. The reaction mixture was diluted with water, and extracted with ethyl ether. The extract was washed with a saturated aqueous sodium chloride solution, and dried over anhydrous sodium sulfate. The desiccant was separated by filtration, and then the solvent was evaporated. The residue was purified by preparative thin-layer chromatography [thin-layer plate: Kieselgel $60F_{254}$, Art. 5744 (a product of E. Merck Co.); developing solvent: hexane/ethyl acetate=3/1] to give the following compounds as colorless oils.

(E)-N-(6,6-dimethyl-2-hepten-4-ynyl)-N-methyl-3-benzoylaminobenzylamine (compound 22)

Amount: 179 mg (yield 50.0 %)

$$IR\ \nu_{max}^{neat}\ cm^{-1}:$$

3304, 2872, 1656, 1614, 1584, 1548, 1494, 1365, 1266, 966, 789.

NMR($CDCl_3$)δ: 1.24(9H, s), 2.20(3H, s), 3.06(2H, dd, J=6.6Hz, 1.5Hz), 3.49(2H, s), 5.66(1H, dt, J=15.9Hz, 1.5Hz), 6.10(1H, dt, J=15.9Hz, 6.6Hz), 7.09(1H, d, J=7.5Hz), 7.32(1H, t, J=7.5Hz), 7.45-7.58(4H, m), 7.64-7.67(1H, m), 7.85-7.88(2H, m).

(Z)-N-(6,6-dimethyl-2-hepten-4-ynyl)-N-methyl-3-benzoylaminobenzylamine (compound 23)

Amount: 93 mg (yield 25.7 %)

$$IR\ \nu_{max}^{neat}\ cm^{-1}:$$

3304, 2974, 2782, 1656, 1614, 1551, 1494, 1440, 1323, 1269, 1029, 792, 696.

NMR($CDCl_3$)δ: 1.24(9H, s), 2.23(3H, s), 3.29(2H, dd, J = 6.8Hz, 1.5Hz), 3.52(2H, s), 5.62(1H, dt, J = 10.7Hz, 1.5Hz), 5.96(1H, dt, J = 10.7Hz, 6.8Hz), 7.10(1H, d, J = 7.5Hz), 7.32(1H, t, J = 7.5Hz), 7.45-7.57(4H, m), 7.65-7.68(1H, m), 7.84-7.88(2H, m).

Compounds of Examples 13 and 14 were obtained by repeating Example 12 except that instead of the starting N-methyl-3-benzoylaminobenzylamine hydrochloride, the corresponding amine hydrochlorides were used.

EXAMPLE 13

(E)-N-(6,6-dimethyl-2-hepten-4-ynyl)-N-methyl-3-(N-phenylcarbamoyl)benzylamine (compound 24)

Melting point: 120-122 °C

$$IR\ \nu_{max}^{KBr}\ cm^{-1}:$$

3382, 2968, 1656, 1599, 1533, 1449, 1323, 1269, 753, 696.

NMR($CDCl_3$)δ: 1.24(9H, s), 2.20(3H, s), 3.08(2H, dd, J = 6.6Hz, 1.5Hz), 3.55(2H, s), 5.67 (1H, dt, J = 15.9Hz, 1.5Hz), 6.10(1H, dt, J = 15.9Hz, 6.6Hz), 7.15(1H, t, J = 7.5Hz), 7.34-7.51-(4H, m), 7.64-7.68 (2H, m), 7.51-7.76(4H, m), 7.76-7.90 (3H, m).

(Z)-N-(6,6-dimethyl-2-hepten-4-ynyl)-N-methyl-3-(N-phenylcarbamoyl)benzylamine (compound 25)

$$IR\ \nu_{max}^{neat}\ cm^{-1}:$$

3304, 2968, 1656, 1602, 1539, 1506, 1446, 1326, 1269, 753, 693.

NMR($CDCl_3$)δ: 1.23(9H, s), 2.26(3H, s), 3.30(2H, dt, J = 6.8Hz, 1.6Hz), 3.60(2H, s), 5.66(1H, dt, J = 10.7Hz, 1.6Hz), 5.97(1H, dt, J = 10.7Hz, 6.8Hz), 7.15(1H, t, J = 7.5Hz), 7.35-7.52(4H, m), 7.67(2H, d, J = 7.5Hz), 7.65-7.92(3H, m).

EXAMPLE 14

(E)-N-(6,6-dimethyl-2-hepten-4-ynyl)-N-methyl-3-phenoxymethylbenzylamine (compound 26)

$$IR\ \nu_{max}^{neat}\ cm^{-1}:$$

2968, 2788, 1602, 1500, 1365, 1242, 753, 690.

NMR($CDCl_3$)δ: 1.21(9H, s), 2.19(3H, s), 3.04(2H, dd, J = 6.6Hz, 1.5Hz), 3.50(2H, s), 5.05 (2H, s), 5.64-(1H, dd, J = 15.9Hz, 1.5Hz), 6.09(1H, dt, J = 15.9Hz, 6.6Hz), 6.93-6.99 (3H, m), 7.25-7.38(6H, m).

(Z)-N-(6,6-dimethyl-2-hepten-4-ynyl)-N-methyl-3-phenoxymethylbenzylamine (compound 27)

$$IR\ \nu_{max}^{neat}\ cm^{-1}:$$

2968, 1602, 1500, 1269, 1242, 1032, 753, 693.

NMR($CDCl_3$)δ: 1.24(9H, s), 2.21(3H, s), 3.28(2H, dd, J=5.7Hz, 1.5Hz), 3.53(2H, s), 5.05 (2H, s), 5.62-(1H, dd, J=10.7Hz, 1.5Hz), 5.95(1H, dt, J=10.7Hz, 5.7Hz), 6.93-6.99 (3H, m), 7.25-7.39(6H, m).

EXAMPLE 15

Production of (E)- and (Z)-N-(6,6-dimethyl-2-hepten-4-ynyl)-N-methyl-3-benzyloxy-4-methylbenzylamine

N-methyl-3-benzyloxy-4-methylbenzylamine hydrochloride (67 mg) was dissolved in 2 ml of dimethylformamide, and 48 mg of 1-bromo-6,6-dimethyl-2-hepten-4-yne and 51 mg of sodium carbonate were added. The mixture was stirred overnight at room temperature. The reaction mixture was concentrated under reduced pressure at below 40 °C, and the residue was purified by preparative thin-layer chromatography [thin-layer plate: Kieselgel $60F_{254}$, Art. 5744 (a product of E. Merck Co.); developing solvent: hexane/ethyl acetate=5/1)] to give the following compounds as colorless oily products.

(E)-(6,6-dimethyl-2-hepten-4-ynyl)-N-methyl-3-benzyloxy-4-methylbenzylamine (compound 28)

Amount: 50 mg (yield 57 %)

$$IR\ \nu_{max}^{neat}\ cm^{-1}:$$

2974, 2926, 1512, 1458, 1422, 1368, 1260, 1155, 1128, 1026, 735.

NMR($CDCl_3$)δ: 1.24(9H, s), 2.18(3H, s), 2.26(3H, s), 3.02(1H, dd, J=6.6Hz, 1.5Hz), 3.46 (2H, s), 5.09-(2H, s), 5.63(1H, dt, J=15.7Hz, 1.5Hz), 6.07(1H, dt, J=15.7Hz, 6.6Hz), 6.78(1H, dd, J=7.6Hz, 1.2Hz), 6.91(1H, d, J=1.2Hz), 7.08(1H, d, J=7.6Hz), 7.28-7.42 (3H, m), 7.42-7.49(2H, m).

(Z)-(6,6-dimethyl-2-hepten-4-ynyl)-N-methyl-3-benzyloxy-4-methylbenzylamine (compound 29)

Amount: 20 mg (yield 23 %)

$$IR\ \nu_{max}^{neat}\ cm^{-1}:$$

2968, 2926, 1458, 1419, 1263, 1128, 1029, 735.

NMR($CDCl_3$)δ: 1.24(9H, s), 2.23(3H, s), 2.26(3H, s), 3.27(2H, d, J=6.9Hz), 3.50(2H, s), 5.09(2H, s), 5.62(1H, d, J=10.7Hz), 5.95(1H, dt, J=10.7Hz, 6.9Hz), 6.81 (1H, dd, J=7.6Hz, 1.2Hz), 6.93(1H, s), 7.09(1H, d, J=7.6Hz), 7.30-7.48(5H, m).

Compounds of Examples 16 to 18 were obtained by repeating Example 15 except that instead of the starting N-methyl-3-benzyloxy-4-methylbenzylamine hydrochloride, the corresponding amine hydrochlorides were used.

EXAMPLE 16

(E)-(6,6-dimethyl-2-hepten-4-ynyl)-N-methyl-5-benzyloxy-2-bromobenzylamine (compound 30)

$$IR\ \nu_{max}^{neat}\ cm^{-1}:$$

2974, 1485, 1458, 1419, 1365, 1281, 1260, 1041, 1026.

NMR($CDCl_3$)δ: 1.24(9H, s), 2.15(3H, s), 3.00(2H, dd, J=6.7Hz, 1.5Hz), 3.41(2H, s), 5.17 (2H, s), 5.62-(1H, dt, J=15.9Hz, 1.5Hz), 6.03(1H, dt, J=15.9Hz, 6.7Hz), 6.77 (1H, dd, J=8.1Hz, 2.1Hz), 6.97(1H, d, J=2.1Hz), 7.30-7.52(6H, m).

(Z)-(6,6-dimethyl-2-hepten-4-ynyl)-N-methyl-5-benzyloxy-2-bromobenzylamine (compound 31)

$$IR\ \nu_{max}^{neat}\ cm^{-1}:$$

2968, 1485, 1458, 1419, 1284, 1266, 1041, 1029, 735.

NMR($CDCl_3$)δ: 1.24(9H, s), 2.19(3H, s), 3.23(2H, dd, J=6.8Hz, 1.3Hz), 3.45(2H, s), 5.16 (2H, s), 5.61-(1H, dt, J=10.7Hz, 1.3Hz), 5.90(1H, dt, J=10.7Hz, 6.8Hz), 6.79 (1H, dd, J=8.1Hz, 1.9Hz), 6.98(1H, d, J=1.9Hz), 7.30-7.42(3H, m), 7.44-7.52 (3H, m).

EXAMPLE 17

(E)-(6,6-dimethyl-2-hepten-4-ynyl)-N-methyl-3-benzyloxy-2-methoxybenzylamine (compound 32)

$$IR\ \nu_{max}^{neat}\ cm^{-1}:$$

2972, 1482, 1458, 1270, 1014, 754.

NMR($CDCl_3$)δ: 1.24(9H, s), 2.20(3H, s), 3.07(2H, dd, J=6.5Hz, 1.7Hz), 3.45(2H, s), 3.86 (3H, s), 5.11-(2H, s), 5.66(1H, dt, J=15.7Hz, 1.7Hz), 6.10(1H, dt, J=15.7Hz, 6.5Hz), 6.87(1H, dd, J=6.7Hz, 3.2Hz), 6.94-7.01(2H, m), 7.28-7.48(5H, m).

(Z)-(6,6-dimethyl-2-hepten-4-ynyl)-N-methyl-3-benzyloxy-2-methoxybenzylamine (compound 33)

$$IR\ \nu_{max}^{neat}\ cm^{-1}:$$

2968, 1518, 1461, 1266, 1137, 1029.

NMR($CDCl_3$)δ: 1.24(9H, s), 2.16(3H, s), 3.21(2H, dd, J=6.8Hz, 1.5Hz), 3.41(2H, s), 3.87 (3H, s), 5.15-(2H, s), 5.60(1H, dt, J=10.7Hz, 1.5Hz), 5.90(1H, dt, J=10.7Hz, 6.8Hz), 6.82-6.92(3H, m), 7.26-7.39 (3H, m). 7.41-7.48(2H, m).

EXAMPLE 18

(E)-N-(6,6-dimethyl-2-hepten-4-ynyl)-N-methyl-3-furfuryloxybenzylamine (compound 34)

$$IR\ \nu_{max}^{neat}\ cm^{-1}:$$

2968, 1599, 1491, 1455, 1368, 1263, 1155, 1017, 741.

NMR($CDCl_3$)δ: 1.24(9H, s), 2.18(3H, s), 3.03(2H, dd, J=6.6Hz, 1.7Hz), 3.46(2H, s), 5.00 (2H, s), 5.65-(1H, dt, J=15.9Hz, 1.7Hz), 6.08(1H, dt, J=15.9Hz, 6.6Hz), 6.38 (1H, dt, J=3.2Hz, 1.6Hz), 6.43(1H, dd, J=3.2Hz, 0.7Hz), 6.84-6.89(1H, m), 6.89-6.94(1H, m), 6.96-7.00-(1H, m), 7.22(1H, t, J=7.8Hz), 7.45(1H, dd, J=1.6Hz, 0.7Hz).

(Z)-N-(6,6-dimethyl-2-hepten-4-ynyl)-N-methyl-3-furfuryloxybenzylamine (compound 35)

$$IR\ \nu_{max}^{neat}\ cm^{-1}:$$

2968, 1605, 1458, 1260, 1155, 1017, 741.

NMR($CDCl_3$)δ: 1.25(9H, s), 2.22(3H, s), 3.26(2H, dd, J=6.8Hz, 1.5Hz), 3.49(2H, s), 5.00 (2H, s), 5.62-(1H, dt, J=10.8Hz, 1.5Hz), 5.95(1H, dt, J=10.8Hz, 6.8Hz), 6.38 (1H, dd, J=3.2Hz, 2.0Hz), 6.43(1H, dd, J=3.2Hz, 0.8Hz), 6.84-6.90(1H, m), 6.91-6.96(1H, m), 6.96-7.00-(1H, m), 7.23(1H, t, J=7.8Hz), 7.45(1H, dd, J=2.0Hz, 0.8Hz).

EXAMPLE 19

Production of (E)-N-(6,6-dimethyl-2-hepten-4-ynyl)-N-ethyl-3-benzyloxybenzylamine hydrochloride (compound 36)

N-Ethyl-3-benzyloxybenzylamine hydrochloride (100 mg) was dissolved in 2 ml of dimethylformamide, and 73 mg of 1-bromo-6,6-dimethyl-2-hepten-4-yne and 69 mg of potassium carbonate were added. The mixture was stirred overnight at room temperature. The reaction mixture was concentrated under reduced pressure, and the residue was dissolved in 10 ml of ethyl ether. The insoluble material was separated by filtration, and the solvent was evaporated. The residue was subjected to silica gel column chromatography [Wakogel C-200, 10 g; eluting solvent: hexane/ethyl acetate = 20/1 → 3/1] to isolate only the E-form. It was dissolved in HCl-methanol and again distilled under reduced pressure. Recrystallization of the residue from a mixture of tetrahydrofuran and hexane gave 58 mg (yield 40 %) of the captioned compound as a colorless crystalline powder having a melting point of 116 to 119 °C.

$$IR\ \nu_{max}^{KBr}\ cm^{-1}:$$

3460, 2974, 2926, 2494, 1458, 1266, 699.

NMR($CDCl_3$)δ: 1.25(9H, s), 1.43(3H, t, J=7.2Hz), 2.90-3.15(2H, m), 3.46-3.75(2H, m), 4.07(2H, s), 5.17(2H, s), 5.81(1H, d, J=15.9Hz), 6.22(1H, dt, J=15.9Hz, 7.7Hz), 7.02-7.07(1H, m), 7.12(1H, d, J=7.5Hz), 7.27-7.52(7H, m).

Compounds of Examples 20 to 30 were obtained by repeating Example 19 except that instead of the starting N-ethyl-3-benzyloxybenzylamine hydrochloride, the corresponding amines were used (when the product was a free base, the hydrochloride production step in the after-treatment was not included).

EXAMPLE 20

(E)-N-(6,6-dimethyl-2-hepten-4-ynyl)-N-propyl-3-benzyloxybenzylamine (compound 37)

$$IR\ \nu_{max}^{neat}\ cm^{-1}:$$

2968, 2872, 1602, 1491, 1263, 1029, 783, 738.

NMR($CDCl_3$)δ: 0.86(3H, t, J=7.3Hz), 1.24(9H, s), 1.43-1.51(2H, m), 2.37(2H, t, J=7.4Hz), 3.06(2H, dd, J=6.3Hz, 1.5Hz), 3.52 (2H, s), 5.06(2H, s), 5.63(1H, dt, J=15.9Hz, 1.5Hz), 6.06(2H, dt, J=15.9Hz, 6.3Hz), 6.85(1H, ddd, J=8.2Hz, 2.8Hz, 0.6Hz), 6.91(1H, d, J=7.5Hz), 6.99-7.01(1H, m), 7.20 (1H, t, J=7.8Hz), 7.32-7.46(5H, m).

EXAMPLE 21

(E)-N-butyl-N-(6,6-dimethyl-2-hepten-4-ynyl)-3-benzyloxybenzylamine (compound 38)

$$IR\ \nu_{max}^{neat}\ cm^{-1}:$$

2968, 2932, 1599, 1458, 735.

NMR($CDCl_3$)δ: 0.87(3H, t, J=7.2Hz), 1.24(9H, s), 1.27-1.33(2H, m), 1.41-1.46(2H, m), 1.56(2H, s), 2.40(2H, t, J=7.2Hz), 3.06(2H, dd, J=6.3Hz, 1.4Hz), 3.52 (2H, s), 5.62(1H, dt, J=15.9Hz, 1.5Hz), 6.05(1H, dt, J=15.9Hz, 6.3Hz), 6.84 (1H, ddd, J=10.8Hz, 2.7Hz, 0.8Hz), 6.90(1H, d, J=7.8Hz), 6.99-7.10(1H, m), 7.20(1H, t, J=7.8Hz), 7.32-7.46(5H, m).

EXAMPLE 22

(E)-N-(6,6-dimethyl-2-hepten-4-ynyl)-N-isobutyl-3-benzyloxybenzylamine (compound 39)

$$IR\ \nu_{max}^{neat}\ cm^{-1}:$$

2968, 2872, 1599, 1458, 1263, 738, 693.

NMR($CDCl_3$)δ: 0.87(6H, d, J=6.6Hz), 1.24(9H, s), 1.76-1.79(1H, m), 2.15(1H, d, J=7.3Hz), 3.03(2H, dd, J=6.3Hz, 1.6Hz), 3.50 (2H, s), 5.06(2H, s), 5.61(1H, dt, J=15.9Hz, 1.6Hz), 6.05-(1H, dt, J=15.9Hz, 6.3Hz), 6.84(1H, ddd, J=8.2Hz, 3.5Hz, 0.8Hz), 6.91(1H, dd, J=8.0Hz, 0.5Hz), 6.99-7.01(1H, m), 7.32-7.46(5H, m).

EXAMPLE 23

(E)-N-(6,6-dimethyl-2-hepten-4-ynyl)-N-pentyl-3-benzyloxybenzylamine (compound 40)

$$IR\ \nu_{max}^{neat}\ cm^{-1}:$$

2968, 2866, 1599, 1458, 1266, 735, 693.

NMR($CDCl_3$)δ: 0.87(3H, t, J=6.8Hz), 1.24(9H, s), 1.25-1.27(4H, m), 1.43-1.45(2H, m), 2.39(2H, t, J=7.0Hz), 3.06(2H, dd, J=6.4Hz, 1.6Hz), 3.52(2H, s), 5.06 (2H, s), 5.62(1H, dt,

J = 15.9Hz, 0.8Hz), 6.05(1H, dt, J = 15.9Hz, 6.4Hz), 6.84(1H, ddd, J = 8.2Hz, 2.6Hz, 0.9Hz), 6.90(1H, d, J = 7.4Hz), 6.99-7.00(1H, m), 7.20(1H, t, J = 7.8Hz), 7.32-7.46(5H, m).

EXAMPLE 24

(E)-N-allyl-N-(6,6-dimethyl-2-hepten-4-ynyl)-3-benzyloxybenzylamine (compound 41)

$$IR\ \nu_{max}^{neat}\ cm^{-1}:$$

2974, 1599, 1491, 1458, 1263, 1152, 1029, 696.

NMR($CDCl_3$)δ: 1.25(9H, s), 3.05-3.10(4H, m), 3.50 (2H, s), 5.07(2H, s), 5.07-5.22(2H, m), 5.64(1H, dt, J = 15.9Hz, 1.9Hz), 5.84 (1H, ddt, J = 16.8Hz, J = 10.8Hz, 6.3Hz), 6.06(1H, dt, J = 15.9Hz, 6.7Hz), 6.84-7.46(9H, m).

EXAMPLE 25

(E)-N-(6,6-dimethyl-2-hepten-4-ynyl)-N-propargyl-3-benzyloxybenzylamine (compound 42)

$$IR\ \nu_{max}^{neat}\ cm^{-1}:$$

2974, 1602, 1491, 1458, 1365, 1263, 1155, 1029, 960, 738, 696.

NMR($CDCl_3$)δ: 1.24(9H, s), 2.22(1H, t, J = 2.4Hz), 3.17(1H, dd, J = 6.5Hz, 1.5Hz), 3.30(2H, d, J = 2.4Hz), 5.06(2H, s), 5.71(1H, dt, J = 15.9Hz, 1.5Hz), 6.02(1H, dt, J = 15.9Hz, 6.5Hz), 6.85-7.48(9H, m).

EXAMPLE 26

(E)-N-cyclopropyl-N-(6,6-dimethyl-2-hepten-4-ynyl)-3-benzyloxybenzylamine (compound 43)

$$IR\ \nu_{max}^{neat}\ cm^{-1}:$$

2974, 2926, 1590, 1491, 1458, 1365, 1263, 696.

NMR($CDCl_3$)δ: 0.38-0.44(4H, m), 1.29(9H, s), 1.80-1.87(1H, m), 3.14(2H, dd, J = 6.8Hz, 1.6Hz), 3.67-(2H, s), 5.06(2H, s), 5.55 (1H, dt, J = 15.9Hz, 1.6Hz), 6.11(1H, dt, J = 15.9Hz, 6.8Hz), 6.83-7.45(9H, m).

EXAMPLE 27

(E)-N-(6,6-dimethyl-2-hepten-4-ynyl)-N-ethyl-3-(2-methylbenzyloxy)benzylamine hydrochloride (compound 44)

Melting point: 125-127 °C

$$IR\ \nu_{max}^{KBr}\ cm^{-1}:$$

2974, 2926, 2566, 2488, 1458, 1266, 750

NMR($CDCl_3$)δ: 1.25(9H, s), 1.44(3H, t, J=7.2Hz), 2.40(3H, s), 2.90-3.16(2H, m), 3.48-3.60 (1H, m), 3.62-3.75(1H, m), 4.09(2H, s), 5.15(2H, s), 5.81(1H, d, J=15.8Hz), 6.23(1H, dt, J=15.8Hz, 7.6Hz), 7.05 (1H, dd, J=7.9Hz, 2.6Hz) 7.14(1H, d, J=7.9Hz), 7.16-7.28(3H, m), 7.34(1H, t, J=7.9Hz), 7.41-7.45(1H, m), 7.52 (1H, br).

EXAMPLE 28

(E)-N-(6,6-dimethyl-2-hepten-4-ynyl)-N-propyl-3-(2-methylbenzyloxy)benzylamine hydrochloride (compound 45)

Melting point: 138-140 °C

$$IR\ \nu_{max}^{KBr}\ cm^{-1}:$$

2968, 2872, 1599, 1491, 1458, 1263, 1152, 747.

NMR($CDCl_3$)δ: 0.93(3H, t, J=7.3Hz), 1.25(9H, s), 1.80(2H, br), 2.40(3H, s), 2.75(2H, br), 3.48(2H, br), 3.97(2H, br), 5.12(2H, s), 5.70(1H, d, J=15.6Hz), 6.18(1H, dt, J=15.6Hz, 7.0Hz) 7.11-(1H, dd, J=7.2Hz, 1.2Hz), 7.08(1H, d, J=7.2Hz), 7.18-7.45 (6H, m).

EXAMPLE 29

(E)-N-(6,6-dimethyl-2-hepten-4-ynyl)-N-ethyl-3-(3-cyanobenzyloxy)benzylamine (compound 46)

$$IR\ \nu_{max}^{neat}\ cm^{-1}:$$

2974, 1491, 1458, 1263, 1155, 1044, 789, 687.

NMR($CDCl_3$)δ: 1.03(3H, t, J=7.1Hz), 1.24(9H, s), 2.50(2H, q, J=7.1Hz), 3.08(2H, dd, J=6.4Hz, 1.5Hz), 3.54(2H, s), 5.09 (2H, s), 5.64(1H, dt, J=15.9Hz, 1.5Hz), 6.06(1H, dt, J=15.9Hz, 6.4Hz), 6.80-6.86(1H, m), 6.94(1H, d, J=7.6Hz), 6.97-7.02(1H, m), 7.23(1H, t, J=7.9Hz), 7.50(1H, t, J=7.6Hz), 7.59-7.64(1H, m), 7.65-7.71(1H, m), 7.75-7.78(1H, m).

EXAMPLE 30

(E)-N-(6,6-dimethyl-2-hepten-4-ynyl)-N-propyl-3-(3-cyanobenzyloxy)benzylamine hydrochloride (compound 47)

Melting point: 136-138 °C

$$IR\ \nu_{max}^{KBr}\ cm^{-1}:$$

2974, 2500, 1605, 1455, 1266, 1182.

NMR($CDCl_3$)δ: 0.95(3H, t, J=7.3Hz), 1.26(9H, s), 1.82-2.02(2H, m), 2.76-2.99(2H, m), 3.51-3.78(2H, m), 4.08(2H, br), 5.26 (2H, s), 5.83(1H, d, J=15.7Hz), 6.19 (2H, dt, J=15.7Hz, 5.8Hz), 6.99-7.08 (2H, m), 7.33(1H, t, J=7.9Hz), 7.49 (1H, t, J=7.7Hz), 7.60(1H, d, J=7.7Hz), 7.74-7.84(3H, m).

EXAMPLE 31

Production of (E)-N-(6,6-dimethyl-2-hepten-4-ynyl)-N-methyl-3-benzyloxy-5-methylbenzylamine hydrochloride (compound 48)

3-Benzyloxy-5-methylbenzyl bromide (118 mg) and 76 mg of (E)-N-methyl-6,6-dimethyl-2-hepten-4-ynylamine hydrochloride were dissolved in 25 ml of dimethylformamide, and 67 mg of potassium carbonate was added. The mixture was stirred overnight at room temperature. The reaction mixture was evaporated under reduced pressure, and the residue was purified by preparative thin-layer chromatography [thin layer plate: Kieselgel $60F_{254}$, Art.5715 (a product of E. Merck Co.); developing solvent: hexane/ethyl acetate = 4/1]. The purified compound was then converted into a hydrochloride using HCl methanol solution. Treatment with a mixture of tetrahydrofuran and hexane gave 102 mg (yield 60.9 %) of the captioned compound as a colorless crystalline powder having a melting point of 180 to 186 °C.

$$IR\ \nu_{max}^{KBr}\ cm^{-1}:$$

3450, 2968, 2500, 1599, 1461, 1326, 1299, 1161, 1062, 969, 729.

NMR($CDCl_3$)δ: 1.25(9H, s), 2.35(3H, s), 2.58 (3H, s), 3.41-3.70(2H, m), 3.91-4.13 (2H, m), 5.14(2H, s), 5.80(1H, d, J = 15.9Hz), 6.25(1H, dt, J = 15.9Hz, 6.6Hz), 6.87-6.91(2H, m), 7.17-7.20 (1H, m), 7.29-7.47(5H, m).

Compounds of Examples 32 and 33 were obtained by repeating Example 31 except that instead of the starting 3-benzyloxy-5-methylbenzyl bromide, the corresponding benzyl bromide derivatives were used (when the product was a free base, the hydrochloride producing step was not included in the after-treatment).

EXAMPLE 32

(E)-N-(6,6-dimethyl-2-hepten-4-ynyl)-N-methyl-3-benzyloxy-4-fluorobenzylamine (compound 49)

$$IR\ \nu_{max}^{neat}\ cm^{-1}:$$

2974, 1518, 1461, 1431, 1269, 1116, 1023.

NMR($CDCl_3$)δ: 1.25(9H, s), 2.14(3H, s), 2.99(2H, dd, J = 6.2Hz, 1.5Hz), 3.40(2H, s), 5.14 (2H, s), 5.62-(1H, dt, J = 15.9Hz, 1.5Hz), 6.04(1H, dt, J = 15.9Hz, 6.2Hz), 6.77-6.83(1H, m), 6.97-7.05(2H, m), 7.28-7.48(5H, m).

EXAMPLE 33

(E)-N-(6,6-dimethyl-2-hepten-4-ynyl)-N-methyl-3-benzyloxy-4-hydroxybenzylamine hydrochloride (compound 50)

Melting point: ~98 °C (colorless crystalline powder containing 0.3 mole of hexane)

$$IR\ \nu_{max}^{KBr}\ cm^{-1}:$$

2968, 2626, 1527, 1461, 1446, 1368, 1284, 1263, 1164, 1131.

NMR($CDCl_3$)δ: 1.25(9H, s), 2.59(3H, s), 3.40-3.52 (1H, m), 3.61-3.73(1H, m), 3.87-3.97 (1H, m), 4.07-4.18(1H, m), 5.27(1H, s), 5.82(1H, d, J = 15.6Hz), 5.89(1H, s), 6.23(1H, dt, J = 15.6Hz, 7.6Hz), 6.81 (1H, dd, J = 7.9Hz, 1.8Hz), 6.91(1H, d, J = 7.9Hz), 7.46-7.52(2H, m), 7.74 (1H, br).

EXAMPLE 34

Production of (E)-N-(6,6-dimethyl-2-hepten-4-ynyl)-N-methyl-3-(2-methoxybenzyloxy)benzylamine hydrochloride (compound 51)

0.33 g of 3-(2-methoxybenzyloxy)benzyl methanesulfonate was dissolved in 4 ml of dimethylformamide, and 0.19 g of (E)-N-methyl-6,6-dimethyl-2-hepten-4-ynylamine hydrochloride and 0.17 g of potassium carbonate were added, and the mixture was stirred overnight at room temperature. The reaction mixture was concentrated under reduced pressure. The residue was dissolved in ethyl ether, and then the insoluble salts were removed by filtration. The filtrate was concentrated under reduced pressure, and the residue was purified by silica gel column chromatography [Wakogel C-200, 6 g; eluting solvent: hexane/ethyl acetate = 2/1] to give 0.40 g of the free base of the captioned compound as a pale yellow oil. The product was dissolved in 5 ml of methanol, and 0.9 ml of 30 % HCl/methanol solution was added, and the mixture was evaporated under reduced pressure. Recrystallization from a mixture of tetrahydrofuran and hexane gave 0.19 g (yield 47 %) of the captioned compound as colorless needles.
Melting point: 138-140 °C

$$IR\ \nu_{max}^{KBr}\ cm^{-1}:$$

3448, 2968, 2494, 1608, 1500, 1464, 1266, 1248, 1047, 1029, 756.

NMR($CDCl_3$)δ: 1.24(9H, s), 2.59(3H, s), 3.56 (2H, br), 3.86(3H, s), 4.09(2H, br), 5.15(2H, s), 5.80(1H, d, J = 15.0Hz), 6.26(1H, br), 6.89-6.98(2H, m), 7.05 (1H, d, J = 7.7Hz), 7.16-7.19(2H, m), 7.26-7.40(2H, m), 7.41(1H, dd, J = 6.7Hz, 1.2Hz).

Compounds of Examples 35 to 46 were obtained by repeating Example 34 except that instead of the starting 3-(2-methoxybenzyloxy)benzyl methanesulfonate, the corresponding benzyl methanesulfonates were used (when the product was a free base, the hydrochloride producing step was not included in the after-treatment).

EXAMPLE 35

(E)-N-(6,6-dimethyl-2-hepten-4-ynyl)-N-methyl-3-(3-methoxybenzyloxy)benzylamine hydrochloride (compound 52)

Melting point: 118-120 °C

$$IR\ \nu_{max}^{KBr}\ cm^{-1}:$$

3450, 2968, 2488, 1590, 1497, 1461, 1266, 1155, 1041, 783.

NMR($CDCl_3$)δ: 1.24(9H, s), 2.60(3H, s), 3.57 (2H, br), 3.82(3H, s), 4.08(2H, br), 5.13(2H, s), 5.82(1H, d, J = 15.4Hz), 6.25(1H, br), 6.85(1H, dd, J = 7.2Hz, 2.1Hz), 7.00-7.11(4H, m), 7.26-7.36 (2H, m), 7.39(1H, s).

EXAMPLE 36

(E)-N-(6,6-dimethyl-2-hepten-4-ynyl)-N-methyl-3-(3-methylbenzyloxy)benzylamine (compound 53)

$$IR\ \nu_{max}^{neat}\ cm^{-1}:$$

2968, 2788, 1596, 1491, 1458, 1365, 1266, 1026, 777.

NMR($CDCl_3$)δ: 1.24(9H, s), 2.18(3H, s), 2.37(3H, s), 3.03(2H, dd, J = 6.5Hz, 1.5Hz), 3.46 (2H, s), 5.02-(2H, s), 5.64(1H, dt, J = 15.7Hz, 1.5Hz), 6.08(1H, dt, J = 15.7Hz, 6.6Hz), 6.83-6.91(2H,

m), 6.97-6.98(1H, m), 7.11-7.14(1H, m), 7.18-7.30(4H, m).

EXAMPLE 37

(E)-N-(6,6-dimethyl-2-hepten-4-ynyl)-N-methyl-3-(4-methylbenzyloxy)benzylamine (compound 54)

$$IR\ \nu_{max}^{neat}\ cm^{-1}:$$

2968, 1602, 1587, 1491, 1458, 1263, 1152, 1032.

NMR($CDCl_3$)δ: 1.24(9H, s), 2.18(3H, s), 2.36(3H, s), 3.03(2H, dd, J=6.5Hz, 1.4Hz), 3.45 (2H, s), 5.01-(2H, s), 5.64(1H, dt, J=15.9Hz, 1.4Hz), 6.08(1H, dt, J=15.9Hz, 6.6Hz), 6.82-6.90(2H, m), 6.95-6.96(1H, m), 7.15-7.25(3H, m), 7.32(2H, d, J=8.0Hz).

EXAMPLE 38

(E)-N-(6,6-dimethyl-2-hepten-4-ynyl)-N-methyl-3-(2,3-dimethylbenzyloxy)benzylamine hydrochloride (compound 55)

Melting point: 182-184 °C

$$IR\ \nu_{max}^{KBr}\ cm^{-1}:$$

3448, 2968, 2482, 1587, 1497, 1461, 1263, 1167, 1032, 783.

NMR($CDCl_3$)δ: 1.24(9H, s), 2.28(3H, s), 2.31 (3H, s), 2.61(3H, s), 3.60(2H, br), 4.08(2H, br), 5.13(2H, s), 5.82(1H, d, J=15.5Hz), 6.26(1H, dt, J=15.5Hz, 7.6Hz), 7.03-7.16(4H, m), 7.25-7.28 (1H, m), 7.34(1H, t, J=7.4Hz), 7.40 (1H, d, J=1.7Hz).

EXAMPLE 39

(E)-N-(6,6-dimethyl-2-hepten-4-ynyl)-N-methyl-3-(2-chlorobenzyloxy)benzylamine (compound 56)

$$IR\ \nu_{max}^{neat}\ cm^{-1}:$$

2974, 1590, 1491, 1452, 1365, 1266, 1152, 1038, 753.

NMR($CDCl_3$)δ: 1.24(9H, s), 2.18(3H, s), 3,03(2H, dd, J=6.6Hz, 1.4Hz), 3.47(2H, s), 5.17 (2H, s), 5.64-(1H, dt, J=15.9Hz, 1.4Hz), 6.08(1H, dt, J=15.9Hz, 6.6Hz), 6.87 (1H, dd, J=7.0Hz, 2.8Hz), 6.92(1H, d, J=7.5Hz), 6.98(1H, d, J=1.8Hz), 7.22 (1H, d, J=7.7Hz), 7.24-7.31-(2H, m), 7.38-7.41(1H, m), 7.55-7.59(1H, m).

EXAMPLE 40

(E)-N-(6,6-dimethyl-2-hepten-4-ynyl)-N-methyl-3-(3-trifluoromethylbenzyloxy)benzylamine hydrochloride (compound 57)

Melting point: 145-146 °C

$$IR\ \nu_{max}^{KBr}\ cm^{-1}:$$

3460, 2974, 2626, 2506, 1458, 1335, 1266, 1197, 1164, 1125, 1074.

NMR($CDCl_3$)δ: 1.25(9H, s), 2.63(3H, s), 3.62 (2H, br), 4.10(2H, br), 5.22(2H, s), 5.85(1H, d, J=15.5Hz), 6.25(1H, dt, J=15.5Hz, 7.6Hz), 7.03-7.09(2H, m), 7.34(1H, t, J=7.7Hz), 7.50(1H, t, J=7.7Hz), 7.57-7.63(2H, m), 7.68(1H, d, J=7.6Hz), 7.74(1H, s).

EXAMPLE 41

(E)-N-(6,6-dimethyl-2-hepten-4-ynyl)-N-methyl-3-(3-phenylpropoxy)benzylamine hydrochloride (compound 58) Melting point: ~100 °C (amorphous powder)

$$IR\ \nu_{max}^{KBr}\ cm^{-1}:$$

3454, 2968, 2548, 2488, 1605, 1587, 1497, 1458, 1266, 699.

NMR($CDCl_3$)δ: 1.24(9H, s), 2.06-2.16(2H, m), 2.62 (3H, s), 2.82(2H, t, J=7.5Hz), 3.62 (2H, d, J=7.3Hz), 4.06(2H, t, J=6.0Hz), 4.07(2H, s), 5.84(1H, d, J=15.9Hz), 6.29(1H, dt, J=15.9Hz, 7.5Hz), 6.95 (1H, dd, J=3.8Hz, 2.5Hz), 7.07(1H, d, J=7.6Hz), 7.16-7.35-(7H, m).

EXAMPLE 42

(E)-N-(6,6-dimethyl-2-hepten-4-ynyl)-N-methyl-3-(2-methyl-2-propenyloxy)benzylamine (compound 59)

$$IR\ \nu_{max}^{neat}\ cm^{-1}:$$

2974, 2788, 1602, 1587, 1491, 1455, 1365, 1266, 1152, 1026.

NMR($CDCl_3$)δ: 1.24(9H, s), 1.84(3H, s), 2.19(3H, s), 3.03(2H, dd, J=6.6Hz, 1.5Hz), 3.45 (3H, s), 4.43-(2H, s), 4.98(1H, m), 5.10(1H, m), 5.65(1H, dt, J=15.8Hz, 1.5Hz), 6.09(1H, dt, J=15.8Hz, 6.6Hz), 6.78-6.82(1H, m), 6.86-6.91(2H, m), 7.20(1H, t, J=7.8Hz).

EXAMPLE 43

N-[(E)-6,6-dimethyl-2-hepten-4-ynyl]-N-methyl-3-[(E)-3,7-dimethyl-2,6-octadienyloxy]benzylamine (compound 60)

$$IR\ \nu_{max}^{neat}\ cm^{-1}:$$

2968, 2926, 2584, 2488, 1602, 1458, 1266, 1188, 963.

NMR($CDCl_3$)δ: 1.24(9H, s), 1.61(3H, s), 1.68(3H, s), 1.74(3H, s), 2.06-2.16(4H, m), 2.24 (3H, s), 3.10-(2H, d, J=6.5Hz), 3.52 (2H, s), 4.54(2H, d, J=6.6Hz), 5.07-5.13(1H, m), 5.47-5.51(1H, m), 5.67(1H, dt, J=15.9Hz, 1.6Hz), 6.11 (1H, dt, J=15.9Hz, 6.5Hz) 6.82(1H, dd, J=8.1Hz, 1.8Hz), 6.88-6.94(2H, m), 7.22(1H, t, J=8.1Hz).

EXAMPLE 44

(E)-N-(6,6-dimethyl-2-hepten-4-ynyl)-N-methyl-3-(3-methyl-2-butenyloxy)benzylamine (compound 61)

$$IR\ \nu_{max}^{neat}\ cm^{-1}:$$

2968, 2866, 2788, 1602, 1491, 1458, 1365, 1266, 1020.

NMR($CDCl_3$)δ: 1.24(9H, s), 1.73(3H, s), 1.80(3H, s), 2.19(3H, s), 3.03(2H, dd, J = 6.2Hz, 1.4Hz), 3.45-(2H, s), 4.51(2H, d, J = 6.8Hz), 5.50(1H, t.sept., J = 6.8Hz, 1.4Hz), 5.65(1H, dt, J = 15.9Hz, 1.4Hz), 6.08(1H, dt, J = 15.9Hz, 6.2Hz), 6.79 (1H, ddd, J = 8.2Hz, 2.9Hz, 1.4Hz), 6.86-6.90(2H, m), 7.20(1H, t, J = 7.8Hz).

EXAMPLE 45

(E)-N-(6,6-dimethyl-2-hepten-4-ynyl)-N-methyl-3-(2-ethylbenzyloxy)benzylamine hydrochloride (compound 62)

Melting point: 146-148 °C

$$IR\ \nu_{max}^{KBr}\ cm^{-1}:$$

3440, 2968, 2872, 2488, 1602, 1497, 1455, 1263, 1176, 1023, 969, 777, 759.

NMR($CDCl_3$)δ: 1.24(9H, s), 1.27(3H, t, J = 7.6Hz), 2.62(3H, s), 2.74(2H, q, J = 7.6Hz), 3.60 (2H, br), 4.07(2H, br), 5.15(2H, s), 5.82(1H, d, J = 15.6Hz), 6.27(1H, dt, J = 15.6Hz, 7.6Hz), 7.03-7.07(1H, m), 7.11-7.15(1H, m), 7.18-7.45(6H, m).

EXAMPLE 46

N-[(E)-6,6-dimethyl-2-hepten-4-ynyl]-N-methyl-3-[(E)-3-phenyl-2-propenyloxy]benzylamine hydrochloride (compound 63)

Melting point: 128-130 °C

$$IR\ \nu_{max}^{KBr}\ cm^{-1}:$$

3448, 2968, 2482, 1602, 1494, 1458, 1263, 969, 693.

NMR($CDCl_3$)δ: 1.25(9H, s), 2.61(3H, s), 3.59 (3H, br), 4.07(2H, br), 4.80(2H, dd, J = 6.9Hz, 1.2Hz), 5.82(1H, d, J = 15.6Hz), 6.25(1H, dt, J = 15.6Hz, 7.2Hz), 6.41 (1H, dt, J = 15.8Hz, 6.9Hz), 6.79(1H, dt, J = 15.8Hz, 1.2Hz), 7.02-7.08 (2H, m), 7.22-7.37(4H, m), 7.39-7.45 (3H, m).

EXAMPLE 47

Production of (E)-N-(6,6-dimethyl-2-hepten-4-ynyl)-N-methyl-3-(3-cyanobenzyloxy)benzylamine hydrochloride (compound 64)

100 mg of (E)-N-(6,6-dimethyl-2-hepten-4-ynyl)-N-methyl-3-hydroxybenzylamine was dissolved in 2 ml of anhydrous tetrahydrofuran, and with stirring under ice cooling, 19 mg of 60 % oily sodium hydride was added. The mixture was stirred for 10 minutes. A dimethylformamide solution (1 ml) of 88 microliters of 3-cyanobenzyl bromide was added to the resulting solution, and the mixture was stirred at room temperature for 2 hours. Water (10 ml) was then added to dilute the mixure. The mixture was extracted with 10 ml of

ethyl ether twice. The extracts were combined, washed with a saturated aqueous sodium chloride solution and dried over anhydrous sodium sulfate. The solvent was then evaporated. The residue was purified by silica gel column chromatography (Wakogel C-200, 5 g; eluting solvent: hexane/ethyl acetate = 4/1) to give 120 mg (yield 77 %) of the free base of the captioned compound as a colorless oil. The product was treated with HCl-methanol, and the solvent was evaporated. Recrystallization of the residue from ethyl ether/hexane gave the captioned compound as colorless needles.
Melting point: 131-133 °C

$$IR\ \nu_{max}^{KBr}\ cm^{-1}:$$

2972, 2624, 2232, 1458, 1268, 1166, 688.

NMR($CDCl_3$)δ: 1.25(9H, s), 2.61(3H, s), 3.48-3.80 (2H, m), 3.85-4.25(2H, m), 5.24(2H, s), 5.83(1H, d, J = 15.9Hz), 6.23(1H, dt, J = 15.9Hz, 7.1Hz), 7.00-7.06(2H, m), 7.33(1H, t, J = 7.8Hz), 7.50(1H, t, J = 7.8Hz), 7.59-7.64(1H, m), 7.75(1H d, J = 8.1Hz), 7.79(1H, s).

Compounds of Examples 48 to 78 were obtained by repeating Example 47 except that instead of the starting 3-cyanobenzyl bromide, the corresponding halogenobenzyl derivatives or methanesulfonyloxybenzyl derivatives were used (when the product was a free base, the hydrochloride producing step was not included in the after-treatment).

EXAMPLE 48

(E)-N-(6,6-dimethyl-2-hepten-4-ynyl)-N-methyl-3-(3-hydroxybenzyloxy)benzylamine (compound 65)

$$IR\ \nu_{max}^{neat}\ cm^{-1}:$$

2968, 1596, 1491, 1458, 1368, 1266, 1155, 780.

NMR($CDCl_3$)δ: 1.24(9H, s), 2.19(3H, s), 3.04(2H, d, J = 5.8Hz), 3.48(2H, s), 5.02(2H, s), 5.65(1H, dt, J = 15.9Hz, 2.1Hz), 6.08(1H, dt, J = 15.9Hz, 6.6Hz), 6.78(1H, dd, J = 7.8Hz, 2.2Hz), 6.85(1H, dd, J = 7.8Hz, 2.2Hz), 6.87-6.93(2H, m), 6.95-6.98 (2H, m), 7.18-7.24(2H, m).

EXAMPLE 49

(E)-N-(6,6-dimethyl-2-hepten-4-ynyl)-N-methyl-3-(2-fluorobenzyloxy)benzylamine hydrochloride (compound 66)

Melting point: 154-155 °C

$$IR\ \nu_{max}^{KBr}\ cm^{-1}:$$

2968, 2626, 2560, 2506, 1587, 1497, 1458, 1272, 1248, 762.

NMR($CDCl_3$)δ: 1.24(9H, s), 2.62(3H, s), 3.60(2H, d, J = 6.4Hz), 4.10(2H, s), 5.19(2H, s), 5.83(1H, d, J = 16.0Hz), 6.26(1H, dt, J = 16.0Hz, 6.4Hz), 7.02-7.20(4H, m), 7.27-7.38(3H, m), 7.51-(1H, dt, J = 7.4Hz, 1.7Hz).

EXAMPLE 50

(E)-N-(6,6-dimethyl-2-hepten-4-ynyl)-N-methyl-3-(2-cyanobenzyloxy)benzylamine hydrochloride (compound 67)

Melting point: 161-163 °C

$$IR\ \nu_{max}^{KBr}\ cm^{-1}:$$

2974, 2620, 2230, 1590, 1497, 1458, 1263, 1167, 771.

NMR($CDCl_3$)δ: 1.25(9H, s), 2.63(3H, s), 3.62(2H, d, J=7.6Hz), 4.10(2H, s), 5.31(2H, s), 5.85(1H, d, J=15.8Hz), 6.30(1H, dt, J=15.8Hz, 7.6Hz), 7.04-7.08(1H, m), 7.36(1H, t, J=7.9Hz), 7.45(1H, dt, J=7.6Hz, 1.2Hz), 7.50(1H, t, J=1.9Hz), 7.63(1H, dt, J=7.6Hz, 1.2Hz), 7.69-7.73 (2H, m).

EXAMPLE 51

(E)-N-(6,6-dimethyl-2-hepten-4-ynyl)-N-methyl-3-(2-trifluoromethylbenzyloxy)benzylamine hydrochloride (compound 68)

Melting point: 204-206 °C

$$IR\ \nu_{max}^{KBr}\ cm^{-1}:$$

2974, 1458, 1317, 1254, 1167, 1119, 777.

NMR($CDCl_3$)δ: 1.25(9H, s), 2.60(3H, s), 3.58(2H, d, J=7.2Hz), 4.07(2H, s), 5.31(2H, s), 5.82(1H, d, J=15.7Hz), 6.25(1H, dt, J=15.7Hz, 7.2Hz), 7.00-7.04(1H, m), 7.21(1H, d, J=7.5Hz), 7.26-7.27 (1H, m), 7.36(1H, t, J=7.9Hz), 7.44 (1H, t, J=7.7Hz), 7.58(1H, t, J=7.6Hz) 7.69-7.75(2H, m).

EXAMPLE 52

(E)-N-(6,6-dimethyl-2-hepten-4-ynyl)-N-methyl-3-(3-isopropenylbenzyloxy)benzylamine (compound 69)

$$IR\ \nu_{max}^{neat}\ cm^{-1}:$$

2974, 1602, 1497, 1458, 1320, 1266, 1179, 966.

NMR($CDCl_3$)δ: 1.25(9H, s), 2.18(3H, s), 2.19(3H, s), 3.03(2H, dd, J=6.5Hz, 1.5Hz), 3.46 (2H, s), 5.06-(2H, s), 5.10(1H, dt, J=3.0Hz, 1.5Hz), 5.39(1H, dt, J=1.5Hz, 0.9Hz), 5.64(1H, dt, J=15.9Hz, 1.5Hz), 6.08(1H, dt, J=15.8Hz, 6.5Hz), 6.85-6.92(2H, m), 6.99(1H, t, J=1.7Hz), 7.22(1H, t, J=8.1Hz), 7.33-7.45(3H, m), 7.53-7.54(1H, m).

EXAMPLE 53

(E)-N-(6,6-dimethyl-2-hepten-4-ynyl)-N-methyl-3-[3-(2-methyl-1-propenyl)benzyloxy]benzylamine (compound 70)

$$IR\ \nu_{max}^{neat}\ cm^{-1}:$$

2968, 1602, 1491, 1458, 1365, 1266, 1152, 1023, 780, 696.

NMR($CDCl_3$)δ: 1.24(9H, s), 1.85(3H, d, J = 1.4Hz), 1.90(3H, d, J = 1.4Hz), 2.18(3H, s), 3.03(2H, dd, J = 6.6Hz, 1.4Hz), 3.56 (2H, s), 5.05(2H, s), 5.64(1H, dt, J = 15.7Hz, 1.4Hz), 6.08(1H, dt, J = 15.7Hz, 6.6Hz), 6.26-6.29(1H, m), 6.83-6.91(2H, m), 6.97(1H, dd, J = 2.2Hz, 2.0Hz), 7.16-7.35(5H, m).

EXAMPLE 54

(E)-N-(6,6-dimethyl-2-hepten-4-ynyl)-N-methyl-3-[3-(2-methyl-2-propenyl)benzyloxy]benzylamine (compound 71)

$$IR\nu_{max}^{neat}\ cm^{-1}:$$

2974, 1599, 1491, 1458, 1368, 1266, 1155, 1026, 888, 780, 696.

NMR($CDCl_3$)δ: 1.24(9H, s), 1.67(3H, s), 2.18 (3H, s), 3.03(2H, dd, J = 6.6Hz, 1.5Hz), 3.34(2H, s), 3.46-(2H, s), 4.74(1H, m), 4.81(1H, m), 5.04(2H, s), 5.64(1H, dt, J = 15.9Hz, 1.5Hz), 6.08(1H, dt, J = 15.9Hz, 6.6Hz), 6.84-6.91(1H, m), 6.96-6.98(1H, m), 7.13-7.16(1H, m), 7.21(1H, t, J = 7.7Hz), 7.25-7.33 (3H, m).

EXAMPLE 55

(E)-N-(6,6-dimethyl-2-hepten-4-ynyl)-N-methyl-3-[3-(3-methyl-2-butenyl)benzyloxy]benzylamine (compound 72)

$$IR\nu_{max}^{neat}\ cm^{-1}:$$

2974, 2920, 1602, 1491, 1455, 1365, 1266, 1155, 1026, 783.

NMR($CDCl_3$)δ: 1.24(9H, s), 1.61(3H, s), 1.72(3H, d, J = 1.5Hz), 1.75(3H, d, J = 1.5Hz), 2.18(3H, s), 3.03(2H, dd, J = 6.6Hz, 1.5Hz), 3.36(2H, d, J = 7.3Hz), 3.46 (2H, s), 5.03(2H, s), 5.33-(1H, t.sept., J = 7.3Hz, 1.5Hz), 5.64(1H, dt, J = 15.9Hz, 1.5Hz), 6.08(1H, dt, J = 15.9Hz, 6.6Hz), 6.84-6.91(2H, m), 6.96-6.98(1H, m), 7.11-7.33(5H, m).

EXAMPLE 56

(E)-N-(6,6-dimethyl-2-hepten-4-ynyl)-N-methyl-3-[3-(3-methyl-2-butenyloxy)benzyloxy]benzylamine (compound 73)

$$IR\nu_{max}^{neat}\ cm^{-1}:$$

2974, 1602, 1494, 1452, 1266, 1152, 1023.

NMR($CDCl_3$)δ: 1.24(9H, s), 1.74(3H, s), 1.79(3H, s), 2.18(3H, s), 3.03(2H, dd, J = 6.6Hz, 1.5Hz), 3.46-(2H, s), 4.51(1H, d, J = 6.5Hz), 5.03(2H, s), 5.47-5.52 (1H, m), 5.64(1H, dt, J = 15.6Hz, 1.4Hz), 6.08(1H, dt, J = 15.6Hz, 6.5Hz), 6.83-6.91(2H, m), 6.96-7.01(3H, m), 7.21(1H, t, J = 8.0Hz), 7.23(1H, dd, J = 8.3Hz, 8.2Hz),

EXAMPLE 57

(E)-N-(6,6-dimethyl-2-hepten-4-ynyl)-N-methyl-3-(3-phenylbenzyloxy)benzylamine hydrochloride (compound 74)

Melting point: 147-148 °C

$$IR\ \nu_{max}^{KBr}\ cm^{-1}:$$

2974, 2486, 1458, 1263, 1164, 1035, 756, 699.

NMR($CDCl_3$)δ: 1.25(9H, s), 2.59(3H, s), 3.47-3.53 (2H, m), 3.99-4.12(2H, m), 5.23(2H, s), 5.81(1H, d, J=15.8Hz), 6.24(1H, dt, J=15.8Hz, 7.2Hz), 7.05-7.10(2H, m), 7.32-7.69(11H, m).

EXAMPLE 58

(E)-N-(6,6-dimethyl-2-hepten-4-ynyl)-N-methyl-3-[3-(2-furyl)benzyloxy]benzylamine hydrochloride (compound 75)

Melting point: 115-116 °C

$$IR\ \nu_{max}^{KBr}\ cm^{-1}:$$

2968, 2488, 1605, 1497, 1461, 1266, 789.

NMR($CDCl_3$)δ: 1.25(9H, s), 2.60(3H, s), 3.50-3.64 (2H, m), 4.00-4.13(2H, m), 5.20(2H, s), 5.78-5.84(1H, m), 6.18-6.32(1H, m), 6.47(1H, dd, J=3.5Hz, 2.0Hz), 6.70 (1H, d, J=3.5Hz), 7.05-7.11-(2H, m), 7.30-7.47(5H, m), 7.62(1H, d, J=7.1Hz), 7.78(1H, s).

EXAMPLE 59

(E)-N-(6,6-dimethyl-2-hepten-4-ynyl)-N-methyl-3-[3-(2-oxazolyl)benzyloxy]benzylamine (compound 76)

$$IR\ \nu_{max}^{neat}\ cm^{-1}:$$

2974, 1590, 1491, 1458, 1365, 1266, 1152, 1026, 798, 729.

NMR($CDCl_3$)δ: 1.24(9H, s), 2.18(3H, s), 3.30(2H, dd, J=6.6Hz, 1.5Hz), 3.46(2H, s), 5.12 (2H, s), 5.63-(1H, dd, J=15.8Hz, 1.5Hz), 6.08(1H, dt, J=15.8Hz, 6.6Hz), 6.85-6.92(2H, m), 6.99-7.15(1H, m), 7.22(1H, t, J=7.7Hz), 7.24(1H, d, J=1.1Hz), 7.48(1H, t, J=7.7Hz), 7.54 (1H, d, J=7.9Hz), 7.72(1H, d, J=1.1Hz), 8.01(1H, dt, J=7.9Hz, 1.2Hz), 8.13-8.16 (1H, m).

EXAMPLE 60

(E)-N-(6,6-dimethyl-2-hepten-4-ynyl)-N-methyl-3-[3-(2-thiazolyl)benzyloxy]benzylamine (compound 77)

$$IR\ \nu_{max}^{neat}\ cm^{-1}:$$

2972, 1588, 1490, 1456, 1364, 1266, 1148, 1022, 788, 692.

NMR ($CDCl_3$)δ: 1.19(9H, s), 2.14(3H, s), 2.99(2H, dd, J=6.6Hz, 1.4Hz), 3.42(2H, s), 5.08 (2H, s), 5.60-

(1H, dt, J=15.8Hz, 1.2Hz), 6.04(1H, dt, J=15.8Hz, 6.6Hz), 6.83 (1H, dd, J=8.1Hz, 1.5Hz), 6.87(1H, d, J=8.1Hz), 6.95(1H, d, J=1.5Hz), 7.18 (1H, t, J=7.8Hz), 7.30(1H, d, J=3.3Hz) 7.42(1H, t, J=8.1Hz), 7.47(1H, d, J=7.6Hz), 7.83(1H, d, J=3.3Hz), 7.87 (1H, d, J=7.3Hz), 8.01(1H, s).

EXAMPLE 61

(E)-N-(6,6-dimethyl-2-hepten-4-ynyl)-N-methyl-3-[3-(5-oxazolyl)benzyloxy]benzylamine (compound 78)

$$IR\ \nu_{max}^{neat}\ cm^{-1}:$$

2968, 1596, 1458, 1263, 1152, 1026, 954, 789, 693.

NMR($CDCl_3$)δ: 1.24(9H, s), 2.19(3H, s), 3.04(2H, dd, J=6.5Hz, 1.4Hz), 3.47(2H, s), 5.11 (2H, s), 5.65-(1H, dt, J=15.9Hz, 1.4Hz), 6.08(1H, dt, J=15.9Hz, 6.5Hz), 6.86-6.93(2H, m), 7.00-7.02(1H, m), 7.23(1H, t, J=7.9Hz), 7.38(1H, s), 7.42-7.43(1H, m), 7.45(1H, t, J=7.6Hz), 7.60-7.64(1H, m), 7.74-7.76(1H, m), 7.93(1H, s).

EXAMPLE 62

(E)-N-(6,6-dimethyl-2-hepten-4-ynyl)-N-methyl-3-[3-(5-thiazolyl)benzyloxy]benzylamine (compound 79)

$$IR\ \nu_{max}^{neat}\ cm^{-1}:$$

2974, 1590, 1458, 1266, 873, 789, 693.

NMR($CDCl_3$)δ: 1.24(9H, s), 2.19(3H, s), 3.04(2H, dd, J=6.6Hz, 1.5Hz), 3.47(2H, s), 5.10 (2H, s), 5.64-(1H, dt, J=15.8Hz, 1.5Hz), 6.08(1H, dt, J=15.8Hz, 6.6Hz), 6.88 (1H, dd, J=8.1Hz, 1.8Hz), 6.92(1H, d, J=7.8Hz), 7.00(1H, t, J=1.8Hz), 7.23 (1H, t, J=7.8Hz), 7.42-7.44-(2H, m), 7.53-7.56(1H, m), 7.65-7.66(1H, m), 8.10(1H, d, J=0.6Hz), 8.76(1H, d, J=0.6Hz).

EXAMPLE 63

(E)-N-(6,6-dimethyl-2-hepten-4-ynyl)-N-methyl-3-[3-(3-pyridyl)benzyloxy]benzylamine (compound 80)

$$IR\ \nu_{max}^{neat}\ cm^{-1}:$$

2968, 1599, 1491, 1458, 1365, 1263, 1152, 1023, 783.

NMR($CDCl_3$)δ: 1.24(9H, s), 2.18(3H, s), 3.30(2H, dd, J=6.6Hz, 1.5Hz), 3.47(2H, s), 5.62 (2H, s), 5.64-(1H, dt, J=15.9Hz, 1.5Hz), 6.07(1H, dt, J=15.9Hz, 6.6Hz), 6.87-6.93(2H, m), 7.01(1H, br), 7.23 (1H, t, J=7.9Hz), 7.37(1H, ddd, J=6.8Hz, 5.0Hz, 0.9Hz), 7.48-7.57(3H, m), 7.67 (1H, br), 7.87-7.91(1H, m), 8.60(1H, dd, J=4.8Hz, 1.7Hz), 8.86(1H, dd, J=2.4Hz, 0.9Hz).

EXAMPLE 64

(E)-N-(6,6-dimethyl-2-hepten-4-ynyl)-N-methyl-3-[3-(1-pyrrolyl)benzyloxy]benzylamine (compound 81)

$$IR\ \nu_{max}^{neat}\ cm^{-1}:$$

1596, 1506, 1488, 1458, 1341, 1266, 1029, 786, 726.

NMR($CDCl_3$)δ: 1.24(9H, s), 2.18(3H, s), 3.03(2H, dd, J=6.6Hz, 1.5Hz), 3.47(2H, s), 5.11 (2H, s), 5.64-(1H, dt, J=15.8Hz, 1.5Hz), 6.07(1H, dt, J=15.8Hz, 6.6Hz), 6.35(2H, t, J=2.2Hz), 6.86-(1H, ddd, J=10.8Hz, 2.3Hz, 0.8Hz), 6.91(1H, d, J=7.8Hz), 6.98-6.99(1H, m), 7.22(1H, t, J=7.8Hz), 7.10(2H, t, J=2.2Hz), 7.25-7.50(4H, m).

EXAMPLE 65

(E)-N-(6,6-dimethyl-2-hepten-4-ynyl)-N-methyl-[(3-methyl-2-furyl)methyloxy]benzylamine (compound 82)

$$IR\ \nu_{max}^{neat}\ cm^{-1}:$$

2974, 1602, 1491, 1458, 1368, 1263, 1158, 1017.

NMR($CDCl_3$)δ: 1.24(9H, s), 2.09(3H, s), 2.19(3H, s), 3.04(2H, d, J=6.6Hz), 3.47(2H, s), 4.95 (2H, s), 5.65(1H, dt, J=15.9Hz, 1.5Hz), 6.09(1H, dt, J=15.9Hz, 6.6Hz), 6.24 (1H, d, J=1.9Hz), 6.85-6.98(3H, m), 7.22(1H, t, J=7.8Hz), 7.35(1H, d, J=1.9Hz).

EXAMPLE 66

(E)-N-(6,6-dimethyl-2-hepten-4-ynyl)-N-methyl-3-[(2-methyl-3-furyl)methyloxy]benzylamine (compound 83)

$$IR\ \nu_{max}^{neat}\ cm^{-1}:$$

2968, 2926, 1599, 1458, 1365, 1265, 1143, 1028.

NMR($CDCl_3$)δ: 1.26(9H, s), 2.19(3H, s), 2.32(3H, s), 3.05(2H, dd, J=6.6Hz, 1.5Hz), 3.46 (2H, s), 4.84-(2H, s), 5.65(1H, dt, J=15.9Hz, 1.5Hz), 6.08(1H, dt, J=15.9Hz, 6.6Hz), 6.48(1H, d, J=2.0Hz), 6.84(1H, dd, J=6.8Hz, 2.7Hz), 6.90(1H, d, J=6.8Hz), 6.95-6.96(1H, m), 7.22(1H, t, J=6.8Hz), 7.86(1H, d, J=2.0Hz).

EXAMPLE 67

(E)-N-(6,6-dimethyl-2-hepten-4-ynyl)-N-methyl-3-(5-isoxazolylmethyloxy)benzylamine (compound 84)

Melting point: 55-56 °C

$$IR\ \nu_{max}^{KBr}\ cm^{-1}:$$

2972, 1600, 1382, 1258, 1164, 1026, 776.

NMR($CDCl_3$)δ: 1.24(9H, s), 2.18(3H, s), 3.04(2H, dd, J=6.5Hz, 1.5Hz), 3.46(2H, s), 5.19 (2H, s), 5.65-(1H, dt, J=15.9Hz, 1.5Hz), 6.08(1H, dt, J=15.9Hz, 6.5Hz), 6.33-6.36 (1H, m), 6.81-6.87(1H, m), 6.92-6.99 (2H, m), 7.24(1H, t, J=8.0Hz), 8.24 (1H, d, J=1.8Hz).

EXAMPLE 68

(E)-N-(6,6-dimethyl-2-hepten-4-ynyl)-N-methyl-3-(4-pyridylmethyloxy)benzylamine hydrochloride (compound 85)

$$IR\ \nu_{max}^{KBr}\ cm^{-1}:$$

3442, 2974, 2626, 1644, 1611, 1266, 792.

NMR($CDCl_3$)δ: 1.25(9H, s), 2.65(3H, s), 3.45-3.77 (2H, m), 3.96-4.31(2H, m), 5.63(2H, s), 5.87(1H, d, J = 14.8Hz), 6.10-6.26(1H, m), 6.97(1H, d, J = 7.5Hz), 7.15(1H, d, J = 7.8Hz), 7.37(1H, t, J = 7.5Hz), 8.00-8.20(3H, m), 8.60-8.80(2H, m).

EXAMPLE 69

(E)-N-(6,6-dimethyl-2-hepten-4-ynyl)-N-methyl-3-(2-thienylmethyloxy)benzylamine (compound 86)

$$IR\ \nu_{max}^{neat}\ cm^{-1}:$$

2974, 1587, 1491, 1458, 1365, 1263, 1023, 699.

NMR($CDCl_3$)δ: 1.24(9H, s), 2.18(3H, s), 3.03(2H, dd, J = 6.6Hz, 1.5Hz), 3.46(2H, s), 5.22(2H, s), 5.64-(1H, dt, J = 15.8Hz, 1.5Hz), 6.08(1H, dt, J = 15.8Hz, 6.5Hz), 6.87(1H, ddd, J = 8.2Hz, 2.6Hz, 0.8Hz), 6.91(1H, d, J = 7.6Hz), 6.97-7.02(2H, m), 7.10-7.12 (1H, m), 7.22(1H, t, J = 7.8Hz), 7.32 (1H, dd, J = 5.0Hz, 1.2Hz).

EXAMPLE 70

(E)-N-(6,6-dimethyl-2-hepten-4-ynyl)-N-methyl-3-(8-quinolylmethyloxy)benzylamine hydrochloride (compound 87)

Melting point: 99-101 °C.

$$IR\ \nu_{max}^{KBr}\ cm^{-1}:$$

2968, 1599, 1506, 1491, 1365, 1269, 1152, 1020, 822, 789.

NMR($CDCl_3$)δ: 1.24(9H, s), 2.17(3H, s), 3.03(1H, dd, J = 6.7Hz, 1.5Hz), 3.46(2H, s), 5.64(1H, dd, J = 16.0Hz, 1.5Hz), 5.84 (2H, s), 6.08(1H, dd, J = 16.0Hz, 6.7Hz), 6.91(1H, d, J = 7.7Hz), 6.98(1H, dd, J = 6.8Hz, 1.6Hz), 7.23(1H, dd, J = 7.7Hz, 6.8Hz), 7.45(1H, dd, J = 8.3Hz, 4.2Hz), 7.57(1H, dd, J = 8.5Hz, 7.0Hz), 7.83(1H, d, J = 8.5Hz), 7.95(1H, dd, J = 7.0Hz, 0.9Hz), 8.19(1H, dd, J = 8.3Hz, 1.6Hz), 8.96(1H, dd, J = 4.2Hz, 1.6Hz).

EXAMPLE 71

(E)-N-(6,6-dimethyl-2-hepten-4-ynyl)-N-methyl-3-(2-benzofuranylmethyloxy)benzylamine (compound 88)

Melting point: 66-67 °C

$$IR\ \nu_{max}^{KBr}\ cm^{-1}:$$

2968, 1602, 1458, 1260, 1014, 966, 792, 753.

NMR($CDCl_3$)δ: 1.24(9H, s), 2.18(3H, s), 3.03(2H, dd, J = 6.6Hz, 1.5Hz), 3.47(2H, s), 5.17(2H, s), 5.65-(1H, dt, J = 15.9Hz, 1.5Hz), 6.08(1H, dt, J = 15.9Hz, 6.6Hz), 6.79(1H, d, J = 0.6Hz), 6.88-6.94(2H, m), 7.01-7.03 (1H, m).

EXAMPLE 72

N-[(E)-6,6-dimethyl-2-hepten-4-ynyl]-N-methyl-3-[(Z) 2-methyl-2-butenyloxy]benzylamine hydrochloride (compound 89)

Melting point: 141-142 °C

$$IR\ \nu_{max}^{KBr}\ cm^{-1}:$$

2974, 2626, 2494, 1458, 1266, 1164, 1026, 975.

NMR($CDCl_3$)δ: 1.25(9H, s), 1.72(3H, dd, J = 6.8Hz, 1.0Hz), 1.80-1.84(3H, m), 2.62(3H, s), 3.43-3.58-(1H, m), 3.62-3.77(1H, m), 3.94-4.06(1H, m), 4.10-4.23(1H, m), 4.59(2H, s), 5.49-5.59-(1H, m), 5.84 (1H, d, J = 15.6Hz), 6.27(1H, dt, J = 15.6Hz, 7.6Hz), 7.00(1H, dd, J = 8.3Hz, 2.1Hz), 7.12(1H, d, J = 6.8Hz), 7.29(1H, br), 7.34(1H, dd, J = 8.3Hz, 6.8Hz).

EXAMPLE 73

N-[(E)-6,6-dimethyl-2-hepten-4-ynyl]-N-methyl-3-[(E)-2-methyl-2-butenyloxy]benzylamine hydrochloride (compound 90)

Melting point: 119-121 °C

$$IR\ \nu_{max}^{KBr}\ cm^{-1}:$$

2974, 2920, 2626, 2560, 2506, 1458, 1266, 1251, 1164.

NMR($CDCl_3$)δ: 1.25(9H, s), 1.66(3H, dd, J = 6.5Hz, 1.0Hz), 1.73-1.76(3H, m), 2.62(3H, s), 3.44-3.78-(2H, m), 3.93-4.24(2H, m), 4.46(2H, s), 5.62-5.72(1H, m), 5.83 (1H, d, J = 15.6Hz), 6.26-(1H, dt, J = 15.6Hz, 7.3Hz), 6.98(1H, dd, J = 8.2Hz, 2.0Hz), 7.08(1H, d, J = 7.6Hz), 7.27 (1H, m), 7.32(1H, dd, J = 8.2Hz, 7.6Hz).

EXAMPLE 74

(E)-N-(6,6-dimethyl-2-hepten-4-ynyl]-N-methyl-3-(2-ethyl-2-propenyloxy)benzylamine (compound 91)

$$IR\ \nu_{max}^{neat}\ cm^{-1}:$$

2968, 1458, 1365, 1266, 1152, 1024.

NMR($CDCl_3$)δ: 1.11(3H, t, J = 7.4Hz), 1.24(9H, s), 2.13-2.22(5H, m), 3.04(2H, dd, J = 6.6Hz, 1.4Hz), 3.45(2H, s), 4.47(2H, s), 4.98 (1H, s), 5.12(1H, s), 5.65(1H, dt, J = 15.9Hz, 1.4Hz), 6.09-(1H, dt, J = 15.9Hz, 6.6Hz), 6.78-6.83(1H, m), 6.86-6.91 (2H, m), 7.20(1H, t, J = 7.8Hz).

EXAMPLE 75

(E)-N-(6,6-dimethyl-2-hepten-4-ynyl)-N-methyl-3-(2,3-dimethyl-2-butenyloxy)benzylamine (compound 92)

$$IR\ \nu_{max}^{neat}\ cm^{-1}:$$

2974, 2926, 1458, 1266, 1149, 1017.

NMR($CDCl_3$)δ: 1.24(9H, s), 1.74(3H, s), 1.79(6H, s), 2.19(3H, s), 3.04(2H, dd, J = 6.6Hz, 1.7Hz), 3.46-(2H, s), 4.49(2H, s), 5.65 (1H, dt, J = 15.9Hz, 1.7Hz), 6.09(1H, dt, J = 15.9Hz, 6.7Hz), 6.78-6.84(1H, m), 6.85-6.93(2H, m), 7.20(1H, t, J = 7.8Hz).

EXAMPLE 76

(E)-N-(6,6-dimethyl-2-hepten-4-ynyl)-N-methyl-3-(4-phenylbutoxy)benzylamine (compound 93)

$$IR\ \nu_{max}^{neat}\ cm^{-1}:$$

2968, 1602, 1458, 1266, 1152, 696.

NMR($CDCl_3$)δ: 1.24(9H, s), 1.75-1.90(4H, m), 2.18 (3H, s), 2.62-2.76(2H, m), 3.03(2H, dd, J = 6.6Hz, 1.6Hz), 3.45(2H, s), 3.92-4.04 (2H, m), 5.64(1H, dt, J = 15.9Hz, 1.5Hz), 6.09(1H, dt, J = 15.9Hz, 6.6Hz), 6.74-6.79(1H, m), 6.84-6.89(2H, m), 7.15-7.24(4H, m), 7.25-7.32(2H, m).

EXAMPLE 77

(E)-N-(6,6-dimethyl-2-hepten-4-ynyl)-N-methyl-3-(5-phenylpentyloxy)benzylamine (compound 94)

$$IR\ \nu_{max}^{neat}\ cm^{-1}:$$

2938, 2866, 2788, 1605, 1458, 1266, 696.

NMR($CDCl_3$)δ: 1.24(9H, s), 1.44-1.58(2H, m), 1.64-1.76(2H, m), 1.76-1.87(2H, m), 2.18(3H, s), 2.65-(2H, t, J = 7.7Hz), 3.04(2H, dd, J = 6.6Hz, 1.5Hz), 3.45 (2H, s), 3.95(2H, t, J = 6.5Hz), 5.64 (1H, dt, J = 15.9Hz, 1.5Hz), 6.09 (1H, dt, J = 15.9Hz, 6.6Hz), 6.74-6.79 (1H, m), 6.84-6.89(2H, m), 7.14-7.23 (4H, m), 7.24-7.31(2H, m).

EXAMPLE 78

(E)-N-(6,6-dimethyl-2-hepten-4-ynyl)-N-methyl-3-(3-formylbenzyloxy)benzylamine (compound 95)

$$IR\ \nu_{max}^{neat}\ cm^{-1}:$$

2974, 1707, 1590, 1455, 1266, 1155, 783, 756.

NMR($CDCl_3$)δ: 2.19(3H, s), 3.04(2H, dd, J = 6.6Hz, 1.2Hz), 3.47(2H, s), 5.15(2H, s), 5.64(1H, dt, J = 15.9Hz, 1.5Hz), 6.08 (1H, dt, J = 15.9Hz, 6.5Hz), 6.86(1H, dd, J = 8.2Hz, 2.5Hz), 6.92(1H, d, J = 7.6Hz), 6.99-7.00(1H, m), 7.22(1H, t, J = 7.9Hz), 7.57(1H, t, J = 7.6Hz), 7.72(1H, dt, J = 8.0Hz, 0.8Hz), 7.85 (1H, dt, J = 7.6Hz, 1.3Hz), 7.96-7.99 (1H, m), 10.05-(1H, s).

EXAMPLE 79

Production of (E)-N-(6,6-dimethyl-2-hepten-4-ynyl)-N-methyl-3-(3-hydroxymethylbenzyloxy)benzylamine (compound 96)

85 mg of the (E)-N-(6,6-dimethyl-2-hept-4-ynyl)-N-methyl-3-(3-formylbenzyloxy)benzylamine obtained as above was dissolved in 2 ml of ethanol, and under ice cooling, 8.6 mg of sodium borohydride was added with stirring for 30 minutes. The solvent was evaporated under reduced pressure. The residue was dissolved in a mixture of ethyl acetate and water. The organic layer was separated, and dried over anhydrous magnesium sulfate. The desiccant was removed by filtration, and the solvent was evaporated. The residue was purified by silica gel column chromatography (Wakogel C-200, 15 g; eluting solvent: hexane/ethyl acetate = 10/1 → 5/1) to give 40 mg (yield 39 %) of the captioned compound as a colorless oil.

$$IR\ \nu_{max}^{neat}\ cm^{-1}:$$

2972, 1588, 1492, 1458, 1366, 1266, 1158, 1028, 784.

NMR($CDCl_3$)δ: 1.24(9H, s), 2.18(3H, s), 3.03(2H, dd, J = 6.6Hz, 1.5Hz), 3.46(2H, s), 4.73(2H, s), 5.07-(2H, s), 5.64(1H, dt, J = 15.8Hz, 1.5Hz), 6.07(1H, dt, J = 15.8Hz, 6.5Hz), 6.84-6.91(2H, m), 6.97-6.99(1H, m), 7.22(1H, t, J = 7.8Hz), 7.31-7.39(3H, m), 7.46-7.47(1H, m).

EXAMPLE 80

Production of N-[(E)-6,6-dimethyl-2-hepten-4-ynyl]-N-methyl-3-[(E)-styryl]benzylamine hydrochloride (compound 97)

133 mg of (E)-3-bromomethylstilbene was dissolved in 5 ml of dimethylformamide, and 86 mg of (E)-N-methyl-6,6-dimethyl-2-hepten-4-ynylamine hydrochloride and 200 mg of potassium carbonate were added. The mixture was stirred overnight at room temperature. The reaction mixture was diluted with water, and extracted with isopropyl ether. The extract was washed with water, and dried over anhydrous sodium sulfate. The desiccant was removed by filtration, and then the solvent was evaporated. The residue was purified by preparative thin-layer chromatography [thin-layer plate: Kieselgel $60F_{254}$, Art. 5744 (a product of E. Merck Co.); developing solvent: hexane/ethyl acetate = 5/1] to give 150 mg (yield 89.7 %) of the captioned compound as a colorless oil.

$$IR\ \nu_{max}^{KBr}\ cm^{-1}:$$

3020, 2866, 2788, 1500, 1455, 1266, 1134, 963, 768, 696.

NMR($CDCl_3$)δ: 1.24(9H, s), 2.21(3H, s), 3.07(2H, dd, J = 6.6Hz, 1.5Hz), 3.51(2H, s), 5.68 (1H, dt, J = 15.9Hz, 1.5Hz), 6.12(1H, dt, J = 15.9Hz, 6.6Hz), 7.11(2H, s), 7.19-7.53(9H, m).

Compounds of Examples 81 to 84 were obtained by repeating Example 80 except that instead of the starting (E)-3-bromomethylstilbene, the corresponding brominated derivatives were used.

EXAMPLE 81

N-[(E)-6,6-dimethyl-2-hepten-4-ynyl]-N-methyl-3-[(Z)-styryl]benzylamine (compound 98)

$$IR\ \nu_{max}^{neat}\ cm^{-1}:$$

3022, 2866, 2788, 1497, 1455, 1308, 1200, 1026, 837, 774.

NMR($CDCl_3$)δ: 1.24(9H, s), 2.11(3H, s), 2.97(1H, dd, J = 6.6Hz, 1.5Hz), 3.37(2H, s), 5.59(1H, dt, J = 15.9Hz, 1.5Hz), 6.02(1H, dt, J = 15.9Hz, 6.6Hz), 6.59(2H, s), 7.11-7.21(9H, m).

EXAMPLE 82

N-[(E)-6,6-dimethyl-2-hepten-4-ynyl]-N-methyl-3-[(E)-o-methylstyryl]benzylamine (compound 99)

$$IR\ \nu_{max}^{neat}\ cm^{-1}:$$

3028, 2968, 2866, 2782, 1605, 1491, 1461, 1365, 1266, 1134, 1026, 963, 783, 714, 693.

NMR($CDCl_3$)δ: 1.25(9H, s), 2.23(3H, s), 2.45(3H, s), 3.08(2H, dd, J=6.7Hz, 1.5Hz), 3.52 (2H, s), 5.68-(1H, dt, J=15.9Hz, 1.5Hz), 6.12(1H, dt, J=15.9Hz, 6.7Hz), 6.99 (1H, d, J=16.1Hz), 7.17-7.61(9H, m).

EXAMPLE 83

N-[(E)-6,6-dimethyl-2-hepten-4-ynyl]-N-methyl-3-[(E)-2-(1-naphthylvinyl)]benzylamine (compound 100)

$$IR\ \nu_{max}^{neat}\ cm^{-1}:$$

3034, 2866, 2788, 1479, 1398, 1266, 1131, 963, 768.

NMR($CDCl_3$)δ: 1.25(9H, s), 2.24(3H, s), 3.10(1H, dd, J=6.6Hz, 1.5Hz), 3.54(2H, s), 5.68 (1H, dt, J=15.9Hz, 1.5Hz), 6.14(1H, dt, J=15.9Hz, 6.6Hz), 7.15(1H, d, J=16.2Hz), 7.24-8.26-(11H, m).

EXAMPLE 84

(E)-N-(6,6-dimethyl-2-hepten-4-ynyl)-N-methyl-3-(2-phenylethynyl)benzylamine (compound 101)

$$IR\ \nu_{max}^{KBr}\ cm^{-1}:$$

2968, 2788, 1605, 1497, 1365, 1026, 966, 756, 690.

NMR($CDCl_3$)δ: 1.24(9H, s), 2.19(3H, s), 3.06(2H, dd, J=6.6Hz, 1.4Hz), 3.48(2H, s), 5.66 (1H, dt, J=15.9Hz, 1.4Hz), 6.10(1H, dt, J=15.9Hz, 6.6Hz), 7.26-7.44(6H, m), 7.50-7.55(3H, m).

EXAMPLE 85

Production of (E)-N-(6,6-dimethyl-2-hepten-4-ynyl)-N-methyl-3-benzylaminobenzylamine (compound 102)

125 mg of 3-benzylaminobenzyl alcohol hydrochloride was suspended in 5 ml of chloroform, and 0.2 ml of thionyl chloride was added. The mixture was stirred at room temperature for 3 hours. The reaction mixture was concentrated under reduced pressure. The residue was washed with ethyl ether and then dissolved in dimethylformamide. 85 mg of (E)-N-methyl-6,6-dimethyl-2-hepten-4-ynylamine hydrochloride and 300 mg of potassium carbonate were added. The mixture was stirred overnight at room temperature. The reaction mixture was diluted with ethyl acetate. The extract was washed with a saturated aqueous sodium chloride solution and dried over anhydrous sodium sulfate. The desiccant was removed by filtration, and the solvent was evaporated under reduced pressure. The residue was purified by preparative thin-layer chromatography [thin-layer plate: Kieselgel $60F_{254}$, Art. 5744 (a product of E. Merck Co.); developing solvent: hexane/ethyl acetate=3/1] to give 100 mg (yield 57.5 %) of the captioned compound as a pale yellow oil.

IR $\nu_{max}^{neat}$ $cm^{-1}$:

3430, 2968, 2788, 1497, 1458, 1335, 1200, 1128, 966, 777, 696.

NMR($CDCl_3$)δ: 1.24(9H, s), 2.17(3H, s), 3.01(2H, dt, J=6.7Hz, 1.5Hz), 3.40(2H, s), 4.32 (2H, s), 5.62-(1H, dt, J=15.9Hz, 1.5Hz), 6.09(1H, dt, J=15.9Hz, 6.7Hz), 6.50-6.53(1H, m), 6.63-6.66(2H, m), 7.10(1H, t, J=7.5Hz), 7.25-7.39 (5H, m).

Compounds of Examples 86 and 87 were obtained by repeating Example 85 except that instead of the starting 3-benzylaminobenzyl alcohol hydrochloride, the corresponding benzyl alcohol hydrochlorides were used. (In Example 87, the resulting free base was treated in a customary manner with a HCl/methanol/ethyl ether solution to convert it to a hydrochloride.)

EXAMPLE 86

(E)-N-(6,6-dimethyl-2-hepten-4-ynyl)-N-methyl-3-(2-methylbenzylamino)benzylamine hydrochloride (compound 103)

IR $\nu_{max}^{neat}$ $cm^{-1}$:

3432, 2972, 2778, 1608, 1364, 772, 746.

NMR($CDCl_3$)δ: 1.24(9H, s), 2.19(3H, s), 2.38(3H, s), 3.03(2H, dd, J=6.6Hz, 1.5Hz), 3.42 (2H, s), 4.27-(2H, s), 5.63(1H, dt, J=15.9Hz, 2.8Hz), 6.08(1H, dt, J=15.9Hz, 6.6Hz), 6.52(1H, ddd, J=8.2Hz, 2.5Hz, 1.2Hz), 6.63-6.67(2H, m), 7.09-7.19 (4H, m), 7.31-7.34(1H, m).

EXAMPLE 87

(E)-N-[6,6-dimethyl-2-hepten-4-ynyl]-N-methyl-3-anilinomethylbenzylamine hydrochloride (compound 104)

Melting point: 120-122 °C

IR $\nu_{max}^{KBr}$ $cm^{-1}$:

3418, 2632, 1635, 1602, 1461, 1365, 1263, 756, 549.

NMR($CDCl_3$)δ: 1.25(9H, s), 2.62(3H, s), 3.65-3.78 (2H, m), 4.05(1H, br), 4.31(1H, br), 4.42(2H, s), 5.92-(1H, d, J=15.9Hz), 6.10-6.30(1H, m), 7.20-8.00(9H, m).

EXAMPLE 88

Production of (E)-N-(6,6-dimethyl-2-hepten-4-ynyl)-N-methyl-3-(N-methylbenzylamino)benzylamine (compound 105)

250 mg of N-benzyl-N-methyl-3-hydroxymethylaniline hydrochloride was dissolved in 10 ml of chloroform, and 1 ml of thionyl chloride was added. The mixture was stirred at room temperature for 3 hours. The reaction mixture was evaporated under reduced pressure, and the residue was dissolved in 3 ml of dimethylformamide. 163 mg of (E)-N-methyl-6,6-dimethyl-2-hepten-4-ynylamine hydrochloride and 120 mg of sodium carbonate were added, and the mixture was stirred overnight at room temperature. Water (15 ml) was added to the reaction mixture, and the mixture was extracted with 15 ml of ethyl ether twice. The extracts were combined, washed with 10 ml of a saturated aqueous sodium chloride solution, and then dried over anhydrous magnesium sulfate. The desiccant was removed by filtration, and the solvent was evaporated. The residue was purified by preparative thin-layer chromatography [thin-layer plate: Kieselgel 6OF$_{254}$, Art. 5744 (a product of E. Merck Co.); developing solvent: chloroform/ethyl acetate=10/1]. Recrystallization from hexane gave 183 mg (yield 58.5 %) of the captioned compound as colorless needles

having a melting point of 62 to 63 °C.

$$IR\ \nu_{max}^{KBr}\ cm^{-1}:$$

2974, 1602, 1458, 1383, 1365.

NMR($CDCl_3$)δ: 1.24(9H, s), 3.00(3H, s), 3.43 (3H, s), 3.43-3.47(2H, m), 4.53 (2H, s), 5.61(1H, dt, J = 15.9Hz, 1.4Hz), 6.05(1H, dt, J = 15.9Hz, 6.6Hz), 6.61-6.67(2H, m), 6.72-6.73(1H, m), 7.15-7.17(1H, m), 7.21-7.34(5H, m).

EXAMPLE 89

(E)-N-(6,6-dimethyl-2-hepten-4-ynyl)-N-methyl-3-benzylthiobenzylamine (compound 106)

Methyl 3-benzylthiobenzoate (120 mg) was dissolved in 5 ml of anhydrous tetrahydrofuran, and with stirring under ice cooling, 10 mg of lithium aluminum hydride was added by portions over the course of 5 minutes. The mixture was stirred at this temperature for 10 minutes. Twenty milliliters of a 5 % aqueous solution of ammonium chloride was added to decompose the excess of the reducing agent. The mixture was then extracted with 20 ml of ethyl ether twice. The extracts were combined, and dried over anhydrous magnesium sulfate. The solvent was evaporated to give 100 ml (yield 93 %) of 3-benzylthiobenzyl alcohol as a colorless oil.

One hundred milligrams of the alcohol obtained above was dissolved in 10 ml of chloroform, and 0.3 ml of thionyl chloride was added. The mixture was reacted at room temperature for 2 hours. The reaction mixture was concentrated under reduced pressure, and the residue was dissolved in 20 ml of water and 20 ml of ethyl ether. The ether layer was separated, and the solvent was evaporated. The residue was purified by medium-pressure liquid chromatography [column: Lobar column, size A, Lichroprep Si 60F (a product of E. Merck Co.); eluting solvent: hexane] to give 55 mg (yield 51 %) of 3-benzylthiobenzyl chloride as a colorless oil.

The resulting chloride (55 mg) was dissolved in 3 ml of dimethylformamide, and 50 mg of (E)-N-methyl-6,6-dimethyl-2-hepten-4-ynylamine hydrochloride and 50 mg of potassium carbonate were added. The mixture was stirred overnight at room temperature. Water (20 ml) and 20 ml of ethyl ether were added to the reaction mixture, and the organic layer was separated, washed with a saturated aqueous sodium chloride solution and then dried over anhydrous magnesium sulfate. The desiccant was removed by filtration, and the solvent was evaporated. The residue was purified by medium-pressure liquid chromatography [column: Lobar column, size A, Lichroprep Si 60F (a product of E. Merck Co.); eluting solvent: hexane/ethyl acetate = 20/1] to give 37 mg (yield 46 %) of the captioned compound as a colorless oil.

$$IR\ \nu_{max}^{neat}\ cm^{-1}:$$

2974, 2788, 1458, 1365, 966, 777, 696.

NMR($CDCl_3$)δ: 1.24(9H, s), 2.13(3H, s), 2.99(2H, dd, J = 6.6Hz, 1.5Hz), 3.40(2H, s), 4.11 (2H, s), 5.63-(1H, dt, J = 15.9Hz, 1.5Hz), 6.06(1H, dt, J = 15.9Hz, 6.6Hz), 7.09-7.30(9H, m).

EXAMPLE 90

(E)-N-(6,6-dimethyl-2-hepten-4-ynyl)-N-methyl-3-(beta-phenethyl)benzylamine (compound 107)

Ethyl 3-phenethylbenzoate (180 mg) was dissolved in 2 ml of anhydrous tetrahydrofuran, and under ice cooling, 42 mg of lithium aluminum hydride was added by portions over 5 minutes. The mixture was stirred for one hour under ice cooling. Water (10 ml) was added to the reaction mixture to decompose the excess of the reducing agent. The mixture was then extracted with 20 ml of ethyl ether. The extract was separated, washed with a saturated aqueous sodium chloride solution, and dried over anhydrous sodium sulfate. The solvent was evaporated to give 140 mg (yield 93 %) of 3-phenethylbenzyl alcohol.

The resulting alcohol (140 mg) was dissolved in 1 ml of chloroform, and 0.3 ml of thionyl chloride was added. The mixture was reacted at room temperature for 30 minutes. The reaction mixture was evaporated

under reduced pressure to given 150 mg (yield 99 %) of 3-phenethylbenzyl chloride as a pale yellow oil.

The resulting chloride (150 mg) was dissolved in 1 ml of dimethylformamide, and 124 mg of (E)-N-methyl-6,6-dimethyl-2-hepten-4-ynylamine hydrochloride and 300 mg of potassium carbonate were added, and the mixture was stirred overnight at room temperature. Water (10 ml) and 20 ml of ethyl acetate were added to the reaction mixture. The organic layer was separated, washed with a saturated aqueous sodium chloride solution and dried over anhydrous sodium sulfate. The dessicant was removed by filtration, and the solvent was evaporated. The residue was purified by silica gel column chromatography [Wakogel C-200, 20 g; eluting solvent: hexane/ethyl acetate = 10/1] to give 150 mg (yield 58%) of the captioned compound as a colorless oil.

$$IR\ \nu_{max}^{neat}\ cm^{-1}:$$

3028, 2866, 1608, 1458, 1365, 1269, 1206, 786, 699.

NMR($CDCl_3$)δ: 1.24(9H, s), 2.16(3H, s), 2.91(4H, s), 3.02(2H, dd, J = 6.6Hz, 1.5 Hz), 3.45 (2H, s), 5.64-(1H, d, J = 15.9Hz), 6.08 (1H, dt, J = 15.9Hz, 6.6Hz), 7.05-7.30 (9H, m).

EXAMPLE 91

Production of (E)-N-(6,6-dimethyl-2-hepten-4-ynyl)-N-methyl-3-[(E)-2-(2-pyridyl)vinyl]benzylamine (compound 108)

3.9 g of (2-pyridyl)methyltriphenylphosphonium chloride was suspended in 20 ml of tetrahydrofuran, and with stirring, 5.3 ml of a 20 % aqueous solution of potassium carbonate and 0.13 g of isophthalaldehyde were added. The mixture was stirred overnight at room temperature. Hexane was added, and the resulting aqueous layer was discarded, and concentrated under reduced pressure. Hexane was added to the residue. The insoluble matter was removed by filtration, and the filtrate was evaporated under reduced pressure. The residue was purified by medium-pressure liquid chromatography [column: Lobar column, size B, Lichroprep Si 60 (a product of E. Merck Co.); eluting solvent: hexane/ethyl acetate = 7/1 → 4/1] to give 0.76 g (yield 36 %) of (E)-2-[2-(3-formylphenyl)vinyl]pyridine.

The resulting vinyl pyridine (573 mg) was dissolved in 5 ml of methanol, and under ice cooling, 104 mg of sodium borohydride was added. The mixture was stirred at room temperature for 30 minutes. The reaction mixture was concentrated under reduced pressure, and water and ethyl acetate were added to the residue. The organic layer was separated and evaporated under reduced pressure. The residue was dissolved in 5 ml of chloroform and under ice cooling, 0.17 ml of thionyl chloride was added. The mixture was stirred at room temperature for 1 hour. The reaction mixture was evaporated under reduced pressure, and the residue was treated with ethyl ether to give 412 mg (yield 57 %) of (E)-2-[2-(3-chloromethylphenyl)-vinyl]pyridine hydrochloride as colorless needles having a melting point of 159 to 160 °C. The resulting hydrochloride was dissolved in 5 ml of dimethylformamide, and 219 mg of (E)-N-methyl-6,6-dimethyl-2-hepten-4-ynylamine hydrochloride and 166 mg of potassium carbonate were added, and the mixture was stirred overnight at room temperature. The reaction mixture was diluted with water and benzene. The organic layer was separated and evaporated, then the residue was purified by medium-pressure liquid chromatography [column: Lobar column, size B, Lichroprep Si 60 (a product of E. Merck Co.); eluting solvent: hexane/ethyl acetate = 6/1 → 3/1] to give 277 mg (yield 67 %) of the captioned compound as a colorless oil.

$$IR\ \nu_{max}^{neat}\ cm^{-1}:$$

2968, 2788, 1587, 1473, 1440, 1365, 1266, 966, 792, 756, 696, 552.

NMR($CDCl_3$)δ: 1.25(9H, s), 2.21(3H, s), 3.07(2H, dd, J = 6.6Hz, 1.5Hz), 3.51(2H, s), 5.67 (1H, d, J = 15.8Hz), 6.11(1H, dt, J = 15.9Hz, 6.6Hz), 7.12-7.69(9H, m), 8.60(1H, dd, J = 5.0Hz, 1.1Hz).

EXAMPLE 92

Production of (E)-N-(6,6-dimethyl-2-hepten-4-ynyl)-2-benzyloxy-6-pyridylmethylamine dihydrochloride (compound 109)

91 mg of 2-benzyloxy-6-chloromethylpyridine was dissolved in 1.2 ml of dimethylformamide, and 110 mg of (E)-N-methyl-6,6-dimethyl-2-hepten-4-ynylamine hydrochloride and 96 mg of potassium carbonate were added. The mixture was stirred overnight at room temperature. The reaction mixture was concentrated under reduced pressure, and the residue was dissolved in chloroform. The inorganic salts were removed by filtration and the filtrate was evaporated and purified by thin-layer chromatography [thin layer plate: Kieselgel $60F_{254}$, Art.5715 (a product of E. Merck Co.); developing solvent: hexane/ethyl acetate = 3/1] to give 115 mg of the free base of the captioned compound as a pale yellow oil.

The product was dissolved in 3 ml of methanol, and 0.26 ml of a 30 % hydrogen chloride/methanol solution was added. The mixture was concentrated under reduced pressure. Recrystallization of the residue from a mixture of ethyl ether and hexane gave 108 mg (yield 68 %) of the captioned compound as a colorless crystalline powder having a melting point of 103 to 106 °C.

$$IR\ \nu_{max}^{KBr}\ cm^{-1}:$$

3448, 2968, 2866, 2596, 1635, 1608, 1578, 1458, 1368, 1320, 1266, 969.

NMR($CDCl_3$)δ: 1.24(9H, s), 2.67(3H, s), 3.54-3.64 (1H, m), 3.71-3.80(1H, m), 4.37(2H, s), 5.49(2H, s), 5.80(1H, d, J = 15.5Hz), 6.25(1H, dt, J = 15.5Hz, 7.5Hz), 6.98 (1H, d, J = 7.9Hz), 7.30-7.44(5H, m), 7.49(1H, d, J = 7.9Hz), 7.82(1H, t, J = 7.9Hz).

Compounds of Examples 93 to 95 were obtained by repeating Example 92 except that instead of the starting 2-benzyloxy-6-chloromethylpyridine, the corresponding chloromethylpyridines were used.

EXAMPLE 93

(E)-N-(6,6-dimethyl-2-hepten-4-ynyl)-N-methyl-4-benzyloxy-2-pyridylmethylamine dihydrochloride (compound 110)

Melting point: 126-128 °C

$$IR\ \nu_{max}^{KBr}\ cm^{-1}:$$

3454, 2968, 2596, 1635, 1500, 1461, 1332, 966.

NMR($CDCl_3$)δ: 1.24(9H, s), 2.85(3H, s), 3.72-3.77 (1H, m), 3.86(1H, d, J = 7.6Hz), 4.90 (2H, s), 5.52-(2H, s), 5.96(1H, d, J = 15.6Hz), 6.27(1H, dt, J = 15.6Hz, 7.6Hz), 7.26-7.54(1H, m), 8.41-(1H, d, J = 6.5Hz), 8.82-8.84(1H, m).

EXAMPLE 94

(E)-N-(6,6-dimethyl-2-hepten-4-ynyl)-N-methyl-2-(2-methylbenzyloxy)-6-pyridylmethylamine (compound 111)

$$IR\ \nu_{max}^{neat}\ cm^{-1}:$$

2968, 1599, 1581, 1455, 1311, 1266, 1005.

NMR($CDCl_3$)δ: 1.24(9H, s), 2.28(3H, s), 2.41(3H, s), 3.14(2H, dd, J = 6.4Hz, 1.4Hz), 3.59 (2H, s), 5.35-(2H, s), 5.68(1H, dd, J = 15.9Hz, 1.4Hz), 6.11(1H, dt, J = 15.9Hz, 6.4Hz), 6.64(1H, d, J = 7.9Hz), 6.98 (1H, d, J = 7.1Hz), 7.19-7.45(4H, m), 7.54(1H, dd, J = 7.9Hz, 7.1Hz).

EXAMPLE 95

(E)-N-(6,6-dimethyl-2-hepten-4-ynyl)-N-methyl-4-(2-methylbenzyloxy)-2-pyridylmethylamine (compound 112)

$$IR\ \nu_{max}^{neat}\ cm^{-1}:$$

2968, 1596, 1566, 1461, 1365, 1308, 1014, 747.

NMR($CDCl_3$)δ: 1.24(9H, s), 2.25(3H, s), 2.38(3H, s), 3.61(2H, s), 5.09(2H, s), 5.66(1H, dt, J = 15.9Hz, 3.4Hz), 6.09(1H, dt, J = 15.9Hz, 6.6Hz), 6.77(1H, dd, J = 6.4Hz, 2.7Hz), 7.08(1H, d, J = 2.4Hz), 7.20-7.31(3H, m), 7.37-7.40(1H, m), 8.35(1H, d, J = 5.6Hz).

EXAMPLE 96

Production of N-[(E)-6,6-dimethyl-2-hepten-4-ynyl]-N-methyl-5-[(E)-styryl]-3-pyrazolylmethylamine dihydrochloride (compound 113)

The same reaction as in Example 92 was repeated except that (E)-3-chloromethyl-5-styrylpyrazole was used instead of 2-benzyloxy-6-chloromethylpyridine. The product was recrystallized from a mixture of acetone and ethyl acetate to give the captioned compound as colorless needles having a melting point of 198 to 200 °C.

$$IR\ \nu_{max}^{KBr}\ cm^{-1}:$$

3130, 2968, 2482, 1464, 966, 750, 696.

NMR($CDCl_3$)δ: 1.24(9H, s), 2.72(3H, s), 3.72(2H, br), 4.23(2H, s), 5.94(1H, d, J = 15.6Hz), 6.14-6.24-(1H, m), 6.78(1H, s), 6.97(1H, d, J = 16.6Hz), 7.15(1H, d, J = 16.6Hz), 7.28-7.40(3H, m), 7.49(2H, d, J = 7.2Hz).

EXAMPLE 97

N-[(E)-6,6-dimethyl-2-hepten-4-ynyl]-N-methyl-5-[(Z)-styryl]furfurylamine (compound 114)

Fifty milligrams of (Z)-2-hydroxymethyl-5-styrylfuran and 24.3 microliters of anhydrous pyridine were dissolved in 1.5 ml of anhydrous ethyl ether, and at -30 °C, 0.5 ml of an anhydrous ethyl ether solution of 18.2 microliters of thionyl chloride was added dropwise with stirring in an atmosphere of nitrogen. After the addition, the mixture was stirred for 2 days at 10 °C. Then, 75 mg of (E)-N-methyl-6,6-dimethyl-2-hepten-4-ynylamine hydrochloride, 111 mg of potassium carbonate and 1 ml of dimethylformamide were added to the resulting solution, and the mixture was stirred overnight at room temperature. The reaction mixture was concentrated under reduced pressure, and the residue was purified by silica gel column chromatography [Wakogel C-200 5 g; eluting solvent: hexane/ethyl acetate = 5/1] to give 45 mg (yield 54 %) of the captioned compound as a pale yellow oil.

$$IR\ \nu_{max}^{neat}\ cm^{-1}:$$

2968, 1455, 1365, 1266, 1020, 966, 795, 696.

NMR($CDCl_3$)δ: 1.25(9H, s), 2.20(3H, s), 3.01(2H, dd, J = 6.7Hz, 1.4Hz), 3.48(2H, s), 5.60(1H, dt, J = 15.7Hz, 1.4Hz), 6.03(1H, dt, J = 15.7Hz, 6.7Hz), 6.10(1H, d, J = 3.4Hz), 6.17(1H, d, J = 3.4Hz), 7.22-7.36(3H, m), 7.42-7.48(2H, m).

EXAMPLE 98

Production of N-[(E)-6,6-dimethyl-2-hepten-4-ynyl]-N-methyl-5-[(E)-styryl]furfurylamine (compound 115)

Example 97 was repeated except that (E)-2-hydroxymethyl-5-styrylfuran was used instead of (Z)-2-hydroxymethyl-5-styrylfuran. The captioned compound was obtained as a pale yellow oil.

$$IR\,\nu_{max}^{neat}\ cm^{-1}:$$

2968, 1662, 1608, 1266, 756.

NMR($CDCl_3$)δ: 1.25(9H, s), 2.29(3H, s), 3.10(2H, dd, J = 6.8Hz, 1.5Hz), 3.59(2H, s), 5.67(1H, dt, J = 15.9Hz, 1.5Hz), 6.10(1H, dt, J = 15.9Hz, 6.8Hz), 6.22(1H, d, J = 16.2Hz), 7.01(1H, d, J = 16,2Hz), 7.19-7.24(1H, m), 7.29-7.37(2H, m), 7.42-7.48(2H, m).

EXAMPLE 99

The same reaction as in Example 97 was carried out except that a mixture of (E)- and (Z)-2-hydroxymethyl-4-styrylthiazoles was used instead of (Z)-2-hydroxymethyl-5-styrylfuran. The product containing a mixture of the (E)-form and (Z)-form were separated and purified by preparative thin-layer chromatography [thin-layer plate: Kieselgel $60F_{254,}$ Art. 5744 (a product of E. Merck Co.); developing solvent: toluene/ethyl acetate/20 % aqueous ammonia = 240/100/1] to give the following compounds as pale yellow oils.

N-[(E)-6,6-dimethyl-2-hepten-4-ynyl]-N-methyl-2-[(Z)-styryl]-4-thiazolylmethylamine (compound 116)

$$IR\,\nu_{max}^{KBr}\ cm^{-1}:$$

2968, 1458, 1365, 1266, 963, 750, 696.

NMR($CDCl_3$)δ: 1.23(9H, s), 2.24(3H, s), 3.09(2H, dd, J = 6.6Hz, 1.5Hz), 3.62(2H, s), 5.63(1H, dt, J = 15.9Hz, 1.5Hz), 6.09(1H, dt, J = 15.9Hz, 6.6Hz), 6.88(1H, dd, J = 11.9Hz, 1.3Hz), 6.91(1H, s), 6.95(1H, d, J = 11.9Hz), 7.36-7.40(5H, m).

N-[(E)-6,6-dimethyl-2-hepten-4-ynyl]-N-methyl-2-[(E)-styryl]-4-thiazolylmethylamine (compound 117)

$$IR\,\nu_{max}^{neat}\ cm^{-1}:$$

2968, 2926, 1458, 1365, 1266, 960, 750, 690.

NMR($CDCl_3$)δ: 1.24(9H, s), 2.29(3H, s), 3.13(2H, dd, J = 6.7Hz, 1.6Hz), 3.67(2H, s), 5.67(1H, dt, J = 15.9Hz, 1.6Hz), 6.19(1H, dt, J = 15.9Hz, 6.7Hz), 7.05(1H, s), 7.30 (1H, d, J = 16.2Hz), 7.38(1H, d, J = 16.2Hz), 7.26-7.40(3H, m), 7.51-7.53 (2H, m).

EXAMPLE 100

N-[(E)-6,6-dimethyl-2-hepten-4-ynyl]-N-methyl-2-[(E)-styryl]-4-oxazolylmethylamine (compound 118)

Example 97 was repeated except that (E)-2-hydroxymethyl-5-styryloxazole was used instead of (Z)-2-hydroxymethyl-5-styrylfuran. The captioned compound was obtained as a colorles oil.

IR $\nu_{max}^{neat}$ $cm^{-1}$:

2968, 1716, 1455, 960, 750, 693.

NMR($CDCl_3$)δ: 1.24(9H, s), 2.35(3H, s), 3.18(2H, dd, J=6.7Hz, 1.4Hz), 3.74(2H, s), 5.70(1H, dt, J=15.7Hz, 1.4Hz), 6.09(1H, dt, J=15.7Hz, 6.7Hz), 6.87(1H, d, J=16.4Hz), 6.99(1H, s), 7.08(1H, d, J=16.4Hz), 7.25-7.40(3H, m), 7.45-7.51(2H, m).

EXAMPLE 101

(E)-N-(6,6-dimethyl-2-hepten-4-ynyl)-N-ethyl-3-[3-(1-pyrrolyl)benzyloxy]benzylamine (compound 119)

One hundred milligrams of (E)-N-(6,6-dimethyl-2-hepten-4-ynyl)-N-ethyl-3-hydroxybenzylamine was dissolved in 4 ml of anhydrous tetrahydrofuran, and with stirring under ice cooling, 20 mg of 60 % oily sodium hydride was added. The mixture was stirred for 10 minutes. To this solution was added 1 ml of a dimethylformamide solution of 100 mg of 3-(1-pyrrolyl)benzyl methanesulfonate, and the mixture was stirred overnight at room temperature. Water (20 ml) and ethyl ether (30 ml) were added. The organic layer was separated, washed with a saturated aqueous sodium chloride solution, and dried over anhydrous sodium sulfate. The solvent was evaporated, and the residue was purified by silica gel column chromatography [Wakogel C-200, 20 g; the eluting solvent: hexane/ethyl acetate=10/1 → 5/1] to give 110 mg (yield 70 %) of the captioned compound as a colorless oil.

IR $\nu_{max}^{neat}$ $cm^{-1}$:

2968, 1596, 1506, 1488, 1455, 1341, 1263, 1071, 786, 726.

NMR($CDCl_3$)δ: 0.98(3H, t, J=7.0Hz), 1.19(9H, s), 2.45(2H, q, J=7.0Hz), 3.03(2H, dd, J=6.4Hz, 1.6Hz) 3.49(2H, s), 5.06 (2H, s), 5.59(1H, dt, J=15.9Hz, 1.6Hz), 6.01(1H, dt, J=15.9Hz, 6.4Hz), 6.30(2H, t, J=2.1Hz), 6.80(1H, ddd, J=8.3Hz, 2.6Hz, 0.8Hz), 6.88(1H, d, J=7.6Hz), 6.96-6.98(1H, m), 7.06(2H, t, J=2.1Hz), 7.17(1H, t, J=7.8Hz), 7.25-7.32(2H, m), 7.39(1H, t, J=7.8Hz), 7.44-7.45(1H, m).

Compounds of Examples 102 to 105 were obtained by repeating Example 101 except that instead of the starting (E)-N-(6,6-dimethyl-2-hept-4-ynyl)-N-ethyl-3-hydroxybenzylamine and/or 3-(1-pyrrolyl)benzyl methanesulfonate, the corresponding amine derivative and/or the benzyl methanesulfonyl derivative was used.

EXAMPLE 102

(E)-N-(6,6-dimethyl-2-hepten-4-ynyl)-N-ethyl-3-[3-(5-oxazolyl)benzyloxy]benzylamine (compound 120)

IR $\nu_{max}^{neat}$ $cm^{-1}$:

2968, 1491, 1458, 1263, 1107, 954, 789.

NMR($CDCl_3$)δ: 1.03(3H, t, J=7.1Hz), 1.24(9H, s), 2.50(2H, q, J=7.1Hz), 3.09(2H, dd, J=6.4Hz, 1.5Hz), 3.54(2H, s), 5.11 (2H, s), 5.64(1H, dt, J=15.8Hz, 1.5Hz), 6.07(1H, dt, J=15.8Hz, 6.4Hz), 6.84-6.89(1H, m), 6.93(1H, d, J=7.7Hz), 7.03(1H, br), 7.23(1H, t, J=7.7Hz), 7.39(1H, s), 7.40-7.49(2H, m), 7.62 (1H, dt, J=6.6Hz, 2.1Hz), 7.75(1H, br), 7.93(1H, s).

EXAMPLE 103

(E)-N-(6,6-dimethyl-2-hepten-4-ynyl)-N-ethyl-3-[3-(5-thiazolyl)benzyloxy]benzylamine (compound 121)

$$IR\ \nu_{max}^{neat}\ cm^{-1}:$$

2972, 1588, 1490, 1456, 1364, 1264, 1152, 1046, 874, 788.

NMR($CDCl_3$)δ: 0.96(3H, t, J = 7.0Hz), 1.19(9H, s), 2.46(2H, q, J = 7.0Hz), 3.04(2H, d, J = 6.0Hz), 3.50-(2H, s), 5.06(2H, s), 5.60(1H, d, J = 15.8Hz), 6.02(1H, dt, J = 15.8Hz, 6.0Hz), 6.82(1H, dd, J = 8.1Hz, 2.1Hz), 6.89(1H, d, J = 8.0Hz), 6.98 (1H, br), 7.18(1H, t, J = 8.0Hz), 7.38-7.41(2H, m), 7.48-7.52(1H, m), 7.62(1H, s), 8.05(1H, d, J = 0.6Hz), 8.71(1H, d, J = 0.6Hz).

EXAMPLE 104

(E)-N-(6,6-dimethyl-2-hepten-4-ynyl)-N-propyl-3-[3-(1-pyrrolyl)benzyloxy]benzylamine (compound 122)

$$IR\ \nu_{max}^{neat}\ cm^{-1}:$$

2968, 1596, 1506, 1488, 1455, 1341, 1263, 1071, 723.

NMR($CDCl_3$)δ: 0.85(3H, t, J = 7.4Hz), 1.24(9H, s), 1.47(2H, sex, J = 7.4Hz), 2.36(2H, t, J = 7.4Hz) 3.06-(2H, dd, J = 6.4Hz, 1.6Hz), 3.53(2H, s), 5.11(2H, s), 5.63(1H, dt, J = 15.9Hz, 1.6Hz), 6.05(1H, dt, J = 15.9Hz, 6.4Hz), 6.35(1H, t, J = 2.2Hz), 6.85(1H, ddd, J = 8.2Hz, 2.6Hz, 0.9Hz), 6.92(1H, d, J = 7.6Hz), 7.00-7.03(1H, m), 7.11(2H, t, J = 2.2Hz), 7.21(1H, t, J = 7.8Hz), 7.28-7.37(2H, m), 7.44(1H, t, J = 7.8Hz), 7.49(1H, t, J = 1.5Hz).

EXAMPLE 105

(E)-N-(6,6-dimethyl-2-hepten-4-ynyl)-N-propyl-3-[3-(5-oxazolyl)benzyloxy]benzylamine (compound 123)

$$IR\ \nu_{max}^{neat}\ cm^{-1}:$$

2968, 1596, 1491, 1455, 1263, 1107, 954, 789.

NMR($CDCl_3$)δ: 0.85(3H, t, J = 7.4Hz), 1.47(2H, sex, J = 7.4Hz), 2.37(2H, t, J = 7.4Hz) 3.07(2H, dd, J = 6.4Hz, 1.5Hz), 3.53(2H, s), 5.10 (2H, s), 5.63(1H, dt, J = 15.9Hz, 1.5Hz), 6.06(1H, dt, J = 15.9Hz, 6.4Hz), 6.84-6.89 (1H, m), 6.92(1H, d, J = 7.8Hz), 7.02 (1H, br), 7.22(1H, t, J = 7.8Hz), 7.38 (1H, s), 7.40-7.49(2H, m), 7.62(1H, dt, J = 6.6Hz, 2.1Hz), 7.75(1H, br), 7.93 (1H, s).

EXAMPLE 106

Production of (E)- and (Z)-N-methyl-N-(2-nonen-4-ynyl)-3-benzyloxybenzylamine

Two hundred milligrams of N-methyl-3-benzyloxybenzylamine hydrochloride was dissolved in 3 ml of dimethylformamide, and 152 mg of 1-bromo-2-nonen-4-yne (a mixture of the E- and Z-forms) and 80 mg of sodium carbonate were added. The mixture was stirred overnight at room temperature. Water (15 ml) was added to the reaction mixture to dilute it. The mixture was extracted with 10 ml of ethyl ether twice. The extracts were combined, washed with 10 ml of a saturated aqueous sodium chloride solution, and dried over anhydrous magnesium sulfate. The solvent was evaporated, and the residue was purified by preparative

chromatography [thin-layer plate: Kieselgel 60$F_{254}$, Art. 5744 (a product of E. Merck Co.); developing solvent: chloroform/ethyl acetate = 10/1] to give the following compounds as colorless oils.

(E)-N-methyl-N-(2-nonen-4-ynyl)-3-benzyloxybenzyl amine (compound 124)

Amount: 112 mg (yield: 42 %)

$$IR\ \nu_{max}^{neat}\ cm^{-1}:$$

2932, 1587, 1491, 1458, 1263, 1026.

NMR($CDCl_3$)δ: 0.91(3H, t, J = 7.2Hz), 1.37-1.54 (4H, m), 2.18(3H, s), 2.30(2H, dt, J = 6.8Hz, 2.0Hz) 3.04(2H, dd, J = 6.6Hz, 1.5Hz), 3.46(2H, s), 5.06(2H, s), 5.63 (1H, dm, J = 15.9Hz), 6.09(1H, dt, J = 15.9Hz, 6.6Hz), 6.84-6.91(2H, m), 6.96-6.97(1H, m), 7.18-7.24(1H, m), 7.26-7.46(5H, m).

(Z)-N-methyl-N-(2-nonen-4-ynyl)-3-benzyloxybenzyl amine (compound 125)

Amount: 36 mg (yield: 14 %)

$$IR\ \nu_{max}^{neat}\ cm^{-1}:$$

2932, 1587, 1458, 1263, 1152, 1026, 738, 696.

NMR($CDCl_3$)δ: 0.92(3H, t, J = 7.0Hz), 1.38-1.54 (4H, m), 2.22(3H, s), 2.33(2H, dt, J = 6.8Hz, 1.9Hz), 3.28(2H, dd, J = 6.8Hz, 1.5Hz), 3.50(2H, s), 5.06(2H, s), 5.61 (1H, dm, J = 11.0Hz), 5.95(1H, dt, J = 11.0Hz, 6.8Hz), 6.85-6.88(1H, m), 6.91-6.93(1H, m), 6.98-6.99(1H, m), 7.19-7.25(1H, m), 7.31-7.46(5H, m).

Compounds of Examples 107 and 108 were obtained by repeating Example 106 except that instead of the starting 1-bromo-2-nonen-4-yne, the corresponding brominated alkene derivatives were used.

EXAMPLE 107

(E)-N-(6-methoxy-6-methyl-2-hepten-4-ynyl)-N-methyl-3-benzyloxybenzylamine (compound 126)

$$IR\ \nu_{max}^{neat}\ cm^{-1}:$$

1458, 1260, 1170, 1152, 1077, 1026.

NMR($CDCl_3$)δ: 1.47(6H, s), 2.19(3H, s), 3.05(2H, dd, J = 6.5Hz, 1.6Hz), 3.36(3H, s), 3.47 (2H, s), 5.06-(2H, s), 5.68(1H, dt, J = 15.8Hz, 1.6Hz), 6.17(1H, dt, J = 15.8Hz, 6.5Hz), 6.85-6.91(2H, m), 6.97-6.98(1H, m), 7.19-7.26(1H, m), 7.31-7.46(5H, m).

(Z)-N-(6-methoxy-6-methyl-2-hepten-4-ynyl)-N-methylbenzyloxybenzylamine (compound 127)

$$IR\ \nu_{max}^{neat}\ cm^{-1}:$$

1458, 1254, 1170, 1152, 1077, 1029.

NMR($CDCl_3$)δ: 1.47(6H, s), 2.22(3H, s), 3.28(2H, dd, J = 6.8Hz, 1.5Hz), 3.35(3H, s), 3.50 (2H, s), 5.06-(2H, s), 5.65(1H, dt, J = 10.6Hz, 1.5Hz), 6.04(1H, dt, J = 10.6Hz, 6.8Hz), 6.85-6.92(2H, m), 6.97-6.99(1H, m), 7.20-7.26(1H, m), 7.31-7.46(5H, m).

EXAMPLE 108

(E)-N-(5-phenyl-2-penten-4-ynyl)-N-methyl-3-benzyloxybenzylamine (compound 128)

$$IR\ \nu_{max}^{neat}\ cm^{-1}:$$

1599, 1491, 1458, 1263, 1026, 756, 693.

NMR($CDCl_3$)δ: 2.22(3H, s), 3.12(2H, dd, J=6.7Hz, 1.4Hz) 3.50(3H, s), 5.07(2H, s), 5.88 (1H, dt, J=15.9Hz, 1.4Hz), 6.28(1H, dt, J=15.9Hz, 6.7Hz), 6.85-6.93(2H, m), 6.98-6.99(1H, m), 7.20-7.26(1H, m), 7.28-7.46(10H, m).

(Z)-N-(5-phenyl-2-penten-4-ynyl)-N-methyl-3-benzyloxybenzylamine (compound 129)

$$IR\ \nu_{max}^{neat}\ cm^{-1}:$$

1599, 1491, 1458, 1263, 1026, 756, 738, 693.

NMR($CDCl_3$)δ: 2.27(3H, s), 3.39(2H, dd, J=6.8Hz, 1.5Hz), 3.54(2H, s), 5.05(2H, s), 5.84 (1H, dt, J=10.7Hz, 1.5Hz), 6.10(1H, dt, J=10.7Hz, 6.8Hz), 6.84-6.88(1H, m), 6.92-6.95(1H, m), 6.99-7.00(1H, m), 7.19-7.25(1H, m), 7.28-7.42(10H, m).

EXAMPLE 109

Production of (E)-N-(3-phenyl-2-propenyl)-N-methyl-3-benzyloxybenzylamine (compound 130)

Ninety milligrams of 3-benzyloxybenzyl chloride was dissolved in 2 ml of dimethylformamide, and 71 mg of (E)-N-methylcinnamylamine hydrochloride and 45 mg of sodium carbonate were added. The mixture was stirred overnight at room temperature. Water (15 ml) was added to the reaction mixture, and the mixture was extracted with 10 ml of ethyl ether twice. The extracts were combined, washed with 10 ml of a saturated aqueous sodium chloride solution, and dried over anhydrous magnesium sulfate. The dessicant was removed by filtration, and the solvent was evaporated. The residue was purified by preparative thin-layer chromatography [thin-layer plate: Kieselgel 60$F_{254}$, Art. 5744 (a product of E. Merck Co.); developing solvent: chloroform/ethyl acetate-10/1]] to give 108 mg (yield 81.3 %) of the captioned compounds as a colorless oil.

$$IR\ \nu_{max}^{neat}\ cm^{-1}:$$

1599, 1491, 1455, 1266, 1152, 1026, 741, 693.

NMR($CDCl_3$)δ: 2.24(3H, s), 3.18(2H, dd, J=6.6Hz, 1.2Hz) 3.53(2H, s), 5.07(2H, s), 6.30 (1H, dt, J=15.9Hz, 6.6Hz), 6.53(1H, dt, J=15.8Hz, 1.2Hz), 6.85-6.89(1H, m), 6.92-6.95(1H, m), 7.00-7.01(1H, m), 7.20-7.46(6H, m).

EXAMPLE 110

(E)-N-methyl-N-[5-(1-methylcyclopropyl)-2-penten-4-ynyl]-3-(2-methylbenzyloxy)benzylamine (compound 131)

Carbon tetrabromide (18.3 g) and 28.9 g of triphenylphosphine were added to 350 ml of methylene chloride, and the mixture was stirred at room temperature for 10 minutes. Then, 4.62 g of 1-methylcyclopropanaldehyde [J. Am. Chem., Soc., 97, 2778 (1975)] was added, and the mixture was stirred for 10 minutes. The reaction mixture was washed with water and concentrated under reduced pressure. Hexane

was added to the residue, and the mixture was filtered. The filtrate was concentrated, and disilled under reduced pressure to give 5.62 g of 1-(2,2-dibromoethenyl)-1-methylcyclopropane. The dibromo compound obtained above (720 mg) was dissolved in 10 ml of tetrahydrofuran, and under cooling at -80 °C, 4.0 ml of a 1.57M hexane solution of butyl lithium was added. The mixture was stirred at this temperature for 1 hour and then at room temperature for 1 hour. The solution was again cooled to -10 °C, and a tetrahydrofuran solution (4.5 ml) of 168 mg of acrolein was added. The mixture was stirred at room temperature for 30 minutes. An aqueous solution of ammonium chloride and ethyl ether were added to the reaction mixture. The organic layer was separated and the solvent was evaporated. The residue was distilled at 100 °C under a reduced pressure of 5 mmHg to give 205 mg of a fraction containing 1-(1-methylcyclopropyl)-4-penten-1-yn-3-ol as a main component.

Two hundred milligrams of the above fraction was dissolved in 5 ml of methylene chloride, and with stirring under ice cooling, 0.42 ml of triethylamine and 0.14 ml of methanesulfonyl chloride was added. The mixture was stirred for 30 minutes, and then 450 mg of N-methyl-3-(2-methylbenzyloxy)benzylamine was added. The mixture was stirred at room temperature for 2 hours. The reaction mixture was diluted with methylene chloride and then washed with water. The solvent was evaporated, and the residue was purified by medium-pressure liquid chromatography (column: Lobar column, size A, Lichroprep Si 60F (a product of E. Merck Co.; eluting solvent: hexane/ethyl acetate = 10/1 → 7/1] to give the captioned compound as a colorless oil.

$$IR\,\nu_{max}^{neat}\ cm^{-1}:$$

2962, 2926, 2788, 2224, 1599, 1458, 1266, 1152, 1020, 747.

NMR($CDCl_3$)δ: 0.58-0.63(2H, m), 0.89-0.93(2H, m), 1.26(3H, s), 2.19(3H, s), 2.38(3H, s), 3.04(2H, dd, J = 6.5Hz, 1.4Hz), 3.47 (2H, s), 5.03(2H, s), 5.62(1H, dt, J = 15.8Hz, 1.4Hz), 6.08(1H, dt, J = 15.8Hz, 6.5Hz, 6.85-6.92(2H, m), 6.97(1H, d, J = 1.7Hz), 7.20-7.26(5H, m), 7.40-7.43(1H, m).

EXAMPLE 111

<u>(E),(E)-N-(6,6-dimethyl-2,4-heptadienyl)-N-methyl-3-(2-methylbenzyloxy)benzylamine compound (132)</u>

Using 200 mg of (E), (E)-6,6-dimethyl-2,4-heptadienol, 0.43 ml of triethylamine, 180 mg of methanesulfonyl chloride and 350 mg of N-methyl-3-(2-methylbenzyloxy)benzylamine, the same reaction as in Example 110 above was carried out. There was obtained 131 mg (yield 25 %) of the captioned comound as a colorless oil.

$$IR\,\nu_{max}^{neat}\ cm^{-1}:$$

2962, 1599, 1491, 1461, 1263, 1020, 990, 747.

NMR($CDCl_3$)δ: 1.03(9H, s), 2.19(3H, s), 2.38(3H, s), 3.04(2H, dd, J = 6.6Hz, 1.5Hz) 3.47(2H, s), 5.03-(2H, s), 5.68(1H, d, J = 14.9Hz), 5.68(1H, dt, J = 14.6Hz, 6.6Hz), 5.98(1H, dd, J = 14.9Hz, 10.1Hz), 6.13(1H, ddt, J = 14.6Hz, 10.1Hz, 1.5Hz), 6.87(1H, ddd, J = 8.4Hz, 2.8Hz, 1.4Hz), 6.90-6.94 (1H, m), 6.98-7.00(1H, m), 7.19-7.26 (4H, m), 7.40-7.44(1H, m).

EXAMPLE 112

<u>(E),(E)-N-(3,7-dimethyl-2,6-octadienyl)-N-methyl-3-benzyloxybenzylamine (compound 133)</u>

Geranyl bromide (313 mg), 290 mg of N-methyl-3-benzyloxybenzylamine hydrochloride and 200 mg of potassium carbonate were added to 3 ml of dimethylformamide, and the mixture was stirred overnight at room temperature. Ethyl ether and water were added to the reaction mixture. The organic layer was separated, and washed with a saturated aqueous sodium chloride solution. The organic layer was dried over anhydrous magnesium sulfate and the solvent was evaporated. The residue was purified by medium-pressure liquid chromatography [column: Lobar column, size B, Lichroprep Si 60F (a product of E. Merck Co.); eluting solvent: hexane/ethyl acetate = 10/1 → 7/1] to give the captioned compound as a colorless oil.

$$IR\ \nu_{max}^{neat}\ cm^{-1}:$$

2920, 1596, 1491, 1455, 1263, 1152, 1026, 696.

NMR($CDCl_3$)δ: 1.60(3H, s), 1.62(3H, s), 1.66(3H, s), 2.01-2.15(4H, m), 2.18(3H, s), 2.98 (2H, d, J = 6.8Hz) 3.45(2H, s), 5.06 (2H, s), 5.07-5.12(1H, m), 5.27-5.33 (1H, m), 6.86(1H, dd, J = 7.8Hz, 2.4Hz), 6.91(1H, d, J = 7.6Hz), 7.19-7.46(6H, m).

EXAMPLE 113

Tablets, capsules and granules containing (E)-N-(6,6-dimethyl-2-hepten-4-ynyl)-N-propyl-3-(2-methylbenzyloxy)benzylamine hydrochloride (compound 45) as an active ingredient

(1) Tablets (25 mg/tablet)

Twenty-five parts of compound 45, 70 parts of lactose, 30 parts of corn starch, 23 parts of crystalline cellulose and 2 parts of magnesium stearate were uniformly mixed, and tableted by a conventional method to give tablets containing 25 mg of the active ingredient per tablet.

(2) Capsules (25 mg/capsule)

Twenty-five parts of compound 45, 125 parts of lactose, 45 parts of corn starch and 5 parts of magnesium stearate were uniformly mixed, and then 200 mg of the mixture was filled into each of hard gelatin capsules (No. 2) to give capsules containing 25 mg of the active ingredient per capsule.

(3) Granules (50 mg/g)

Fifty parts of compound 45, 700 parts of lactose and 230 parts of corn starch were uniformly mixed, and then kneaded with a paste prepared from 20 parts of hydroxypropylcellulose and purified water. The kneaded mixture was granulated by a conventional method to give granules containing 50 mg of the active ingredient per gram.

General methods of synthesizing the starting compounds used in the above Example will be described below.

REFERENTIAL EXAMPLE 1

Production of 3-benzyloxybenzylamine hydrochloride

Three hundred milligrams of 3-benzyloxybenzaldehyde was dissolved in 15 % ammonia/ethanol, and the solution was stirred for 3 hours. Then, 100 mg of sodium borohydride was added, and the mixture was stirred for 1 hour. The reaction mixture was distilled under reduced pressure, and 20 ml of water and 20 ml of ethyl ether were added. The organic layer was separated, washed with a saturated aqueous solution chloride solution and dried over anhydrous sodium sulfate. The dessicant was removed by filtration, and the solvent was evaporated. The residue was recrystallized from a mixture of tetrahydrofuran and ethyl ether containing hydrogen chloride to give 140 mg (yield 40 %) of the captioned compound as colorless scales having a melting point of 155 to 159 °C.

REFERENTIAL EXAMPLE 2

Production of N-methyl-3-benzyloxybenzylamine hydrochloride

15.0 g of m-hydroxybenzaldehyde was dissolved in 200 ml of ethanol, and 25.0 g of potassium carbonate and 32 g of benzyl bromide were added. With stirring, the mixture was heated under reflux for 6 hours. After the reaction, the insoluble inorganic salts were removed by filtration. The filtrate was evaporated

under reduced pressure, and purified by silica gel column chromatography [Wakogel C-200, 150 g; hexane/chloroform = 1/1] and then recrystallized from hexane to give 21.1 g (yield 81.1 %) of 3-benzyloxybenzaldehyde having a melting point of 56 to 57 °C.

The resulting benzyloxy compound (7.23 g) was dissolved in 40 ml of a 40 % methanol solution of monomethylamine, and 2.4 g of sodium borohydride was added. The mixture was stirred at room temperature for 3 hours. The reaction mixture was concentrated under reduced pressure, and the residue was dissolved in a mixture of 100 ml of water and 100 ml of ethyl ether. The organic layer was separated and then dried over anhydrous sodium sulfate. The dessicant was removed by filtration, and then the solvent was evaporated. The residue was recrystallized from a mixture of methanol and ethyl ether containing hydrogen chloride to give 7.68 g (yield 85.5 %) of the captioned compound as colorless needles having a melting point of 127 to 129 °C.

The N-methylbenzylamine derivatives used in Examples 4 to 18 can be synthesized by a similar method.

REFERENTIAL EXAMPLE 3

Production of N-isopropyl-3-benzyloxybenzylamine hydrochloride

Three hundred grams of 3-benzyloxybenzaldehyde was dissolved in 10 ml of ethanol, and 84 mg of isopropylamine was added. The mixture was stirred at room temperature for 3 hours. Then, 100 mg of sodium cyanoborohydride was added, and the mixture was stirred for 1 hour. Water (20 ml) and 20 ml of ethyl ether were added, and the organic layer was separated. The organic layer was dried over anhydrous magnesium sulfate, and the dessicant was removed by filtration. The solvent was evaporated, and the residue was recrystallized from a mixture of tetrahydrofuran and ethyl ether containing hydrogen chloride to give 320 mg (yield 78 %) of the captioned compound as colorless scales having a melting point of 150 to 151 °C.

The N-substituted benzylamine derivatives used in Examples 19 to 30 can be synthesized by a similar method.

REFERENTIAL EXAMPLE 4

Production of 3-benzyloxy-4-fluorobenzyl bromide

54 mg of 4-fluoro-3-hydroxybenzaldehyde was dissolved in 2 ml of 2-propanol, and 73 mg of benzyl chloride, 80 mg of potassium carbonate and 5 mg of sodium iodide were added to the solution. The mixture was heated under reflux for 7 hours with stirring. The reaction mixture was diluted with ethyl ether, and the inorganic salts were removed by filtration. The filtrate was evaporated under reduced pressure, and purified by silica gel column chromatography (Wakogel C-200, 5 g; eluting solvent: hexane/ethyl acetate = 20/1), and then recrystallized from chloroform/hexane to give 77 mg of 3-benzyloxy-4-fluorobenzaldehyde as colorless needles having a melting point of 68 to 69 °C.

The benzyloxy compound obtained above (71 mg) was dissolved in 15 ml of ethanol, and 22 mg of sodium borohydride was added. The mixture was stirred at room temperature for 30 minutes. The reaction mixture was concentrated under reduced pressure, and ethyl ether and water were added. The organic layer was separated, washed with water and then dried over anhydrous magnesium sulfate. The desiccant was removed by filtration, the filtrate was evaporated under reduced pressure. The residue was dissolved in 1.5 ml of methylene chloride, and then 41 microliters of phosphorus tribromide was added. The mixture was stirred at room temperature for 30 minutes. Water and methylene chloride were added to the reaction mixture. The organic layer was separated, washed with water and dried over anhydrous magnesium sulfate. The desiccant was removed by filtration, and the filtrate was concentrated under reduced pressure. The residue was purified by silica gel column chromatography (Wakogel, C-200, 5 g; eluting solvent: hexane/ethyl acetate = 20/1) to give 68 mg (yield 25 %) of the captioned compound as colorless crystals having a melting point of 81 to 82 °C.

REFERENTIAL EXAMPLE 5

Production of 3-benzyloxy-5-methylbenzyl bromide

420 mg of benzyl 3-benzyloxy-5-methylbenzoate obtained by benzylating 3-hydroxy-5-methylbenzoic acid was dissolved in 10 ml of tetrahydrofuran, and 72 mg of lithium aluminum hydride was added. The mixture was stirred under ice cooling for 1.5 hours. Acetone was added to the reaction mixture to decompose the excess of the reducing agent. Water and ethyl ether were added, and the organic layer was separated, and dried over anhydrous magnesium sulfate. The desiccant was removed by filtration, and the filtrate was evaporated under reduced pressure to give a mixture of 3-benzyloxy-5-methylbenzyl alcohol and benzyl alcohol.

The mixture was dissolved in 10 ml of methylene chloride, and 0.23 ml of phosphorus tribromide was added. The mixture was stirred at room temperature for 30 minutes. Water and methylene chloride were added to the reaction mixture. The organic layer was separated and dried over anhydrous sodium sulfate. The desiccant was removed by filtration, and the filtrate was concentrated under reduced pressure, and purified by medium-pressure liquid chromatography [column: Lobar column, size A, Lichroprep Si 60 (a product of E. Merck Co.); eluting solvent: hexane/ethyl acetate = 100/1] to give 137 mg (yield 37.2 %) of the captioned compound as a colorless oil.

When 3-benzyloxy-4-hydroxybenzoic acid is used as a starting material and similarly reduced and brominated, 3-benzyloxy-4-hydroxybenzyl bromide used in Example 33 can be obtained.

REFERENTIAL EXAMPLE 6

Production of 3-(2-methoxybenzyloxy)benzyl methanesulfonate

530 mg of 3-(2-methoxybenzyloxy)benzyl alcohol obtained by reducing 3-(2-methoxybenzyloxy)-benzaldehyde with sodium borohydride in ethanol was dissolved in 8 ml of methylene chloride. The solution was stirred at -5 to 0 °C, 0.20 ml of methanesulfonyl chloride and 0.65 ml of triethylamine were added. The mixture was stirred at this temperature for 5 minutes. The reaction mixture was diluted with methylene chloride, washed with a saturated aqueous solution of sodium hydrogen carbonate, and dried over anhydrous magnesium sulfate. The solvent was evaporated to give 660 mg (yield 95.3 %) of the captioned compound as a colorless oil.

By a similar method, the benzyl methanesulfonates used in Examples 35 to 46 can be synthesized.

REFERENTIAL EXAMPLE 7

Production of (E)-N-(6,6-dimethyl-2-hepten-4-ynyl)-N-methyl-3-hydroxybenzylamine

10.0 g of 3-hydroxybenzaldehyde and 9.55 g of a 40 % methanol solution of methylamine were mixed, and then the solvent was evaporated. The resulting Schiff base was dissolved in 50 ml of ethanol, and with stirring under ice cooling, 10.0 g of sodium borohydride was added. The mixture was stirred overnight at room temperature. The solvent was evaporated under reduced pressure, and the residue was extracted with ethyl acetate and a saturated aqueous sodium chloride solution. The organic layer was separated, and dried over anhydrous sodium sulfate. The solvent was evaporated, and the residue was purified by silica gel column chromatography [Wakogel C-100, 100g; eluting solvent: methylene chloride/methanol = 10/1 → 5/1] to give 8.88 g (yield 79 %) of N-methyl-3-hydroxybenzylamine as pale yellow crystals having a melting point of 138 to 140 °C.

The resulting N-methylamine compound (8.88 g) and 18.0 g of potassium carbonate were added to 30 ml of dimethylformamide, and with stirring at room temperature, a dimethylformamide solution (10 ml) of 13.0 g of 1-bromo-6,6-dimethyl-2-hepten-4-yne (a mixture of the E- and Z-forms in a ratio of about 3:1) was added. The mixture was stirred overnight at room temperature. After the reaction, the solvent was evaporated. The residue was extracted with ethyl acetate and a saturated aqueous sodium chloride solution. The organic layer was separated, and dried over anhydrous magnesium sulfate. The desiccant was removed by filtration, and the solvent was evaporated. The residue was purified by silica gel column chromatography (Wakogel C-200, 300 g; the eluting solvent: hexane/ethyl acetate = 10/1) to give 7.94 g (overall yield 39 %) of the captioned compound as pale yellow crystals.

When the same reaction as above is carried out using ethylamine or propylamine instead of methylamine, (E)-N-(6,6-dimethyl-2-hepten-4-ynyl)-N-ethyl-3-hydoxybenzylamine used in Examples 101 to

103 or (E)-N-(6,6-dimethyl-2-hepten-4-ynyl)-N-propyl-3-hydroxybenzylamine used in Examples 104 and 105 is obtained.

REFERENTIAL EXAMPLE 8

Production of ethyl 3-(beta-phenethyl)benzoate

863 mg of 3-ethoxycarbonylbenzyltriphenyl phosphonium bromide was suspended in 20 ml of anhydrous tetrahydrofuran, and with stirring under cooling at -30 °C, 1.6 ml of 1.53M hexane solution of butyl lithium was added dropwise. After the dropwise addition, the mixture was stirred at room temperature for 1 hour. The reaction mixture was poured into water, and then extracted with ethyl acetate. The extract was washed with a saturated aqueous sodium chloride solution and dried over anhydrous sodium sulfate. The desiccant was removed by filtration and the solvent was evaporated. The residue was purified by silica gel column chromatography (Wakogel C-200, 50 g; eluting solvent: toluene) to give 260 mg (yield 52 %) of a mixture of ethyl (E)- and (Z)-3-styrylbenzoates. Two hundred milligrams of the stilbene compound obtained above was dissolved in 2 ml of ethanol, and hydrogenated at room temperature under atmospheric pressure for 5 hours in the presence of 10 mg of a 10 % palladium-carbon catalyst. The catalyst was separated by filtration, and then the filtrate was evaporated under reduced pressure to give 200 mg (yield 99 %) of the captioned compound as a colorless oil.

REFERENTIAL EXAMPLE 9

Production of (E)-3-styrylbenzyl bromide

The mixture of ethyl (E)- and (Z)-3-styrylbenzoates obtained in Referential Example 8 was reduced by using lithium aluminum hydride in anhydrous tetrahydrofuran to give a mixture of (E)- and (Z)-3-styrylbenzyl alcohols. The mixture was separated and purified by medium-pressure liquid chromatography [column: Lobar column, size A, Lichroprep Si 60 (a product of E. Merck Co.); eluting solvent: hexane/ethyl acetate = 5/1] to obtain (E)-3-styrylbenzyl alcohol (colorless crystals; m.p. 87-88 °C) and (Z)-3-styrylbenzyl alcohol (colorless oil).

(E)-3-styrylbenzyl alcohol obtained above (105 mg) was dissolved in 5 ml of chloroform, and 80 microliters of phosphorus tribromide was added, and the mixture was stirred for 40 minutes. The reaction mixture was poured into ice water, and extracted with chloroform. The extract was dried over anhydrous sodium sulfate. The desiccant was removed by filtration, and the filtrate was evaporated under reduced pressure to give 133 mg (yield 93.8 %) of the captioned compound.

By a similar method, (Z)-3-styrylbenzyl bromide, (E)-3-(o-methylstyryl)benzyl bromide, and (E)-3-[2-(1-naphthyl)vinyl]benzyl bromide are obtained.

REFERENTIAL EXAMPLE 10

Production of 3-benzylaminobenzyl alcohol hydrochloride

334 mg of 3-aminobenzaldehyde dimethyl acetal was dissolved in 10 ml of anhydrous benzene, and 212 mg of benzaldehyde and 2 g of molecular sieve 4A (a product of Nippon Chromato Kogyo Co., Ltd.) were added. The mixture was stirred at room temperature for 4 hours. The molecular sieve was removed by filtration, and the filtrate was evaporated under reduced pressure. The residue was dissolved in 20 ml of methanol, and 100 mg of sodium borohydride was added. The mixture was stirred for 2 hours. The reaction mixture was diluted with water, and extracted with ethyl ether. The extract was dried over anhydrous sodium sulfate, and the solvent was evaporated. The residue was recrystallized from hexane to give 451 mg (yield 87.7 %) of 3-benzylaminobenzaldehyde dimethyl acetal having a melting point of 55 to 56 °C.

The resulting acetal (257 mg) was added to 10 ml of 1N HCl, and the mixture was heated at 80 °C for 10 minutes. The reaction mixture was evaporated under reduced pressure. The residue was dissolved in 25 ml of 20 % hydrous methanol. With stirring, 200 mg of sodium borohydride was added. The mixture was stirred at room temperature for 1 hour. The reaction mixture was alkalified with sodium carbonate, and extracted with ethyl ether. The extract was dried over anhydrous sodium sulfate and evaporated under reduced pressure. The residue was purified by silica gel column chromatography (Wakogel C-200, 20 g; eluting solvent: hexane/ethyl acetate = 5/1), and then treated with HCl-methanol to give 222 mg (yield 89 %) of the captioned compound having a melting point of 136 to 137 °C.

REFERENTIAL EXAMPLE 11

Production of 3-(2-phenylethynyl)benzyl bromide

213 mg of 3-acetoxymethylstilbene was dissolved in 4 ml of ethyl ether, and 148 mg of bromine was added. The mixture was stirred at room temperature for 2 hours. The crystals were collected by filtration, and dried to give 253 mg (yield 73 %) of 1,2-dibromo-2-phenyl-(3-acetoxyphenyl)ethane having a melting point of 156 to 157 °C.

The resulting dibromo compound (250 mg) was dissolved in 1 ml of ethanol, and 360 mg of potassium hydroxide was added. The mixture was heated under reflux for 8 hours. The reaction mixture was diluted with water, and then extracted with ethyl ether. The extract was concentrated under reduced pressure, and the residue was purified by medium-pressure liquid chromatography [column: Lobar column, size B, Lichroprep Si 60 (a product of E. Merck Co.); eluting solvent: hexane/ethyl acetate = 3/1] to give 120 mg (yield 95 %) of 3-(2-phenylethynyl)benzyl alcohol as a colorless oil.

The resulting acetylene compound (116 mg) was dissolved in 1 ml of chloroform, and 0.1 ml of phosphorus tribromide was added. The mixture was stirred at room temperature for 1 hour. Water and chloroform were added to the reaction mixture. The organic layer was separated, washed with a saturated aqueous solution of sodium hydrogen carbonate and water, and then dried over anhydrous sodium sulfate. The desiccant was removed by filtration, and the filtrate was evaporated under reduced pressure to give 150 mg (yield 99 %) of the captioned compound as a colorles oil.

REFERENTIAL EXAMPLE 12

Production of N-methyl-3-benzyloxy-alpha-phenethylamine

1.0 g of 3-benzyloxybenzaldehyde was dissolved in 10 ml of a 40 % methanol solution of methylamine, and after 1 hour, the solution was concentrated to dryness under reduced pressure. The residue was dissolved in 20 ml of anhydrous ethyl ether, and 12 ml of a 0.89M ethyl ether solution of methyl lithium was added. The mixture was heated under reflux with stirring for 3 hours. The reaction mixture was allowed to cool, and poured into 20 ml of ice water. The organic layer was then separated and dried over anhydrous magnesium sulfate. The desiccant was removed by filtration, and the filtrate was evaporated under reduced pressure to give 800 mg (yield 70 %) of the captioned compound as a colorless oil.

REFERENTIAL EXAMPLE 13

Production of 3-anilinomethylbenzyl alcohol hydrochloride

1.34 g of isophthalaldehyde was dissolved in 20 ml of anhydrous benzene, and 0.93 g of aniline and 10 g of molecular sieve 4A (a product of Nippon Chromato Kogyo Co., Ltd.) were added. The mixture was stirred at room temperature for 4 hours. The molecular sieve was removed by filtration, and the filtrate was evaporated under reduce pressure. The residue was dissolved in 10 ml of methanol, and then 0.5 g of sodium borohydride was added. The mixture was stirred for 3 hours. The reaction mixture was diluted with water, and extracted with ethyl acetate. The extract was dried over anhydrous sodium sulfate, and the solvent was evaporated. The residue was purified by silica gel column chromatography (Wakogel C-200, 50 g; eluting solvent: hexane/ethyl acetate = 3/1) and treated with HCl-methanol to give 1.21 g (yield 48.4 %) of the captioned compound having a melting point of 124 to 126 °C.

REFERENTIAL EXAMPLE 14

Production of N-methyl-3-benzoylaminobenzylamine hydrochloride

1.06 g of 3-aminobenzaldehyde dimethyl acetal was dissolved in a mixture of 5 ml of toluene and 10 ml of water containing 0.8 g of sodium hydroxide, and 5 ml of a toluene solution of 1.3 g of benzoyl chloride was added. The mixture was stirred at room temperature for 2 hours. The organic layer was separated, and evaporated under reduced pressure. The residue was dissolved in a mixture of 10 ml of methanol and 10 ml of 10 % hydrochloric acid, and heated at 90 °C for 30 minutes. Water (20 ml) was added to the reaction mixture to allow it to cool. There was obtained 1.16 g (yield 87.3 %) of 3-benzoylaminobenzaldehyde as colorless needles having a melting point of 122 to 123 °C.

The above aldehyde (314 mg) was dissolved in 5 ml of a 40 % methanol solution of methylamine, and the solution was left to stand at room temperature. Sodium borohydride (110 mg) was added, and the mixture was stirred for 2 hours. The reaction mixture was diluted with water and concentrated under reduced pressure to remove methanol. Ethyl ether was added to the residue to extract the product. The ether layer was washed with a saturated aqueous sodium chloride solution and dried over anhydrous sodium sulfate. The solvent was evaporated, and the residue was treated with HCl/methanol to give 305 mg (yield 79.1 %) of the captioned compound as colorless needles having a melting point of 213 to 214 °C.

When a similar reaction is carried out by using 3-(N-phenylcarbamoyl)benzaldehyde obtained by condensation of 3-formylbenzoyl chloride and aniline, N-methyl-3-(N-phenylcarbamoyl)benzylamine hydrochloride can be synthesized.

REFERENTIAL EXAMPLE 15

Production of N-methyl-3-furfuryloxybenzylamine hydrochloride

Two grams of furfuryl alcohol was dissolved in 20 ml of anhydrous ethyl ether, and 3 ml of an anhydrous ethyl ether solution containing 2 g of phosphorus tribromide was added. The mixture was stirred for 30 minutes under ice cooling. The reaction mixture was washed with a 30 % aqueous solution of sodium hydroxide, and dried over granular sodium hydroxide. The solution was then added to a previously prepared solution of 3-hydroxybenzaldehyde sodium salt (obtained by dissolving 2.44 g of 3-hydroxybenzaldehyde and 0.8 g of 60 % oily sodium hydride in a mixture of 5 ml of anhydrous dimethylformamide and 10 ml of anhydrous ethyl ether), and the mixture was stirred at room temperature for 1 hour. To the reaction mixture were added isopropyl ether and water. The organic layer was separated, washed with a 10 % aqueous solution of sodium hydroxide, and then dried over anhydrous sodium sulfate. The desiccant was removed by filtration, and the solvent was evaporated. The residue was purified by silica gel column chromatography (Wakogel C-200, 100 g; eluting solvent: hexane/ethyl acetate = 3/1) to give 3-furfuryloxybenzaldehyde as a pale yellow oil.

The resulting furfuryloxy compound (0.5 g) was reductively aminated in the same method as in Referential Example 2 to give 0.38 g (yield 60 %) of the captioned compound as a crystalline powder having a melting point of 144 to 146 °C.

REFERENTIAL EXAMPLE 16

Production of 2-benzyloxy-6-chloromethylpyridine

0.80 g of 6-chloro-2-picoline 1-oxide was added to an alcoholate solution prepared from 0.33 g of 60 % oily sodium hydride, 0.69 ml of benzyl alcohol and 5 ml of dimethyl sulfoxide. The mixture was stirred overnight at room temperature. 1N-HCl (1.7 ml) was added to the reaction mixture and then water and chloroform were added. The organic layer was separated, and dried over anhydrous sodium sulfate. The dessicant was removed by filtration, and the filtrate was evaporated under reduced pressure. The residue was purified by silica gel column chromatography (Wakogel C-200, 100 g; eluting solvent: chloroform/methanol = 80/1 → 40/1) to give 0.64 g (yield 54 %) of 6-benzyloxy-2-picoline 1-oxide.

The resulting benzyloxy compound (0.42 g) was dissolved in 12.5 ml of acetic anhydride. The mixture was heated under reflux for 1 hour, and then concentrated under reduced pressure. Water and chloroform were added to the residue to extract the product. The organic layer was separated, and dried over anhydrous magnesium sulfate. The dessicant was removed by filtration, and the filtrate was evaporated under reduced pressure, and the residue was purified by silica gel column chromatography (Wakogel C-200, 40 g; eluting solvent: hexane/ethyl acetate = 4/1) to give 0.26 g (yield 51 %) of 2-acetoxymethyl-6-benzyloxypyridine.

The above acetoxymethyl compound (151 mg) was dissolved in 6 ml of ethanol, and 195 microliters of a 40 % aqueous solution of sodium hydroxide was added. The mixture was stirred overnight at room temperature. After the reaction, the solvent was evaporated under reduced pressure. Water and chloroform were added to the residue to extract the product. The organic layer was separated and dried over anhydrous magnesium sulfate. The desiccant was removed by filtration, and the filtrate was evaporated under reduced pressure. The residue was dissolved in 3 ml of chloroform, and 90 microliters of thionyl chloride was added. The mixture was stirred overnight at room temperature. The reaction mixture was extracted with a saturated aqueous solution of sodium hydrogen carbonate and chloroform. The organic layer was separated, dried over anhydrous magnesium sulfate, and concentrated to dryness under reduced

pressure. The residue was purified by silica gel column chromatography (Wakogel C-100, 10 g; eluting solvent: hexane/ethyl acetate = 3/1) to give 100 mg (yield 69 %) of the captioned compound as a pale yellow oil.

By a similar method to above, the chloromethylpyridine derivatives used in Examples 93 to 95 can be synthesized.

REFERENTIAL EXAMPLE 17

Production of (E)-3-chloromethyl-5-styrylpyrazole

228 mg of (E)-5-styrylpyrazole-3-carboxylic acid [Ann., 453, 151 (1927)] was dissolved in 5 ml of anhydrous tetrahydrofuran and 68 mg of lithium aluminum hydride was added by portions. The mixture was stirred at room temperature for 1 hour. Hydrous ethyl ether was added to decompose the excess of the reducing agent, and then the mixture was extracted with a 10 % aqueous solution of citric acid and ethyl acetate. The organic layer was separated and evaporated under reduced pressure. The residue was washed with ethyl ether, dissolved in 1 ml of 10 % HCl/methanol, and again evaporated under reduced pressure. The residue was washed with ethyl ether and filtered to give 96 mg (yield 38 %) of (E)-3-hydroxymethyl-5-styrylpyrazole hydrochloride.

90 mg of the hydrochloride was dissolved in 3 ml of chloroform, and 0.5 ml of thionyl chloride was added. The mixture was heated at 50 °C for 20 hours. The reaction mixture was evaporated under reduced pressure. The residue was washed with ethyl acetate and filtered to give 60 mg (yield 82 %) of the captioned compound as a colorless crystalline powder.

REFERENTIAL EXAMPLE 18

Production of (E)- and (Z)-2-hydroxymethyl-5-styrylfurans

389 mg of benzyltriphenylphosphonium chloride was suspended in 5 ml of anhydrous tetrahydrofuran, and in a nitrogen atmosphere, 0.75 ml of a 1.59M hexane solution of n-butyl lithium at -30 °C was added. The mixture was stirred at this temperature for 30 minutes. One milliliters of a tetrahydrofuran solution of 5-hydroxymethylfurfural (126 mg) was added to the resulting solution. The mixture was heated to room temperature, and 6 ml of methylene chloride was added. The mixture was stirred overnight. After the reaction, water and chloroform were added to the reaction mixture. The organic layer was separated and dried over anhydrous magnesium sulfate. The dessicant was removed by filtration, and the filtrate was evaporated under reduced pressure. The residue was coarsely purified by silica gel column chromatography (Wakogel C-200, 5 g; eluting solvent: hexane/ethyl acetate = 3/1), and again purified by high-performance liquid chromatography (column: Senshu Pack, silica gel-5301N; eluting solvent: hexane/ethyl acetate = 3/1) to give 103 mg (yield 51 %) of the E-form and 58 mg (yield 29 %) of the Z-form of the captioned compounds as pale yellow oils.

REFERENTIAL EXAMPLE 19

Production of a mixture of of (E)- and (Z)-2-hydroxymethyl-4-styrylthiazoles

Diethyl thiazole-2,4-dicarboxylate (1.5 g) was suspended in 15 ml of ethanol, and at -10 °C, 3 ml of an ethanol solution of 0.16 g of sodium borohydride and 0.58 g of calcium chloride was added. The mixture was stirred at this temperature for 2 hours. Acetone was added to decompose the excess of the reducing agent, and then the solvent was evaporated under reduced pressure. Dilute sulfuric acid was added to the residue, and insoluble calcium sulfate was separated by filtration. The filtrate was adjusted to pH 10 with an aqueous solution of potassium carbonate, and then extracted with chloroform. The chloroform layer was dried over anhydrous magnesium sulfate, and then further concentrated under reduced pressure. The residue was treated with isopropyl ether to give 0.78 g (yield 64 %) of ethyl 2-hydroxymethylthiazole-4-carboxylate as a colorless crystalline powder.

The resulting hydroxymethyl ester (0.78 g) was dissolved in 40 ml of chloroform, and 25 g of active manganese dioxide was added. The mixture was stirred at room temperature for 5 days. The precipitate was removed by filtration, and the filtrate was evaporated under reduced pressure to give 0.67 g (yield 87 %) of ethyl 2-formylthiazole-4-carboxylate as colorless needles.

The resulting formyl compound (150 mg) was added to a phosphoran solution prepared in advance from 351 mg of benzyltriphenylphosphonium bromide, 56.9 mg of powdery sodium methoxide and 0.8 ml of methanol, and the mixture was stirred at room temperature for 2.5 hours. The insoluble precipitate was removed by filtration. The filtrate was evaporated under reduced pressure, and the residue was extracted by adding water and methylene chloride. The organic layer was separated, and dried over anhydrous magnesium sulfate. The desiccant was separated by filtration. The filtrate was evaporated under reduced pressure, and the residue was purified by silica gel column chromatography (Wakogel C-200, 24 g; eluting solvent: hexane/ethyl acetate = 5/1). The resulting mixture of ethyl (E)- and (Z)-2-styrylthiazole-4-carboxylates was dissolved in 4 ml of ethanol. To the solution was added at -5 °C, 1.2 ml of an ethanol solution of 29 mg of sodium borohydride and 100 mg of calcium chloride. The temperature of the mixture was returned to room temperature, and it was stirred overnight at this temperature. Acetone was added to decompose the excess of the reducing agent, and then the solvent was evaporated under reduced pressure. Dilute sulfuric acid was added to the residue, and insoluble calcium sulfate was removed by filtration. Then, potassium carbonate was added to the filtrate to adjust its pH to 10. The solution was concentrated under reduced pressure, and the residue was purified by silica gel column chromatography (Wakogel C-200, 15 g; eluting solvent: hexane/ethyl acetate = 2/1 → 1/1) to give 80 mg (yield 63 %) of the captioned mixture as a pale yellow oil.

REFERENTIAL EXAMPLE 20

Production of (E)-2-hydroxymethyl-5-styryloxazole

150 mg of ethyl (E)-5-styryloxazole-2-carboxylate [Yakugaku Zasshi, vol. 91, page 425 (1971)] was suspended in 15 ml of ethanol, and at -10 °C, 5 ml of an ethanol solution of 35 mg of sodium borohydride and 123 mg of calcium chloride was added, and the mixture was stirred at room temperature for 3 hours. The reaction mixture was concentrated under reduced pressure, and water and ethyl ether were added to the residue to perform extraction. The organic layer was separated, and then dried over anhydrous magnesium sulfate. The dessicant was removed by filtration and the filtrate was concentrated under reduced pressure to give 120 mg (yield 97 %) of the captioned compound as a colorless oil.

REFERENTIAL EXAMPLE 21

Production of 3-isopropenylbenzyl methanesulfonate

Methyl 3-formylbenzoate (0.67 g) was dissolved in 10 ml of methanol, and 0.74 g of methyl orthoformate and 8 mg of p-toluenesulfonic acid were added. The mixture was stirred at room temperature for 10 hours. Sodium methoxide was added to the reaction mixture to make it basic, and then the solvent was evaporated. The residue was dissolved in ethyl acetate, and washed with a 5 % aqueous solution of sodium hydrogen carbonate. The solvent was then evaporated to give 0.75 g of methyl 3-formylbenzoate dimethyl acetal.

0.6 g of the acetal obtained above was dissolved in 12 ml of anhydrous tetrahydrofuran, and with stirring at -20 °C, 3.6 ml of a 2N hexane solution of methyl magnesium bromide was added, and the mixture was stirred for 2 hours. A saturated aqueous solution of ammonium chloride was added to the reaction mixture to terminate the reaction. Ethyl acetate was added to the reaction mixture to perform extraction. The extract was worked up in a customary manner to give 0.48 g of crude 3-(1-hydroxy-1-methylethyl)benzaldehyde dimethyl acetal.

0.4 g of the alcohol compound obtained above was dissolved in 8 ml of purified methylene chloride, and with stirring under ice cooling, 0.24 g of methanesulfonyl chloride and 0.38 g of triethylamine were added. The mixture was stirred at room temperature for 2 hours. The reaction mixture was evaporatd under reduced pressure and then extracted by adding ethyl ether and water. The extract was worked up in a customary manner to give 0.35 g of crude 3-isopropenylbenzaldehyde dimethyl acetal as a pale yellow oil.

0.30 g of the isopropenyl compound obtained above was dissolved in 3 ml of 50 % hydrous trifluoroacetic acid, and the solution was left to stand for 1 hour at room temperature. The solvent was evaporated, and methylene chloride and water were added to the residue to perform extraction, and the extract was worked up in a customary manner to give 0.28 g of crude 3-isopropenylbenzaldehyde.

0.25 g of the resulting aldehyde compound was dissolved in 5 ml of ethanol, and 80 mg of sodium borohydride was added. The mixture was stirred at room temperature for 1 hour. The reaction mixture was concentrated under reduced pressure, and extracted by adding ethyl ether and water. The extract was

worked up in a customary manner, and the product was purified by medium-pressure liquid chromatography [column: Lobar column, size A, Lichroprep Si 60F (a product of E. Merck Co.); eluting solvent: hexane/ethyl acetate = 10/1 → 5/1] to give 0.07 g (overall yield from 3-formylbenzoic acid 23 %) of 3-isopropenylbenzyl alcohol as a colorless oil.

50 mg of the resulting alcohol compound was dissolved in 1 ml of chloroform, and with stirring under ice cooling, 43 mg of methanesulfonyl chloride and 69 mg of triethylamine were added. The mixture was stirred at the above temperature for 2 hours. The reaction mixture was distilled under reduced pressure, and the residue was extracted by adding ethyl ether and a saturated aqueous sodium chloride solution. The extract was worked up in a customary manner to give 75 mg of the captioned compound as a pale yellow oil.

REFERENTIAL EXAMPLE 22

Production of 3-(3-methyl-2-butenyl)benzyl bromide

Magnesium metal (1.2 g) was added to 2 ml of tetrahydrofuran. With stirring at room temperature, 20 ml of a solution of 2.0 g of 3-bromobenzaldehyde dimethyl acetal in a 1:1 mixture of tetrahydrofuran and ethyl ether was added dropwise over the course of 1 hour. With stirring under ice cooling, 15 ml of a tetrahydrofuran solution of 2.25 g of 3-methyl-2-butenyl bromide was added to the resulting solution. The temperature of the mixture was returned to room temperature, and then it was stirred for 1 hour. A saturated aqueous solution of ammonium chloride was added to the reaction mixture to terminate the reaction. Ethyl ether was added, and the reaction mixture was treated in a customary manner to give 1.71 g (yield 90 %) of 3-(3-methyl-2-butenyl)benzaldehyde dimethyl acetal as a colorless oil.

250 mg of the acetal compound obtained above was dissolved in a mixture of 20 ml of tetrahydrofuran and 5 ml of water, and 1 ml of 35 % hydrochloric acid was added. The mixture was stirred at 50 °C for 1 hour. The reaction mixture was diluted with water and ethyl ether, and worked up in a customary manner to give 188 mg (yield 45 %) of 3-(3-methyl-2-butenyl)benzaldehyde.

170 mg of the aldehyde compound was dissolved in 20 ml of ethanol, and 100 mg of sodium borohydride was added. The mixture was stirred at room temperature for 1 hour. Water and ethyl ether were added to the reaction mixture to dilute it, and it was then worked up in a customary manner to give 158 mg (yield 92 %) of 3-(3-methyl-2-butenyl)benzyl alcohol.

130 mg of the resulting alcohol compound was dissolved in 20 ml of ethyl ether, and 2 ml of pyridine and 270 mg of phosphorus tribromide were added. The mixture was stirred for 1 hour under ice cooling. The reaction mixture was poured into ice water, and ethyl ether was added. The mixture was worked up in a customary manner to give 109 mg (yield 62 %) of the captioned compound as a pale yellow oil.

The 3-(2-methyl-1-propenyl)benzyl bromide and 3-(2-methyl-2-propenyl)benzyl bromide used in Examples 53 and 54 can be formed by substantially the same methods as in Referential Examples 21 and 22.

REFERENTIAL EXAMPLE 23

Production of 2-(2-furyl)benzyl chloride

Ethyl 3-(2-furyl)benzoate [J. Chem. Soc., (B), 1971, 2305] was dissolved in 5 ml of anhydrous ethyl ether, and with stirring under ice cooling, 23 mg of lithium aluminum hydride was added. The mixture was stirred for 40 minutes. After the reaction, the reaction mixture was extracted by adding water and ethyl ether. The extract was worked up in a customary manner to give 170 mg (yield 83 %) of 3-(2-furyl)benzyl alcohol.

160 mg of the resulting alcohol compound was dissolved in 3 ml of anhydrous ethyl ether. Thionyl chloride (73 microliters) was added, and under ice coolng, the mixture was stirred for 3 hours. After the reaction, the reaction mixture was washed with a saturated aqueous solution of sodium chloride and 5 % sodium hydrogen carbonate to give an ethyl ether solution of the captioned compound. It was used directly in the reaction of Example 58.

REFERENTIAL EXAMPLE 24

Production of 3-(2-oxazolyl)benzyl bromide

200 mg of 3-(2-ozazolyl)toluene [synthesized in accordance with the method described in Angew. Chem., 75, 165 (1963)] was dissolved in 5 ml of carbon tetrachloride, and 231 mg of N-bromosuccinimide and a catalytic amount of benzoyl peroxide were added. The mixture was refluxed with stirring for 2 hours. The insoluble material was removed after the reaction, and the solvent was evaporated to give the captioned compound.

By a similar method, 3-(2-thiazolyl)benzyl bromide used in Example 60 can be synthesized.

REFERENTIAL EXAMPLE 25

Production of 3-(1-pyrrolyl)benzyl methanesulfonate

Ethyl m-aminobenzoate (1.6 g) was dissolved in 10 ml of glacial acetic acid, and 1.3 g of 2,5-dimethoxytetrahydrofuran was added. The mixture was refluxed for 2 hours, and the solvent was evaporated. The residue was dissolved in a mixture of ethyl acetate and water. The organic layer was separated, and then worked up in a customary manner. Finally, recrystallization from hexane gave 1.7 g (yield 82 %) of ethyl 3-(1-pyrrolyl)benzoate as colorless needles having a melting point of 64 to 65 °C. The resulting pyrrolyl compound (1.1 g) was dissolved in 30 ml of ethyl ether, and with stirring under ice cooling, 0.2 g of lithium aluminum hydride was added. The mixture was stirred for 1 hour. The reaction mixture was extracted by adding water and ethyl ether. The extract was worked up in a customary manner. Recrystallization from a mixture of ethyl acetate and hexane gave 0.80 g (yield 91 %) of 3-(1-pyrrolyl)benzyl alcohol as colorless needles having a melting point of 66 to 68 °C.

170 mg of the resulting alcohol compound was dissolved in 10 ml of methylene chloride, and 120 mg of methanesulfonyl chloride and 150 mg of triethylamine were added. The mixture was stirred for 1 hour under ice cooling. The reaction mixture was washed with water, and dried over anhydrous magnesium sulfate. The solvent was evaporated to give 230 mg (yield 92 %) of the captioned compound as a pale yellow oil.

By a similar method, 3-(5-oxazolyl)benzyl methanesulfonate used in Examples 61, 102 and 105 can be synthesized [see Chem. Pharm. Bull., 27, 793 (1979); Tetrahedron Lett., 1972 , 2369].

The compounds provided by this invention inhibit biosynthesis of cholesterol by inhibiting the squalene-epoxidase of mammals, and thus lower the blood cholesterol level. Accordingly, the compounds are expected to be useful for the treatment and prevention of diseases induced by the excess of cholesterol, such as obesity, hyperlipemia, and arteriosclerosis, and heart and brain diseases incident to them.

## Claims

**Claims for the following Contracting States : AT, BE, CH, DE, FR, GB, IT, LI, LU, NL, SE**

**1.** A substituted alkylamine derivative represented by the general formula

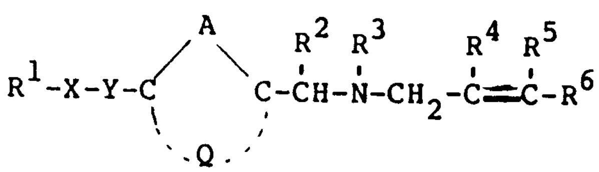 [I]

wherein A represents a methine group, a nitrogen atom, an oxygen atom or a sulfur atom; Q represents a group which may contain one or two hetero atoms selected from the group consisting of nitrogen, oxygen and sulfur atoms and forms a 5- or 6-membered aromatic ring together with the adjacent carbon atoms and A (the aromatic ring may contain one substituent, or two identical or different substituents, selected from the class consisting of halogen atoms, hydroxyl groups, lower alkyl groups and lower alkoxy groups); X and Y may be identical or different and each represents an oxygen atom, a sulfur atom, a carbonyl group, a group of the formula -CHR$^a$- in which R$^a$ represents a hydrogen atom or a lower alkyl group, or a group of the formula -NR$^b$- in which R$^b$ represents a hydrogen atom or a lower alkyl group, or when taken together, X and Y represent a vinylene or

ethynylene group; $R^1$ represents a lower alkenyl group which may be substituted, a cycloalkenyl group which may be substituted, a lower alkynyl group which may be substituted, an aryl group which may be substituted, or a heterocyclic group which may be substituted; $R^2$ represents a hydrogen atom or a lower alkyl group; $R^3$ represents a hydrogen atom, a lower alkyl group, a lower alkenyl group, a lower alkynyl group or a cycloalkyl group; $R^4$ and $R^5$ may be identical or different and each represents a hydrogen atom or a halogen atom; and $R^6$ represents an acyclic hydrocarbon group which may be substituted (which may contain 1 or 2 unsaturated bonds selected from double and triple bonds); a cycloalkyl group which may be substituted, or a phenyl group which may be substituted; provided that when either one of X and Y represents an oxygen or sulfur atom, or the group $-NR^b-$ in which $R^b$ is as defined above, the other represents a carbonyl group or the group $-CHR^a-$ in which $R^a$ is as defined above,
and a nontoxic salt thereof.

2. The substituted alkylamine derivative and its nontoxic salt of claim 1 in which $R^1$ represents an aryl or heterocyclic group which may be substituted; X represents a methylene group and Y represents an oxygen atom or an imino group, or X and Y, taken together, represent an ethylene group or an (E)-vinylene group; the aromatic ring of the formula

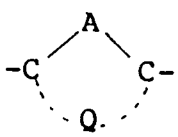

is a benzene, furan, thiophene, oxazole, isoxazole, thiazole, pyridine or pyrimidine ring; $R^2$ represents a hydrogen atom; $R^3$ represents an alkyl group having 1 to 5 carbon atoms, an alkenyl group having 3 to 5 carbon atoms, an alkynyl group having 3 to 5 carbon atoms, or a cycloalkyl group having 3 to 5 carbon atoms; $R^4$ and $R^5$ each represent a hydrogen atom, and the double bond formed by the two carbon atoms to which they are bonded is of a trans-form (E-form); and $R^6$ is a group of the formula $-CH=CH-R^c$ in which $R^c$ represents an alkyl or alkenyl group having 3 to 6 carbon atoms which may be substituted by one alkoxy group having 1 to 4 carbon atoms, or a cycloalkyl group having 3 to 6 carbon atoms which may be substituted by 1 or 2 alkyl groups having 1 to 4 carbon atoms, or a group of the formula $-C\equiv C-R^c$ in which $R^c$ is as defined above.

3. The substituted alkylamine derivative and its nontoxic salt of claim 2 in which $R^1$ is an aryl group or an aromatic heterocyclic group, which may have one substituent, or two identical or different substituents, selected from the group consisting of halogen atoms and cyano, formyl, hydroxymethyl, lower alkyl, lower alkenyl, lower alkynyl, lower alkoxy, lower alkenyloxy, phenyl, furyl, thienyl, oxazolyl, thiazolyl, isoxazolyl, isothiazolyl, pyrrolyl, imidazolyl, furazanyl, pyridyl, pyrazinyl, pyrimidinyl, pyridazinyl, 1,3,5-triazinyl, 1,3,4-oxadiazolyl and 1,3,4-thiadiazolyl groups.

4. The substituted alkylamine derivative and its nontoxic salt of claim 3 in which the aromatic heterocyclic group is a 5- to 12-membered aromatic heterocyclic group containing 1 to 3 hetero atoms selected from the group consisting of nitrogen, oxygen and sulfur atoms.

5. The substituted alkylamine derivative and its nontoxic salt of claim 2 in which $R^1$ is a phenyl, naphthyl, furyl, thienyl, pyridyl, oxazolyl, thiazolyl, isoxazolyl, isothiazolyl, pyrazinyl, pyrimidinyl, pyridazinyl, 1,3,5-triazinyl, benzofuranyl, benzothienyl, benzoxazolyl, benzothiazolyl or benzofurazanyl group which may contain one substituent, or identical or different two substituents, selected from the class consisting of halogen atoms and hydroxyl, cyano, formyl, hydroxymethyl, $C_1$-$C_3$ alkyl, $C_3$-$C_5$ alkenyl, $C_3$-$C_5$ alkynyl, $C_1$-$C_3$ alkoxy, $C_3$-$C_5$ alkenyloxy, phenyl, furyl, thienyl, oxazolyl, thiazolyl, isoxazolyl, pyrrolyl and imidazolyl groups.

6. The substituted alkylamine derivative and its nontoxic salt of claim 2 in which $R^1$ is a 2-methylphenyl, 2-fluorophenyl, 3-cyanophenyl, 3-(2-furyl)phenyl, 3-(2-thienyl)phenyl, 3-(5-oxazolyl)phenyl, 3-(1-pyrrolyl)phenyl, 3-(1-imidazolyl)phenyl or 3-(5-thiazolyl)phenyl group.

7. The substituted alkylamine derivative and its nontoxic salt of claim 1 in which $R^3$ represents an alkyl group having 1 to 3 carbon atoms, an allyl group, a propargyl group or a cyclopropyl group; and $R^6$ represents a group of the formula

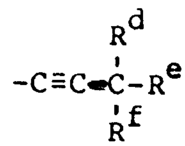

in which $R^d$ represents a hydrogen atom, an alkyl group having 1 to 4 carbon atoms or an alkoxy group having 1 to 4 carbon atoms, and $R^e$ and $R^f$ may be identical or different and each represents a methyl or ethyl group, or taken together, they may represents a cyclopropyl group.

8. The substituted alkylamine derivative and its nontoxic salt of claim 7 in which $R^3$ represents a methyl, ethyl or propyl group.

9. A process for producing a substituted alkylamine derivative of general formula [I] set forth in claim 1 and its nontoxic salt, which comprises

(a) reacting a substituted amine derivative represented by the general formula

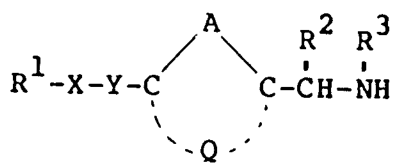

[II]

wherein A, Q, X, Y, $R^1$, $R^2$ and $R^3$ are as defined in claim 1,

or its properly protected derivative with a compound represented by the general formula

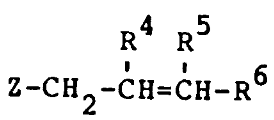

[III]

wherein Z represents a leaving group, and $R^4$, $R^5$ and $R^6$ are as defined in claim 1,

or its properly protected derivative, and thereafter, as required, removing the protective group, or

(b) reacting a compound represented by the general formula

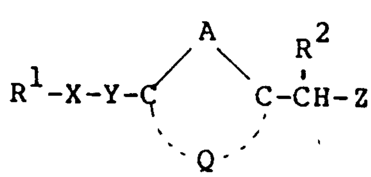

[IV]

wherein A, Q, X, Y, $R^1$, $R^2$ and Z are as defined above,

or its properly protected derivative with a substituted amine represented by the general formula

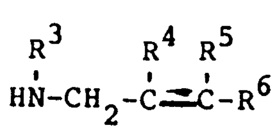

[V]

wherein $R^3$, $R^4$, $R^5$ and $R^6$ are as defined above, o rits properly protected derivative, and thereafter, as required, removing the protective group,

and, as required, converting the resulting compound into a nontoxic salt.

**10.** A process for producing a substituted alkylamine derivative represented by the general formula

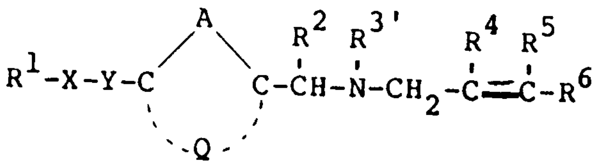

[$I^b$]

wherein A, Q, X, Y, $R^1$, $R^2$, $R^4$, $R^5$ and $R^6$ are as defined in claim 1, and $R^{3'}$ represents a lower alkyl group, a lower alkenyl group, a lower alkynyl group or a cycloalkyl group,
and its nontoxic salt, which comprises reacting a substituted alkylamine derivative represented by the general formula

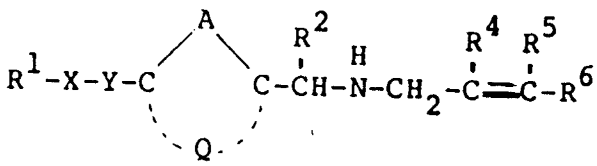

[$I^a$]

wherein A, Q, X, Y, $R^1$, $R^2$, $R^4$, $R^5$ and $R^6$ are as defined above,
or its properly protected compound with a compound represented by the general formula

$Z-R^{3'}$ [VI]

wherein Z represents a leaving group and $R^{3'}$ is as defined above,
then, as required, removing the protective group, and as required, converting the resulting compound into a non-toxic salt.

**11.** A process for producing a substituted alkylamine derivative represented by the general formula

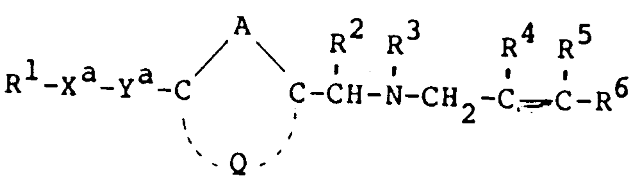

[$I^c$]

wherein A, Q, $R^1$, $R^2$, $R^3$, $R^4$, $R^5$ and $R^6$ are as defined in claim 1, $X^a$ represents a carbonyl group or a group of the formula $-CHR^a-$ in which $R^a$ represents a hydrogen atom or a lower alkyl group, and $Y^a$ represents an oxygen or sulfur atom or a group of the formula $-NR^b-$ in which $R^b$ represents a hydrogen atom or a lower alkyl group,
or its nontoxic salt, which comprises reacting a compound represented by the general formua

$R^1-X^a-Z$ [VII]

wherein Z represents a leaving group and $R^1$ and $X^a$ are as defined above,
or its properly proteced derivative with a substituted amine derivative represented by the general formula

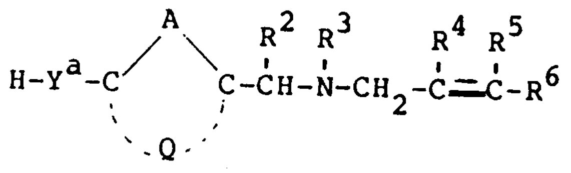

[VIII]

wherein A, Q, $R^2$, $R^3$, $R^4$, $R^5$, $R^6$ and $Y^a$ are as defined, or its properly protected compound, then, as required, removing the protective group, and then, as required, converting the resulting compound into a nontoxic salt.

**12.** A process for producing a substituted alkylamine derivative represented by the general formula

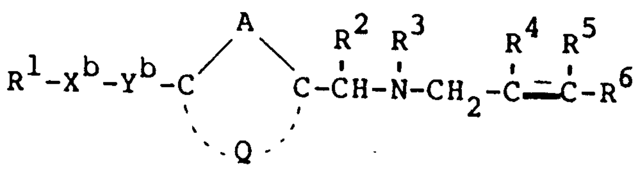

[$I^d$]

in which A, Q, $R^1$, $R^2$, $R^3$, $R^4$, $R^5$ and $R^6$ are as defined in claim 1, $X^b$ represents an oxygen or sulfur atom, or a group of the formula $-NR^b-$ in which $R^b$ represents a hydrogen atom or a lower alkyl group, and $Y^b$ represents a carbonyl group or a group of the formula $-CHR^a-$ in which $R^a$ represents a hydrogen atom or a lower alkyl group,
and its nontoxic salt, which comprises reacting a compound represented by the general formula

$R^1-X^b-H$ [IX]

wherein $R^1$ and $X^b$ are as defined above,
or its properly protected derivative with a substituted amine derivative represented by the general formula

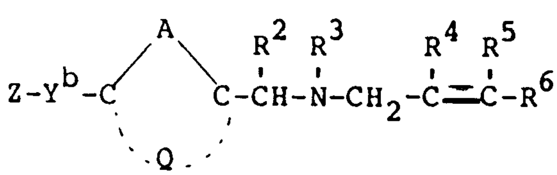

[X]

wherein A, Q, $R^2$, $R^3$, $R^4$, $R^5$, $R^6$ and $Y^b$ are as defined above, and Z represents a leaving group, or its properly protected compound, and, as required, removing the protecting group, and then, as required, converting the resulting compound into a nontoxic salt.

**13.** A medicament containing a compound of general formula [I] according to claim 1 or a pharmaceutically acceptable salt thereof.

**14.** An agent for treating hypercholesterolemia, hyperlipemia or arteriosclerosis, comprising a compound of general formula [I] or a pharmaceutically acceptable salt thereof.

**15.** A pharmaceutical preparation comprising an effective amount of a compound of general formula [I] according to claim 1 or a pharmaceutically acceptable salt thereof and a pharmaceutically acceptable carrier or diluent.

**16.** A squalene oxidase inhibitor comprising a compound of general formula [I] of claim 1 or a pharmaceutically acceptable salt thereof.

**Claims for the following Contracting State : ES**

1. A process for producing a substituted alkylamine derivative of the general formula

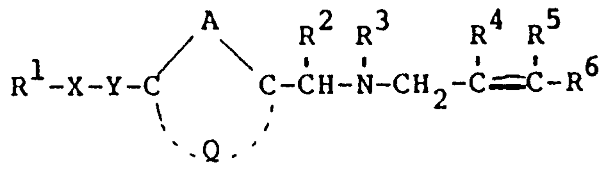

[I]

wherein A represents a methine group, a nitrogen atom, an oxygen atom or a sulfur atom; Q represents a group which may contain one or two hetero atoms selected from the group consisting of nitrogen, oxygen and sulfur atoms and forms a 5- or 6-membered aromatic ring together with the adjacent carbon atoms and A (the aromatic ring may contain one substituent, or two identical or different substituents, selected from the class consisting of halogen atoms, hydroxyl groups, lower alkyl groups and lower alkoxy groups); X and Y may be identical or different and each represents an oxygen atom, a sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group, or when taken together, X and Y represent a vinylene or ethynylene group; $R^1$ represents a lower alkenyl group which may be substituted, a cycloalkenyl group which may be substituted, a lower alkynyl group which may be substituted, an aryl group which may be substituted, or a heterocyclic group which may be substituted; $R^2$ represents a hydrogen atom or a lower alkyl group; $R^3$ represents a hydrogen atom, a lower alkyl group, a lower alkenyl group, a lower alkynyl group or a cycloalkyl group; $R^4$ and $R^5$ may be identical or different and each represents a hydrogen atom or a halogen atom; and $R^6$ represents an acyclic hydrocarbon group which may be substituted (which may contain 1 or 2 unsaturated bonds selected from double and triple bonds); a cycloalkyl group which may be substituted, or a phenyl group which may be substituted; provided that when either one of X and Y represents an oxygen or sulfur atom, or the group $-NR^b-$ in which $R^b$ is as defined above, the other represents a carbonyl group or the group $-CHR^a-$ in which $R^a$ is as defined above,
and a nontoxic salt thereof, which comprises
(a) reacting a substituted amine derivative represented by the general formula

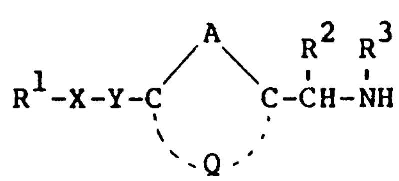

[II]

wherein A, Q, X, Y, $R^1$, $R^2$ and $R^3$ are as defined above,
or its properly protected derivative with a compound represented by the general formula

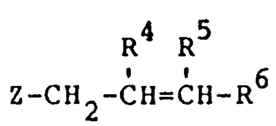

[III]

wherein Z represents a leaving group, and $R^4$, $R^5$ and $R^6$ are as defined above,
or its properly protected derivative, and thereafter, as required, removing the protective group, or

(b) reacting a compound represented by the general formula

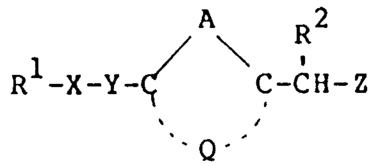

[IV]

wherein A, Q, X, Y, $R^1$, $R^2$ and Z are as defined above,
or its properly protected derivative with a substituted amine represented by the general formula

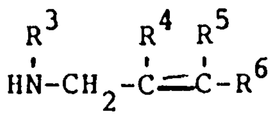

[V]

wherein $R^3$, $R^4$, $R^5$ and $R^6$ are as defined above, o rits properly protected derivative, and thereafter, as required, removing the protective group,
and, as required, converting the resulting compound into a nontoxic salt.

**2.** A process for producing a substituted alkylamine derivative represented by the general formula

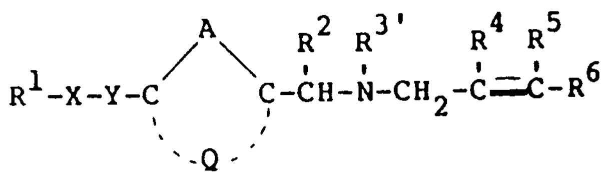

$[I^b]$

wherein A, Q, X, Y, $R^1$, $R^2$, $R^4$, $R^5$ and $R^6$ are as defined in claim 1, and $R^{3'}$ represents a lower alkyl group, a lower alkenyl group, a lower alkynyl group or a cycloalkyl group,
and its nontoxic salt, which comprises reacting a substituted alkylamine derivative represented by the general formula

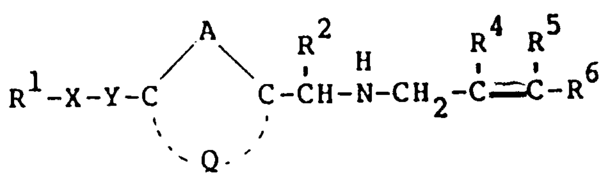

$[I^a]$

wherein A, Q, X, Y, $R^1$, $R^2$, $R^4$, $R^5$ and $R^6$ are as defined above,
or its properly protected compound with a compound represented by the general formula

$Z\text{-}R^{3'}$ [VI]

wherein Z represents a leaving group and $R^{3'}$ is as defined above,
then, as required, removing the protective group, and as required, converting the resulting compound into a non-toxic salt.

**3.** A process for producing a substituted alkylamine derivative represented by the general formula

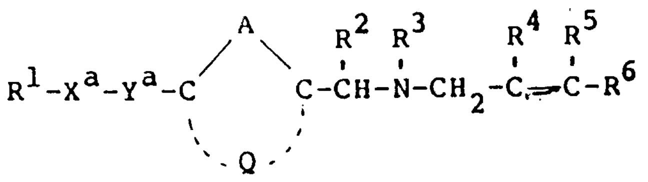
[$I^c$]

wherein A, Q, $R^1$, $R^2$, $R^3$, $R^4$, $R^5$ and $R^6$ are as defined in claim 1, $X^a$ represents a carbonyl group or a group of the formula $-CHR^a-$ in which $R^a$ represents a hydrogen atom or a lower alkyl group, and $Y^a$ represents an oxygen or sulfur atom or a group of the formula $-NR^b-$ in which $R^b$ represents a hydrogen atom or a lower alkyl group,
or its nontoxic salt, which comprises reacting a compound represented by the general formua

$R^1-X^a-Z$ [VII]

wherein Z represents a leaving group and $R^1$ and $X^a$ are as defined above,
or its properly proteced derivative with a substituted amine derivative represented by the general formula

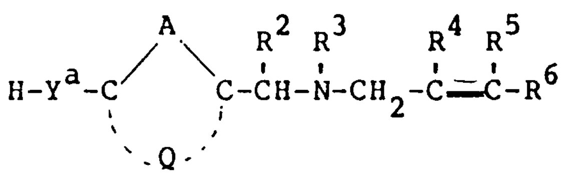
[VIII]

wherein A, Q, $R^2$, $R^3$, $R^4$, $R^5$, $R^6$ and $Y^a$ are as defined,
or its properly protected compound, then, as required, removing the protective group, and then, as required, converting the resulting compound into a nontoxic salt.

**4.** A process for producing a substituted alkylamine derivative represented by the general formula

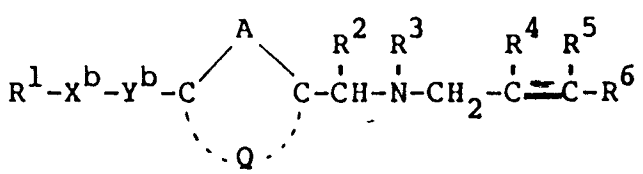
[$I^d$]

in which A, Q, $R^1$, $R^2$, $R^3$, $R^4$, $R^5$ and $R^6$ are as defined in claim 1, $X^b$ represents an oxygen or sulfur atom, or a group of the formula $-NR^b-$ in which $R^b$ represents a hydrogen atom or a lower alkyl group, and $Y^b$ represents a carbonyl group or a group of the formula $-CHR^a-$ in which $R^a$ represents a hydrogen atom or a lower alkyl group,
and its nontoxic salt, which comprises reacting a compound represented by the general formula

$R^1-X^b-H$ [IX]

wherein $R^1$ and $X^b$ are as defined above,
or its properly protected derivative with a substituted amine derivative represented by the general formula

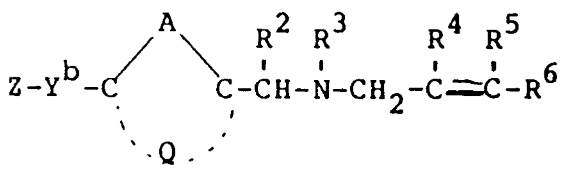 [X]

wherein A, Q, $R^2$, $R^3$, $R^4$, $R^5$, $R^6$ and $Y^b$ are as defined above, and Z represents a leaving group, or its properly protected compound, and, as required, removing the protecting group, and then, as required, converting the resulting compound into a nontoxic salt.

**5.** The process of claims 1 to 4 for the production of compounds in which $R^1$ represents an aryl or heterocyclic group which may be substituted; X represents a methylene group and Y represents an oxygen atom or an imino group, or X and Y, taken together, represent an ethylene group or an (E)-vinylene group; the aromatic ring of the formula

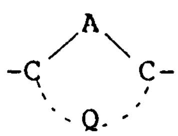

is a benzene, furan, thiophene, oxazole, isoxazole, thiazole, pyridine or pyrimidine ring; $R^2$ represents a hydrogen atom; $R^3$ represents an alkyl group having 1 to 5 carbon atoms, an alkenyl group having 3 to 5 carbon atoms, an alkynyl group having 3 to 5 carbon atoms, or a cycloalkyl group having 3 to 5 carbon atoms; $R^4$ and $R^5$ each represent a hydrogen atom, and the double bond formed by the two carbon atoms to which they are bonded is of a trans-form (E-form); and $R^6$ is a group of the formula $-CH=CH-R^c$ in which $R^c$ represents an alkyl or alkenyl group having 3 to 6 carbon atoms which may be substituted by one alkoxy group having 1 to 4 carbon atoms, or a cycloalkyl group having 3 to 6 carbon atoms which may be substituted by 1 or 2 alkyl groups having 1 to 4 carbon atoms, or a group of the formula $-C \equiv C-R^c$ in which $R^c$ is as defined above.

**6.** The process of claim 5 for the production of compounds in which $R^1$ is an aryl group or an aromatic heterocyclic group, which may have one substituent, or two identical or different substituents, selected from the group consisting of halogen atoms and cyano, formyl, hydroxymethyl, lower alkyl, lower alkenyl, lower alkynyl, lower alkoxy, lower alkenyloxy, phenyl, furyl, thienyl, oxazolyl, thiazolyl, isoxazolyl, isothiazolyl, pyrrolyl, imidazolyl, furazanyl, pyridyl, pyrazinyl, pyrimidinyl, pyridazinyl, 1,3,5-triazinyl, 1,3,4-oxadiazolyl and 1,3,4-thiadiazolyl groups.

**7.** The process of claim 6 for the production of compounds in which the aromatic heterocyclic group is a 5- to 12-membered aromatic heterocyclic group containing 1 to 3 hetero atoms selected from the group consisting of nitrogen, oxygen and sulfur atoms.

**8.** The process of claim 5 for the production of compounds in which $R^1$ is a phenyl, naphthyl, furyl, thienyl, pyridyl, oxazolyl, thiazolyl, isoxazolyl, isothiazolyl, pyrazinyl, pyrimidinyl, pyridazinyl, 1,3,5-triazinyl, benzofuranyl, benzothienyl, benzoxazolyl, benzothiazolyl or benzofurazanyl group which may contain one substituent, or identical or different two substituents, selected from the class consisting of halogen atoms and hydroxyl, cyano, formyl, hydroxymethyl, $C_1$-$C_3$ alkyl, $C_3$-$C_5$ alkenyl, $C_3$-$C_5$ alkynyl, $C_1$-$C_3$ alkoxy, $C_3$-$C_5$ alkenyloxy, phenyl, furyl, thienyl, oxazolyl, thiazolyl, isoxazolyl, pyrrolyl and imidazolyl groups.

**9.** The process of claim 5 for the production of compounds in which $R^1$ is a 2-methylphenyl, 2-fluorophenyl, 3-cyanophenyl, 3-(2-furyl)phenyl, 3-(2-thienyl)phenyl, 3-(5-oxazolyl)phenyl, 3-(1-pyrrolyl)-phenyl, 3-(1-imidazolyl)phenyl or 3-(5-thiazolyl)phenyl group.

**10.** The process of claims 1 to 4 for the production of compounds in which $R^3$ represents an alkyl group having 1 to 3 carbon atoms, an allyl group, a propargyl group or a cyclopropyl group; and $R^6$ represents a group of the formula

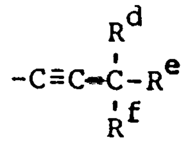

in which $R^d$ represents a hydrogen atom, an alkyl group having 1 to 4 carbon atoms or an alkoxy group having 1 to 4 carbon atoms, and $R^e$ and $R^f$ may be identical or different and each represents a methyl or ethyl group, or taken together, they may represents a cyclopropyl group.

**11.** The process of claim 10 for the production of compounds in which $R^3$ represents a methyl, ethyl or propyl group.

**Claims for the following Contracting State : GR**

**1.** A substituted alkylamine derivative represented by the general formula

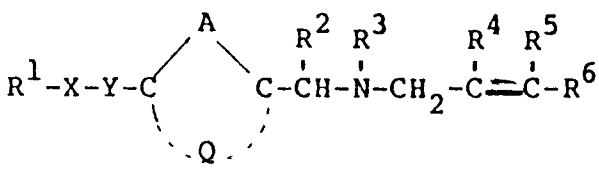

[I]

wherein A represents a methine group, a nitrogen atom, an oxygen atom or a sulfur atom; Q represents a group which may contain one or two hetero atoms selected from the group consisting of nitrogen, oxygen and sulfur atoms and forms a 5- or 6-membered aromatic ring together with the adjacent carbon atoms and A (the aromatic ring may contain one substituent, or two identical or different substituents, selected from the class consisting of halogen atoms, hydroxyl groups, lower alkyl groups and lower alkoxy groups); X and Y may be identical or different and each represents an oxygen atom, a sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group, or when taken together, X and Y represent a vinylene or ethynylene group; $R^1$ represents a lower alkenyl group which may be substituted, a cycloalkenyl group which may be substituted, a lower alkynyl group which may be substituted, an aryl group which may be substituted, or a heterocyclic group which may be substituted; $R^2$ represents a hydrogen atom or a lower alkyl group; $R^3$ represents a hydrogen atom, a lower alkyl group, a lower alkenyl group, a lower alkynyl group or a cycloalkyl group; $R^4$ and $R^5$ may be identical or different and each represents a hydrogen atom or a halogen atom; and $R^6$ represents an acyclic hydrocarbon group which may be substituted (which may contain 1 or 2 unsaturated bonds selected from double and triple bonds); a cycloalkyl group which may be substituted, or a phenyl group which may be substituted; provided that when either one of X and Y represents an oxygen or sulfur atom, or the group $-NR^b-$ in which $R^b$ is as defined above, the other represents a carbonyl group or the group $-CHR^a-$ in which $R^a$ is as defined above,
and a nontoxic salt thereof.

**2.** The substituted alkylamine derivative and its nontoxic salt of claim 1 in which $R^1$ represents an aryl or heterocyclic group which may be substituted; X represents a methylene group and Y represents an oxygen atom or an imino group, or X and Y, taken together, represent an ethylene group or an (E)-vinylene group; the aromatic ring of the formula

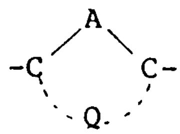

is a benzene, furan, thiophene, oxazole, isoxazole, thiazole, pyridine or pyrimidine ring; $R^2$ represents a hydrogen atom; $R^3$ represents an alkyl group having 1 to 5 carbon atoms, an alkenyl group having 3 to 5 carbon atoms, an alkynyl group having 3 to 5 carbon atoms, or a cycloalkyl group having 3 to 5 carbon atoms; $R^4$ and $R^5$ each represent a hydrogen atom, and the double bond formed by the two carbon atoms to which they are bonded is of a trans-form (E-form); and $R^6$ is a group of the formula $-CH=CH-R^c$ in which $R^c$ represents an alkyl or alkenyl group having 3 to 6 carbon atoms which may be substituted by one alkoxy group having 1 to 4 carbon atoms, or a cycloalkyl group having 3 to 6 carbon atoms which may be substituted by 1 or 2 alkyl groups having 1 to 4 carbon atoms, or a group of the formula $-C{\equiv}C-R^c$ in which $R^c$ is as defined above.

**3.** The substituted alkylamine derivative and its nontoxic salt of claim 2 in which $R^1$ is an aryl group or an aromatic heterocyclic group, which may have one substituent, or two identical or different substituents, selected from the group consisting of halogen atoms and cyano, formyl, hydroxymethyl, lower alkyl, lower alkenyl, lower alkynyl, lower alkoxy, lower alkenyloxy, phenyl, furyl, thienyl, oxazolyl, thiazolyl, isoxazolyl, isothiazolyl, pyrrolyl, imidazolyl, furazanyl, pyridyl, pyrazinyl, pyrimidinyl, pyridazinyl, 1,3,5-triazinyl, 1,3,4-oxadiazolyl and 1,3,4-thiadiazolyl groups.

**4.** The substituted alkylamine derivative and its nontoxic salt of claim 3 in which the aromatic heterocyclic group is a 5- to 12-membered aromatic heterocyclic group containing 1 to 3 hetero atoms selected from the group consisting of nitrogen, oxygen and sulfur atoms.

**5.** The substituted alkylamine derivative and its nontoxic salt of claim 2 in which $R^1$ is a phenyl, naphthyl, furyl, thienyl, pyridyl, oxazolyl, thiazolyl, isoxazolyl, isothiazolyl, pyrazinyl, pyrimidinyl, pyridazinyl, 1,3,5-triazinyl, benzofuranyl, benzothienyl, benzoxazolyl, benzothiazolyl or benzofurazanyl group which may contain one substituent, or identical or different two substituents, selected from the class consisting of halogen atoms and hydroxyl, cyano, formyl, hydroxymethyl, $C_1$-$C_3$ alkyl, $C_3$-$C_5$ alkenyl, $C_3$-$C_5$ alkynyl, $C_1$-$C_3$ alkoxy, $C_3$-$C_5$ alkenyloxy, phenyl, furyl, thienyl, oxazolyl, thiazolyl, isoxazolyl, pyrrolyl and imidazolyl groups.

**6.** The substituted alkylamine derivative and its nontoxic salt of claim 2 in which $R^1$ is a 2-methylphenyl, 2-fluorophenyl, 3-cyanophenyl, 3-(2-furyl)phenyl, 3-(2-thienyl)phenyl, 3-(5-oxazolyl)phenyl, 3-(1-pyrrolyl)phenyl, 3-(1-imidazolyl)phenyl or 3-(5-thiazolyl)phenyl group.

**7.** The substituted alkylamine derivative and its nontoxic salt of claim 1 in which $R^3$ represents an alkyl group having 1 to 3 carbon atoms, an allyl group, a propargyl group or a cyclopropyl group; and $R^6$ represents a group of the formula

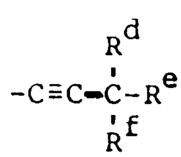

in which $R^d$ represents a hydrogen atom, an alkyl group having 1 to 4 carbon atoms or an alkoxy group having 1 to 4 carbon atoms, and $R^e$ and $R^f$ may be identical or different and each represents a methyl or ethyl group, or taken together, they may represents a cyclopropyl group.

**8.** The substituted alkylamine derivative and its nontoxic salt of claim 7 in which $R^3$ represents a methyl, ethyl or propyl group.

**9.** A process for producing a substituted alkylamine derivative of general formula [I] set forth in claim 1 and its nontoxic salt, which comprises

(a) reacting a substituted amine derivative represented by the general formula

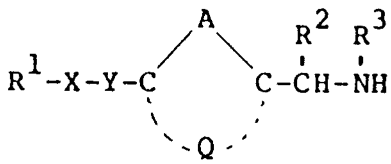

[II]

wherein A, Q, X, Y, $R^1$, $R^2$ and $R^3$ are as defined in claim 1,

or its properly protected derivative with a compound represented by the general formula

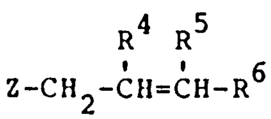

[III]

wherein Z represents a leaving group, and $R^4$, $R^5$ and $R^6$ are as defined in claim 1,

or its properly protected derivative, and thereafter, as required, removing the protective group, or

(b) reacting a compound represented by the general formula

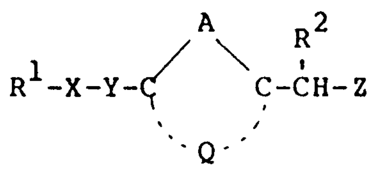

[IV]

wherein A, Q, X, Y, $R^1$, $R^2$ and Z are as defined above,

or its properly protected derivative with a substituted amine represented by the general formula

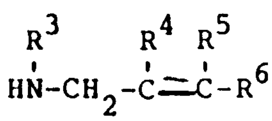

[V]

wherein $R^3$, $R^4$, $R^5$ and $R^6$ are as defined above, o rits properly protected derivative, and thereafter, as required, removing the protective group,

and, as required, converting the resulting compound into a nontoxic salt.

**10.** A process for producing a substituted alkylamine derivative represented by the general formula

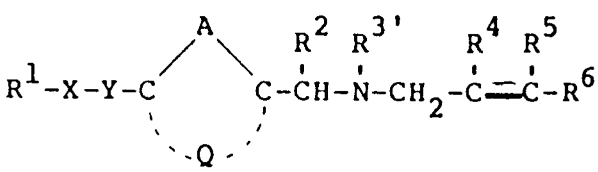

[$I^b$]

wherein A, Q, X, Y, $R^1$, $R^2$, $R^4$, $R^5$ and $R^6$ are as defined in claim 1, and $R^{3'}$ represents a lower alkyl group, a lower alkenyl group, a lower alkynyl group or a cycloalkyl group,

and its nontoxic salt, which comprises reacting a substituted alkylamine derivative represented by the general formula

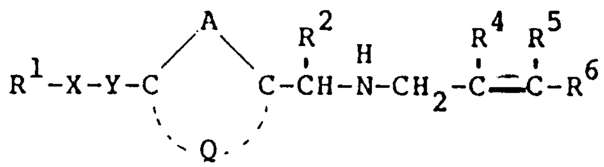

[$I^a$]

wherein A, Q, X, Y, $R^1$, $R^2$, $R^4$, $R^5$ and $R^6$ are as defined above,
or its properly protected compound with a compound represented by the general formula

$Z\text{-}R^{3'}$ [VI]

wherein Z represents a leaving group and $R^{3'}$ is as defined above,
then, as required, removing the protective group, and as required, converting the resulting compound into a non-toxic salt.

**11.** A process for producing a substituted alkylamine derivative represented by the general formula

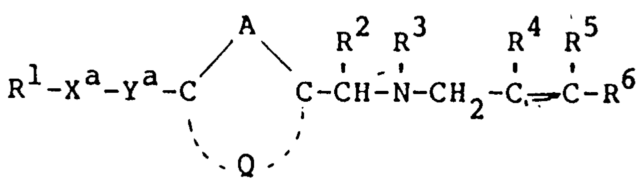

[$I^c$]

wherein A, Q, $R^1$, $R^2$, $R^3$, $R^4$, $R^5$ and $R^6$ are as defined in claim 1, $X^a$ represents a carbonyl group or a group of the formula $-CHR^a-$ in which $R^a$ represents a hydrogen atom or a lower alkyl group, and $Y^a$ represents an oxygen or sulfur atom or a group of the formula $-NR^b-$ in which $R^b$ represents a hydrogen atom or a lower alkyl group,
or its nontoxic salt, which comprises reacting a compound represented by the general formua

$R^1\text{-}X^a\text{-}Z$ [VII]

wherein Z represents a leaving group and $R^1$ and $X^a$ are as defined above,
or its properly proteced derivative with a substituted amine derivative represented by the general formula

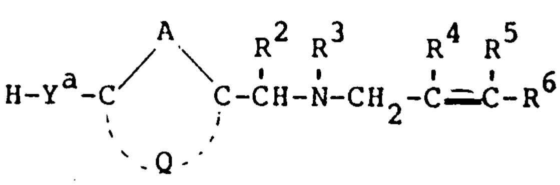

[VIII]

wherein A, Q, $R^2$, $R^3$, $R^4$, $R^5$, $R^6$ and $Y^a$ are as defined,
or its properly protected compound, then, as required, removing the protective group, and then, as required, converting the resulting compound into a nontoxic salt.

**12.** A process for producing a substituted alkylamine derivative represented by the general formula

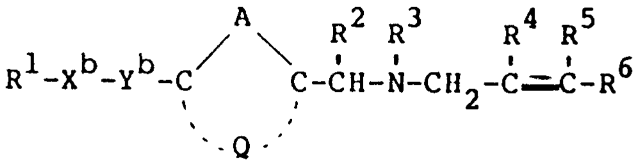

[$I^d$]

in which A, Q, $R^1$, $R^2$, $R^3$, $R^4$, $R^5$ and $R^6$ are as defined in claim 1, $X^b$ represents an oxygen or sulfur atom, or a group of the formula $-NR^b-$ in which $R^b$ represents a hydrogen atom or a lower alkyl group, and $Y^b$ represents a carbonyl group or a group of the formula $-CHR^a-$ in which $R^a$ represents a hydrogen atom or a lower alkyl group,
and its nontoxic salt, which comprises reacting a compound represented by the general formula

$R^1-X^b-H$ [IX]

wherein $R^1$ and $X^b$ are as defined above,
or its properly protected derivative with a substituted amine derivative represented by the general formula

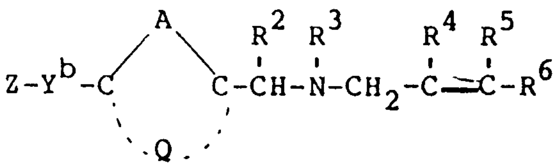

[X]

wherein A, Q, $R^2$, $R^3$, $R^4$, $R^5$, $R^6$ and $Y^b$ are as defined above, and Z represents a leaving group, or its properly protected compound, and, as required, removing the protecting group, and then, as required, converting the resulting compound into a nontoxic salt.

## Patentansprüche
**Patentansprüche für folgende Vertragsstaaten : AT, BE, CH, DE, FR, GB, IT, LI, LU, NL, SE**

**1.** Substituiertes Alkylaminderivat der allgemeinen Formel:

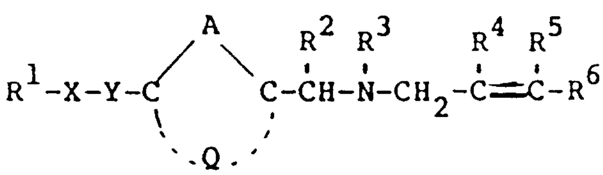

[I]

worin A für eine Methingruppe, ein Stickstoffatom, ein Sauerstoffatom oder ein Schwefelatom steht; Q für eine Gruppe, die ein oder zwei Heteroatome, ausgewählt aus der Gruppe bestehend aus Stickstoff-, Sauerstoff- und Schwefelatomen, enthalten kann, steht, die zusammen mit den angrenzenden Kohlenstoffatomen und A einen 5- oder 6gliedrigen aromatischen Ring bildet (wobei der aromatische Ring einen Substituenten oder zwei gleiche oder verschiedene Substituenten, ausgewählt aus der Klasse bestehend aus Halogenatomen, Hydroxylgruppen, Niedrigalkylgruppen und Niedrigalkoxygruppen, enthalten kann); X und Y gleich oder verschieden sein können und jeweils für ein Sauerstoffatom, ein Schwefelatom, eine Carbonylgruppe, eine Gruppe der Formel $-CHR^a-$, worin $R^a$ ein Wasserstoffatom oder eine Niedrigalkylgruppe darstellt, oder eine Gruppe der Formel $-NR^b-$, worin $R^b$ ein Wasserstoffatom oder eine Niedrigalkylgruppe darstellt, stehen, oder wobei zusammengenommen X und Y eine Vinylen- oder Ethinylengruppe darstellen; $R^1$ für eine Niedrigalkenylgruppe, die substituiert sein kann,

eine Cycloalkenylgruppe, die substituiert sein kann, eine Niedrigalkinylgruppe, die substituiert sein kann, eine Arylgruppe, die substituiert sein kann, oder eine heterocyclische Gruppe, die substituiert sein kann, steht; $R^2$ für ein Wasserstoffatom oder eine Niedrigalkylgruppe steht; $R^3$ für ein Wasserstoffatom, eine Niedrigalkylgruppe, eine Niedrigalkenylgruppe, eine Niedrigalkinylgruppe oder eine Cycloalkylgruppe steht; $R^4$ und $R^5$ gleich oder verschieden sein können und jeweils für ein Wasserstoffatom oder ein Halogenatom stehen; und $R^6$ für eine acyclische Kohlenwasserstoffgruppe, die substituiert sein kann (die 1 oder 2 ungesättigte Bindungen, ausgewählt aus Doppel- und Dreifachbindungen, enthalten kann), eine Cycloalkylgruppe, die substituiert sein kann, oder eine Phenylgruppe, die substituiert sein kann, steht; mit der Maßgabe, daß, wenn eines von X und Y ein Sauerstoff- oder Schwefelatom oder die Gruppe -$NR^b$-, worin $R^b$ die obige Definition hat, ist, das andere für eine Carbonylgruppe oder die Gruppe -$CHR^a$-, worin $R^a$ die obige Definition hat, steht,
und ein nichttoxisches Salz davon.

2. Substituiertes Alkylaminderivat und sein nichttoxisches Salz nach Anspruch 1, dadurch **gekennzeichnet,** daß $R^1$ für eine Aryl- oder heterocyclische Gruppe, die substituiert sein kann, steht; X für eine Methylengruppe steht; und Y für ein Sauerstoffatom oder eine Iminogruppe steht oder X und Y zusammengenommen eine Ethylengruppe oder eine (E)-Vinylengruppe darstellen; der aromatische Ring der Formel:

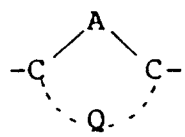

ein Benzol-, Furan-, Thiophen-, Oxazol-, Isoxazol-, Thiazol-, Pyridin- oder Pyrimidinring ist; $R^2$ für ein Wasserstoffatom steht; $R^3$ für eine Alkylgruppe mit 1 bis 5 Kohlenstoffatomen, eine Alkenylgruppe mit 3 bis 5 Kohlenstoffatomen, eine Alkinylgruppe mit 3 bis 5 Kohlenstoffatomen oder eine Cycloalkylgruppe mit 3 bis 5 Kohlenstoffatomen steht; $R^4$ und $R^5$ jeweils für ein Wasserstoffatom stehen und die zwischen den zwei Kohlenstoffatomen, an die sie gebunden sind, gebildete Doppelbindung die trans-Form (E-Form) hat; und $R^6$ für eine Gruppe der Formel $-CH=CH-R^c$, worin $R^c$ eine Alkyl- oder Alkenylgruppe mit 3 bis 6 Kohlenstoffatomen, die mit einer Alkoxygruppe mit 1 bis 4 Kohlenstoffatomen substituiert sein kann, darstellt oder eine Cycloalkylgruppe mit 3 bis 6 Kohlenstoffatomen, die mit 1 oder 2 Alkylgruppen mit 1 bis 4 Kohlenstoffatomen substituiert sein kann, oder eine Gruppe der Formel $-C\equiv C-R^c$,worin $R^c$ die obige Definition hat, steht.

3. Substituiertes Alkylaminderivat und sein nichttoxisches Salz nach Anspruch 2, dadurch **gekennzeichnet,** daß $R^1$ für eine Arylgruppe oder eine aromatische heterocyclische Gruppe, die einen Substituenten oder zwei identische oder verschiedene Substituenten, ausgewählt aus der Gruppe bestehend aus Halogenatomen und Cyano-, Formyl-, Hydroxymethyl-, Niedrigalkyl-, Niedrigalkenyl-, Niedrigalkinyl, Niedrigalkoxy-, Niedrigalkenyloxy-, Phenyl-, Furyl-, Thienyl-, Oxazolyl-, Thiazolyl-, Isoxazolyl-, Isothiazolyl-, Pyrrolyl-, Imidazolyl-, Furazanyl-, Pyridyl-, Pyrazinyl-, Pyrimidinyl-, Pyridazinyl-, 1,3,5-Triazinyl-, 1,3,4-Oxadiazolyl- und 1,3,4-Thiadiazolylgruppen, haben kann, steht.

4. Substituiertes Alkylaminderivat und sein nichttoxisches Salz nach Anspruch 3, dadurch **gekennzeichnet,** daß die aromatische heterocyclische Gruppe eine 5- bis 12gliedrige aromatische heterocyclische Gruppe ist, die 1 bis 3 Heteroatome, ausgewählt aus der Gruppe bestehend aus Stickstoff-, Sauerstoff- und Schwefelatomen, enthält.

5. Substituiertes Alkylaminderivat und sein nichttoxisches Salz nach Anspruch 2, dadurch **gekennzeichnet,** daß $R^1$ für eine Phenyl-, Naphthyl-, Furyl-, Thienyl-, Pyridyl-, Oxazolyl-, Thiazolyl-, Isoxazolyl-, Isothiazolyl-, Pyrazinyl-, Pyrimidinyl-, Pyridazinyl-, 1,3,5-Triazinyl-, Benzofuranyl-, Benzothienyl-, Benzoxazolyl-, Benzothiazolyl- oder Benzofurazanylgruppe steht, die einen Substituenten oder identische oder verschiedene zwei Substituenten, ausgewählt aus der Klasse bestehend aus Halogenatomen und Hydroxyl-, Cyano-, Formyl-, Hydroxymethyl-, $C_1$-$C_3$-Alkyl-, $C_3$-$C_5$-Alkenyl-, $C_3$-$C_5$-Alkinyl-, $C_1$-$C_3$-Alkoxy-, $C_3$-$C_5$-Alkenyloxy-, Phenyl-, Furyl-, Thienyl-, Oxazolyl-, Thiazolyl-, Isoxazolyl-, Pyrrolyl- und Imidazolylgruppen, aufweisen kann.

6. Substituiertes Alkylaminderivat und sein nichttoxisches Salz nach Anspruch 2, dadurch **gekennzeichnet,** daß $R^1$ für eine 2-Methylphenyl-, 2-Fluorphenyl-, 3-Cyanophenyl-, 3-(2-Furyl)phenyl-, 3-(2-Thienyl)-phenyl-, 3-(5-Oxazolyl)phenyl-,3-(1-Pyrrolyl)phenyl-, 3-(1-Imidazolyl)phenyl- oder 3-(5-Thiazolyl)-phenylgruppe steht.

7. Substituiertes Alkylaminderivat und sein nichttoxisches Salz nach Anspruch 1, dadurch **gekennzeichnet,** daß $R^3$ für eine Alkylgruppe mit 1 bis 3 Kohlenstoffatomen, eine Allylgruppe, eine Propargylgruppe oder eine Cyclopropylgruppe steht; und $R^6$ für eine Gruppe der Formel:

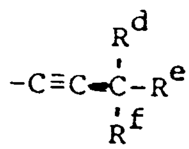

steht,
worin $R^d$ für ein Wasserstoffatom, eine Alkylgruppe mit 1 bis 4 Kohlenstoffatomen oder eine Alkoxygruppe mit 1 bis 4 Kohlenstoffatomen steht; und $R^e$ und $R^f$, die gleich oder verschieden sein können, jeweils für eine Methyl- oder Ethylgruppe stehen oder zusammengenommen eine Cyclopropylgruppe darstellen können.

8. Substituiertes Alkylaminderivat und sein nichttoxisches Salz nach Anspruch 7, dadurch **gekennzeichnet,** daß $R^3$ für eine Methyl-, Ethyl- oder Propylgruppe steht.

9. Verfahren zur Herstellung eines substituierten Alkylaminderivats der allgemeinen Formel [I] nach Anspruch 1 und seines nichttoxischen Salzes, dadurch **gekennzeichnet,** daß man
(a) ein substituiertes Aminderivat der allgemeinen Formel:

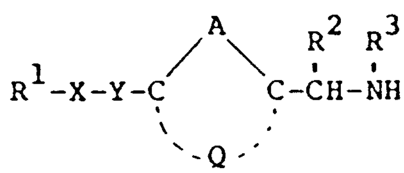

[II]

worin A, Q, X, Y, $R^1$, $R^2$ und $R^3$ wie in Anspruch 1 definiert sind,
oder ihr geschütztes Derivat mit einer Verbindung der allgemeinen Formel:

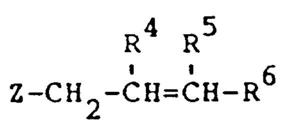

[III]

worin Z für eine Austrittsgruppe steht und $R^4$, $R^5$ und $R^6$ wie in Anspruch 1 definiert sind,
oder ihrem geschützten Derivat umsetzt und danach erforderlichenfalls die Schutzgruppe entfernt; oder daß man
(b) eine Verbindung der allgemeinen Formel:

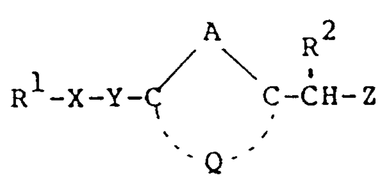

[IV]

worin A, Q, X, Y, $R^1$, $R^2$ und Z wie oben definiert sind, oder ihr geschütztes Derivat mit einem substituierten Amin der allgemeinen Formel:

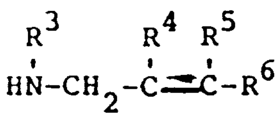

[V]

worin $R^3$, $R^4$, $R^5$ und $R^6$ wie oben definiert sind, oder ihrem geschützten Derivat umsetzt und danach erforderlichenfalls die Schutzgruppe entfernt, und daß man erforderlichenfalls die resultierende Verbindung in ein nichttoxisches Salz umwandelt.

**10.** Verfahren zur Herstellung eines substituierten Alkylaminderivats der allgemeinen Formel:

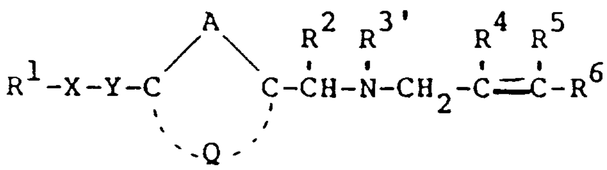

[$I^b$]

worin A, Q, X, Y, $R^1$, $R^2$, $R^4$, $R^5$ und $R^6$ wie in Anspruch 1 definiert sind und $R^{3'}$ für eine Niedrigalkylgruppe, eine Niedrigalkenylgruppe, eine Niedrigalkinylgruppe oder eine Cycloalkylgruppe steht,
und seines nichttoxischen Salzes, dadurch **gekennzeichnet,** daß man ein substituiertes Alkylaminderivat der allgemeinen Formel:

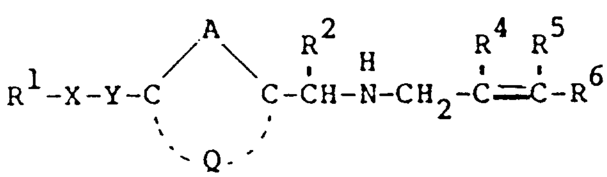

[$I^a$]

worin A, Q, X, Y, $R^1$, $R^2$, $R^4$, $R^5$ und $R^6$ wie oben definiert sind,
oder ihre geschützte Verbindung mit einer Verbindung der allgemeinen Formel:

$Z\text{-}R^{3'}$ [VI]

worin Z für eine Austrittsgruppe steht und $R^{3'}$ wie oben definiert ist,
umsetzt, dann erforderlichenfalls die Schutzgruppe entfernt, und daß man erforderlichenfalls die resultierende Verbindung in ein nichttoxisches Salz umwandelt.

**11.** Verfahren zur Herstellung eines substituierten Alkylaminderivats der allgemeinen Formel:

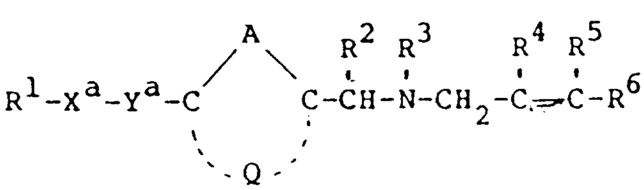

[$I^c$]

worin A, Q, $R^1$, $R^2$, $R^3$, $R^4$, $R^5$ und $R^6$ wie in Anspruch 1 definiert sind, $X^a$ für eine Carbonylgruppe oder eine Gruppe der Formel $-CHR^a-$, worin $R^a$ ein Wasserstoffatom oder eine Niedrigalkylgruppe ist,

steht und $Y^a$ für ein Sauerstoff- oder Schwefelatom oder eine Gruppe der Formel $-NR^b-$, worin $R^b$ ein Wasserstoffatom oder eine Niedrigalkylgruppe ist, steht, oder seines nichttoxischen Salzes, dadurch **gekennzeichnet,** daß man eine Verbindung der allgemeinen Formel:

$R^1-X^a-Z$ [VII]

worin Z für eine Austrittsgruppe steht und $R^1$ und $X^a$ wie oben definiert sind,
oder ihr geschütztes Derivat mit einem substituierten Aminderivat der allgemeinen Formel:

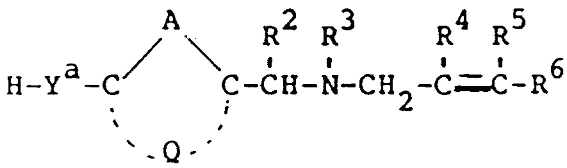

[VIII]

worin A, Q, $R^2$, $R^3$, $R^4$, $R^5$, $R^6$ und $Y^a$ wie definiert sind,
oder ihrer geschützten Verbindung umsetzt, sodann erforderlichenfalls die Schutzgruppe entfernt, und daß man hierauf erforderlichenfalls die resultierende Verbindung in ein nichttoxisches Salz umwandelt.

**12.** Verfahren zur Herstellung eines substituierten Alkylaminderivats der allgemeinen Formel:

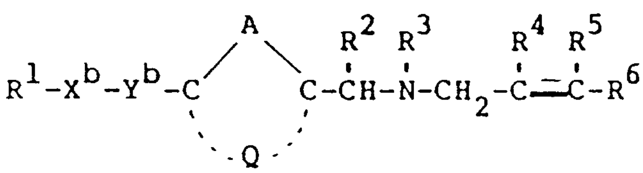

$[I^d]$

worin A, Q, $R^1$, $R^2$, $R^3$, $R^4$, $R^5$ und $R^6$ wie in Anspruch 1 definiert sind, $X^b$ für ein Sauerstoff- oder Schwefelatom oder eine Gruppe der Formel $-NR^b-$, worin $R^b$ ein Wasserstoffatom oder eine Niedrigalkylgruppe darstellt, steht und $Y^b$ für eine Carbonylgruppe oder eine Gruppe der Formel $-CHR^a-$, worin $R^a$ ein Wasserstoffatom oder eine Niedrigalkylgruppe darstellt, steht,
und seines nichttoxischen Salzes, dadurch **gekennzeichnet,** daß man eine Verbindung der allgemeinen Formel:

$R^1-X^b-H$ [IX]

worin $R^1$ und $X^b$ wie oben definiert sind,
oder ihr geschütztes Derivat mit einem substituierten Aminderivat der allgemeinen Formel:

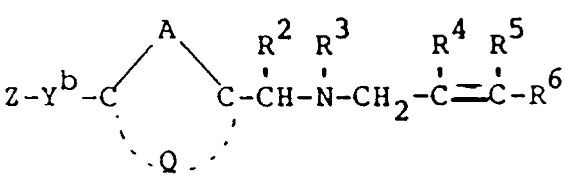

[X]

worin A, Q, $R^2$, $R^3$, $R^4$, $R^5$, $R^6$ und $Y^b$ wie oben definiert sind und Z für eine Austrittsgruppe steht,
oder ihrer geschützten Verbindung umsetzt und erforderlichenfalls die Schutzgruppe entfernt, und daß man hierauf erforderlichenfalls die resultierende Verbindung in ein nichttoxisches Salz umwandelt.

**13.** Arzneimittel, dadurch **gekennzeichnet,** daß es eine Verbindung der allgemeinen Formel [I] nach Anspruch 1 oder ein pharmazeutisch annehmbares Salz davon enthält.

**14.** Mittel zur Behandlung von Hypercholesterinämie, Hyperlipämie oder Arteriosklerose, dadurch **gekennzeichnet,** daß es eine Verbindung der allgemeinen Formel [I] oder ein pharmazeutisch annehmbares Salz davon enthält.

**15.** Pharmazeutisches Präparat, dadurch **gekennzeichnet,** daß es eine wirksame Menge einer Verbindung der allgemeinen Formel [I] nach Anspruch 1 oder eines pharmazeutisch annehmbaren Salzes davon und einen pharmazeutisch annehmbaren Träger oder ein pharmazeutisch annehmbares Verdünnungsmittel enthält.

**16.** Squalen-Oxidase-Inhibitor, dadurch **gekennzeichnet,** daß er eine Verbindung der allgemeinen Formel [I] nach Anspruch 1 oder ein pharmazeutisch annehmbares Salz davon umfaßt.

**Patentansprüche für folgenden Vertragsstaat : ES**

**1.** Verfahren zur Herstellung eines substituierten Alkylaminderivats der allgemeinen Formel:

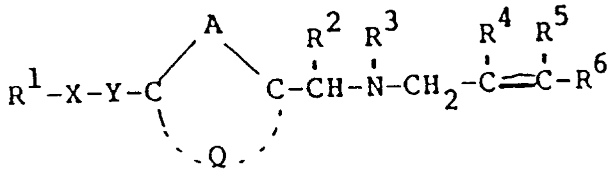

[I]

worin A für eine Methingruppe, ein Stickstoffatom, ein Sauerstoffatom oder ein Schwefelatom steht; Q für eine Gruppe, die ein oder zwei Heteroatome, ausgewählt aus der Gruppe bestehend aus Stickstoff-, Sauerstoff- und Schwefelatomen, enthalten kann, steht, die zusammen mit den angrenzenden Kohlenstoffatomen und A einen 5- oder 6gliedrigen aromatischen Ring bildet (wobei der aromatische Ring einen Substituenten oder zwei gleiche oder verschiedene Substituenten, ausgewählt aus der Klasse bestehend aus Halogenatomen, Hydroxylgruppen, Niedrigalkylgruppen und Niedrigalkoxygruppen, enthalten kann); X und Y gleich oder verschieden sein können und jeweils für ein Sauerstoffatom, ein Schwefelatom, eine Carbonylgruppe, eine Gruppe der Formel $-CHR^a-$, worin $R^a$ ein Wasserstoffatom oder eine Niedrigalkylgruppe darstellt, oder eine Gruppe der Formel $-NR^b-$, worin $R^b$ ein Wasserstoffatom oder eine Niedrigalkylgruppe darstellt, stehen, oder wobei zusammengenommen X und Y eine Vinylen- oder Ethinylengruppe darstellen; $R^1$ für eine Niedrigalkenylgruppe, die substituiert sein kann, eine Cycloalkenylgruppe, die substituiert sein kann, eine Niedrigalkinylgruppe, die substituiert sein kann, eine Arylgruppe, die substituiert sein kann, oder eine heterocyclische Gruppe, die substituiert sein kann, steht; $R^2$ für ein Wasserstoffatom oder eine Niedrigalkylgruppe steht; $R^3$ für ein Wasserstoffatom, eine Niedrigalkylgruppe, eine Niedrigalkenylgruppe, eine Niedrigalkinylgruppe oder eine Cycloalkylgruppe steht; $R^4$ und $R^5$ gleich oder verschieden sein können und jeweils für ein Wasserstoffatom oder ein Halogenatom stehen; und $R^6$ für eine acyclische Kohlenwasserstoffgruppe, die substituiert sein kann (die 1 oder 2 ungesättigte Bindungen, ausgewählt aus Doppel- und Dreifachbindungen, enthalten kann), eine Cycloalkylgruppe, die substituiert sein kann, oder eine Phenylgruppe, die substituiert sein kann, steht; mit der Maßgabe, daß, wenn eines von X und Y ein Sauerstoff- oder Schwefelatom oder die Gruppe $-NR^b-$, worin $R^b$ die obige Definition hat, ist, das andere für eine Carbonylgruppe oder die Gruppe $-CHR^a-$, worin $R^a$ die obige Definition hat, steht,
und eines nichttoxischen Salzes davon, dadurch **gekennzeichnet,** daß man
(a) ein substituiertes Aminderivat der allgemeinen Formel:

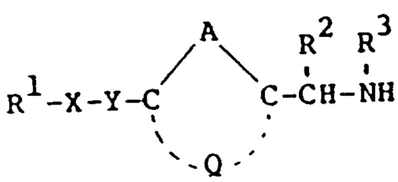

[II]

worin A, Q, X, Y, $R^1$, $R^2$ und $R^3$ wie oben definiert sind,

oder ihr geschütztes Derivat mit einer Verbindung der allgemeinen Formel:

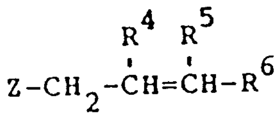

[III]

worin Z für eine Austrittsgruppe steht und $R^4$, $R^5$ und $R^6$ wie oben definiert sind,
oder ihrem geschützten Derivat umsetzt und danach erforderlichenfalls die Schutzgruppe entfernt; oder daß man
(b) eine Verbindung der allgemeinen Formel:

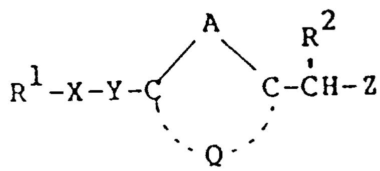

[IV]

worin A, Q, X, Y, $R^1$, $R^2$ und Z wie oben definiert sind,
oder ihr geschütztes Derivat mit einem substituierten Amin der allgemeinen Formel:

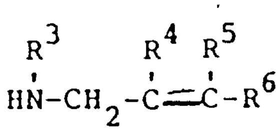

[V]

worin $R^3$, $R^4$, $R^5$ und $R^6$ wie oben definiert sind,
oder ihrem geschützten Derivat umsetzt und danach erforderlichenfalls die Schutzgruppe entfernt, und daß man erforderlichenfalls die resultierende Verbindung in ein nichttoxisches Salz umwandelt.

**2.** Verfahren zur Herstellung eines substituierten Alkylaminderivats der allgemeinen Formel:

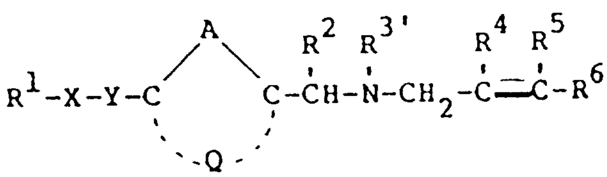

[$I^b$]

worin A, Q, X, Y, $R^1$, $R^2$, $R^4$, $R^5$ und $R^6$ wie in Anspruch 1 definiert sind und $R^{3'}$ für eine Niedrigalkylgruppe, eine Niedrigalkenylgruppe, eine Niedrigalkinylgruppe oder eine Cycloalkylgruppe steht,
und seines nichttoxischen Salzes, dadurch **gekennzeichnet,** daß man ein substituiertes Alkylaminderivat der allgemeinen Formel:

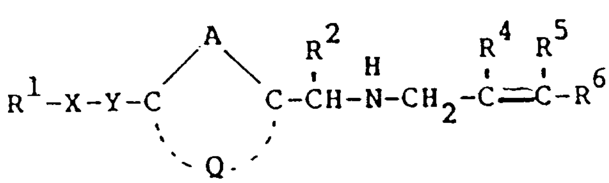

[$I^a$]

worin A, Q, X, Y, $R^1$, $R^2$, $R^4$, $R^5$ und $R^6$ wie oben definiert sind, oder ihre geschützte Verbindung mit einer Verbindung der allgemeinen Formel:

$Z\text{-}R^{3'}$ [VI]

worin Z für eine Austrittsgruppe steht und $R^{3'}$ wie oben definiert ist, umsetzt, dann erforderlichenfalls die Schutzgruppe entfernt, und daß man erforderlichenfalls die resultierende Verbindung in ein nichttoxisches Salz umwandelt.

**3.** Verfahren zur Herstellung eines substituierten Alkylaminderivats der allgemeinen Formel:

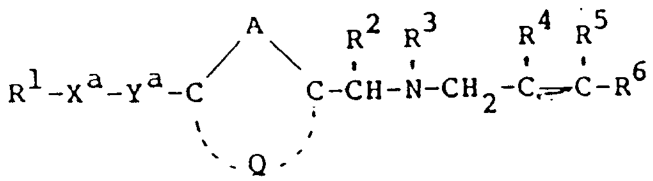

[$I^c$]

worin A, Q, $R^1$, $R^2$, $R^3$, $R^4$, $R^5$ und $R^6$ wie in Anspruch 1 definiert sind, $X^a$ für eine Carbonylgruppe oder eine Gruppe der Formel $\text{-CHR}^a\text{-}$, worin $R^a$ ein Wasserstoffatom oder eine Niedrigalkylgruppe ist, steht und $Y^a$ für ein Sauerstoff- oder Schwefelatom oder eine Gruppe der Formel $\text{-NR}^b\text{-}$, worin $R^b$ ein Wasserstoffatom oder eine Niedrigalkylgruppe ist, steht, oder seines nichttoxischen Salzes, dadurch **gekennzeichnet,** daß man eine Verbindung der allgemeinen Formel:

$R^1\text{-}X^a\text{-}Z$ [VII]

worin Z für eine Austrittsgruppe steht und $R^1$ und $X^a$ wie oben definiert sind, oder ihr geschütztes Derivat mit einem substituierten Aminderivat der allgemeinen Formel:

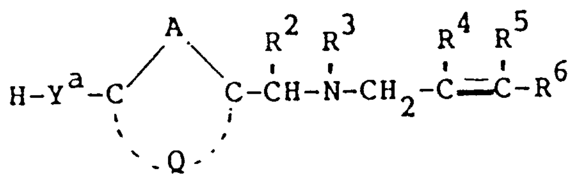

[VIII]

worin A, Q, $R^2$, $R^3$, $R^4$, $R^5$, $R^6$ und $Y^a$ wie definiert sind, oder ihrer geschützten Verbindung umsetzt, sodann erforderlichenfalls die Schutzgruppe entfernt, und daß man hierauf erforderlichenfalls die resultierende Verbindung in ein nichttoxisches Salz umwandelt.

**4.** Verfahren zur Herstellung eines substituierten Alkylaminderivats der allgemeinen Formel:

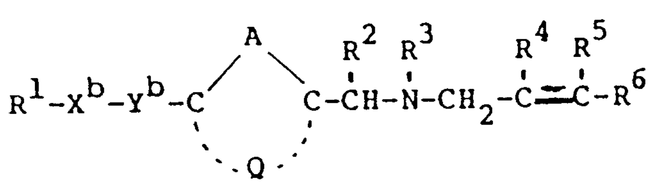

[$I^d$]

worin A, Q, $R^1$, $R^2$, $R^3$, $R^4$, $R^5$ und $R^6$ wie in Anspruch 1 definiert sind, $X^b$ für ein Sauerstoff- oder Schwefelatom oder eine Gruppe der Formel $\text{-NR}^b\text{-}$, worin $R^b$ ein Wasserstoffatom oder eine Niedrigalkylgruppe darstellt, steht und $Y^b$ für eine Carbonylgruppe oder eine Gruppe der Formel $\text{-CHR}^a\text{-}$, worin $R^a$ ein Wasserstoffatom oder eine Niedrigalkylgruppe darstellt, steht,

und seines nichttoxischen Salzes, dadurch **gekennzeichnet,** daß man eine Verbindung der allgemeinen Formel:

$R^1$-$X^b$-H [IX]

worin $R^1$ und $X^b$ wie oben definiert sind,
oder ihr geschütztes Derivat mit einem substituierten Aminderivat der allgemeinen Formel:

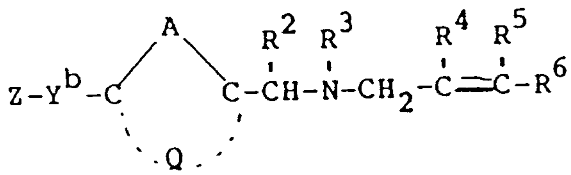

[X]

worin A, Q, $R^2$, $R^3$, $R^4$, $R^5$, $R^6$ und $Y^b$ wie oben definiert sind und Z für eine Austrittsgruppe steht, oder ihrer geschützten Verbindung umsetzt und erforderlichenfalls die Schutzgruppe entfernt, und daß man hierauf erforderlichenfalls die resultierende Verbindung in ein nichttoxisches Salz umwandelt.

5. Verfahren nach den Ansprüchen 1 bis 4 zur Herstellung von Verbindungen, bei denen $R^1$ für eine Aryl- oder heterocyclische Gruppe, die substituiert sein kann, steht; X für eine Methylengruppe steht; und Y für ein Sauerstoffatom oder eine Iminogruppe steht oder X und Y zusammengenommen eine Ethylengruppe oder eine (E)-Vinylengruppe darstellen; der aromatische Ring der Formel:

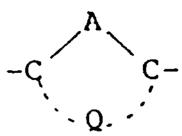

ein Benzol-, Furan-, Thiophen-, Oxazol-, Isoxazol-, Thiazol-, Pyridin- oder Pyrimidinring ist; $R^2$ für ein Wasserstoffatom steht; $R^3$ für eine Alkylgruppe mit 1 bis 5 Kohlenstoffatomen, eine Alkenylgruppe mit 3 bis 5 Kohlenstoffatomen, eine Alkinylgruppe mit 3 bis 5 Kohlenstoffatomen oder eine Cycloalkylgruppe mit 3 bis 5 Kohlenstoffatomen steht; $R^4$ und $R^5$ jeweils für ein Wasserstoffatom stehen und die zwischen den zwei Kohlenstoffatomen, an die sie gebunden sind, gebildete Doppelbindung die trans-Form (E-Form) hat; und $R^6$ für eine Gruppe der Formel -CH = CH-$R^c$, worin $R^c$ eine Alkyl- oder Alkenylgruppe mit 3 bis 6 Kohlenstoffatomen, die mit einer Alkoxygruppe mit 1 bis 4 Kohlenstoffatomen substituiert sein kann, darstellt oder eine Cycloalkylgruppe mit 3 bis 6 Kohlenstoffatomen, die mit 1 oder 2 Alkylgruppen mit 1 bis 4 Kohlenstoffatomen substituiert sein kann, oder eine Gruppe der Formel -C≡C-$R^c$,worin $R^c$ die obige Definition hat, steht.

6. Verfahren nach Anspruch 5 zur Herstellung von Verbindungen, bei denen $R^1$ für eine Arylgruppe oder eine aromatische heterocyclische Gruppe, die einen Substituenten oder zwei identische oder verschiedene Substituenten, ausgewählt aus der Gruppe bestehend aus Halogenatomen und Cyano-, Formyl-, Hydroxymethyl-, Niedrigalkyl-, Niedrigalkenyl-, Niedrigalkinyl-, Niedrigalkoxy-, Niedrigalkenyloxy-, Phenyl-, Furyl-, Thienyl-, Oxazolyl-, Thiazolyl-, Isoxazolyl-, Isothiazolyl-, Pyrrolyl-, Imidazolyl-, Furazanyl-, Pyridyl-, Pyrazinyl-, Pyrimidinyl-, Pyridazinyl-, 1,3,5-Triazinyl-, 1,3,4-Oxadiazolyl- und 1,3,4-Thiadiazolylgruppen, haben kann, steht.

7. Verfahren nach Anspruch 6 zur Herstellung von Verbindungen, bei denen die aromatische heterocyclische Gruppe eine 5- bis 12gliedrige aromatische heterocyclische Gruppe ist, die 1 bis 3 Heteroatome, ausgewählt aus der Gruppe bestehend aus Stickstoff-, Sauerstoff- und Schwefelatomen, enthält.

8. Verfahren nach Anspruch 5 zur Herstellung von Verbindungen, bei denen $R^1$ für eine Phenyl-, Naphthyl-, Furyl-, Thienyl-, Pyridyl-, Oxazolyl-, Thiazolyl-, Isoxazolyl-, Isothiazolyl-, Pyrazinyl-, Pyrimidinyl-, Pyridazinyl-, 1,3,5-Triazinyl-, Benzofuranyl-, Benzothienyl-, Benzoxazolyl-, Benzothiazolyl- oder Benzofurazanylgruppe steht, die einen Substituenten oder identische oder verschiedene zwei

Substituenten, ausgewählt aus der Klasse bestehend aus Halogenatomen und Hydroxyl-, Cyano-, Formyl-, Hydroxymethyl-, $C_1$-$C_3$-Alkyl-, $C_3$-$C_5$-Alkenyl-, $C_3$-$C_5$-Alkinyl-, $C_1$-$C_3$-Alkoxy-, $C_3$-$C_5$-Alkenyloxy-, Phenyl-, Furyl-, Thienyl-, Oxazolyl-, Thiazolyl-, Isoxazolyl-, Pyrrolyl- und Imidazolylgruppen, aufweisen kann.

**9.** Verfahren nach Anspruch 5 zur Herstellung von Verbindungen, bei denen $R^1$ für eine 2-Methylphenyl-, 2-Fluorphenyl-, 3-Cyanophenyl-, 3-(2-Furyl)phenyl-, 3-(2-Thienyl)phenyl-, 3-(5-Oxazolyl)phenyl-, 3-(1-Pyrrolyl)phenyl-, 3-(1-Imidazolyl)phenyl- oder 3-(5-Thiazolyl)phenylgruppe steht.

**10.** Verfahren nach den Ansprüchen 1 bis 4 zur Herstellung von Verbindungen, bei denen $R^3$ für eine Alkylgruppe mit 1 bis 3 Kohlenstoffatomen, eine Allylgruppe, eine Propargylgruppe oder eine Cyclopropylgruppe steht; und $R^6$ für eine Gruppe der Formel:

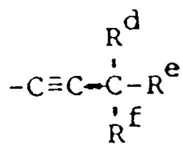

steht,

worin $R^d$ für ein Wasserstoffatom, eine Alkylgruppe mit 1 bis 4 Kohlenstoffatomen oder eine Alkoxygruppe mit 1 bis 4 Kohlenstoffatomen steht; und $R^e$ und $R^f$, die gleich oder verschieden sein können, jeweils für eine Methyl- oder Ethylgruppe stehen oder zusammengenommen eine Cyclopropylgruppe darstellen können.

**11.** Verfahren nach Anspruch 10 zur Herstellung von Verbindungen, bei denen $R^3$ für eine Methyl-, Ethyl- oder Propylgruppe steht.

## Patentansprüche für folgenden Vertragsstaat : GR

**1.** Substituiertes Alkylaminderivat der allgemeinen Formel:

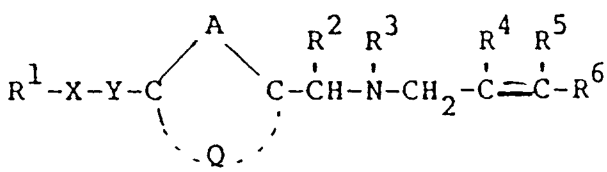

[I]

worin A für eine Methingruppe, ein Stickstoffatom, ein Sauerstoffatom oder ein Schwefelatom steht; Q für eine Gruppe, die ein oder zwei Heteroatome, ausgewählt aus der Gruppe bestehend aus Stickstoff-, Sauerstoff- und Schwefelatomen, enthalten kann, steht, die zusammen mit den angrenzenden Kohlenstoffatomen und A einen 5- oder 6gliedrigen aromatischen Ring bildet (wobei der aromatische Ring einen Substituenten oder zwei gleiche oder verschiedene Substituenten, ausgewählt aus der Klasse bestehend aus Halogenatomen, Hydroxylgruppen, Niedrigalkylgruppen und Niedrigalkoxygruppen, enthalten kann); X und Y gleich oder verschieden sein können und jeweils für ein Sauerstoffatom, ein Schwefelatom, eine Carbonylgruppe, eine Gruppe der Formel $-CHR^a-$, worin $R^a$ ein Wasserstoffatom oder eine Niedrigalkylgruppe darstellt, oder eine Gruppe der Formel $-NR^b-$, worin $R^b$ ein Wasserstoffatom oder eine Niedrigalkylgruppe darstellt, stehen, oder wobei zusammengenommen X und Y eine Vinylen- oder Ethinylengruppe darstellen; $R^1$ für eine Niedrigalkenylgruppe, die substituiert sein kann, eine Cycloalkenylgruppe, die substituiert sein kann, eine Niedrigalkinylgruppe, die substituiert sein kann, eine Arylgruppe, die substituiert sein kann, oder eine heterocyclische Gruppe, die substituiert sein kann, steht; $R^2$ für ein Wasserstoffatom oder eine Niedrigalkylgruppe steht; $R^3$ für ein Wasserstoffatom, eine Niedrigalkylgruppe, eine Niedrigalkenylgruppe, eine Niedrigalkinylgruppe oder eine Cycloalkylgruppe steht; $R^4$ und $R^5$ gleich oder verschieden sein können und jeweils für ein Wasserstoffatom oder ein Halogenatom stehen; und $R^6$ für eine acyclische Kohlenwasserstoffgruppe, die substituiert sein

kann (die 1 oder 2 ungesättigte Bindungen, ausgewählt aus Doppel- und Dreifachbindungen, enthalten kann), eine Cycloalkylgruppe, die substituiert sein kann, oder eine Phenylgruppe, die substituiert sein kann, steht; mit der Maßgabe, daß, wenn eines von X und Y ein Sauerstoff- oder Schwefelatom oder die Gruppe $-NR^b-$, worin $R^b$ die obige Definition hat, ist, das andere für eine Carbonylgruppe oder die Gruppe $-CHR^a-$, worin $R^a$ die obige Definition hat, steht,
und ein nichttoxisches Salz davon.

2. Substituiertes Alkylaminderivat und sein nichttoxisches Salz nach Anspruch 1, dadurch **gekennzeichnet,** daß $R^1$ für eine Aryl- oder heterocyclische Gruppe, die substituiert sein kann, steht; X für eine Methylengruppe steht; und Y für ein Sauerstoffatom oder eine Iminogruppe steht oder X und Y zusammengenommen eine Ethylengruppe oder eine (E)-Vinylengruppe darstellen; der aromatische Ring der Formel:

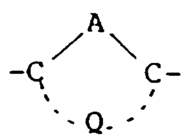

ein Benzol-, Furan-, Thiophen-, Oxazol-, Isoxazol-, Thiazol-, Pyridin- oder Pyrimidinring ist; $R^2$ für ein Wasserstoffatom steht; $R^3$ für eine Alkylgruppe mit 1 bis 5 Kohlenstoffatomen, eine Alkenylgruppe mit 3 bis 5 Kohlenstoffatomen, eine Alkinylgruppe mit 3 bis 5 Kohlenstoffatomen oder eine Cycloalkylgruppe mit 3 bis 5 Kohlenstoffatomen steht; $R^4$ und $R^5$ jeweils für ein Wasserstoffatom stehen und die zwischen den zwei Kohlenstoffatomen, an die sie gebunden sind, gebildete Doppelbindung die trans-Form (E-Form) hat; und $R^6$ für eine Gruppe der Formel $-CH=CH-R^c$, worin $R^c$ eine Alkyl- oder Alkenylgruppe mit 3 bis 6 Kohlenstoffatomen, die mit einer Alkoxygruppe mit 1 bis 4 Kohlenstoffatomen substituiert sein kann, darstellt oder eine Cycloalkylgruppe mit 3 bis 6 Kohlenstoffatomen, die mit 1 oder 2 Alkylgruppen mit 1 bis 4 Kohlenstoffatomen substituiert sein kann, oder eine Gruppe der Formel $-C \equiv C-R^c$,worin $R^c$ die obige Definition hat, steht.

3. Substituiertes Alkylaminderivat und sein nichttoxisches Salz nach Anspruch 2, dadurch **gekennzeichnet,** daß $R^1$ für eine Arylgruppe oder eine aromatische heterocyclische Gruppe, die einen Substituenten oder zwei identische oder verschiedene Substituenten, ausgewählt aus der Gruppe bestehend aus Halogenatomen und Cyano-, Formyl-, Hydroxymethyl-, Niedrigalkyl-, Niedrigalkenyl-, Niedrigalkinyl-, Niedrigalkoxy-, Niedrigalkenyloxy-, Phenyl-, Furyl-, Thienyl-, Oxazolyl-, Thiazolyl-, Isoxazolyl-, Isothiazolyl-, Pyrrolyl-, Imidazolyl-, Furazanyl-, Pyridyl-, Pyrazinyl-, Pyrimidinyl-, Pyridazinyl-, 1,3,5-Triazinyl-, 1,3,4-Oxadiazolyl- und 1,3,4-Thiadiazolylgruppen, haben kann, steht.

4. Substituiertes Alkylaminderivat und sein nichttoxisches Salz nach Anspruch 3, dadurch **gekennzeichnet,** daß die aromatische heterocyclische Gruppe eine 5- bis 12gliedrige aromatische heterocyclische Gruppe ist, die 1 bis 3 Heteroatome, ausgewählt aus der Gruppe bestehend aus Stickstoff-, Sauerstoff- und Schwefelatomen, enthält.

5. Substituiertes Alkylaminderivat und sein nichttoxisches Salz nach Anspruch 2, dadurch **gekennzeichnet,** daß $R^1$ für eine Phenyl-, Naphthyl-, Furyl-, Thienyl-, Pyridyl-, Oxazolyl-, Thiazolyl-, Isoxazolyl-, Isothiazolyl-, Pyrazinyl-, Pyrimidinyl-, Pyridazinyl-, 1,3,5-Triazinyl-, Benzofuranyl-, Benzothienyl-, Benzoxazolyl-, Benzothiazolyloder Benzofurazanylgruppe steht, die einen Substituenten oder identische oder verschiedene zwei Substituenten, ausgewählt aus der Klasse bestehend aus Halogenatomen und Hydroxyl-, Cyano-, Formyl-, Hydroxymethyl-, $C_1$-$C_3$-Alkyl-, $C_3$-$C_5$-Alkenyl-, $C_3$-$C_5$-Alkinyl-, $C_1$-$C_3$-Alkoxy -, $C_3$-$C_5$-Alkenyloxy-, Phenyl-, Furyl-, Thienyl-, Oxazolyl-, Thiazolyl-, Isoxazolyl-, Pyrrolyl- und Imidazolylgruppen, aufweisen kann.

6. Substituiertes Alkylaminderivat und sein nichttoxisches Salz nach Anspruch 2, dadurch **gekennzeichnet,** daß $R^1$ für eine 2-Methylphenyl-, 2-Fluorphenyl-, 3-Cyanophenyl-, 3-(2-Furyl)phenyl-, 3-(2-Thienyl)-phenyl-, 3-(5-Oxazolyl)phenyl-,3-(1-Pyrrolyl)phenyl-, 3-(1-Imidazolyl)phenyl- oder 3-(5-Thiazolyl)-phenylgruppe steht.

7. Substituiertes Alkylaminderivat und sein nichttoxisches Salz nach Anspruch 1, dadurch **gekennzeichnet,** daß $R^3$ für eine Alkylgruppe mit 1 bis 3 Kohlenstoffatomen, eine Allylgruppe, eine Propargylgruppe oder eine Cyclopropylgruppe steht; und $R^6$ für eine Gruppe der Formel:

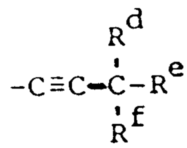

steht,

worin $R^d$ für ein Wasserstoffatom, eine Alkylgruppe mit 1 bis 4 Kohlenstoffatomen oder eine Alkoxygruppe mit 1 bis 4 Kohlenstoffatomen steht; und $R^e$ und $R^f$, die gleich oder verschieden sein können, jeweils für eine Methyl- oder Ethylgruppe stehen oder zusammengenommen eine Cyclopropylgruppe darstellen können.

8. Substituiertes Alkylaminderivat und sein nichttoxisches Salz nach Anspruch 7, dadurch **gekennzeichnet,** daß $R^3$ für eine Methyl-, Ethyl- oder Propylgruppe steht.

9. Verfahren zur Herstellung eines substituierten Alkylaminderivats der allgemeinen Formel [I] nach Anspruch 1 und seines nichttoxischen Salzes, dadurch **gekennzeichnet,** daß man

(a) ein substituiertes Aminderivat der allgemeinen Formel:

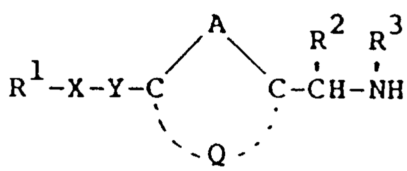

[II]

worin A, Q, X, Y, $R^1$, $R^2$ und $R^3$ wie in Anspruch 1 definiert sind,

oder ihr geschütztes Derivat mit einer Verbindung der allgemeinen Formel:

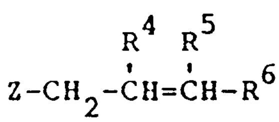

[III]

worin Z für eine Austrittsgruppe steht und $R^4$, $R^5$ und $R^6$ wie in Anspruch 1 definiert sind,

oder ihrem geschützten Derivat umsetzt und danach erforderlichenfalls die Schutzgruppe entfernt; oder daß man

(b) eine Verbindung der allgemeinen Formel:

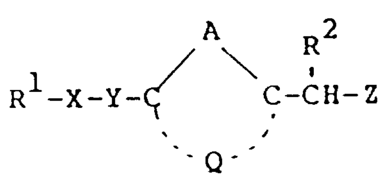

[IV]

worin A, Q, X, Y, $R^1$, $R^2$ und Z wie oben definiert sind,

oder ihr geschütztes Derivat mit einem substituierten Amin der allgemeinen Formel:

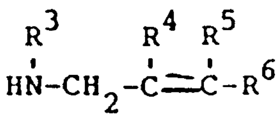

[V]

worin $R^3$, $R^4$, $R^5$ und $R^6$ wie oben definiert sind, oder ihrem geschützten Derivat umsetzt und danach erforderlichenfalls die Schutzgruppe entfernt, und daß man erforderlichenfalls die resultierende Verbindung in ein nichttoxisches Salz umwandelt.

**10.** Verfahren zur Herstellung eines substituierten Alkylaminderivats der allgemeinen Formel:

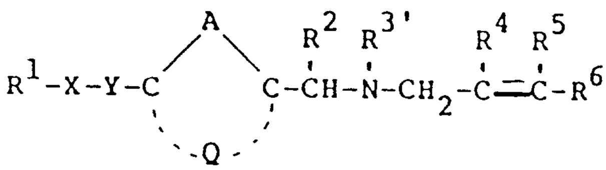

[$I^b$]

worin A, Q, X, Y, $R^1$, $R^2$, $R^4$, $R^5$ und $R^6$ wie in Anspruch 1 definiert sind und $R^{3'}$ für eine Niedrigalkylgruppe, eine Niedrigalkenylgruppe, eine Niedrigalkinylgruppe oder eine Cycloalkylgruppe steht,
und seines nichttoxischen Salzes, dadurch **gekennzeichnet,** daß man ein substituiertes Alkylaminderivat der allgemeinen Formel:

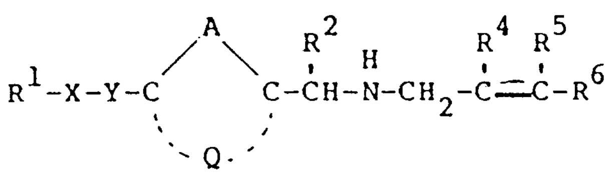

[$I^a$]

worin A, Q, X, Y, $R^1$, $R^2$, $R^4$, $R^5$ und $R^6$ wie oben definiert sind,
oder ihre geschützte Verbindung mit einer Verbindung der allgemeinen Formel:

$Z\text{-}R^{3'}$ [VI]

worin Z für eine Austrittsgruppe steht und $R^{3'}$ wie oben definiert ist,
umsetzt, dann erforderlichenfalls die Schutzgruppe entfernt, und daß man erforderlichenfalls die resultierende Verbindung in ein nichttoxisches Salz umwandelt.

**11.** Verfahren zur Herstellung eines substituierten Alkylaminderivats der allgemeinen Formel:

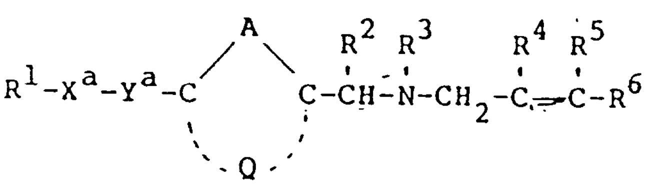

[$I^c$]

worin A, Q, $R^1$, $R^2$, $R^3$, $R^4$, $R^5$ und $R^6$ wie in Anspruch 1 definiert sind, $X^a$ für eine Carbonylgruppe oder eine Gruppe der Formel $-CHR^a-$, worin $R^a$ ein Wasserstoffatom oder eine Niedrigalkylgruppe ist, steht und $Y^a$ für ein Sauerstoff- oder Schwefelatom oder eine Gruppe der Formel $-NR^b-$, worin $R^b$ ein Wasserstoffatom oder eine Niedrigalkylgruppe ist, steht,

oder seines nichttoxischen Salzes, dadurch **gekennzeichnet,** daß man eine Verbindung der allgemeinen Formel:

$R^1$-$X^a$-Z [VII]

worin Z für eine Austrittsgruppe steht und $R^1$ und $X^a$ wie oben definiert sind,
oder ihr geschütztes Derivat mit einem substituierten Aminderivat der allgemeinen Formel:

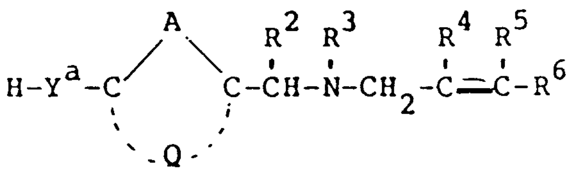 [VIII]

worin A, Q, $R^2$, $R^3$, $R^4$, $R^5$, $R^6$ und $Y^a$ wie definiert sind,
oder ihrer geschützten Verbindung umsetzt, sodann erforderlichenfalls die Schutzgruppe entfernt, und daß man hierauf erforderlichenfalls die resultierende Verbindung in ein nichttoxisches Salz umwandelt.

**12.** Verfahren zur Herstellung eines substituierten Alkylaminderivats der allgemeinen Formel:

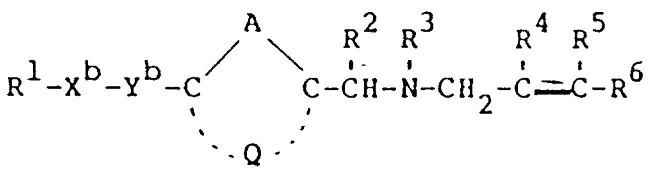 [$I^d$]

worin A, Q, $R^1$, $R^2$, $R^3$, $R^4$, $R^5$ und $R^6$ wie in Anspruch 1 definiert sind, $X^b$ für ein Sauerstoff- oder Schwefelatom oder eine Gruppe der Formel -$NR^b$-, worin $R^b$ ein Wasserstoffatom oder eine Niedrigalkylgruppe darstellt, steht und $Y^b$ für eine Carbonylgruppe oder eine Gruppe der Formel -$CHR^a$-, worin $R^a$ ein Wasserstoffatom oder eine Niedrigalkylgruppe darstellt, steht,
und seines nichttoxischen Salzes, dadurch **gekennzeichnet,** daß man eine Verbindung der allgemeinen Formel:

$R^1$-$X^b$-H [IX]

worin $R^1$ und $X^b$ wie oben definiert sind,
oder ihr geschütztes Derivat mit einem substituierten Aminderivat der allgemeinen Formel:

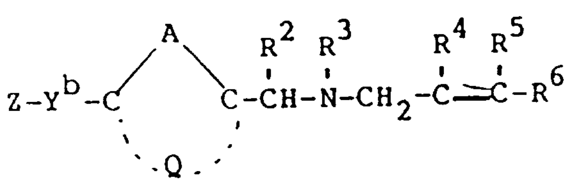 [X]

worin A, Q, $R^2$, $R^3$, $R^4$, $R^5$, $R^6$ und $Y^b$ wie oben definiert sind und Z für eine Austrittsgruppe steht,
oder ihrer geschützten Verbindung umsetzt und erforderlichenfalls die Schutzgruppe entfernt, und daß man hierauf erforderlichenfalls die resultierende Verbindung in ein nichttoxisches Salz umwandelt.

**Revendications**

**Revendications pour les Etats contractants suivants : AT, BE, CH, DE, FR, GB, IT, LI, LU, NL, SE**

1. Dérivé substitué d'alkylamine représenté par la formule générale

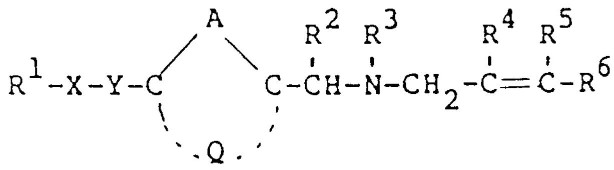

[I]

dans laquelle A représente un groupe méthyne, un atome d'azote, un atome d'oxygène ou un atome de soufre ; Q représente un groupe qui peut contenir un ou deux hétéroatomes choisis dans la classe formée par les atomes d'azote, d'oxygène et de soufre et forme un noyau aromatique penta- ou hexagonal avec les atomes de carbone adjacents et A (le noyau aromatique peut contenir un substituant ou deux substituants identiques ou différents, choisis dans la classe formée par les atomes d'halogènes, les groupes hydroxyle, les groupes alkyles inférieurs et les groupes alcoxy inférieurs) ; X et Y peuvent être identiques ou différents et représentent chacun un atome d'oxygène, un atome de soufre, un groupe carbonyle, un groupe de formule $-CHR^a-$ où $R^a$ représente un atome d'hydrogène ou un groupe alkyle inférieur, ou un groupe de formule $-NR^b-$ où $R^b$ représente un atome d'hydrogène ou un groupe alkyle inférieur, ou bien X et Y, pris ensemble, représentent un groupe vinylène ou éthynylène ; $R^1$ représente un groupe alcényle inférieur qui peut être substitué, un groupe cycloalcényle qui peut être substitué, un groupe alcynyle inférieur qui peut être substitué, un groupe aryle qui peut être substitué ou un groupe hétérocyclique qui peut être substitué ; $R^2$ représente un atome d'hydrogène ou un groupe alkyle inférieur ; $R^3$ représente un atome d'hydrogène, un groupe alkyle inférieur, un groupe alcényle inférieur, un groupe alcynyle inférieur ou un groupe cycloalkyle ; $R^4$ et $R^5$ peuvent être identiques ou différents et chacun représente un atome d'hydrogène ou un atome d'halogène ; et $R^6$ représente un groupe hydrocarboné acyclique qui peut être substitué (qui peut contenir 1 ou 2 liaisons insaturées choisies parmi les double et triple liaisons), un groupe cycloalkyle qui peut être substitué ou un groupe phényle qui peut être substitué ; à condition que si l'un de X et Y représente un atome d'oxygène ou de soufre, ou le groupe $-NR^b-$ où $R^b$ est tel que défini ci-dessus, l'autre représente le groupe carbonyle ou le groupe $-CHR^a-$ où $R^a$ est tel que défini ci-dessus,
et un sel non toxique de ce dérivé.

2. Dérivé substitué d'alkylamine et son sel non toxique selon la revendication 1, dans lesquels $R^1$ représente un groupe aryle ou hétérocyclique qui peut être substitué ; X représente un groupe méthylène et Y représente un atome d'oxygène ou un groupe imino, ou bien X et Y, pris ensemble, représentent un groupe éthylène ou un groupe (E)-vinylène ; le noyau aromatique de formule

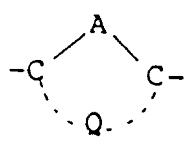

est un noyau de benzène, furanne, thiophène, oxazole, isoxazole, thiazole, pyridine ou pyrimidine ; $R^2$ représente un atome d'hydrogène ; $R^3$ représente un groupe alkyle ayant 1 à 5 atomes de carbone, un groupe alcényle ayant 3 à 5 atomes de carbone, un groupe alcynyle ayant 3 à 5 atomes de carbone ou un groupe cycloalkyle ayant 3 à 5 atomes de carbone ; $R^4$ et $R^5$ représentent chacun un atome d'hydrogène, et la double liaison formée par les deux atomes de carbone auxquels ils sont liés est de forme *trans* (forme E) ; et $R^6$ est un groupe de formule $-CH=CH-R^c$ où $R^c$ représente un groupe alkyle ou alcényle ayant 3 à 6 atomes de carbone qui peut être substitué par un seul groupe alcoxy ayant 1 à 4 atomes de carbone, ou un groupe cycloalkyle ayant 3 à 6 atomes de carbone qui peut être substitué par 1 ou 2 groupes alkyles ayant 1 à 4 atomes de carbone, ou un groupe de formule $-C{\equiv}C-R^c$ où $R^c$ est tel que défini ci-dessus.

3. Dérivé substitué d'alkylamine et son sel non toxique selon la revendication 2, dans lesquels $R^1$ est un groupe aryle ou un groupe hétérocyclique aromatique, qui peut porter un substituant ou deux substituants identiques ou différents choisis dans la classe formée par les atomes d'halogènes et les groupes cyano, formyle, hydroxyméthyle, alkyles inférieurs, alcényles inférieurs, alcynyles inférieurs, alcoxy inférieurs, alcényloxy inférieurs, phényle, furyle, thiényle, oxazolyle, thiazolyle, isoxazolyle, isothiazolyle, pyrrolyle, imidazolyle, furazanyle, pyridyle, pyrazinyle, pyrimidinyle, pyridazinyle, 1,3,5-triazinyle, 1,3,4-oxadiazolyle et 1,3,4-thiadiazolyle.

4. Dérivé substitué d'alkylamine et son sel non toxique selon la revendication 3, dans lesquels le groupe hétérocyclique aromatique est un groupe hétérocyclique aromatique penta- à dodécagonal contenant 1 à 3 hétéroatomes choisis dans la classe formée par les atomes d'azote, d'oxygène et de soufre.

5. Dérivé substitué d'alkylamine et son sel non toxique selon la revendication 2, dans lesquels $R^1$ est un groupe phényle, naphtyle, furyle, thiényle, pyridyle, oxazolyle, thiazolyle, isoxazolyle, isothiazolyle, pyrazinyle, pyrimidinyle, pyridazinyle, 1,3,5-triazinyle, benzofurannyle, benzothiényle, benzoxazolyle, benzothiazolyle ou benzofurazanyle qui peut contenir un substituant ou deux substituants identiques ou différents choisis dans la classe formée par les atomes d'halogènes et les groupes hydroxyle, cyano, formyle, hydroxyméthyle, alkyles en $C_1$-$C_3$, alcényles en $C_3$-$C_5$, alcynyles en $C_3$-$C_5$, alcoxy en $C_1$-$C_3$, alcényloxy en $C_3$-$C_5$, phényle, furyle, thiényle, oxazolyle, thiazolyle, isoxazolyle, pyrrolyle et imidazolyle.

6. Dérivé substitué d'alkylamine et son sel non toxique selon la revendication 2, dans lesquels $R^1$ est un groupe 2-méthylphényle, 2-fluorophényle, 3-cyanophényle, 3-(2-furyl)phényle, 3-(2-thiényl)phényle, 3-(5-oxazolyl)phényle, 3-(1-pyrrolyl)phényle, 3-(1-imidazolyl)phényle ou 3-(5-thiazolyl)phényle.

7. Dérivé substitué d'alkylamine et son sel non toxique selon la revendication 1, dans lesquels $R^3$ représente un groupe alkyle ayant 1 à 3 atomes de carbone, un groupe allyle, un groupe propargyle ou un groupe cyclopropyle ; et $R^6$ représente un groupe de formule

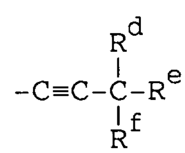

où $R^d$ représente un atome d'hydrogène, un groupe alkyle ayant 1 à 4 atomes de carbone ou un groupe alcoxy ayant 1 à 4 atomes de carbone, et $R^e$ et $R^f$ peuvent être identiques ou différents et représentent chacun un groupe méthyle ou éthyle, ou bien, pris ensemble, ils peuvent représenter un groupe cyclopropyle.

8. Dérivé substitué d'alkylamine et son sel non toxique selon la revendication 7, dans lesquels $R^3$ représente un groupe méthyle, éthyle ou propyle.

9. Procédé de production d'un dérivé substitué d'alkylamine de la formule générale [I] définie la revendication 1 et de son sel non toxique, qui consiste à
(a) faire réagir un dérivé substitué d'amine représenté par la formule générale

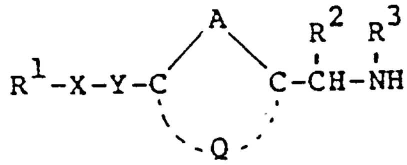

[II]

dans laquelle A, Q, X, Y, $R^1$, $R^2$ et $R^3$ sont tels que définis dans la revendication 1,
ou un dérivé convenablement protégé de celui-ci, avec un composé représenté par la formule générale

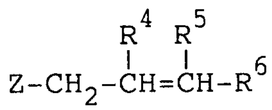 [III]

dans laquelle Z représente un groupe partant, et $R^4$, $R^5$ et $R^6$ sont tels que définis dans la revendication 1,
ou un dérivé convenablement protégé de celui-ci, puis, si nécessaire, éliminer le groupe protecteur, ou
(b) faire réagir un composé représenté par la formule générale

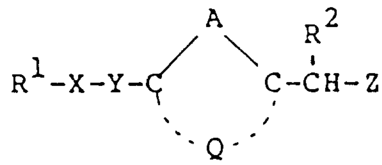 [IV]

dans laquelle A, Q, X, Y, $R^1$, $R^2$ et Z sont tels que définis ci-dessus,
ou un dérivé convenablement protégé de celui-ci, avec une amine substituée représentée par la formule générale

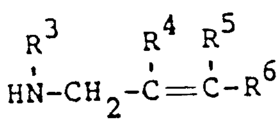 [V]

dans laquelle $R^3$, $R^4$, $R^5$ et $R^6$ sont tels que définis ci-dessus,
ou un dérivé convenablement protégé de celle-ci, puis, si nécessaire, éliminer le groupe protecteur, et, si nécessaire, convertir le composé résultant en un sel non toxique.

**10.** Procédé de production d'un dérivé substitué d'alkylamine représenté par la formule générale

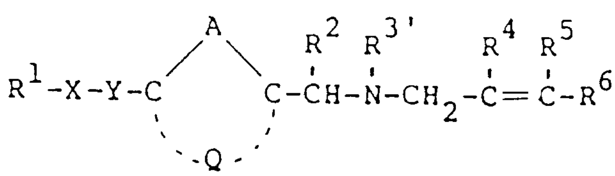 [$I^b$]

dans laquelle A, Q, X, Y, $R^1$, $R^2$, $R^4$, $R^5$ et $R^6$ sont tels que définis dans la revendication 1, et $R^{3'}$ représente un groupe alkyle inférieur, un groupe alcényle inférieur, un groupe alcynyle inférieur ou un groupe cycloalkyle,
et d'un sel non toxique de celui-ci, qui consiste à faire réagir un dérivé substitué d'alkylamine représenté par la formule générale

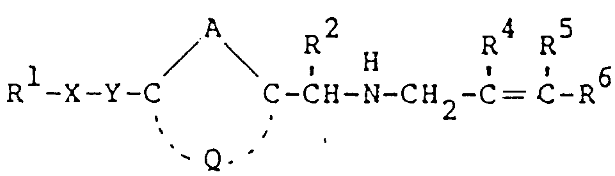 [$I^a$]

dans laquelle A, Q, X, Y, $R^1$, $R^2$, $R^4$, $R^5$ et $R^6$ sont tels que définis ci-dessus,
ou un dérivé convenablement protégé de celui-ci, avec un composé représenté par la formule générale

$Z-R^{3'}$ [VI]

dans laquelle Z représente un groupe partant et $R^{3'}$ est tel que défini ci-dessus,
puis, si nécessaire, éliminer le groupe protecteur et, si nécessaire, convertir le composé résultant en un sel non toxique.

**11.** Procédé de production d'un dérivé substitué d'alkylamine représenté par la formule générale

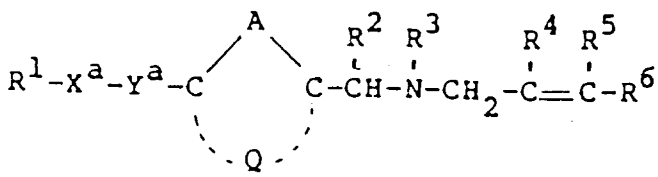

$[I^c]$

dans laquelle A, Q, $R^1$, $R^2$, $R^3$, $R^4$, $R^5$ et $R^6$ sont tels que définis dans la revendication 1, $X^a$ représente un groupe carbonyle ou un groupe de formule $-CHR^a-$ où $R^a$ représente un atome d'hydrogène ou un groupe alkyle inférieur, et $Y^a$ représente un atome d'oxygène ou de soufre ou un groupe de formule $-NR^b-$ où $R^b$ représente un atome d'hydrogène ou un groupe alkyle inférieur,
ou d'un sel non toxique de celui-ci, qui consiste à faire réagir un composé représenté par la formule générale

$R^1-X^a-Z$ [VII]

dans laquelle Z représente un groupe partant et $R^1$ et $X^a$ sont tels que définis ci-dessus,
ou un dérivé convenablement protégé de celui-ci, avec un dérivé substitué d'amine représenté par la formule générale

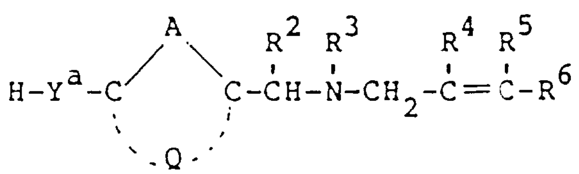

[VIII]

dans laquelle A, Q, $R^2$, $R^3$, $R^4$, $R^5$, $R^6$ et $Y^a$ sont tels que définis,
ou un dérivé convenablement protégé de celui-ci, puis, si nécessaire, éliminer le groupe protecteur, puis, si nécessaire, convertir le composé résultant en un sel non toxique.

**12.** Procédé de production d'un dérivé substitué d'alkylamine représenté par la formule générale

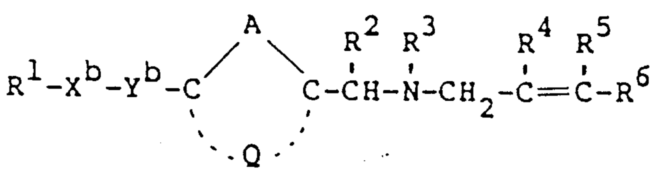

$[I^d]$

dans laquelle A, Q, $R^1$, $R^2$, $R^3$, $R^4$, $R^5$ et $R^6$ sont tels que définis dans la revendication 1, $X^b$ représente un atome d'oxygène ou de soufre ou un groupe de formule $-NR^b-$ où $R^b$ représente un atome d'hydrogène ou un groupe alkyle inférieur, et $Y^b$ représente un groupe carbonyle ou un groupe de formule $-CHR^a-$ où $R^a$ représente un atome d'hydrogène ou un groupe alkyle inférieur,
et d'un sel non toxique de celui-ci, qui consiste à faire réagir un composé représenté par la formule générale

$R^1$-$X^b$-H [IX]

dans laquelle $R^1$ et $X^b$ sont tels que définis ci-dessus,
ou un dérivé convenablement protégé de celui-ci, avec un dérivé substitué d'amine représenté par la formule générale

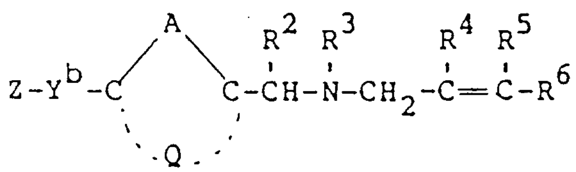

[X]

dans laquelle A, Q, $R^2$, $R^3$, $R^4$, $R^5$, $R^6$ et $Y^b$ sont tels que définis ci-dessus, et Z représente un groupe partant,
ou un dérivé convenablement protégé de celui-ci, et, si nécessaire, éliminer le groupe protecteur, puis, si nécessaire, convertir le composé résultant en un sel non toxique.

**13.** Médicament contenant un composé de formule générale [I] selon la revendication 1 ou un sel pharmaceutiquement acceptable de ce composé.

**14.** Agent pour traiter l'hypercholestérolémie, l'hyperlipémie ou l'artériosclérose, comprenant un composé de formule générale [I] ou un sel pharmaceutiquement acceptable de ce composé.

**15.** Préparation pharmaceutique comprenant une quantité efficace d un composé de formule générale [I] selon la revendication 1 ou d'un sel pharmaceutiquement acceptable de ce composé et un support ou diluant pharmaceutiquement acceptable.

**16.** Inhibiteur de squalène-oxydase comprenant un composé de formule générale [I] selon la revendication 1 ou un sel pharmaceutiquement acceptable de ce composé.

**Revendications pour l'Etat contractant suivant : ES**

**1.** Procédé de production d'un dérivé substitué d'alkylamine représenté par la formule générale

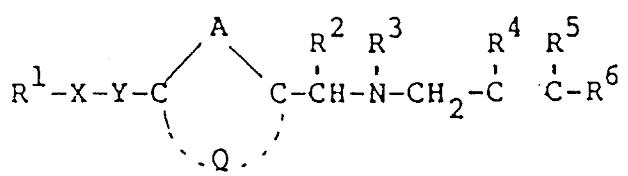

[I]

dans laquelle A représente un groupe méthyne, un atome d'azote, un atome d'oxygène ou un atome de soufre ; Q représente un groupe qui peut contenir un ou deux hétéroatomes choisis dans la classe formée par les atomes d'azote, d'oxygène et de soufre et forme un noyau aromatique penta- ou hexagonal avec les atomes de carbone adjacents et A (le noyau aromatique peut contenir un substituant ou deux substituants identiques ou différents, choisis dans la classe formée par les atomes d'halogènes, les groupes hydroxyle, les groupes alkyles inférieurs et les groupes alcoxy inférieurs) ; X et Y peuvent être identiques ou différents et représentent chacun un atome d'oxygène, un atome de soufre, un groupe carbonyle, un groupe de formule -$CHR^a$- où $R^a$ représente un atome d'hydrogène ou un groupe alkyle inférieur, ou un groupe de formule -$NR^b$- où $R^b$ représente un atome d'hydrogène ou un groupe alkyle inférieur, ou bien X et Y, pris ensemble, représentent un groupe vinylène ou éthynylène ; $R^1$ représente un groupe alcényle inférieur qui peut être substitué, un groupe cycloalcényle qui peut être substitué, un groupe alcynyle inférieur qui peut être substitué, un groupe aryle qui peut être substitué ou un groupe hétérocyclique qui peut être substitué ; $R^2$ représente un atome d'hydrogène ou un groupe alkyle inférieur ; $R^3$ représente un atome d'hydrogène, un groupe alkyle

inférieur, un groupe alcényle inférieur, un groupe alcynyle inférieur ou un groupe cycloalkyle ; $R^4$ et $R^5$ peuvent être identiques ou différents et chacun représente un atome d'hydrogène ou un atome d'halogène ; et $R^6$ représente un groupe hydrocarboné acyclique qui peut être substitué (qui peut contenir 1 ou 2 liaisons insaturées choisies parmi les double et triple liaisons), un groupe cycloalkyle qui peut être substitué ou un groupe phényle qui peut être substitué ; à condition que si l'un de X et Y représente un atome d'oxygène ou de soufre, ou le groupe $-NR^b-$ ou $R^b$ est tel que défini ci-dessus, l'autre représente le groupe carbonyle ou le groupe $-CHR^a-$ où $R^a$ est tel que défini ci-dessus,
et d'un sel non toxique de ce dérivé, qui consiste à

(a) faire réagir un dérivé substitué d'amine représenté par la formule générale

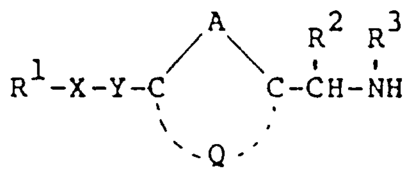

[II]

dans laquelle A, Q, X, Y, $R^1$, $R^2$ et $R^3$ sont tels que définis ci-dessus,
ou un dérivé convenablement protégé de celui-ci, avec un composé représenté par la formule générale

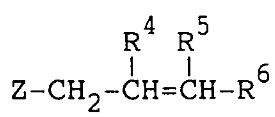

[III]

dans laquelle Z représente un groupe partant, et $R^4$, $R^5$ et $R^6$ sont tels que définis ci-dessus,
ou un dérivé convenablement protégé de celui-ci, puis, si nécessaire, éliminer le groupe protecteur, ou

(b) faire réagir un composé représenté par la formule générale

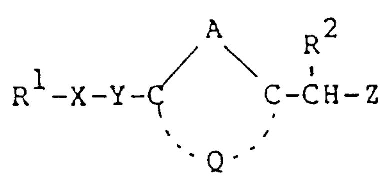

[IV]

dans laquelle A, Q, X, Y, $R^1$, $R^2$ et Z sont tels que définis ci-dessus,
ou un dérivé convenablement protégé de celui-ci, avec une amine substituée représentée par la formule générale

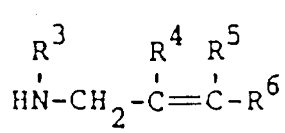

[V]

dans laquelle $R^3$, $R^4$, $R^5$ et $R^6$ sont tels que définis ci-dessus,
ou un dérivé convenablement protégé de celle-ci, puis, si nécessaire, éliminer le groupe protecteur,
et, si nécessaire, convertir le composé résultant en un sel non toxique.

2. Procédé de production d'un dérivé substitué d'alkylamine représenté par la formule générale

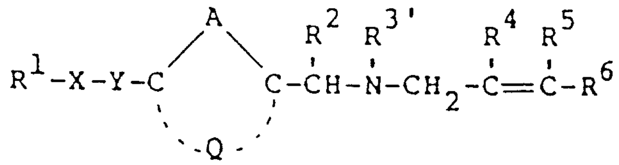

[I$^b$]

dans laquelle A, Q, X, Y, $R^1$, $R^2$, $R^4$, $R^5$ et $R^6$ sont tels que définis dans la revendication 1, et $R^{3'}$ représente un groupe alkyle inférieur, un groupe alcényle inférieur, un groupe alcynyle inférieur ou un groupe cycloalkyle,
ou d'un sel non toxique de celui-ci, qui consiste à faire réagir un dérivé substitué d'alkylamine représenté par la formule générale

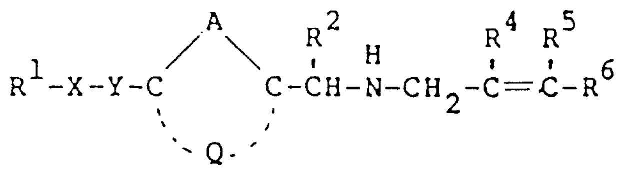

[I$^a$]

dans laquelle A, Q, X, Y, $R^1$, $R^2$, $R^4$, $R^5$ et $R^6$ sont tels que définis ci-dessus,
ou un composé convenablement protégé de celui-ci, avec un composé représenté par la formule générale

$Z-R^{3'}$ [VI]

dans laquelle Z représente un groupe partant et $R^{3'}$ est tel que défini ci-dessus,
puis, si nécessaire, éliminer le groupe protecteur et, si nécessaire, convertir le composé résultant en un sel non toxique.

3. Procédé de production d'un dérivé d'alkylamine substitué représenté par la formule générale

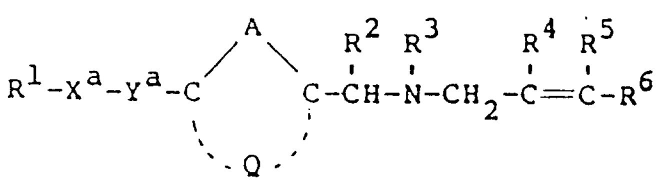

[I$^c$]

dans laquelle A, Q, $R^1$, $R^2$, $R^3$, $R^4$, $R^5$ et $R^6$ sont tels que définis dans la revendication 1, $X^a$ représente un groupe carbonyle ou un groupe de formule $-CHR^a-$ où $R^a$ représente un atome d'hydrogène ou un groupe alkyle inférieur, et $Y^a$ représente un atome d'oxygène ou de soufre ou un groupe de formule $-NR^b-$ où $R^b$ représente un atome d'hydrogène ou un groupe alkyle inférieur,
ou d'un sel non toxique de celui-ci, qui consiste à faire réagir un composé représenté par la formule générale

$R^1-X^a-Z$ [VII]

dans laquelle Z représente un groupe partant et $R^1$ et $X^a$ sont tels que définis ci-dessus,
ou un dérivé convenablement protégé de celui-ci, avec un dérivé substitué d'amine représenté par la formule générale

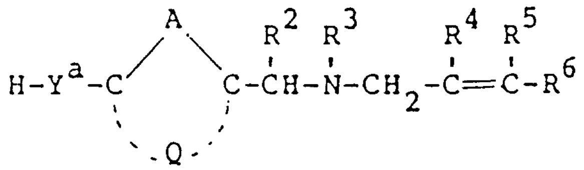

[VIII]

dans laquelle A, Q, $R^2$, $R^3$, $R^4$, $R^5$, $R^6$ et $Y^a$ sont tels que définis,
ou un dérivé convenablement protégé de celui-ci, puis, si nécessaire, éliminer le groupe protecteur, puis, si nécessaire, convertir le composé résultant en un sel non toxique.

**4.** Procédé de production d'un dérivé substitué d'alkylamine représenté par la formule générale

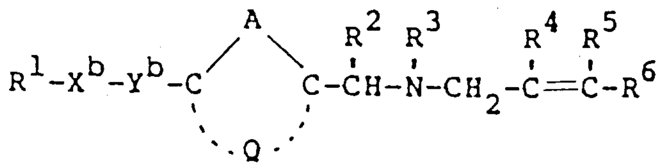

[$I^d$]

dans laquelle A, Q, $R^1$, $R^2$, $R^3$, $R^4$, $R^5$ et $R^6$ sont tels que définis dans la revendication 1, $X^b$ représente un atome d'oxygène ou de soufre ou un groupe de formule $-NR^b-$ où $R^b$ représente un atome d'hydrogène ou un groupe alkyle inférieur, et $Y^b$ représente un groupe carbonyle ou un groupe de formule $-CHR^a-$ où $R^a$ représente un atome d'hydrogène ou un groupe alkyle inférieur,
et d'un sel non toxique de celui-ci, qui consiste à faire réagir un composé représenté par la formule générale

$R^1-X^b-H$ [IX]

dans laquelle $R^1$ et $X^b$ sont tels que définis ci-dessus,
ou un dérivé convenablement protégé de celui-ci, avec un dérivé substitué d'amine représenté par la formule générale

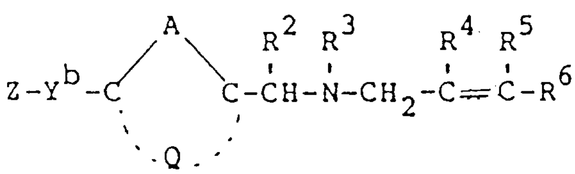

[X]

dans laquelle A, Q, $R^2$, $R^3$, $R^4$, $R^5$, $R^6$ et $Y^b$ sont tels que définis ci-dessus, et Z représente un groupe partant,
ou un dérivé convenablement protégé de celui-ci, et, si nécessaire, éliminer le groupe protecteur, puis, si nécessaire, convertir le composé résultant en un sel non toxique.

**5.** Procédé selon les revendications 1 à 4, pour la production de composés dans lesquels $R^1$ représente un groupe aryle ou hétérocyclique qui peut être substitué ; X représente un groupe méthylène et Y représente un atome d'oxygène ou un groupe imino, ou bien X et Y, pris ensemble, représentent un groupe éthylène ou un groupe (E)-vinylène ; le noyau aromatique de formule

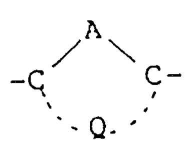

est un noyau de benzène, furanne, thiophène, oxazole, isoxazole, thiazole, pyridine ou pyrimidine ; $R^2$ représente un atome d'hydrogène ; $R^3$ représente un groupe alkyle ayant 1 à 5 atomes de carbone, un groupe alcényle ayant 3 à 5 atomes de carbone, un groupe alcynyle ayant 3 à 5 atomes de carbone ou un groupe cycloalkyle ayant 3 à 5 atomes de carbone ; $R^4$ et $R^5$ représentent chacun un atome d'hydrogène, et la double liaison formée par les deux atomes de carbone auxquels ils sont liés est de forme ***trans*** (forme E) ; et $R^6$ est un groupe de formule $-CH=CH-R^c$ où $R^c$ représente un groupe alkyle ou alcényle ayant 3 à 6 atomes de carbone qui peut être substitué par un seul groupe alcoxy ayant 1 à 4 atomes de carbone, ou un groupe cycloalkyle ayant 3 à 6 atomes de carbone qui peut être substitué par 1 ou 2 groupes alkyles ayant 1 à 4 atomes de carbone, ou un groupe de formule $-C\equiv C-R^c$ où $R^c$ est tel que défini ci-dessus.

**6.** Procédé selon la revendication 5, pour la production de composés dans lesquels $R^1$ est un groupe aryle ou un groupe hétérocyclique aromatique, qui peut porter un substituant ou deux substituants identiques ou différents choisis dans la classe formée par les atomes d'halogènes et les groupes cyano, formyle, hydroxyméthyle, alkyles inférieurs, alcényles inférieurs, alcynyles inférieurs, alcoxy inférieurs, alcényloxy inférieurs, phényle, furyle, thiényle, oxazolyle, thiazolyle, isoxazolyle, isothiazolyle, pyrrolyle, imidazolyle, furazanyle, pyridyle, pyrazinyle, pyrimidinyle, pyridazinyle, 1,3,5-triazinyle, 1,3,4-oxadiazolyle et 1,3,4-thiadiazolyle.

**7.** Procédé selon la revendication 6, pour la production de composés dans lesquels le groupe hétérocyclique aromatique est un groupe hétérocyclique aromatique penta- à dodécagonal contenant 1 à 3 hétéroatomes choisis dans la classe formée par des atomes d'azote, d'oxygène et de soufre.

**8.** Procédé selon la revendication 5, pour la production de composés dans lesquels $R^1$ est un groupe phényle, naphtyle, furyle, thiényle, pyridyle, oxazolyle, thiazolyle, isoxazolyle, isothiazolyle, pyrazinyle, pyrimidinyle, pyridazinyle, 1,3,5-triazinyle, benzofurannyle, benzothiényle, benzoxazolyle, benzothiazolyle ou benzofurazanyle qui peut contenir un substituant ou deux substituants identiques ou différents choisis dans la classe formée par les atomes d'halogènes et les groupes hydroxyle, cyano, formyle, hydroxyméthyle, alkyles en $C_1$-$C_3$, alcényles en $C_3$-$C_5$, alcynyles en $C_3$-$C_5$, alcoxy en $C_1$-$C_3$, alcényloxy en $C_3$-$C_5$, phényle, furyle, thiényle, oxazolyle, thiazolyle, isoxazolyle, pyrrolyle et imidazolyle.

**9.** Procédé selon la revendication 5, pour la production de composés dans lesquels $R^1$ est un groupe 2-méthylphényle, 2-fluorophényle, 3-cyanophényle, 3-(2-furyl)phényle, 3-(2-thiényl)phényle, 3-(5-oxazolyl)phényle, 3-(1-pyrrolyl)phényle, 3-(1-imidazolyl)phényle ou 3-(5-thiazolyl)phényle.

**10.** Procédé selon les revendications 1 à 4, pour la production de composés dans lesquels $R^3$ représente un groupe alkyle ayant 1 à 3 atomes de carbone, un groupe allyle, un groupe propargyle ou un groupe cyclopropyle ; et $R^6$ représente un groupe de formule

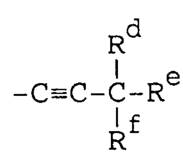

où $R^d$ représente un atome d'hydrogène, un groupe alkyle ayant 1 à 4 atomes de carbone ou un groupe alcoxy ayant 1 à 4 atomes de carbone, et $R^e$ et $R^f$ peuvent être identiques ou différents et représentent chacun un groupe méthyle ou éthyle, ou bien, pris ensemble, ils peuvent représenter un groupe cyclopropyle.

**11.** Procédé selon la revendication 10, pour la production de composés dans lesquels $R^3$ représente un groupe méthyle, éthyle ou propyle.

**Revendications pour l'Etat contractant suivant : GR**

1. Dérivé substitué d'alkylamine représenté par la formule générale

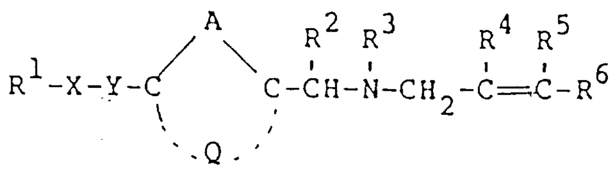

[I]

dans laquelle A représente un groupe méthyne, un atome d'azote, un atome d'oxygène ou un atome de soufre ; Q représente un groupe qui peut contenir un ou deux hétéroatomes choisis dans la classe formée par les atomes d'azote, d'oxygène et de soufre et forme un noyau aromatique penta- ou hexagonal avec les atomes de carbone adjacents et A (le noyau aromatique peut contenir un substituant ou deux substituants identiques ou différents, choisis dans la classe formée par les atomes d'halogènes, les groupes hydroxyle, les groupes alkyles inférieurs et les groupes alcoxy inférieurs) ; X et Y peuvent être identiques ou différents et représentent chacun un atome d'oxygène, un atome de soufre, un groupe carbonyle, un groupe de formule -$CHR^a$- où $R^a$ représente un atome d'hydrogène ou un groupe alkyle inférieur, ou un groupe de formule -$NR^b$- où $R^b$ représente un atome d'hydrogène ou un groupe alkyle inférieur, ou bien X et Y, pris ensemble, représentent un groupe vinylène ou éthynylène ; $R^1$ représente un groupe alcényle inférieur qui peut être substitué, un groupe cycloalcényle qui peut être substitué, un groupe alcynyle inférieur qui peut être substitué, un groupe aryle qui peut être substitué ou un groupe hétérocyclique qui peut être substitué ; $R^2$ représente un atome d'hydrogène ou un groupe alkyle inférieur ; $R^3$ représente un atome d'hydrogène, un groupe alkyle inférieur, un groupe alcényle inférieur, un groupe alcynyle inférieur ou un groupe cycloalkyle ; $R^4$ et $R^5$ peuvent être identiques ou différents et chacun représente un atome d'hydrogène ou un atome d'halogène ; et $R^6$ représente un groupe hydrocarboné acyclique qui peut être substitué (qui peut contenir 1 ou 2 liaisons insaturées choisies parmi les double et triple liaisons), un groupe cycloalkyle qui peut être substitué ou un groupe phényle qui peut être substitué ; à condition que si l'un de X et Y représente un atome d'oxygène ou de soufre, ou le groupe -$NR^b$- où $R^b$ est tel que défini ci-dessus, l'autre représente le groupe carbonyle ou le groupe -$CHR^a$- où $R^a$ est tel que défini ci-dessus,
et un sel non toxique de ce dérivé.

2. Dérivé substitué d'alkylamine et son sel non toxique selon la revendication 1, dans lesquels $R^1$ représente un groupe aryle ou hétérocyclique qui peut être substitué ; X représente un groupe méthylène et Y représente un atome d'oxygène ou un groupe imino, ou bien X et Y, pris ensemble, représentent un groupe éthylène ou un groupe (E)-vinylène ; le noyau aromatique de formule

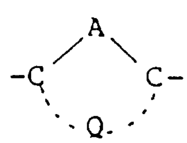

est un noyau de benzène, furanne, thiophène, oxazole, isoxazole, thiazole, pyridine ou pyrimidine ; $R^2$ représente un atome d'hydrogène ; $R^3$ représente un groupe alkyle ayant 1 à 5 atomes de carbone, un groupe alcényle ayant 3 à 5 atomes de carbone, un groupe alcynyle ayant 3 à 5 atomes de carbone ou un groupe cycloalkyle ayant 3 à 5 atomes de carbone ; $R^4$ et $R^5$ représentent chacun un atome d'hydrogène, et la double liaison formée par les deux atomes de carbone auxquels ils sont liés est de forme *trans* (forme E) ; et $R^6$ est un groupe de formule $-CH=CH-R^c$ où $R^c$ représente un groupe alkyle ou alcényle ayant 3 à 6 atomes de carbone qui peut être substitué par un seul groupe alcoxy ayant 1 à 4 atomes de carbone, ou un groupe cycloalkyle ayant 3 à 6 atomes de carbone qui peut être substitué par 1 ou 2 groupes alkyles ayant 1 à 4 atomes de carbone, ou un groupe de formule $-C\equiv C-R^c$ où $R^c$ est tel que défini ci-dessus.

3. Dérivé substitué d'alkylamine et son sel non toxique selon la revendication 2, dans lesquels $R^1$ est un groupe aryle ou un groupe hétérocyclique aromatique, qui peut porter un substituant ou deux

substituants identiques ou différents choisis dans la classe formée par les atomes d'halogènes et les groupes cyano, formyle, hydroxyméthyle, alkyles inférieurs, alcényles inférieurs, alcynyles inférieurs, alcoxy inférieurs, alcényloxy inférieurs, phényle, furyle, thiényle, oxazolyle, thiazolyle, isoxazolyle, isothiazolyle, pyrrolyle, imidazolyle, furazanyle, pyridyle, pyrazinyle, pyrimidinyle, pyridazinyle, 1,3,5-triazinyle, 1,3,4-oxadiazolyle et 1,3,4-thiadiazolyle.

4. Dérivé substitué d'alkylamine et son sel non toxique selon la revendication 3, dans lesquels le groupe hétérocyclique aromatique est un groupe hétérocyclique aromatique penta- à dodécagonal contenant 1 à 3 hétéroatomes choisis dans la classe formée par les atomes d'azote, d'oxygène et de soufre.

5. Dérivé substitué d'alkylamine et son sel non toxique selon la revendication 2, dans lesquels $R^1$ est un groupe phényle, naphtyle, furyle, thiényle, pyridyle, oxazolyle, thiazolyle, isoxazolyle, isothiazolyle, pyrazinyle, pyrimidinyle, pyridazinyle, 1,3,5-triazinyle, benzofurannyle, benzothiényle, benzoxazolyle, benzothiazolyle ou benzofurazanyle qui peut contenir un substituant ou deux substituants identiques ou différents choisis dans la classe formée par les atomes d'halogènes et les groupes hydroxyle, cyano, formyle, hydroxyméthyle, alkyles en $C_1$-$C_3$, alcényles en $C_3$-$C_5$, alcynyles en $C_3$-$C_5$, alcoxy en $C_1$-$C_3$, alcényloxy en $C_3$-$C_5$, phényle, furyle, thiényle, oxazolyle, thiazolyle, isoxazolyle, pyrrolyle et imidazolyle.

6. Dérivé substitué d'alkylamine et son sel non toxique selon la revendication 2, dans lesquels $R^1$ est un groupe 2-méthylphényle, 2-fluorophényle, 3-cyanophényle, 3-(2-furyl)phényle, 3-(2-thiényl)phényle, 3-(5-oxazolyl)phényle, 3-(1-pyrrolyl)phényle, 3-(1-imidazolyl)phényle ou 3-(5-thiazolyl)phényle.

7. Dérivé substitué d'alkylamine et son sel non toxique selon la revendication 1, dans lesquels $R^3$ représente un groupe alkyle ayant 1 à 3 atomes de carbone, un groupe allyle, un groupe propargyle ou un groupe cyclopropyle ; et $R^6$ représente un groupe de formule

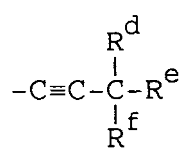

où $R^d$ représente un atome d'hydrogène, un groupe alkyle ayant 1 à 4 atomes de carbone ou un groupe alcoxy ayant 1 à 4 atomes de carbone, et $R^e$ et $R^f$ peuvent être identiques ou différents et représentent chacun un groupe méthyle ou éthyle, ou bien, pris ensemble, ils peuvent représenter un groupe cyclopropyle.

8. Dérivé substitué d'alkylamine et son sel non toxique selon la revendication 7, dans lesquels $R^3$ représente un groupe méthyle, éthyle ou propyle.

9. Procédé de production d'un dérivé substitué d'alkylamine de la formule générale [I] définie la revendication 1 et de son sel non toxique, qui consiste à

(a) faire réagir un dérivé substitué d'amine représenté par la formule générale

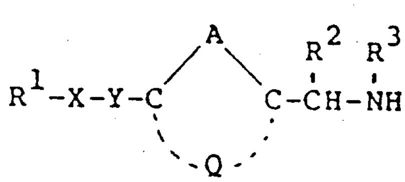

[II]

dans laquelle A, Q, X, Y, $R^1$, $R^2$ et $R^3$ sont tels que définis dans la revendication 1,

ou un dérivé convenablement protégé de celui-ci, avec un composé représenté par la formule générale

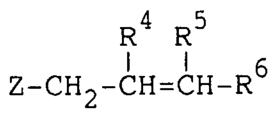

[III]

dans laquelle Z représente un groupe partant, et $R^4$, $R^5$ et $R^6$ sont tels que définis dans la revendication 1,
ou un dérivé convenablement protégé de celui-ci, puis, si nécessaire, éliminer le groupe protecteur, ou
(b) faire réagir un composé représenté par la formule générale

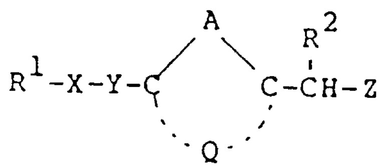

[IV]

dans laquelle A, Q, X, Y, $R^1$, $R^2$ et Z sont tels que définis ci-dessus,
ou un dérivé convenablement protégé de celui-ci, avec une amine substituée représentée par la formule générale

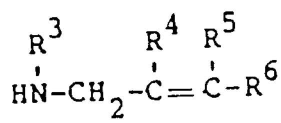

[V]

dans laquelle $R^3$, $R^4$, $R^5$ et $R^6$ sont tels que définis ci-dessus,
ou un dérivé convenablement protégé de celle-ci, puis, si nécessaire, éliminer le groupe protecteur,
et, si nécessaire, convertir le composé résultant en un sel non toxique.

**10.** Procédé de production d'un dérivé substitué d'alkylamine représenté par la formule générale

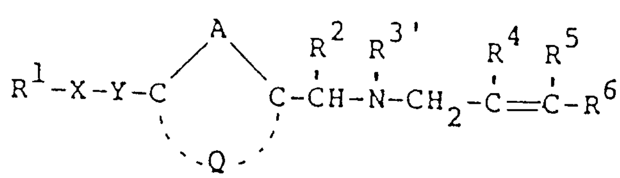

[$I^b$]

dans laquelle A, Q, X, Y, $R^1$, $R^2$, $R^4$, $R^5$ et $R^6$ sont tels que définis dans la revendication 1, et $R^{3'}$ représente un groupe alkyle inférieur, un groupe alcényle inférieur, un groupe alcynyle inférieur ou un groupe cycloalkyle,
et d'un sel non toxique de celui-ci, qui consiste à faire réagir un dérivé substitué d'alkylamine représenté par la formule générale

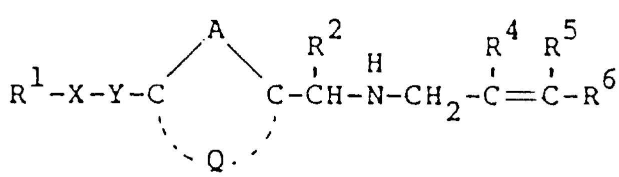

[$I^a$]

dans laquelle A, Q, X, Y, $R^1$, $R^2$, $R^4$, $R^5$ et $R^6$ sont tels que définis ci-dessus,
ou un dérivé convenablement protégé de celui-ci, avec un composé représenté par la formule générale

Z-$R^{3'}$ [VI]

dans laquelle Z représente un groupe partant et $R^{3'}$ est tel que défini ci-dessus, puis, si nécessaire, éliminer le groupe protecteur et, si nécessaire, convertir le composé résultant en un sel non toxique.

**11.** Procédé de production d'un dérivé substitué d'alkylamine représenté par la formule générale

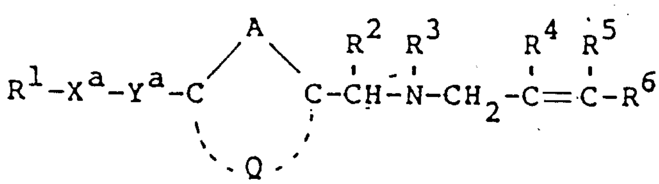

[$I^c$]

dans laquelle A, Q, $R^1$, $R^2$, $R^3$, $R^4$, $R^5$ et $R^6$ sont tels que définis dans la revendication 1, $X^a$ représente un groupe carbonyle ou un groupe de formule -$CHR^a$- où $R^a$ représente un atome d'hydrogène ou un groupe alkyle inférieur, et $Y^a$ représente un atome d'oxygène ou de soufre ou un groupe de formule -$NR^b$-où $R^b$ représente un atome d'hydrogène ou un groupe alkyle inférieur, ou d'un sel non toxique de celui-ci, qui consiste à faire réagir un composé représenté par la formule générale

$R^1$-$X^a$-Z [VII]

dans laquelle Z représente un groupe partant et $R^1$ et $X^a$ sont tels que définis ci-dessus, ou un dérivé convenablement protégé de celui-ci, avec un dérivé substitué d'amine représenté par la formule générale

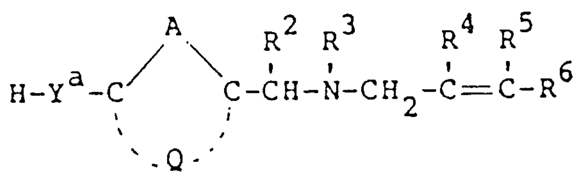

[VIII]

dans laquelle A, Q, $R^2$, $R^3$, $R^4$, $R^5$, $R^6$ et $Y^a$ sont tels que définis, ou un dérivé convenablement protégé de celui-ci, puis, si nécessaire, éliminer le groupe protecteur, puis, si nécessaire, convertir le composé résultant en un sel non toxique.

**12.** Procédé de production d'un dérivé substitué d'alkylamine représenté par la formule générale

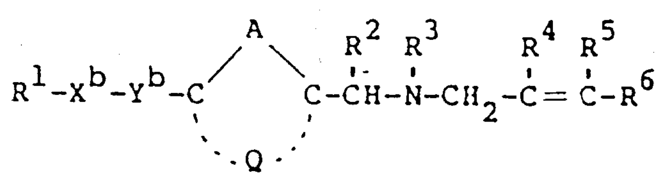

[$I^d$]

dans laquelle A, Q, $R^1$, $R^2$, $R^3$, $R^4$, $R^5$ et $R^6$ sont tels que définis dans la revendication 1, $X^b$ représente un atome d'oxygène ou de soufre ou un groupe de formule -$NR^b$- où $R^b$ représente un atome d'hydrogène ou un groupe alkyle inférieur, et $Y^b$ représente un groupe carbonyle ou un groupe de formule -$CHR^a$- où $R^a$ représente un atome d'hydrogène ou un groupe alkyle inférieur, et d'un sel non toxique de celui-ci, qui consiste à faire réagir un composé représenté par la formule générale

$R^1$-$X^b$-H [IX]

dans laquelle $R^1$ et $X^b$ sont tels que définis ci-dessus,
ou un dérivé convenablement protégé de celui-ci, avec un dérivé substitué d'amine représenté par la formule générale

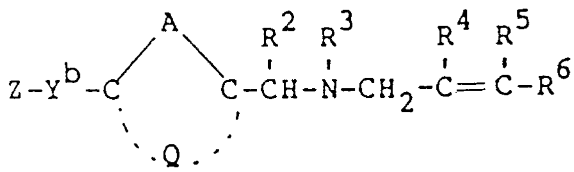

[X]

dans laquelle A, Q, $R^2$, $R^3$, $R^4$, $R^5$, $R^6$ et $Y^b$ sont tels que définis ci-dessus, et Z représente un groupe partant,
ou un dérivé convenablement protégé de celui-ci, et, si nécessaire, éliminer le groupe protecteur, puis, si nécessaire, convertir le composé résultant en un sel non toxique.